# EUROPEAN PATENT APPLICATION

(11) **EP 4 265 616 A1**
(43) Date of publication of application: **25.10.2023**
(21) Application number: 21909541.1
(22) Date of filing: 23.12.2021
(51) Int. Cl.: C07D 487/10, C07D 487/20, C07D 491/107, A61K 31/519, A61K 31/527, A61P 35/00

(54) **SPIROCYCLIC COMPOUND AS KRAS-G12C INHIBITOR**

(30) Priority: 25.12.2020 CN 202011556213; 03.06.2021 CN 202110619679
(71) Applicant: Shanghai Euregen Biopharma Co., Ltd., Shanghai 201422 (CN); Shanghai Youli Biopharma Co., Ltd., Shanghai 201210 (CN)
(72) Inventor: CHEN, Xuxing, Shanghai 201210 (CN); LI, Jing, Shanghai 201210 (CN); CHEN, Yanhong, Shanghai 201210 (CN); ZHAO, Zhao, Shanghai 201210 (CN)
(74) Representative: dompatent von Kreisler Selting Werner - Partnerschaft von Patent- und Rechtsanwälten mbB
(86) International application number: PCT/CN2021/140991
(87) International publication number: WO 2022/135546

(57) **Abstract**

The present invention relates to a spirocyclic compound, represented by formula (I), as a KRAS-G12C inhibitor, which can be used in the preparation of a drug for treating KRAS G12C-mediated tumors.

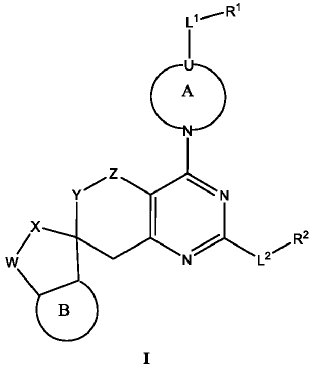

## Description

### Technical Field

The invention relates to the field of pharmaceutical chemistry. In particular, the present invention relates to a class of spirocyclic compound, which can be used as KRAS-G12C inhibitors to prepare drugs for the treatment of KRAS G12C-mediated tumors.

### Background

The Kirsten rat sarcoma 2 viral oncogene homolog ("KRAS") is a GTPase enzyme and is a member of the RAS oncogene family. KRAS acts as a molecular switch of cycles between inactive (guanosine diphosphate (GDP)-binding) and active (GTP-binding) states to transduce upstream cellular signals received from a variety of tyrosine kinases to downstream effectors to regulate a variety of processes, including cell proliferation (Current Opin Pharmcol. 2013(13): 394-401).

KRAS is one of the most frequently mutated oncogene in cancers. About 22% of cancer patients have KRAS mutations, especially in pancreatic cancer (68%), cholangiocarcinoma (27%) and lung cancer (17%). Wherein, the incidence of KRAS G12C in non-small cell lung cancer, colorectal cancer and pancreatic cancer is 48%, 10% and 1%, respectively. The lack of ideal small molecule binding pockets for KRAS proteins and high affinity of the KRAS protein for the abundant GTP make it challenging to design specific small molecule drugs. Among various known KRAS mutations, KRAS G12C mutation is considered to be the most potent drug target. Several covalent inhibitors targeting KRAS G12C are currently in clinical trials, such as AMG 510 of Amgen and MRTX849 from Mirati Therapeutics. All of these compounds covalently bind to KRAS G12C at cysteine residue 12, keeping KRAS G12C in an inactive state of GDP-binding and inhibiting KRAS-dependent signal transduction.

After more than thirty years of research, the development of KRAS inhibitors have made certain progress, but there is still no KRAS inhibitor that has shown sufficient safety and efficacy to gain regulatory approval. Therefore, pharmaceutical companies need to make continuous efforts to develop novel KRAS inhibitors so as to treat various diseases, such as cancers.

WO2020/236940 discloses a compound 819, the binding affinity between compound 819 and KRAS G12C was predicted using software based on two methods: MMGBSA and CovDock, but does not provide the synthetic method, the characterization results or any biological test results of the compound.

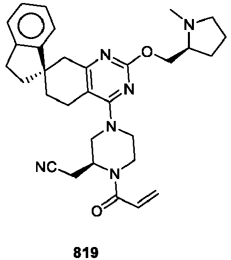

### SUMMARY

The purpose of the present invention is to provide a class of spirocyclic compounds or pharmaceutically acceptable salts thereof.

Another purposeof the present invention is to provide a pharmaceutical composition comprising the spirocyclic compounds or the pharmaceutically acceptable salts thereof.

A further purpose of the present invention is to provide the use of the spirocyclic compounds or the pharmaceutically acceptable salts thereof or the compositions comprising the spirocyclic compounds or the pharmaceutically acceptable salts thereof in the preparation of anti-tumor drugs.

In the first aspect of the present invention, provided is a compound of formula I, or pharmaceutically acceptable salts thereof, enantiomers, diastereomers, tautomers, cis-trans isomers, solvates, polymorphs, deuterated compounds, or combinations thereof, 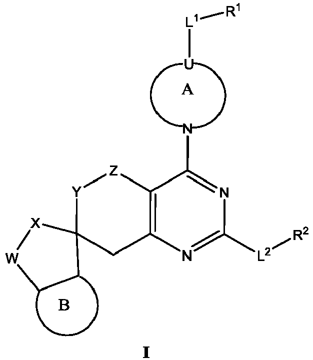 wherein,
A is a 4-12 membered saturated or partially saturated monocyclic ring, fused ring, bridged ring or spiro ring, wherein the saturated or partially saturated monocyclic ring, fused ring, bridged ring, or spiro ring is optionally substituted by one or more R³;
each R³ is independently oxo, C₁₋₃ alkyl, C₂₋₄ alkenyl, C₂₋₄ alkynyl, cyano, -C(O)OR⁴, -CON(R⁴)₂, or -N(R⁴)₂, wherein the C₁₋₃ alkyl is optionally substituted by cyano, halogen, -OR⁴, or -N(R⁴)₂;
each R⁴ is independently hydrogen or C₁₋₃ alkyl;
U is N or CH;
L¹ is absent or NR^{N}, and when U is N, L¹ is absent, and when U is CH, L¹ is NR^{N};
R^{N} is hydrogen or C₁₋₃ alkyl;
R¹ is 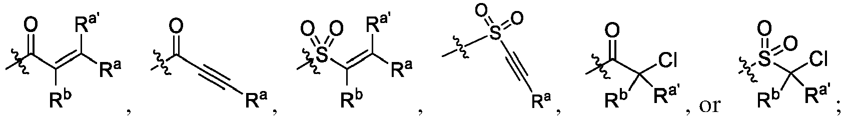 wherein,
R^{a} is hydrogen, halogen, optionally substituted C₁₋₃ alkyl, -N(R⁵)₂, optionally substituted 4-6 membered saturated heterocyclyl, C₁₋₃ alkoxy, C₁₋₃ alkylthio or acetyl, wherein the substituents for optionally substituted described in R^{a} is selected from the group consisting of: methyl, ethyl, -N(R⁵)₂, halogen, C₁₋₃ alkoxy, and 4-6 membered saturated heterocyclyl;
each R⁵ is independently hydrogen or C₁₋₃ alkyl;
R^{a'} and R^{b} are independently hydrogen, halogen or C₁₋₃ alkyl;
L² is absent, -O-, -S-, -NR⁶-, -SO-, -SO₂- or -CO-, wherein, R⁶ is hydrogen or C₁₋₄ alkyl;
R² is hydrogen, optionally substituted C₁₋₄ alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused 6-10 membered aryl, optionally substituted saturated or unsaturated 3-8 membered carbocyclyl fused 5-10 membered heteroaryl, optionally substituted saturated or unsaturated 3-8 membered heterocyclyl fused 6-10 membered aryl, optionally substituted saturated or unsaturated 3-8 membered heterocyclyl fused 5-10 membered heteroaryl, optionally substituted 6-10 membered aryl or optionally substituted 5-10 membered heteroaryl; wherein the substituents for optionally substituted described in R² is selected from the group consisting of: deuterium, halogen, hydroxy, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl, and optionally substituted 4-8 membered saturated and unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl;
W is -(CR⁸R⁹)-, -NR¹⁰-, O or S, wherein R⁸, R⁹ and R¹⁰ are independently hydrogen or C₁₋₃ alkyl;
X is -(CR¹¹R¹²)ₘ- or -(C=O)-, wherein each R¹¹ is independently hydrogen or C₁₋₃ alkyl, and each R¹² is independently hydrogen or C1-C3 alkyl;
m=1, 2 or 3;
B is 6-10 membered aryl or 5-8 membered heteroaryl, wherein the 6-10 membered aryl or the 5-8 membered heteroaryl is unsubstituted or optionally substituted by one or more R¹³;
each R¹³ is independently halogen, hydroxy, amino, C₁₋₃ alkylamino, (C₁₋₃ alkyl)₂amino, C₁₋₆ alkyl, C₁₋₆ haloalkyl, C₃₋₆ carbocyclyl, C₁₋₃ alkoxy, or C₁₋₃ haloalkoxy;
Y is -NR¹⁴-, wherein R¹⁴ is hydrogen, C₁₋₆ alkyl, C₁₋₆ haloalkyl or C₃₋₆ carbocyclyl;
Z is -(CR¹⁵R¹⁶)-, wherein R¹⁵ and R¹⁶ are independently hydrogen or C₁₋₃ alkyl.

In a preferred embodiment, R² is hydrogen, optionally substituted C₁₋₄ alkyl, optionally substituted saturated or unsaturated 3-8 membered carbocyclyl, optionally substituted saturated or unsaturated 4-12 membered heterocyclyl, optionally substituted saturated or unsaturated 3-8 membered heterocyclyl fused 6-10 membered aryl, optionally substituted saturated or unsaturated 3-8 membered heterocyclyl fused 5-10 membered heteroaryl, optionally substituted 6-10 membered aryl or optionally substituted 5-10 membered heteroaryl, wherein the substituents for optionally substituted described in the R² are selected from: deuterium, halogen, hydroxyl, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl and optionally substituted 4-8 membered saturated or unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl;
preferably, L² is absent, -O-, -S-, or -NR⁶-, wherein R⁶ is hydrogen or C₁₋₄ alkyl;
preferably, R^{a} is hydrogen, fluorine, optionally substituted C₁₋₃ alkyl, optionally substituted 4-6 membered saturated heterocyclyl or acetyl;
wherein the substituents for optionally substituted described in R^{a} are selected from the group consisting of methyl, ethyl, -N(R⁵)₂, halogen, C₁₋₃ alkoxy, and 4-6 membered saturated heterocyclyl;
each R⁵ is independently hydrogen or C₁₋₃ alkyl;
preferably, R^{a'} and R^{b} are independently hydrogen, fluorine or C₁₋₃ alkyl;
preferably, W is -(CR⁸R⁹)- or -NR¹⁰-, wherein R⁸, R⁹ and R¹⁰ are independently hydrogen or C₁₋₃ alkyl;
preferably, X is -(CR¹¹R¹²)ₘ-; wherein each R¹¹ is independently hydrogen or C₁₋₃ alkyl, and each R¹² is independently hydrogen or C₁₋₃ alkyl; m is 1, 2 or 3; further preferably, m is 1 or 2.

In another preferred embodiment, the moiety represented by 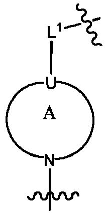 is selected from the group consisting of: 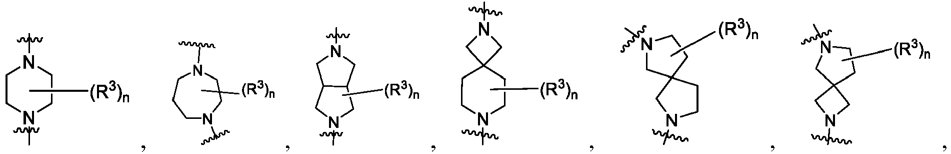 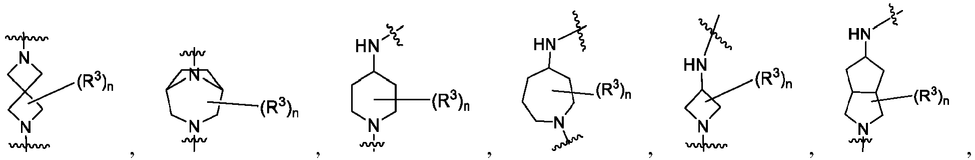 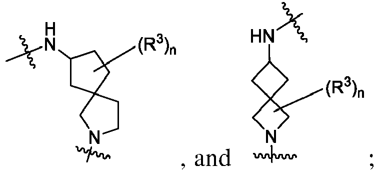
n represents the number of R³, and n is 0, 1, 2 or 3;
each R³ is independently oxo, C₁₋₃ alkyl, C₂₋₄ alkenyl, C₂₋₄ alkynyl, cyano, -C(O)OR⁴, -CON(R⁴)₂, or -N(R⁴)₂, wherein C₁₋₃ alkyl can be optionally substituted by cyano, halogen, -OR⁴, or -N(R⁴)₂;
each R⁴ is independently hydrogen or C₁₋₃ alkyl;
preferably, the moiety represented by 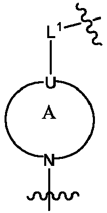 is selected from the group consisting of:

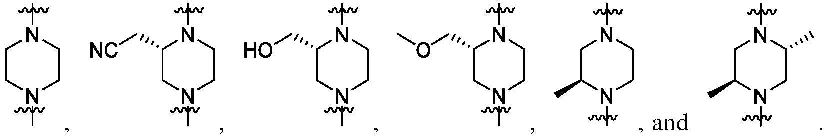

In another preferred embodiment, R¹ is 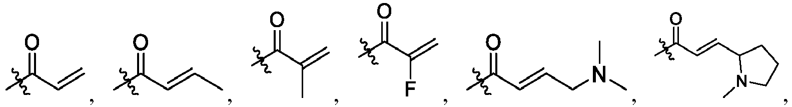 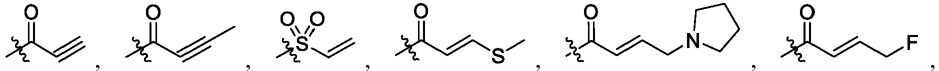 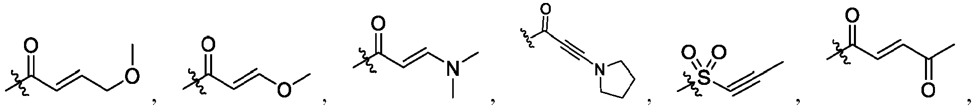 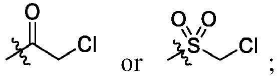 preferably, R¹ is

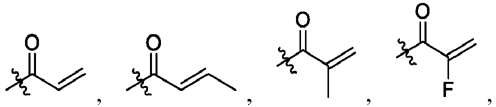

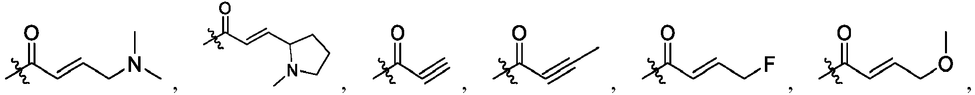

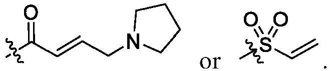

In another preferred embodiment, R² is hydrogen, optionally substituted C₁₋₄ alkyl, optionally substituted 6-10 membered aryl or optionally substituted 5-10 membered heteroaryl; wherein the substituents for optionally substituted discribed in R² are selected from: halogen, hydroxy, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl, and optionally substituted 4-8 membered saturated or unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl.
Preferably, R² is 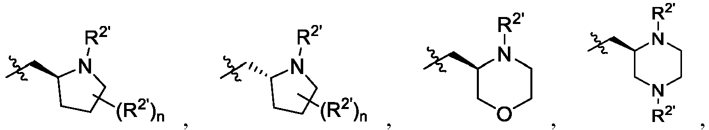 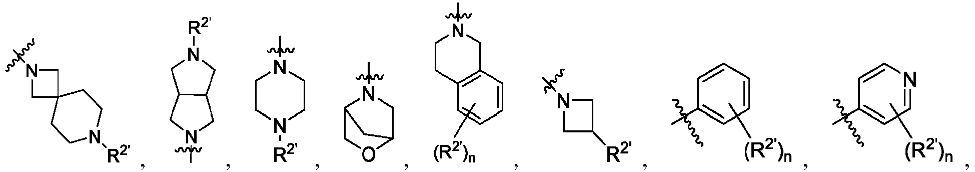 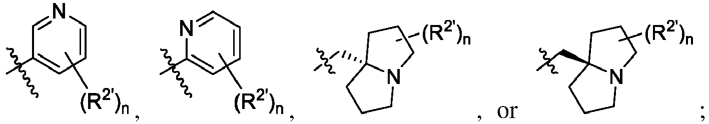 wherein n represents the number of R^{2'}, and selected from: 0, 1, 2 and 3; each R^{2'} is independently halogen, C₁₋₃ alkyl, C₁₋₃ haloalkyl, hydroxy, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl, and optionally substituted 4-8 membered saturated or unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl.

In another preferred embodiment, the spiro ring represented by 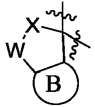 is selected from the group consisting of: 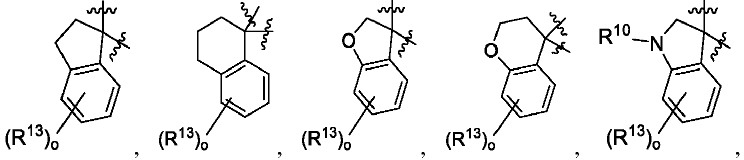 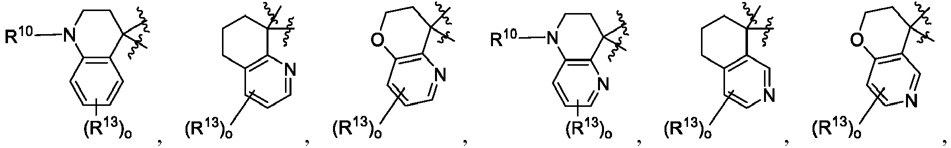 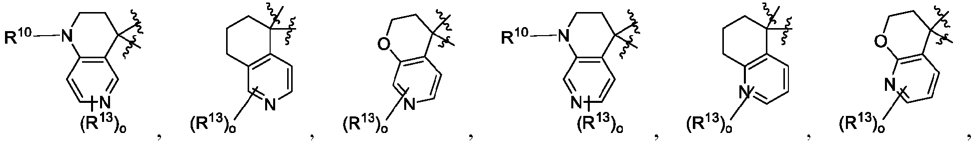 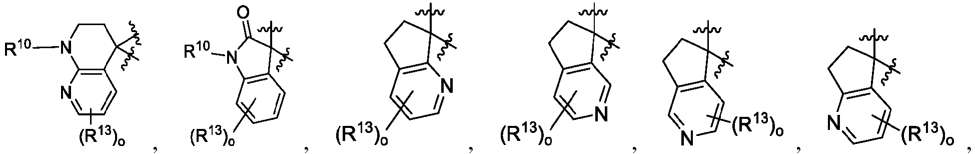 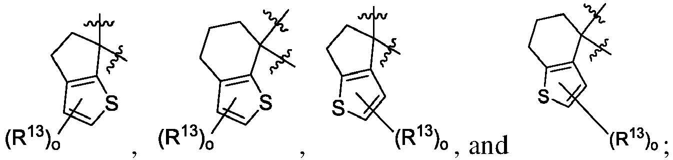
wherein, R¹⁰ is independently hydrogen or C₁₋₃ alkyl;
o represents the number of R¹³, and o is 0, 1, 2 or 3; and
each R¹³ is independently halogen, C₁₋₆ alkyl, C₁₋₆ haloalkyl, C₃₋₆ carbocyclyl, C₁₋₃ alkoxy, or C₁₋₃ haloalkoxy.

In another preferred embodiment, the compound is selected from the group consisting of:

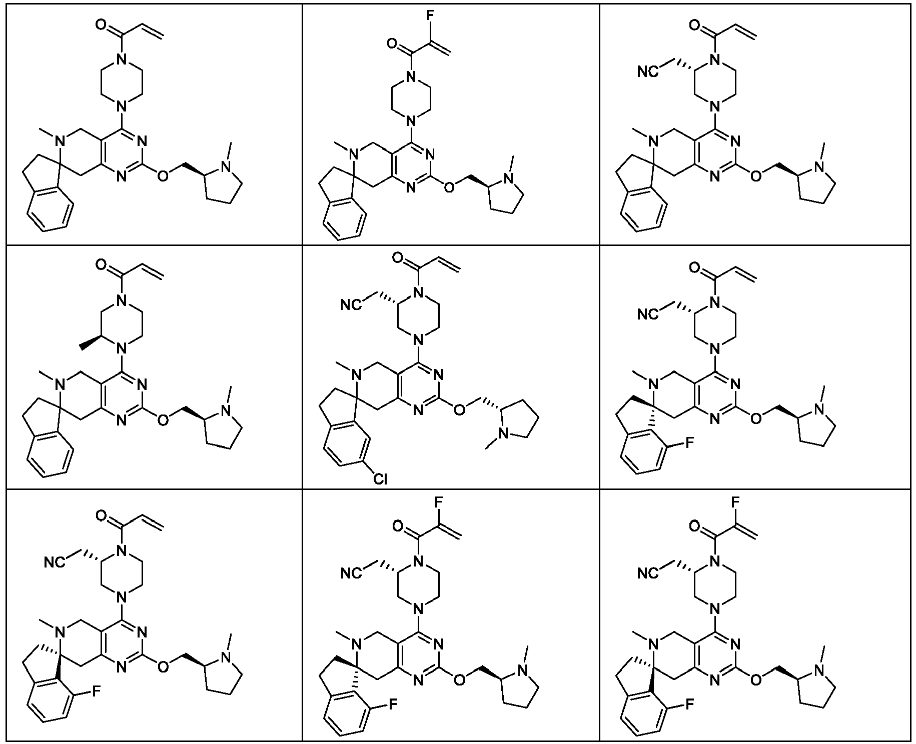

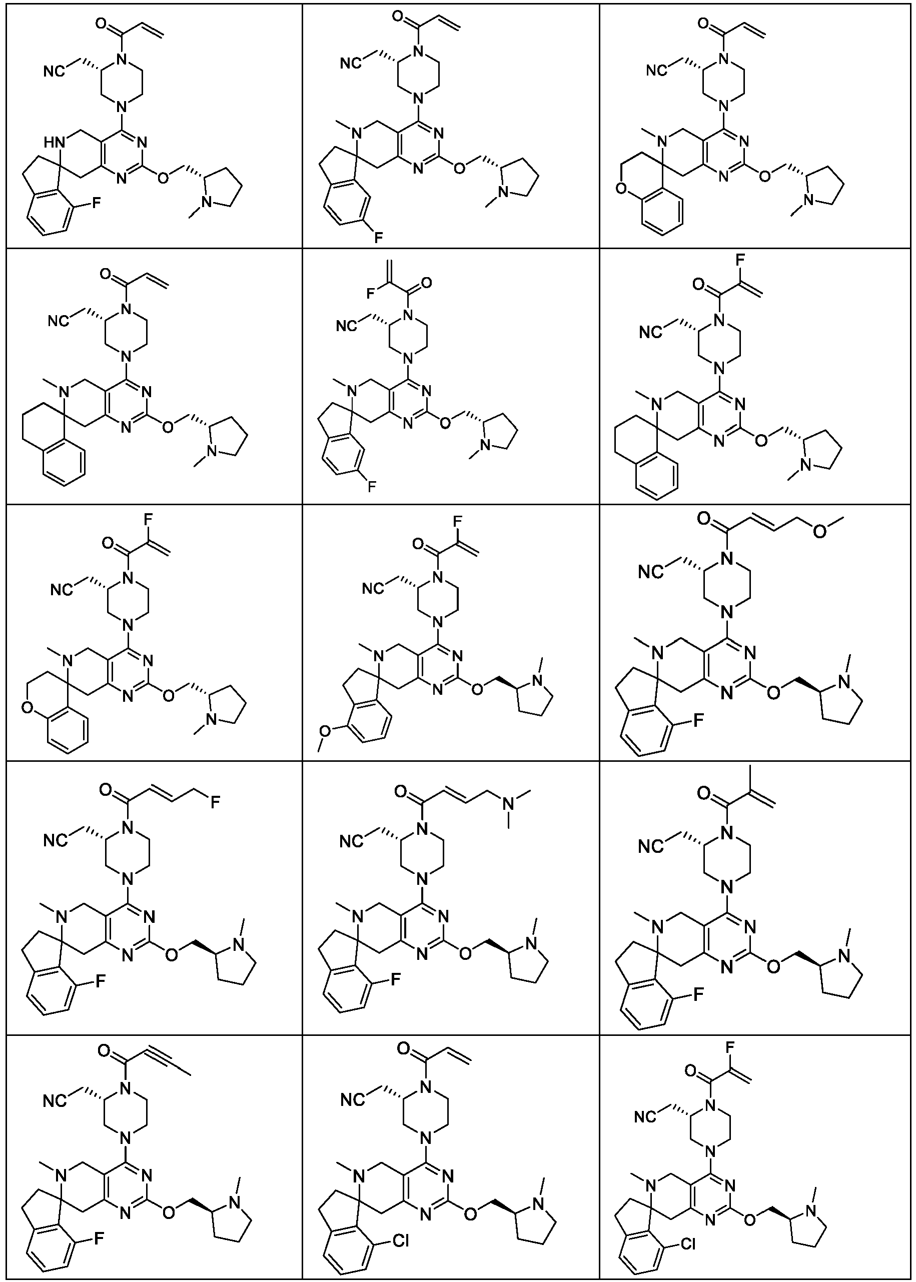

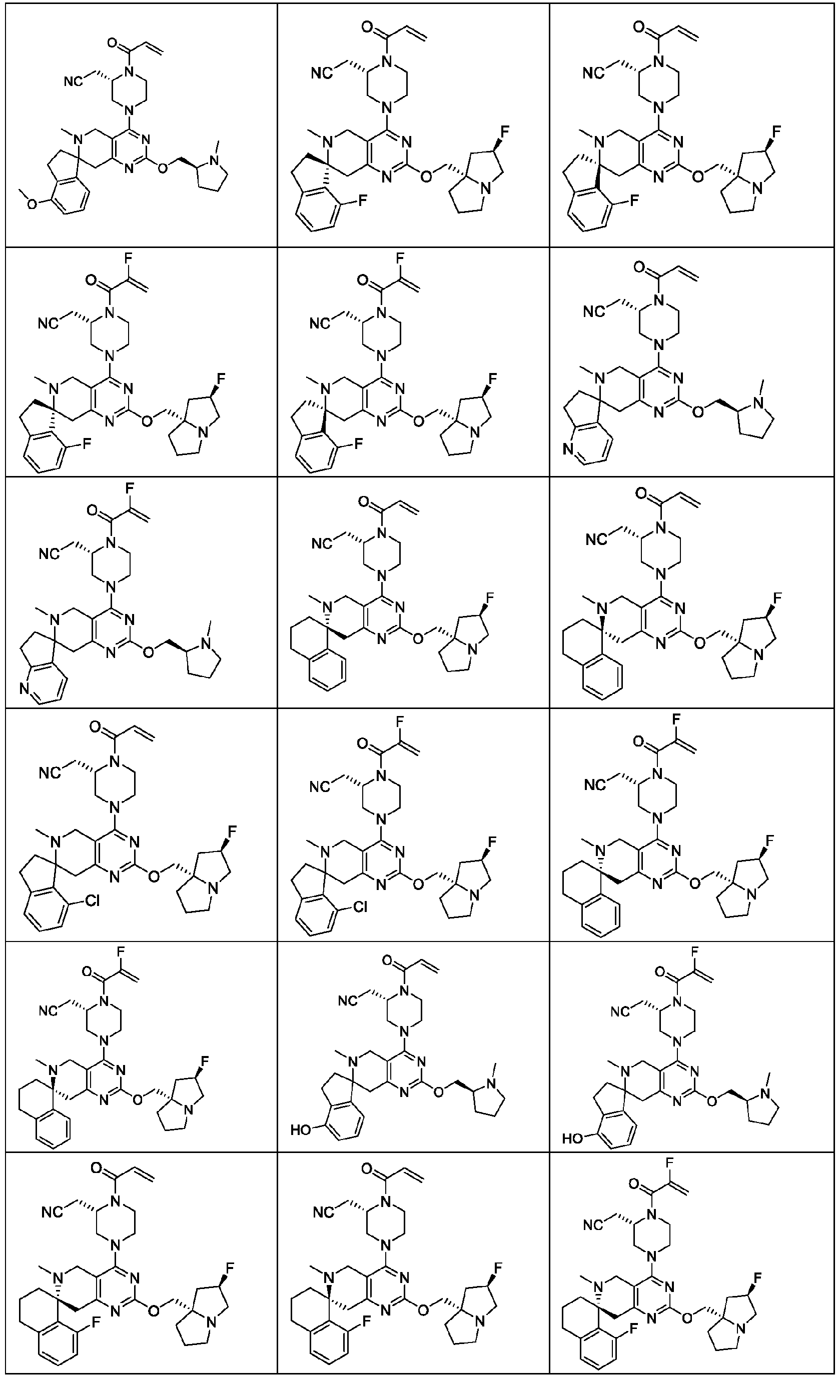

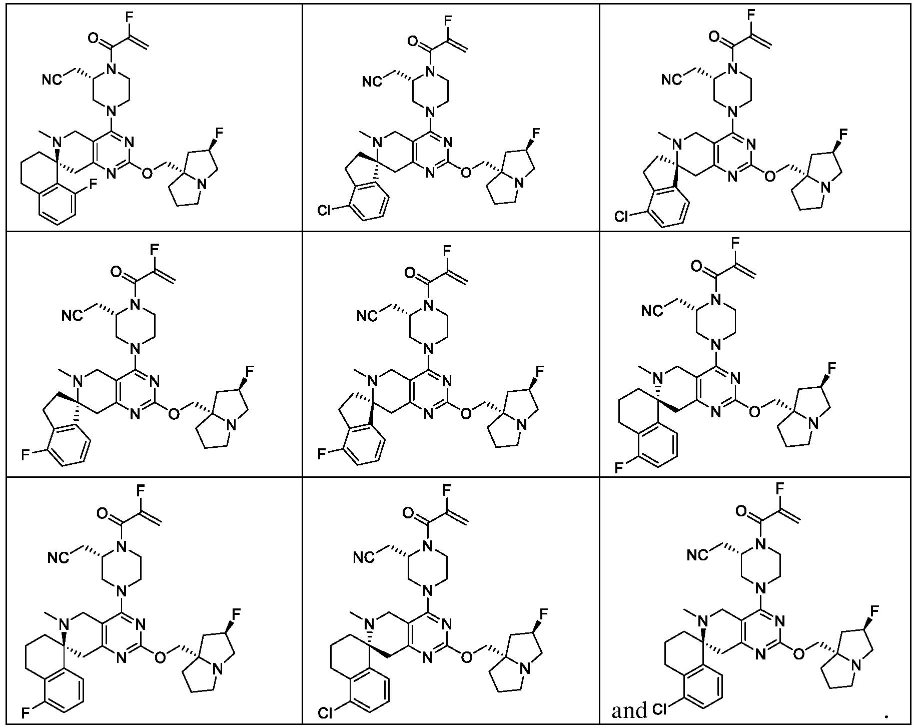

The compound of the present invention can be prepared according to the synthetic methods described in examples or methods similar thereto.

In the second aspect of the present invention, provided is a pharmaceutical composition comprising:
(1) a therapeutically effective amount of one or more of the compounds of the present invention, pharmaceutically acceptable salts thereof, enantiomers, diastereomers, tautomers, cis-trans isomers, solvates, polymorphs, and deuterates thereof as an active ingredient; and
(2) optionally, pharmaceutically acceptable excipients.

In addition, the pharmaceutical composition may further include other pharmaceutically acceptable therapeutic agents or be used in combination with other pharmaceutically acceptable therapeutic agents, especially other anti-tumor drugs. The therapeutic agents include, but are not limited to: anti-tumor drugs that act on the chemical structure of DNA, such as cisplatin; anti-tumor drugs that affect nucleic acid synthesis, such as methotrexate (MTX), 5-fluorouracil (5FU), etc.; anti-tumor drugs that affect nucleic acid transcription, such as adriamycin, epirubicin, aclacinomycin, mithramycin, etc.; anti-tumor drugs that act on tubulin synthesis, such as paclitaxel, vinorelbine; aromatase inhibitors such as aminoglutethimide, lentaron, letrozole, anastrozole, etc; cell signaling pathway inhibitors such as epidermal growth factor receptor inhibitors gefitinib (Gefitinib), erlotinib (Erlotinib), lapatinib (Lapatinib), etc; mitogen-activated extracellular signal-regulated kinase (MEK) inhibitors such as trametinib (Trametinib), cobimetinib (Cobimetinib), etc.; cyclin-dependent kinase 4/6(CDK4/6) inhibitors such as Palbociclib, etc.; Src homologous tyrosine phosphatase (SHP2) inhibitor; SOS1 inhibitor; programmed death receptor -1/programmed death ligand -1(PD-1/PD-L1) inhibitors such as nivolumab (Nivolumab), pembrolizumab (Pembrolizumab), etc.

In the third aspect of the present invention, provided is a use of the compound of the present invention as described above, or pharmaceutically acceptable salts thereof, enantiomers, diastereomers, tautomers, cis-trans isomers, solvates, polymorphs, deuterates, or a pharmaceutical composition thereof in the preparation of medicaments for preventing or treating cancer mediated by KRAS G12C mutation. In a preferred embodiment, the cancer mediated by KRAS G12C is selected from the group consisting of: lung cancer (including non-small cell lung cancer), pancreatic cancer, colorectal cancer, leukemia, Ewing's sarcoma, breast cancer, prostate cancer, T-cell lymphoma, B-cell lymphoma, malignant rhabdomyoma, synovial sarcoma, endometrioma, gastric cancer, liver cancer, renal cancer, melanoma, ovarian cancer, brain glioma, cholangiocarcinoma, nasopharyngeal carcinoma, cervical cancer, head and neck cancer, esophageal cancer, thyroid cancer and bladder cancer. In another preferred embodiment, the cancer mediated by KRAS G12C is selected from non-small cell lung cancer, pancreatic cancer and colorectal cancer.

It should be understood that, within the scope of the present invention, each of the above technical features of the present invention and each of the technical features specifically described in the following (such as the examples) can be combined with each other to constitute a new or preferred technical solution. Due to space limitations, it will not be repeated herein.

### DESCRIPTION OF THE DRAWINGS

Figure 1 shows the in vivo efficacy results of the test substances in the female BALB/c nude mouse model with subcutaneous transplantation of NCI-H358 tumors.
Figure 2 shows the in vivo efficacy results of the test substances in the BALB/c nude mouse NCI-H1373-luc intracranial inoculation model.

### Description of terms

Unless otherwise defined, all technical and scientific terms used herein have the same meaning as those commonly understood by those of ordinary skill in the art of the invention.

As used herein, term "contain" or "include (comprise)" may be open, semi-closed, and closed. In other words, said term also includes "consisting essentially of" or "consisting of".

### Definition of groups

Definitions for standard chemical terms can be found in references including Carey and Sundberg "ADVANCED ORGANIC CHEMISTRY 4TH ED." Vols.A (2000) and B (2001), Plenum Press, New York. Unless otherwise indicated, conventional methods within the skill of the art are used, such as mass spectrometry, NMR, IR and UV/VIS spectroscopy and pharmacological methods. Unless specifically defined, the terms used herein in the relevant descriptions of analytical chemistry, organic synthetic chemistry, and pharmaceuticals and medicinal chemistry are known in the art. Standard techniques can be used in chemical synthesis, chemical analysis, pharmaceutical preparation, formulation and delivery, and treatment of patients. For example, the reaction and purification can be carried out using the manufacturer's instructions for use of the kit, or in a manner well known in the art or as described herein. The above-mentioned techniques and methods can generally be carried out in accordance with conventional methods well known in the art, or as described in the various general and more specific documents cited and discussed in this specification. In the present specification, the groups and substituents thereof can be selected by those skilled in the art to provide stable structural moieties and compounds.

When a substituent is described by a conventional formula written from left to right, the substituent likewise includes the chemically equivalent substituent obtained when the formula is written from right to left. For example, -CH₂O- is equivalent to -OCH₂-.

The section headings used herein are for the purpose of organizing the article only and should not be interpreted as limitations on the subject matter described. All documents or part of documents cited in this application, including but not limited to patents, patent applications, articles, books, manuals, and papers, are hereby incorporated by reference in their entirety.

Certain chemical groups defined herein are preceded by a simplified symbol to indicate the total number of carbon atoms present in that group. For example, C₁₋₆ alkyl refers to an alkyl group as defined below having 1 to 6 carbon atoms. The total number of carbon atoms in the simplified symbol does not include carbons that may be present in substituent of the group.

In addition to the foregoing, the following terms, when used in the present specification and claims, have the meanings set forth below unless otherwise specified.

As used herein, the term "halogen" refers to fluorine, chlorine, bromine or iodine. "Hydroxy" refers to -OH group.

"Hydroxyalkyl" refers to an alkyl group, as defined below, substituted by a hydroxy (-OH) group.

"Carbonyl" refers to -C(= O)- group.

"Nitro" refers to -NO₂.

"Cyano" refers to -CN.

"Amino" refers to -NH₂.

"Substituted amino" refers to an amino group substituted by one or two substituents as defined below: alkyl, alkylcarbonyl, aryl alkyl, heteroarylalkyl group, e.g., monoalkylamino, dialkylamino, alkylamido, arylalkylamino, heteroarylalkylamino.

"Carboxyl" refers to -COOH.

As used herein, the term "alkyl" as a group or part of another group (e. g., in the case of halogen-substituted alkyl.etc.) refers to a fully saturated straight or branched hydrocarbon chain group consisting only of carbon and hydrogen atoms, having, for example, from 1 to 12 (preferably from 1 to 8, more preferably from 1 to 6) carbon atoms, and linked to the rest of the molecule by a single bond, for example including but not limited to methyl, ethyl, n-propyl, isopropyl, n-butyl, isobutyl, sec-butyl, tert-butyl, n-pentyl, 2-methylbutyl, 2,2-dimethylpropyl, n-hexyl, heptyl, 2-methylhexyl, 3-methylhexyl, octyl, nonyl, and decyl, and the like. For the purposes of the present invention, the term "alkyl" preferably refers to an alkyl group containing 1 to 6 carbon atoms.

As used herein, the term "alkenyl", as a group or part of another group, refers to a straight or branched hydrocarbon chain group consisting only of carbon and hydrogen atoms, and at least one double bond, having, for example, from 2 to 14 (preferably from 2 to 10, more preferably from 2 to 6) carbon atoms, linked to the rest of the molecule by a single bond, for example, but not limited to, vinyl, propenyl, allyl group, but-1-enyl, but-2-enyl, pent-1-enyl, pent-1,4-dienyl, and the like.

As a group or part of another group, the term "alkynyl" refers to a straight or branched hydrocarbon chain group consisting only of carbon and hydrogen atoms, containing at least one C=C, having, for example, from 2 to 14 (preferably from 2 to 10, more preferably from 2 to 6) carbon atoms, and linked to the rest of the molecule by a single bond, for example, but not limited to, ethynyl, 1-propynyl, 1-butynyl, heptynyl, octynyl, and the like.

As used herein,, as a group or part of another group, the term "carbocyclyl (carbocyclic group, carbocycle)" refers to a stable non-aromatic monocyclic or polycyclic hydrocarbon group consisting only of carbon and hydrogen atoms, which may include fused ring system, bridged ring system or spiro ring system, having from 3 to 15 carbon atoms, preferably from 3 to 10 carbon atoms, more preferably from 3 to 8 carbon atoms, and it is saturated or unsaturated and can be attached to the rest of the molecule by a single bond via any suitable carbon atom. Unless otherwise specifically indicated in the specification, the carbon atom in the carbocyclyl may be optionally oxidized. Examples of the carbocyclic group include, but are not limited to, cyclopropyl, cyclobutyl, cyclopentyl, cyclohexyl, cycloheptyl, cyclooctyl, adamantyl, 2,3-dihydroindenyl, octahydro-4, 7-methylene -1H-indenyl, 1,2, 3,4-tetrahydro-naphthyl, 5,6,7, 8-tetrahydro-naphthyl, cyclopentenyl, cyclohexenyl, cyclohexadienyl, 1H-indenyl, 8,9-dihydro-7H-benzocyclohepten-6-yl, 6,7,8,9-tetrahydro-5H-benzocycloheptenyl, 5,6,7,8,9, 10-hexahydro-benzocyclooctenyl, fluorenyl, bicyclo [2.2.1] heptyl, 7,7-dimethyl-bicyclo [2.2.1] heptyl, bicyclo [2.2.1] heptenyl, bicyclo [2.2.2] octyl, bicyclo [3.1.1] heptyl, bicyclo [3.2.1] octyl, bicyclo [2.2.2] octenyl, bicyclo [3.2.1] octenyl and octahydro-2, 5-methylene-cyclopentadienyl, etc.

As used herein, as a group or part of another group, the term "heterocyclyl (heterocycle)" refers to a stable 3-to 20-membered non-aromatic cyclic group consisting of 2 to 14 carbon atoms and 1 to 6 heteroatoms selected from nitrogen, phosphorus, oxygen and sulfur. Unless otherwise specifically indicated in the specification, a heterocyclyl may be a monocyclic, bicyclic, tricyclic, or more ring systems, which may include fused ring system, bridged ring system, or spiro ring system; and the nitrogen, carbon, or sulfur atom in heterocyclyl may be optionally oxidized; the nitrogen atom may be optionally quaternized; and the heterocyclyl may be partially or fully saturated. A heterocyclyl may be attached to the rest of the molecule by a single bond via a carbon atom or a heteroatom. In heterocyclyl containing fused rings, one or more of the rings may be an aryl or heteroaryl as defined below, provided that the point of attachment to the rest of the molecule is a non-aromatic ring atom. For the purposes of the present invention, the heterocyclyl is preferably a stable 4-to 11-membered non-aromatic monocyclic, bicyclic, bridged or spiro ring group containing 1 to 3 heteroatoms selected from nitrogen, oxygen and sulfur, more preferred a stable 4-to 8-membered non-aromatic monocyclic, bicyclic, bridged or spirocyclic groups comprising 1 to 3 heteroatoms selected from nitrogen, oxygen and sulfur. Examples of heterocyclyl include, but are not limited to, pyrrolidinyl, morpholinyl, piperazinyl, homopiperazinyl, piperidinyl, thiomorpholinyl, 2,7-diaza-spiro [3.5] nonan-7-yl, 2-oxa-6-aza-spiro [3.3] heptan-6-yl, 2, 5-diaza-bicyclo [2.2.1] heptan-2-yl, azetidinyl, pyranyl, tetrahydropyranyl, thiopyranyl, tetrahydrofuryl, oxazinyl, dioxocyclopentyl, tetrahydroisoquinolinyl, decahydroisoquinolinyl, imidazolinyl, imidazolidinyl, quinazinyl, thiazolidinyl, isothiazolidinyl, isoxazolidinyl, dihydroindolyl, octahydroindolyl, pyrrolidinyl, pyrazolidinyl, phthalimide, etc.

As used herein,, the term "aromatic ring (aryl)", as a group or part of another group, refers to a conjugated hydrocarbon ring system group having from 6 to 18 carbon atoms, preferably having from 6 to 10 carbon atoms. For the purposes of the present invention, an aromatic ring (aryl) may be a monocyclic, bicyclic, tricyclic or polycyclic ring system and may also be fused with the carbocyclyl or heterocyclyl as defined above, provided that the aromatic ring (aryl) attached to the rest of the molecule by a single bond via an atom on the aromatic ring. Examples of the aromatic ring (aryl) include, but are not limited to, phenyl, naphthyl, anthryl, phenanthryl, fluorenyl, 2,3-dihydro-1H-isoindolyl, 2-benzoxazoliabsent, 2H-1, 4-benzoxazin-3 (4H)-one-7-yl, and the like.

As used herein, the term "arylalkyl" refers to an alkyl group as defined above substituted by an aryl as defined above.

As used herein, the term "heteroaromatic ring (heteroaryl)" as a group or part of another group refers to a 5-to 16-membered conjugated ring system group having from 1 to 15 carbon atoms (preferably from 1 to 10 carbon atoms) and 1 to 6 heteroatoms selected from nitrogen, oxygen and sulfur in the ring. Unless specifically indicated in the specification, the heteroaromatic ring (heteroaryl) may be a monocyclic, bicyclic, tricyclic or polycyclic ring system and may also be fused with a carbocyclyl or heterocyclyl as defined above, provided that the heteroaromatic ring (heteroaryl) is attached to the rest of the molecule by a single bond via an atom on the heteroaromatic ring. The nitrogen, carbon or sulfur atom in the heteroaromatic ring (heteroaryl) may optionally be oxidized; the nitrogen atom may optionally be quaternized. For the purposes of the present invention, the heteroaromatic ring (heteroaryl) is preferably a stable 5-to 12-membered aromatic group containing 1 to 5 heteroatoms selected from nitrogen, oxygen and sulfur, more preferred a stable 5 -to 10-membered aromatic group containing 1 to 4 heteroatoms selected from nitrogen, oxygen and sulfur or 5-to 6-membered aromatic group containing 1 to 3 heteroatoms selected from nitrogen, oxygen and sulfur. Examples of heteroaromatic ring (heteroaryl) include, but are not limited to, thienyl, imidazolyl, pyrazolyl, thiazolyl, oxazolyl, oxadiazolyl, isoxazolyl, pyridinyl, pyrimidinyl, pyrazinyl, pyridazinyl, benzimidazolyl, benzopyrazolyl, indolyl, furyl, pyrrolyl, triazolyl, tetrazolyl, triazinyl, indolizinyl, isoindolyl, indazolyl, isoindazolyl, purinyl, quinolinyl, isoquinolinyl, diazanaphthalenyl, naphthyridyl, quinoxalinyl, pteridinyl, carbazolyl, carbolinyl, phenanthridinyl, phenanthrolinyl, acridinyl, phenazinyl, isothiazolyl, benzothiazolyl, benzothiophenyl, oxatriazolyl, cinnolinyl, quinazolinyl, thiophenyl, indolizinyl, phenanthrolinyl, isoxazolyl, phenoxazinyl, phenothiazinyl, 4,5,6, 7-tetrahydrobenzo [b] thienyl, naphthopyridyl, [1,2,4] triazolo [4,3-b] pyridazine, [1,2,4] triazolo [4,3-a] pyrazine, [1,2,4] triazolo [4,3-c] pyrimidine, [1,2,4] triazolo [4,3-a] pyridine, imidazo [1,2-a] pyridine, imidazo [1,2-b] pyridazine, imidazo [1,2-a] pyrazine and the like.

As used herein, the term "heteroarylalkyl" refers to an alkyl group as defined above substituted by a heteroaryl as defined above.

In the present application, the term "absent" means that the two sides of the group defined above are directly linked by chemical bonds. For example, "B is absent in A- B- C" means "A-C".

In the present application, " 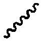 " in " 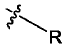 " represents the connection position of group R.

In the present application, unless otherwise specified in the claims, "optionally" and "optionally substituted" mean that the subsequently described event or condition may or may not occur, and the description includes both the occurrence and non-occurrence of the event or condition. For example, "optionally substituted aryl " means that the hydrogen on the aryl is substituted or unsubstituted, and the description includes both substituted aryl and unsubstituted aryl. For example, without explicit listing of substituents, as used herein, the term "optionally substituted", "substituted" or "substituted by" refer to one or more hydrogen atoms on a given atom or group independently substituted by one or more, e.g., 1, 2, 3, or 4, substituents independently selected from: deuterium (D), halogen, -OH, sulfydryl, cyano, -CD₃, -C₁-C₆alkyl (preferred-C₁₋₃alkyl),C₂-C₆alkenyl, C₂-C₆alkynyl, cycloalkyl (preferably 3-8 membered cycloalkyl), aryl, heterocyclyl (preferably 3-8 membered heterocyclyl), heteroaryl, aryl-C₁-C₆alkyl, heteroaryl C₁-C₆alkyl, C₁-C₆haloalkyl-, -OC₁-C₆alkyl (preferred-OC₁-C₃alkyl), -OC₂-C₆alkenyl, -OC₁-C₆alkyl phenyl, -C₁-C₆alkyl-OH (preferred -C₁-C₄alkyl OH), -C₁-C₆alkyl SH, -C₁-C₆alkyl O-C₁-C₆alkyl, -OC₁-C₆haloalkyl, -NH₂, C₁-C₆alkyl-NH₂ (preferably -C₁-C₃alkyl-NH₂), -N(C₁-C₆alkyl)₂(preferably -N(C₁-C₃alkyl)₂), -NH(C₁-C₆alkyl) (preferably -NH(C₁-C₃alkyl)), -N(C₁-C₆alkyl)(C₁-C₆alkyl phenyl), -NH(C₁-C₆alkyl phenyl), nitro, -C(O)-OH, C(O)OC₁-C₆alkyl (preferably -C(O)OC₁-C₃alkyl), -CONRⁱRⁱⁱ (wherein, R' and Rⁱⁱ is H, D and C₁₋₆alkyl, preferably C₁₋₃alkyl), -NHC(O)(C₁-C₆alkyl), -NHC(O)(phenyl), -N(C₁-C₆alkyl) C(O)(C₁-C₆alkyl), -N(C₁-C₆alkyl) C(O)(phenyl), -C(O)C₁-C₆alkyl, -C(O) heteroaryl (preferably -C(O)-5-7 membered heteroaryl), -C(O)C₁-C₆alkyl phenyl, -C(O)C₁-C₆haloalkyl, -OC(O)C₁-C₆alkyl (preferably -OC(O)C₁-C₃alkyl), -S(O)₂-C₁-C₆alkyl, -S(O)-C₁-C₆alkyl, -S(O)₂-phenyl, -S(O)₂-C₁-C₆haloalkyl, -S(O)₂NH₂, -S(O)₂NH(C₁-C₆alkyl), -S(O)₂NH (phenyl), -NHS(O)₂(C₁-C₆alkyl), -NHS(O)₂(phenyl) and -NHS(O)₂(C₁-C₆haloalkyl), wherein each of the alkyl, cycloalkyl, phenyl, aryl, heterocyclyl and heteroaryl is optionally further substituted by one or more substituents selected from halogen, -OH, -NH₂, cycloalkyl, 3-8 membered heterocyclyl, C₁-C₄alkyl, C₁-C₄haloalkyl-, -OC₁-C₄alkyl, -C₁-C₄alkyl-OH, -C₁-C₄alkyl O-C₁-C₄alkyl, -OC₁-C₄haloalkyl, cyano, nitro, -C(O)-OH, -C(O)OC₁-C₆alkyl, -CON(C₁-C₆alkyl)₂, -CONH(C₁-C₆alkyl), -CONH₂-NHC(O)(C₁-C₆alkyl), -NH(C₁-C₆alkyl) C(O)(C₁-C₆alkyl), -SO₂(C₁-C₆alkyl), -SO₂(phenyl), -SO₂(C₁-C₆haloalkyl), -SO₂NH₂, -SO₂NH(C₁-C₆alkyl), -SO₂NH (phenyl), -NHSO₂(C₁-C₆alkyl), -NHSO₂(phenyl) and -NHSO₂(C₁-C₆haloalkyl). When an atom or group is substituted with various substituents, the substituents may be the same or different. As used herein, the terms "moiety," "structure moiety," "chemical moiety," "group," "chemical group" refer to a specific fragment or functional group in a molecule. A chemical moiety is generally considered to be a chemical entity embedded in or attached to a molecule.

"Stereoisomer" refers to compound composed of the same atoms, bonded by the same bonds, but with different three-dimensional structures. The present invention is intended to encompass various stereoisomers and mixtures thereof.

When the compound of the present invention contains olefinic double bonds, the compound of the present invention is intended to include both E-and Z-geometric isomers unless otherwise specified.

The term "tautomer" refers to an isomer formed by the transfer of a proton from one atom of a molecule to another atom of the same molecule. All tautomeric forms of the compound of the invention will also be included within the scope of the invention.

The compounds of the present invention, or pharmaceutically acceptable salts thereof, may contain one or more chiral carbon atoms, and thus may give rise to enantiomers, diastereomers, and other stereoisomeric forms. Each chiral carbon atom may be defined as (R)- or (S)- based on stereochemistry. The present invention is intended to include all possible isomers, as well as racemates and optically pure forms thereof. Racemates, diastereomers or enantiomers can be selected as starting materials or intermediates in the preparation of the compounds of the present invention. Isomers with optical activity can be prepared using chiral synthons or chiral reagents, or resolved using conventional techniques, such as crystallization and chiral chromatography.

Conventional techniques for the preparation/separation of individual isomers include chiral synthesis from appropriate optically pure precursors, or resolution of the racemate (or racemate of salts or derivatives) using, for example, chiral high performance liquid chromatography, see, for example, Gerald Gübitz and Martin G. Schmid (Eds.), Chiral Separations, Methods and Protocols, Methods in Molecular Biology, Vol. 243, 2004;A.M. Stalcup, Chiral Separations. Annu. Rev. Anal. Chem. 3:341-63, 2010**;** Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res.1990, 23, 128.

As used herein, the term "pharmaceutically acceptable salts" includes pharmaceutically acceptable acid addition salts and pharmaceutically acceptable base addition salts.

The term "pharmaceutically acceptable acid addition salts" refers to salts formed with inorganic or organic acids that retain the bioavailability of the free base without exerting other adverse effects. The inorganic acid salts include but are not limited to hydrochloride, hydrobromide, sulfate, nitrate, phosphate, etc.; the organic acid salts include but are not limited to formate, acetate, 2, 2-dichloro acetate, trifluoroacetate, propionate, caproate, caprylate, caprate, undecylenate, glycolate, gluconate, lactate, sebacate, adipate, glutarate, malonate, oxalate, maleate, succinate, fumarate, tartrate, citrate, palmitate, stearate, oleate, cinnamate, lauroleate, malate, glutamate, pyroglutamate, aspartate, benzoate, mesylate, benzene sulfonate, p-toluenesulfonate, alginate, ascorbate, salicylate, 4-aminosalicylate, naphthalene disulfonate, etc. These salts can be prepared by methods known in the art.

The term "pharmaceutically acceptable base addition salts" refers to salts formed with inorganic or organic bases that retain bioavailability of the free acid without exerting other adverse effects. Salts derived from inorganic bases include, but are not limited to, sodium salts, potassium salts, lithium salts, ammonium salts, calcium salts, magnesium salts, ferric salts, zinc salts, copper salts, manganese salts, aluminum salts, and the like. Preferred inorganic salts are ammonium salts, sodium salts, potassium salts, calcium salts and magnesium salts. Salts derived from organic bases include, but are not limited to, primary, secondary and tertiary amines; substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucamine, methylglucamine, theobromine, purine, piperazine, piperidine, N-ethylpiperidine, polyamine resin, and the like. Preferred organic bases include isopropylamine, diethylamine, ethanolamine, trimethylamine, dicyclohexylamine, choline and caffeine. These salts can be prepared by methods known in the art.

As used herein, the term "pharmaceutical composition" refers to a formulation of a compound of the present invention in combination with a medium generally acceptable in the art for the delivery of a biologically active compound to a mammal (e. g., a human). The medium includes pharmaceutically acceptable carriers. The purpose of the pharmaceutical composition is to promote the administration of the organism, facilitate the absorption of active ingredients and thereby exert biological activity.

As used herein, the term "pharmaceutically acceptable" refers to a substance (e. g., a carrier or diluent) that does not affect the biological activity or properties of the compound of the invention, and is relatively non-toxic, i.e., the substance can be administered to an individual without causing any adverse biological reaction or interacting in an adverse manner with any of the components contained in the composition.

In the present application, "pharmaceutically acceptable carriers" include, but are not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier licensed by relevant governmental regulatory authority as acceptable for human or livestock use.

As used herein, the term "tumors" include but are not limited to lung cancer, pancreatic cancer, colorectal cancer, leukemia, Ewing's sarcoma, breast cancer, prostate cancer, T cell lymphoma, B cell lymphoma, malignant rhabdomyoma, synovial sarcoma, endometrioma, gastric cancer, liver cancer, renal cancer, melanoma, ovarian cancer, brain glioma, cholangiocarcinoma, nasopharyngeal carcinoma, cervical carcinoma, head and neck cancer, esophageal cancer, thyroid cancer and bladder cancer, etc.

As used herein, the terms "prophylactic", "preventing" and "prevention" include reducing the possibility of the occurrence or worsening of a disease or disorder in patients.

As used herein, the term "treatment" and other similar synonyms include the following meanings:
(i) Preventing the occurrence of the disease or disorder in mammals, especially when such mammals are susceptible to such diseases or disorders but have not been diagnosed as having it;
(ii) Inhibiting the disease or disorder, i.e. curbing its development;
(iii) Alleviating disease or disorder, i.e., abating the condition of the disease or disorder; or
(iv) Alleviating the symptoms caused by the disease or disorder.

As used herein, the terms "effective amount," "therapeutically effective amount," or "pharmaceutically effective amount" refer to an amount of at least one agent or compound that, upon administration, is sufficient to alleviate to some extent of one or more of the symptoms of the disease or condition being treated. The result can be the reduction and/or alleviation of signs, symptoms, or causes, or any other desired change in a biological system. For example, an "effective amount" for treatment is the amount of a composition comprising the compound disclosed herein required to provide a clinically significant condition-relieving effect. An effective amount appropriate in any individual case can be determined using techniques such as dose escalation assays.

As used herein, the terms "administration," "administed" "administering" and the like refer to methods that enable the delivery of a compound or composition to the desired site for biological action. These methods include, but are not limited to, oral routes, transduodenal routes, parenteral injection (including intravenous, subcutaneous, intraperitoneal, intramuscular, intra-arterial injection or infusion), topical administration, and rectal administration. Those skilled in the art are familiar with the application techniques of the compounds and methods described herein, such as those discussed in Goodman and Gilman, The Pharmacological of Therapeutics, current ed.; Pergamon; and Remington's, Pharmaceutical Sciences (current edition), Mack Publishing Co., Easton, Pa. In preferred examples, the compounds and compositions discussed herein are administered orally.

As used herein, the terms "pharmaceutical combination", "administratd in combination", " combined administration ", "administration of other treatments", "administration of other therapeutic agents" and the like refer to pharmaceutical treatments obtained by admixing or combining more than one active ingredients, which includes both fixed and non-fixed combinations of active ingredients. The term "fixed combination" refers to the simultaneous administration of at least one compound described herein and at least one synergists to a patient in the form of a single entity or single dosage form. The term "non-fixed combination" refers to that at least one compound described herein and at least one synergistic formulation are administered simultaneously, concomitantly, or sequentially at variable intervals to a patient as separate entities. These are also applied to cocktail therapies, for example the administration of three or more active ingredients.

It should also be understood by those of skill in the art that in the methods described below, the functional group of the intermediate compound may need to be protected by an appropriate protecting group. Such functional groups include hydroxyl, amino, sulfydryl, and carboxylic acid. Suitable hydroxyl protecting groups include trialkylmethanosilyl or diaromatic cyclic alkylmethanosilyl (e.g. tert-butyldimethylmethanosilyl, tert-butyl diphenylmethanosilyl or trimethylmethanosilyl), tetrahydropyranyl, benzyl, etc. Suitable protecting groups for amino, amidinyl and guanidinyl include tert-butoxycarbonyl, benzyloxycarbonyl and the like. Suitable thiol protecting groups include -C(O)-R" (wherein R" is alkyl, aryl or arylalkyl), p-methoxybenzyl, trityl, and the like. Suitable carboxy protecting groups include alkyl, aryl or aryl alkyl esters.

Protecting groups can be introduced and removed according to standard techniques known to those skilled in the art and as described herein. The use of protecting groups is described in detail in Greene, T. W. and P. G. M. Wuts, Protective Group s in Organi Synthesis, (1999), 4th Ed., in Wiley. The protecting group may also be polymer resin.

### Beneficial effect

1. Provided a compound of formula **I** or pharmaceutically acceptable salts thereof.
2. Provided a structurally novel pharmaceutical composition for the prevention and treatment of diseases mediated by KRAS G12C mutation.

### Detailed Description of Embodiments

The present invention was further specified with specific examples. It should be clear to those skilled in the art that the examples are merely intended to assist in understanding the present invention and should not be considered as specific limitations to the present invention.

The experimental methods without specific conditions in the following examples generally follow the conventional conditions or the conditions suggested by the manufacturer. Unless otherwise stated, percentages and portions are caculated by weight.

Unless otherwise specified, the experimental materials and reagents used in the following examples are commercially available.

In each example, ¹H NMR was recorded on a BRUKER AVANCE NEO 400 MHz Nuclear Magnetic Resonance instrument with chemical shifts reported as *δ* (ppm); Liquid chromatography-mass spectrometry (LCMS) was recorded on Shimadzu LC-20AD, SIL-20A, CTO-20AC, SPD-M20A, CBM-20A, LCMS-2020 mass spectrometer; preparative HPLC separation used Gilson -281 liquid chromatograph.

### Preparation of intermediates

### 1. Preparation of intermediate A

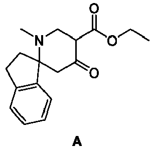

The synthetic route of intermediate A is shown below: 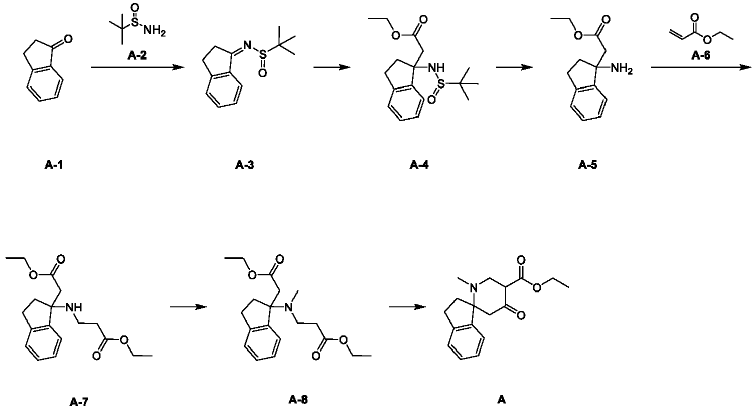
(1) To a solution of compound **A- 1** (59.9 g, 454 mmol) in toluene (500 mL) was added compound **A- 2** (50.0g, 413 mmol) and tetraethyl titanate (188g, 825 mmol). The reaction solution was stirred at 130°C for 12h under nitrogen protection. Then the reaction solution was added with water(400 mL), and extracted with ethyl acetate (400 mL × 2). The combined organic phase was washed with saturated NaCl aqueous solution (500 mL × 1), dried over anhydrous sodium sulfate, filtered, and concentrated under reduced pressure. The crude product was isolated by silica gel column chromatography (petroleum ether/ethyl acetate = 1:0 to 0:1) to obtain compound **A- 3.**
   MS-ESI [M+H]⁺, calculated: 236, found: 236.
(2) To a solution of compound **A- 3** (50.8 g, 216 mmol), and ethyl acetate (95.1g, 1.08 mol) in tetrahydrofuran (500 mL) was added dropwise with lithium diisopropylamide (2 mol/L, 216 mL, tetrahydrofuran solution) at -78°C under nitrogen protection. The reaction solution was stirred at -78°C for 3 hours. After warming to 0°C, the reaction solution was quenched with saturated aqueous ammonium chloride solution(500 mL), and extracted with ethyl acetate (1000 mL × 2). The combined organic phase was dried over anhydrous sodium sulfate, filtered, and concentrated under reduced pressure. The crude product was separated by silica gel column chromatography (petroleum ether/ethyl acetate = 1:0 to 0:1) to obtain compound **A- 4.**
   MS-ESI [M+H]⁺, calculated: 324, found: 324.
   ¹H NMR (400 MHz, CDCl₃) *δ* 7.18-7.26 (m, 4H), 5.21 (s, 1H), 4.17 (qd, *J* = 7.2, 3.2 Hz, 2H), 3.17 (ddd, *J =* 16.4, 8.4, 5.6 Hz, 1H), 2.88-2.93 (m, 1H), 2.73-2.87 (m, 2H), 2.64-2.72 (m, 1H), 2.32 (ddd, *J =* 13.6, 8.4, 5.6 Hz, 1H), 1.24-1.27 (m, 3H), 1.19 (s, 9H).
(3) To a solution of compound A- **4** (15.6 g, 48.2 mmol) in ethanol (100 mL) was added a solution of hydrochloric acid in dioxane (4 mol/L, 30 mL). The reaction solution was stirred at 25°C for 5 hours under nitrogen protection. The reaction solution was concentrated under reduced pressure to afford crude product of hydrochloride of compound A-**5.**
   MS-ESI [M-NH₂+H]⁺, calculated: 203, found: 203.
(4) To a solution of hydrochloride of compound A-**5** (12.0 g, 46.9 mmol) in ethanol (50.0 mL) was added compound A- **6** (46.9g, 469 mmol), copper oxide (373 mg, 4.69 mmol) and triethylamine (4.75g, 46.9 mmol). The reaction solution was reacted at 85°C for 4 hours in a sealed tube under nitrogen protection. Then the reaction solution was added with water(500 mL), and extracted with ethyl acetate (500 mL × 2). The combined organic phase was washed with saturated NaCl aqueous solution (500 mL × 1), dried over anhydrous sodium sulfate, filtered and concentrated under reduced pressure. The crude product was separated by silica gel column chromatography (petroleum ether/ethyl acetate = 1:0 to 0:1) to obtain compound A- **7.**
   MS-ESI [M+H]⁺, calculated: 320, found: 320.
   ¹H NMR (400 MHz, CDCl₃) *δ* 7.13-7.26 (m, 4H), 4.09-4.15 (m, 4H), 2.87-2.99 (m, 2H), 2.76 (q, *J* = 6.8 Hz, 2H), 2.63 (br d, *J* = 14.8 Hz, 1H), 2.54 (dd, *J* = 11.2, 6.4 Hz, 1H), 2.42-2.45 (m, 2H), 2.28-2.36 (m, 1H), 2.17 (ddd, *J* = 13.2, 8.4, 4.8 Hz, 1H), 1.18-1.26 (m, 6H).
(5) To a solution of compound A-**7** (13.7 g, 42.9 mmol) in ethanol (100 mL) was added paraformaldehyde (6.44g) and sodium cyanoborohydride (8.09g, 129 mmol). The reaction solution was reacted at 25°C for 1 hour under nitrogen protection. Then the reaction solution was added with water (150 mL), and extracted with ethyl acetate (200 mL × 2). The combined organic phase was washed with saturated NaCl aqueous solution (150 mL × 1), dried over anhydrous sodium sulfate, filtered, and concentrated under reduced pressure. The crude product was isolated by silica gel column chromatography (petroleum ether/ethyl acetate = 3:1) to obtain compound A- **8.**
   MS-ESI [M+H]⁺, calculated: 334, found: 334.
   ¹H NMR (400 MHz, CDCl₃) *δ* 7.11-7.26 (m, 4H), 4.07-4.12 (m, 2H), 3.81-3.97 (m, 2H), 2.79-3.00 (m, 3H), 2.74-2.78 (m, 1H), 2.57-2.73 (m, 2H), 2.24-2.48 (m, 4H), 2.17 (s, 3H), 1.22-1.26 (m, 3H), 1.01 (t, *J* = 7.2 Hz, 3H).
(6) To a solution of compound A- **8** (13.4 g, 40.2 mmol) in tetrahydrofuran (500 mL) was added potassium bis (trimethylsilyl) amide (1 mol/L, 121 mL, tetrahydrofuran solution) at -78°C. The reaction solution was reacted at -78°C for 1 hour under nitrogen protection. Then the reaction soluton was added with water (500 mL), and extracted with ethyl acetate (500 mL × 2). The combined organic phase was washed with saturated NaCl aqueous solution (500 mL × 1), dried over anhydrous sodium sulfate, filtered, and concentrated under reduced pressure to obtain the crude compound **A.**
   MS-ESI [M+H]⁺, calculated: 288, found: 288.
   ¹H NMR (400 MHz, CDCl₃) *δ* 7.19-7.27 (m, 4H), 4.20-4.31 (m, 2H), 3.44-3.61 (m, 1H), 3.18-3.31 (m, 1H), 2.90-2.99 (m, 2H), 2.69 (br t, *J* = 13.2 Hz, 1H), 2.30-2.44 (m, 1H), 2.16-2.29 (m, 1H), 2.06-2.15 (m, 3H), 1.61-1.96 (m, 2H), 1.30-1.34 (m, 3H).

### Example 1 Synthesis of Compound 1

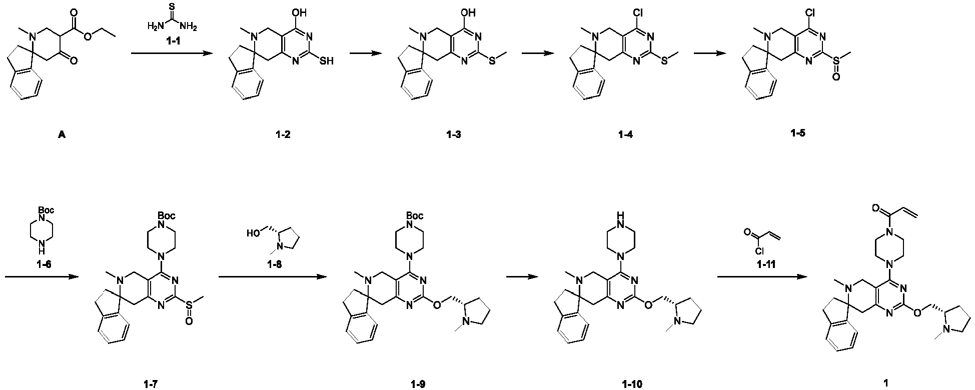

(1) To a solution of intermediate **A** (2.0 g, 6.96 mmol) in ethanol (30.0 mL) was added compound **1-1**(1.06g, 13.9 mmol) and sodium ethoxide (1.42g, 20.9 mmol), and the reaction solution was stirred at 80°C for 16 hours under nitrogen protection. After concentrated under reduced pressure, the reaction solution was added with ethanol (15.0 mL) and water (5.0 mL) and its pH was adjusted to 6 with hydrochloric acid (1 mol/L). The resulting solution was precipitated, and filtered, and the filter cake was dried to obtain compound **1-2.**
   MS-ESI [M+H]⁺, calculated: 300, found: 300.
   ¹H NMR (400 MHz, DMSO-d6) *δ* 12.43 (br s, 1H), 12.28 (br s, 1H), 7.23-7.27 (m, 3H), 7.21 (br s, 1H), 3.49 (br d, *J* = 16.8 Hz, 1H), 3.05 (br d, *J* = 16.4 Hz, 1H), 2.84-2.95 (m, 2H), 2.57-2.69 (m, 1H), 2.40-2.48 (m, 1H), 2.15 (dt, *J =* 13.2, 8.4 Hz, 1H), 1.98 (s, 3H), 1.59-1.69 (m, 1H).
(2) To a solution of compound **1-2** (1.66 g, 5.54 mmol) in water (15.0 mL) was added sodium hydroxide (443 mg, 11.1 mmol), and dimethyl sulfate (1.93 mg, 15.3 mmol), and the reaction solution was stirred at 0°C for 2 hours under nitrogen protection. The reaction solution was added with water(100 mL), and extracted with ethyl acetate (100 mL × 3). The combined organic phase was dried over anhydrous sodium sulfate, filtered, and concentrated under reduced pressure. The crude product was separated by silica gel column chromatography (petroleum ether/ethyl acetate = 1:0 to 1:2) to obtain compound **1-3.**
   MS-ESI [M+H]⁺, calculated: 314, found: 314.
   ¹H NMR (400 MHz, DMSO-d6) *δ* 12.66 (br s, 1H), 7.21-7.26 (m, 3H), 7.15-7.20 (m, 1H), 3.55 (br d, *J* = 17.2 Hz, 1H), 3.12-3.20 (m, 1H), 2.90 (br t, *J* = 7.2 Hz, 2H), 2.77 (br d, *J* = 17.6 Hz, 1H), 2.54 (br d, *J* = 18.4 Hz, 1H), 2.44 (s, 3H), 2.18 (dt, *J* = 13.2, 8.4 Hz, 1H), 2.00 (s, 3H), 1.61 (dt, *J* = 13.2, 6.4 Hz, 1H).
(3) To a solution of compound **1-3** (840 mg, 2.68 mmol) in chloroform (4.0 mL) was added phosphorus oxychloride (6.60g, 43.0 mmol), and the reaction solution was stirred at 80°C for 1 hour under nitrogen protection. Then the reaction solution was concentrated under reduced pressure, added with ice water (50.0 mL), and extracted with dichloromethane (50.0 mL × 1). The organic phase was washed with saturated sodium bicarbonate aqueous solution (50.0 mL × 2) and saturated NaCl aqueous solution (500 mL × 1), dried over anhydrous sodium sulfate, filtered, and concentrated under reduced pressure to obtain compound **1-4.**
   MS-ESI [M+H]⁺, calculated: 332, found: 332.
   ¹H NMR (400 MHz, CDCl₃) *δ* 7.27 (br s, 1H), 7.18-7.26 (m, 3H), 3.95 (br d, *J* = 16.8 Hz, 1H), 3.48-3.60 (m, 1H), 3.04-3.22 (m, 1H), 2.97-3.04 (m, 1H), 2.89-2.97 (m, 2H), 2.54 (s, 3H), 2.23-2.31 (m, 1H), 2.20 (s, 3H), 1.65-1.71 (m, 1H).
(4) To a solution of compound **1-4** (200 mg, 603 µmol) in dichloromethane (5.0 mL) was added m-chloroperoxybenzoic acid (123 mg, 606 µmol), and the reaction solution was stirred at 0°C for 5 hours under nitrogen protection. Then the reaction solution was added with saturated sodium sulfite aqueous solution (30.0 mL), and extracted with dichloromethane (20.0 mL). The organic phase was washed with saturated sodium bicarbonate aqueous solution (20.0 mL × 2) and saturated NaCl aqueous solution (20.0 mL × 1), dried over anhydrous sodium sulfate, filtered, and concentrated under reduced pressure to obtain compound **1-5.**
   MS-ESI [M+H]⁺, calculated: 348, found: 348.
(5) To a solution of compound **1-5** (206 mg, 592 µmol) in *N*,*N*-Dimethylformamide (10.0 mL) was added compound **1-6** (552 mg 2.96 mmol), and the reaction solution was stirred at 25°C for 16 hours under nitrogen protection. Then the reaction solution was added with ethyl acetate (30.0 mL), washed with saturated NaCl aqueous solution (20.0 mL × 5), dried over anhydrous sodium sulfate, filtered, and concentrated under reduced pressure. The crude product was separated by preparative thin layer chromatography (dichloromethane/methanol = 10:1) to obtain compound **1-7.**
   MS-ESI [M+H]⁺, calculated: 498, found: 498.
(6) To a solution of compound **1-7** (32.0 mg, 64.3 µmol) in tetrahydrofuran (3.0 mL) was added compound **1-8** (22.2 mg, 193 µmol), and sodium tert-butoxide (18.6 mg, 194 µmol), and the reaction solution was stirred at 25°C for 1 hour under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure to afford compound **1-9.**
   MS-ESI [M+H]⁺, calculated: 549, found: 549.
(7) To a solution of compound **1-9** (35.0 mg, 63.8 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **1-10.**
   MS-ESI [M+H]⁺, calculated: 449, found: 449.
(8) To a solution of trifluoroacetate of compound **1-10** (35.0 mg, 62.2 µmol) in dichloromethane (2.0 mL) was added triethylamine (31.5 mg, 331 µmol) and compound **1-11** (16.9 mg, 187 µmol), and the reaction solution was stirred at -78°C for 1 hour under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (3_Phenomenex Luna C18, 70mm × 30 mm 3 µm, A: water (10 mmol/L ammonium bicarbonate); B: acetonitrile, 20%-70%: 40 min) to obtain compound **1.**
   MS-ESI [M+H]⁺, calculated: 503, found: 503.
   ¹H NMR (400 MHz, MeOD) *δ* 7.20-7.31 (m, 4H), 6.81 (dd, *J* = 16.8, 10.4 Hz, 1H), 6.26 (dd, *J* = 16.8, 1.6 Hz, 1H), 5.80 (dd, *J* = 10.4, 1.6 Hz, 1H), 4.34-4.41 (m, 1H), 4.27-4.33 (m, 1H), 3.85 (br d, *J* = 3.2 Hz, 2H), 3.74-3.81 (m, 2H), 3.63-3.72 (m, 4H), 3.54 (br s, 2H), 3.08 (dt, *J* = 9.6, 4.8 Hz, 1H), 2.95-3.04 (m, 2H), 2.82-2.94 (m, 2H), 2.70-2.78 (m, 1H), 2.49 (s, 3H), 2.39-2.46 (m, 1H), 2.35 (q, *J =* 8.8 Hz, 1H), 2.20 (s, 3H), 2.05-2.13 (m, 1H), 1.85-1.91 (m, 1H), 1.76-1.84 (m, 2H), 1.65-1.74 (m, 1H).

### Example 2 Synthesis of Compound 2

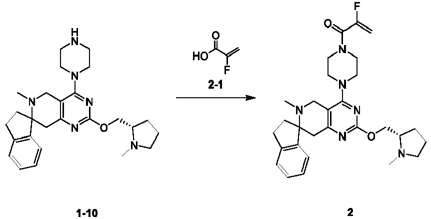

To a solution of compound **2-1** (19.2 mg, 213 µmol) in dichloromethane (2.0 mL) was added triethylamine (108 mg, 1.07 µmol) and 2-(7-azabenzotriazole)-*N,N,N',N'*-tetramethylurea hexafluorophosphate (81.2 mg, 214 µmol), and the reaction solution was stirred at 25°C for 0.5 hours under nitrogen protection, and the reaction solution was added with trifluoroacetate of compound **1-10** (60.0 mg, 107 µmol), and continued stirring for 0.5 hours at 25°C. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 70mm × 30 mm 3 µm, A: water (0.225% formic acid); B: acetonitrile, 0%-30%: 35 min) to obtain formate of compound **2.**
MS-ESI [M+H]⁺, calculated: 521, found: 521.
¹H NMR (400 MHz, MeOD) *δ* 7.25-7.30 (m, 4H), 5.28-5.35 (m, 1H), 5.24 (dd, *J* = 19.2, 4.0 Hz, 1H), 4.70 (dt, *J* = 12.4, 3.6 Hz, 1H), 4.47-4.55 (m, 1H), 3.81-3.90 (m, 3H), 3.73-3.78 (m, 4H), 3.61-3.71 (m, 3H), 3.52-3.61 (m, 2H), 3.21 (dt, *J =* 11.2, 8.0 Hz, 1H), 3.04-3.12 (m, 1H), 3.02 (s, 3H), 2.95-3.01 (m, 2H), 2.88-2.94 (m, 1H), 2.47 (dt, *J* = 13.2, 8.4 Hz, 1H), 2.32-2.40 (m, 1H), 2.24 (s, 3H), 2.14-2.20 (m, 1H), 2.09 (br dd, *J* = 14.4, 7.2 Hz, 1H), 1.97-2.04 (m, 1H), 1.89 (ddd, *J* = 13.2, 8.4, 4.4 Hz, 1H).

### Example 3 Synthesis of Compound 3

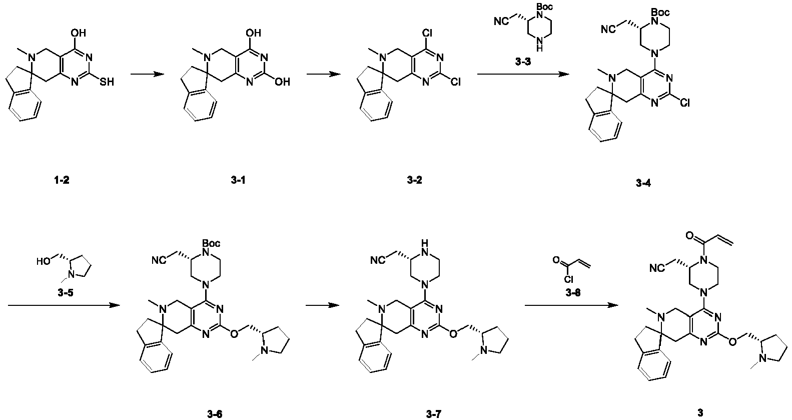

(1) To a solution of intermediate **1-2** (500 mg, 1.67 mmol) in water (10.0 mL) was added chloroacetic acid (1.27g, 13.9 mmol), and the reaction solution was stirred at 100°C for 16 hours under nitrogen protection. After the reaction solution was filtered, the filtrate was adjusted to pH 8 with saturated sodium bicarbonate aqueous solution to give precipitate. After filtration, the filter cake was dried to obtain compound **3-1.**
   MS-ESI [M+H]⁺, calculated: 284, found: 284.
   ¹H NMR (400 MHz, DMSO-*d*6)*δ* 11.01 (s, 1H), 10.76 (s, 1H), 7.22-7.27 (m, 3H), 7.18-7.22 (m, 1H), 3.41-3.45 (m, 1H), 3.02 (br d, *J* = 16.0 Hz, 1H), 2.81-2.93 (m, 2H), 2.56 (br d, *J* = 17.2 Hz, 1H), 2.32 (d, *J* = 17.2 Hz, 1H), 2.16 (dt, *J* = 13.2, 8.4 Hz, 1H), 1.97 (s, 3H), 1.62 (ddd, *J* = 13.2, 8.4, 4.4 Hz, 1H).
(2) Compound **3-1** (328 mg, 1.16 mmol) was dissolved in phosphorus oxychloride (4.95g, 32.3 mmol), and the reaction solution was stirred at 80°C for 16 hours under nitrogen protection. Then the reaction solution was concentrated under reduced pressure, added with ice water(50.0 mL), and extracted with dichloromethane (50.0 mL × 1). The organic phase was washed with saturated sodium bicarbonate aqueous solution (50.0 mL × 2) and saturated NaCl aqueous solution (500 mL × 1), dried over anhydrous sodium sulfate, filtered, and concentrated under reduced pressure to obtain compound **3-2.**
   MS-ESI [M+H]⁺, calculated: 320, found: 320.
   ¹H NMR (400 MHz, CDCl₃) *δ* 7.27 (br d, *J* = 5.2 Hz, 3H), 7.19 (br d, *J* = 4.4 Hz, 1H), 3.98 (d, *J =* 17.6 Hz, 1H), 3.55 (br d, *J* = 17.6 Hz, 1H), 3.11-3.22 (m, 1H), 2.91-3.05 (m, 3H), 2.23-2.31 (m, 1H), 2.20 (s, 3H), 1.62 (td, *J =* 8.8, 4.4 Hz, 1H).
(3) To a solution of compound **3-2** (140 mg, 437 µmol) in *N*,*N*-Dimethylformamide (5.0 mL) was added potassium carbonate (151 mg, 1.09 mmol) and compound **3-3** (82.1 mg, 364 µmol), and the reaction solution was stirred at 50°C for 6 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure. The crude product was separated by preparative thin layer chromatography (petroleum ether/ethyl acetate = 1:1) to obtain compound **3-4.**
   MS-ESI [M+H]⁺, calculated: 509, found: 509.
(4) To a solution of compound **3-4** (120 mg, 236 µmol) in dioxane (5.0 mL) was added compound **3-5** (81.5 mg 708 µmol), cesium carbonate (231 mg, 709 µmol) and (2-dicyclohexylphosphino-2',6'-diisopropoxy-1,1'-biphenyl) [2-(2'-amino-1,1'- biphenyl)] palladium(II) methanesulfonate (39.4 mg, 47.1 µmol), and the reaction solution was stirred at 110°C for 4 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by preparative thin layer chromatography (petroleum ether/ethyl acetate = 1:2) to obtain compound **3-6.**
   MS-ESI [M+H]⁺, calculated: 588, found: 588.
   ¹H NMR (400 MHz, MeOD)*δ* 7.19-7.29 (m, 4H), 4.59-4.65 (m, 1H), 4.30-4.41 (m, 2H), 3.92-4.16 (m, 3H), 3.68-3.76 (m, 2H), 3.41 (br dd, *J* = 14.0, 3.6 Hz, 1H), 3.14-3.25 (m, 2H), 2.96-3.11 (m, 4H), 2.73-2.95 (m, 4H), 2.54 (br d, *J* = 3.6 Hz, 3H), 2.43 (td, *J =* 13.2, 8.4 Hz, 2H), 2.21 (d, *J* = 2.8 Hz, 3H), 2.07-2.17 (m, 1H), 1.81-1.92 (m, 3H), 1.67-1.77 (m, 1H), 1.51 (s, 9H).
(5) To a solution of compound **3-6** (30.0 mg, 51.0 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **3-7.**
   MS-ESI [M+H]⁺, calculated: 488, found: 488.
(6) To a solution of trifluoroacetate of compound **3-7** (30.0 mg, 49.9 µmol) in dichloromethane (2.0 mL) was added triethylamine (25.2 mg, 249 µmol) and compound **3-8** (13.6 mg, 150 µmol), and the reaction solution was stirred at -78°C for 1 hour under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **3.**
   MS-ESI [M+H]⁺, calculated: 542, found: 542.
   ¹H NMR (400 MHz, MeOD) *δ* 7.25-7.31 (m, 4H), 6.82 (br d, *J =* 11.6 Hz, 1H), 6.30 (br d, *J* = 16.4 Hz, 1H), 5.84 (br d, *J* = 10.4 Hz, 1H), 5.04 (br s, 1H), 4.72 (dt, *J* = 12.4, 3.2 Hz, 1H), 4.54 (dd, *J* = 12.4, 7.2 Hz, 1H), 4.18-4.40 (m, 1H), 3.96-4.18 (m, 2H), 3.79-3.88 (m, 3H), 3.65-3.71 (m, 1H), 3.45-3.62 (m, 1H), 3.31-3.44 (m, 1H), 3.13-3.26 (m, 2H), 3.04-3.13 (m, 2H), 3.03 (d, *J =* 5.2 Hz, 3H), 2.89-3.01 (m, 4H), 2.43-2.52 (m, 1H), 2.32-2.40 (m, 1H), 2.26 (d, *J* = 2.4 Hz, 3H), 2.14-2.20 (m, 1H), 2.09 (br dd, *J* = 14.0, 7.6 Hz, 1H), 1.99-2.05 (m, 1H), 1.87-1.96 (m, 1H).

### Example 4 Synthesis of Compound 4

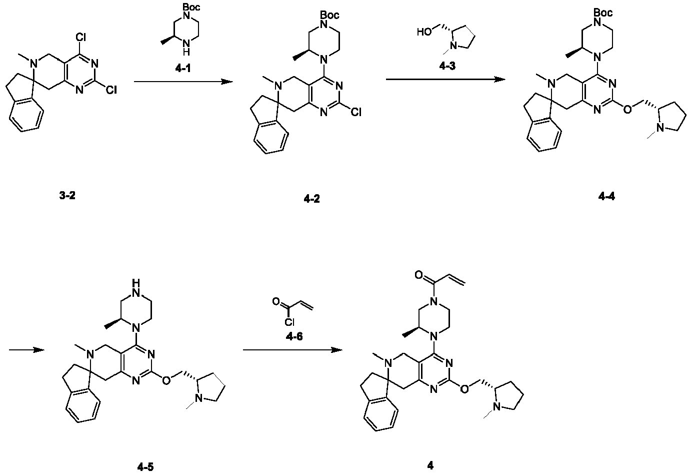

(1) To a solution of compound **3-2** (200 mg, 625 µmol) in N-methylpyrrolidone (5.0 mL) was added potassium carbonate (259 mg, 1.87 mmol) and compound **4-1** (250 mg, 1.25 mmol), and the reaction solution was stirred at 50°C for 16 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure. The crude product was separated by preparative thin layer chromatography (petroleum ether/ethyl acetate = 1:0 to 4:1) to give compound **4-2.**
   MS-ESI [M+H]⁺, calculated: 484, found: 484.
(2) To a solution of compound **4-2** (160 mg, 331 µmol) in dioxane (5.0 mL) was added compound **4-3** (115 mg, 999 µmol), cesium carbonate (323 mg, 991 µmol) and ((2-dicyclohexylphosphino-2',6'-diisopropoxy-1,1'-biphenyl) [2-(2'-amino-1,1'- biphenyl)] palladium(II) methanesulfonate (55.3 mg, 66.1 µmol), and the reaction solution was stirred at 110°C for 4 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by preparative thin layer chromatography (dichloromethane/methanol = 10:1) to obtain compound **4-4.**
   MS-ESI [M+H]⁺, calculated: 563, found: 563.
   ¹H NMR (400 MHz, MeOD)*δ* 7.18-7.30 (m, 4H), 4.32-4.40 (m, 2H), 4.21 (br s, 1H), 4.02 (br s, 1H), 3.76-3.98 (m, 2H), 3.50-3.75 (m, 3H), 3.38-3.50 (m, 1H), 3.32-3.38 (m, 1H), 3.07-3.20 (m, 2H), 2.97-3.05 (m, 2H), 2.87-2.96 (m, 2H), 2.84 (br d, *J* = 2.8 Hz, 1H), 2.54 (s, 2H), 2.38-2.48 (m, 2H), 2.19 (s, 3H), 2.05-2.14 (m, 1H), 1.80-1.93 (m, 3H), 1.68-1.77 (m, 1H), 1.49 (d, *J* = 0.8 Hz, 9H), 1.17-1.37 (m, 3H).
(3) To a solution of compound **4-4** (80.0 mg, 142 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **4-5.**
   MS-ESI [M+H]⁺, calculated: 463, found: 463.
(4) To a solution of trifluoroacetate of compound **4-5** (70.0 mg, 121 µmol) in dichloromethane (2.0 mL) was added triethylamine (61.4 mg, 607 µmol) and compound **4-6** (33.0 mg, 365 µmol), and the reaction solution was stirred at -78°C for 1 hour under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **4.**
   MS-ESI [M+H]⁺, calculated: 517, found: 517.
   ¹H NMR (400 MHz, MeOD) *δ* 7.23-7.33 (m, 4H), 6.73-6.90 (m, 1H), 6.29 (br dd, *J* = 16.4, 2.4 Hz, 1H), 5.81 (dd, *J* = 10.4, 1.6 Hz, 1H), 4.71 (dd, *J* = 12.4, 3.2 Hz, 1H), 4.40-4.54 (m, 2H), 4.11-4.33 (m, 1H), 3.90-4.02 (m, 1H), 3.64-3.87 (m, 5H), 3.50-3.62 (m, 1H), 3.37-3.49 (m, 1H), 3.09-3.29 (m, 2H), 3.04-3.09 (m, 1H), 3.03 (s, 3H), 2.87-3.02 (m, 3H), 2.43-2.53 (m, 1H), 2.31-2.42 (m, 1H), 2.25 (s, 3H), 2.14-2.22 (m, 1H), 2.06-2.13 (m, 1H), 1.98-2.05 (m, 1H), 1.88-1.97 (m, 1H), 1.17-1.38 (m, 3H).

### Example 5 Synthesis of Compound 5

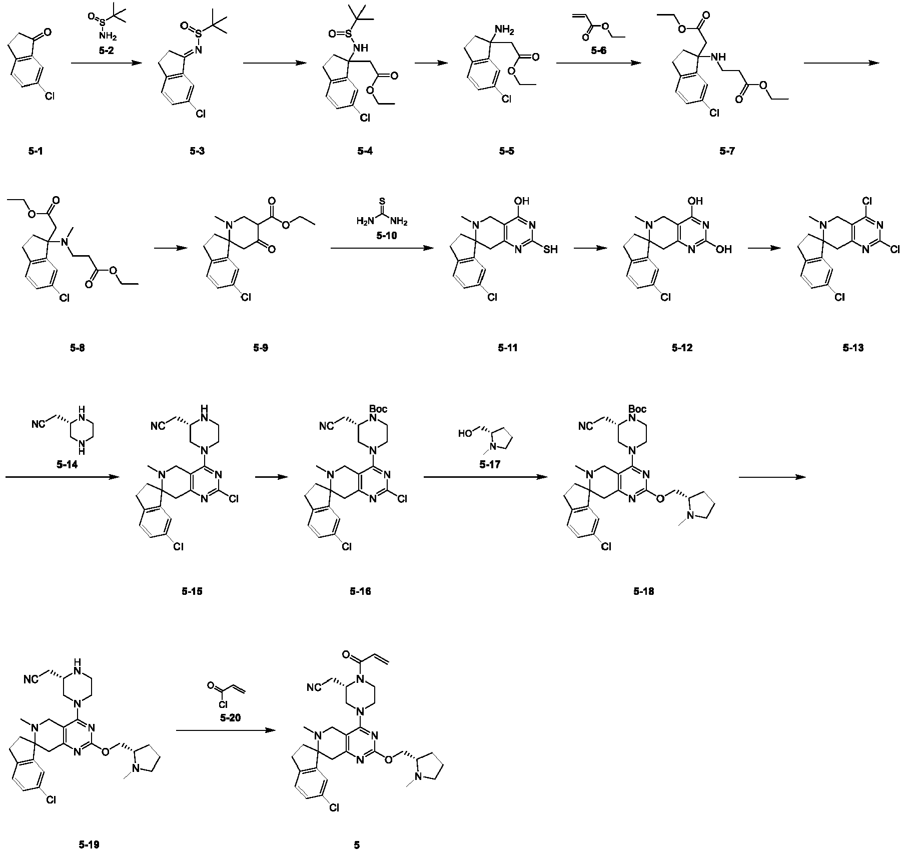

(1) To a solution of compound **5-1** (25.0 g, 150 mmol) in toluene (400 mL) was added compound **5-2** (16.6g, 137 mmol), tetraethyl titanate (62.3g, 273 mmol), and the reaction solution was stirred at 120°C for 16 hours under nitrogen protection. Then the reaction solution was added with water (200 mL), and extracted with ethyl acetate (400 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 2:1) to obtain compound **5-3.**
   MS-ESI [M+H]⁺, calculated: 270, found: 270.
   ¹H NMR (400 MHz, CDCl₃) *δ* 7.73 (d, *J* = 2.0 Hz, 1H), 7.44 (dd, *J* = 8.0, 2.0 Hz, 1H), 7.33 (d, *J* = 8.0 Hz, 1H), 3.43-3.53 (m, 1H), 3.10-3.13 (m, 2H), 3.04-3.10 (m, 1H), 1.32 (s, 9H).
(2) To a solution of ethyl acetate (22.9g, 260 mmol) in tetrahydrofuran (300 mL) was added dropwise with lithium diisopropylamide (2 mol/L, 52.0 mL, tetrahydrofuran solution) at -78°C under nitrogen protection and the reaction solution was stirred at -78°C for 1 hour. Then the reaction solution was added dropwise with a solution of compound **5-3** (14.0 g, 51.9 mmol) in tetrahydrofuran (50.0 mL), and continued stirring for 3 hours at -78°C. After warming to 0°C, then quenched with saturated ammonium chloride aqueous solution (200 mL), and extracted with ethyl acetate (200 mL × 1). The combined organic phase was washed with saturated ammonium chloride aqueous solution (200 mL × 1) and saturated NaCl aqueous solution (200 mL × 1), dried over anhydrous sodium sulfate, filtered, and concentrated under reduced pressure. The crude product was separated by silica gel column chromatography (petroleum ether/ethyl acetate = 1:0 to 4:1) to obtain compound **5-4.**
   MS-ESI [M+H]⁺, calculated: 358, found: 358.
   ¹H NMR (400 MHz, CDCl₃) *δ* 7.21-7.24 (m, 1H), 7.15-7.20 (m, 2H), 5.24 (s, 1H), 4.15-4.21 (m, 2H), 3.13 (ddd, *J* = 16.4, 8.8, 5.2 Hz, 1H), 2.83-2.90 (m, 1H), 2.73-2.83 (m, 2H), 2.67-2.73 (m, 1H), 2.33 (ddd, *J* = 13.2, 8.4, 5.2 Hz, 1H), 1.25-1.28 (m, 3H), 1.20 (s, 9H).
(3) To a solution of compound **5-4** (5.10 g, 14.3 mmol) in ethanol (40.0 mL) was added hydrochloric acid in dioxane (4 mol/L, 15.0 mL), and the reaction solution was stirred at 25°C for 2 hours under nitrogen protection. Then the reaction solution was concentrated under reduced pressure to obtain crude product of hydrochloride of compound **5-5.**
   MS-ESI [M-NH₂+H]⁺, calculated: 237, found: 237.
(4) To a solution of hydrochloride of compound **5-5** (4.0 g, 13.8 mmol) in ethanol (20.0 mL) was added compound **5-6** (15.7g, 157 mmol), copper oxide (219 mg, 2.75 mmol), triethylamine (4.18g, 41.4 mmol), and the reaction solution was reacted at 85°C in a sealed tube for 8 hours under nitrogen protection. Then the reaction solution was added with water (200 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 6:1) to obtain compound **5-7.**
   MS-ESI [M+H]⁺, calculated: 354, found: 354.
   ¹H NMR (400 MHz, CDCl₃) *δ* 7.02-7.25 (m, 3H), 4.07-4.16 (m, 4H), 2.86-2.91 (m, 1H), 2.77-2.85 (m, 1H), 2.69-2.77 (m, 2H), 2.58-2.65 (m, 1H), 2.37-2.58 (m, 4H), 2.32 (dt, *J =* 13.2, 8.4 Hz, 1H), 2.16 (ddd, *J* = 13.2, 8.4, 4.4 Hz, 1H), 1.25 (t, *J* = 7.2 Hz, 3H), 1.20 (t, *J =* 7.2 Hz, 3H).
(5) To a solution of compound **5-7** (1.0 g, 2.83 mmol) in ethanol (15.0 mL) was added paraformaldehyde (500 mg) and sodium cyanoborohydride (533 mg, 8.48 mmol), and the reaction solution was reacted for 16 hours at 25°C under nitrogen protection. Then the reaction solution was added with water (50.0 mL), and extracted with ethyl acetate (50 mL × 1). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 2:1) to obtain compound **5-8.**
   MS-ESI [M+H]⁺, calculated: 368, found: 368.
   ¹H NMR (400 MHz, CDCl₃) *δ* 7.13-7.21 (m, 2H), 7.06-7.10 (m, 1H), 4.08-4.16 (m, 2H), 3.87-3.94 (m, 2H), 2.83-2.89 (m, 3H), 2.73-2.77 (m, 1H), 2.59-2.70 (m, 2H), 2.41 (dt, *J* = 7.6, 6.4 Hz, 2H), 2.31-2.37 (m, 1H), 2.20-2.28 (m, 1H), 2.17 (s, 3H), 1.25 (t, *J* = 7.2 Hz, 3H), 1.04 (t, *J* = 7.2 Hz, 3H).
(6) To a solution of compound **5-8** (600 mg, 1.63 mmol) in tetrahydrofuran (10.0 mL) was added potassium bis (trimethylsilyl) amide (1 mol/L, 4.89 mL, tetrahydrofuran solution) at -78°C, and the reaction solution was reacted at -78°C for 2 hours under nitrogen protection. Then the reaction solution was added with saturated ammonium chloride aqueous solution (50.0 mL), and extracted with ethyl acetate (50.0 mL × 1). The combined organic phase was washed with saturated NaCl aqueous solution (50.0 mL × 1), dried over anhydrous sodium sulfate, filtered, and concentrated under reduced pressure. The crude product was separated by silica gel column chromatography (petroleum ether/ethyl acetate = 1:0 to 6:1) to obtain compound **5-9.**
   MS-ESI [M+H]⁺, calculated: 322, found: 322.
   ¹H NMR (400 MHz, CDCl₃) *δ* 7.17-7.23 (m, 2H), 7.11-7.15 (m, 1H), 4.22-4.32 (m, 2H), 3.50 (d, *J* = 15.2 Hz, 1H), 3.22-3.33 (m, 1H), 3.08-3.16 (m, 1H), 2.86-2.93 (m, 2H), 2.54-2.64 (m, 1H), 2.20-2.37 (m, 2H), 2.09 (s, 3H), 1.78 (ddd, *J =* 13.2, 8.4, 4.4 Hz, 1H), 1.30-1.34 (m, 3H).
(7) To a solution of compound **5-9** (440 mg, 1.37 mmol) in ethanol (10.0 mL) was added compound **5-10** (208 mg, 2.37 mmol) and sodium ethoxide (279 mg, 4.10 mmol), and the reaction solution was stirred at 80°C for 12 hours under nitrogen protection. After concentrated under reduced pressure, the reaction solution was added with ethanol(15.0 mL) and water (5.0 mL) and its pH was adjusted to 6 with hydrochloric acid (1 mol/L). The resulting solution was precipitated, and filtered, and the filter cake was dried to obtain compound **5-11.**
   MS-ESI [M+H]⁺, calculated: 334, found: 334.
   ¹H NMR (400 MHz, DMSO-d6) *δ* 12.20-12.60 (m, 2H), 7.19-7.36 (m, 3H), 3.36-3.56 (m, 1H), 3.06 (br s, 1H), 2.84-2.94 (m, 2H), 2.65-2.77 (m, 1H), 2.37-2.49 (m, 3H), 2.20 (br s, 1H), 1.99 (br s, 1H), 1.68 (br s, 1H).
(8) To a solution of intermediate **5-11** (360 mg, 1.08 mmol) in water (10.0 mL) was added chloroacetic acid (590 mg, 6.24 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **5-12.**
   MS-ESI [M+H]⁺, calculated: 318, found: 318.
   ¹H NMR (400 MHz, DMSO-*d*6)*δ* 11.03 (s, 1H), 10.78 (s, 1H), 7.27-7.32 (m, 2H), 7.21 (d, *J* = 1.6 Hz, 1H), 3.43 (br d, *J* = 16.0 Hz, 1H), 3.01 (br d, *J* = 16.0 Hz, 1H), 2.83-2.93 (m, 2H), 2.62 (br d, *J* = 17.6 Hz, 1H), 2.32 (d, *J* = 17.2 Hz, 1H), 2.19 (dt, *J* = 13.2, 8.4 Hz, 1H), 1.97 (s, 3H), 1.65 (ddd, *J* = 13.2, 8.4, 4.4 Hz, 1H).
(9) Compound **5-12** (210 mg, 661 µmol) was dissolved in phosphorus oxychloride (8.25g, 53.8 mmol), and the reaction solution was stirred at 80°C for 2 hours under nitrogen protection, and the reaction solution was concentrated under reduced pressure, added with iced water (50.0 mL), and extracted with dichloromethane (50.0 mL × 1). The organic phase was washed with saturated sodium bicarbonate aqueous solution (50.0 mL × 2) and saturated NaCl aqueous solution (500 mL × 1), dried over anhydrous sodium sulfate, filtered, and concentrated under reduced pressure to obtain compound **5-13.**
   MS-ESI [M+H]⁺, calculated: 356, found: 356.
   ¹H NMR (400 MHz, CDCl₃) *δ* 7.28-7.37 (m, 2H), 7.22 (s, 1H), 3.87-4.04 (m, 1H), 3.51 (br d, *J* = 18.0 Hz, 1H), 3.14 (br d, *J* = 17.6 Hz, 1H), 2.88-2.96 (m, 3H), 2.23 (dt, *J* = 13.2, 8.4 Hz, 1H), 2.10 (s, 3H), 1.58-1.69 (m, 1H).
(10) To a solution of compound **5-13** (150 mg, 423 µmol) in N-methylpyrrolidone (5.0 mL) was added potassium carbonate (292 mg, 2.11 mmol) and hydrochloride of compound **5-14** (126 mg, 107 µmol), and the reaction solution was stirred at 50°C for 12 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure to obtain compound **5-15.**
   MS-ESI [M+H]⁺, calculated: 443, found: 443.
(11) To a solution of compound **5-15** (187 mg, 422 µmol) in dichloromethane (10.0 mL) was added triethylamine (214 mg, 2.11 mmol) and di-tert-butyl carbonate (920 mg, 4.22 mmol), and the reaction solution was stirred at 25°C for 12 hours. Then the reaction solution was added with dichloromethane (20.0 mL), washed with saturated NaCl aqueous solution (20.0 mL × 2), dried over anhydrous sodium sulfate, and concentrated under reduced pressure. The crude product was separated by silica gel column chromatography (petroleum ether/ethyl acetate = 6:1) to obtain compound **5-16.**
   MS-ESI [M+H]⁺, calculated: 543, found: 543.
(12) To a solution of compound **5-16** (80.0 mg, 147 µmol) in dioxane (5.0 mL) was added compound **5-17** (50.9 mg, 442 µmol), potassium carbonate (61.0 mg, 441 µmol), tris (dibenzylideneacetone) dipalladium (27.0 mg, 29.5 µmol) and dicyclohexyl(2',4',6'-triisopropyl-[1,1'-biphenyl]-2-yl)phosphine (28.1 mg, 58.9 µmol), and the reaction solution was stirred at 110°C for 6 hours under nitrogen protection. Then the reaction solution was added with ethyl acetate (40.0 mL), washed with saturated NaCl aqueous solution (40.0 mL × 2), dried over anhydrous sodium sulfate, filtered, and concentrated under reduced pressure. The crude product was separated by preparative thin layer chromatography (dichloromethane/methanol = 1:0 to 15:1) to obtain compound**5-18.**
   MS-ESI [M+H]⁺, calculated: 622, found: 622.
   ¹H NMR (400 MHz, MeOD)*δ* 7.18-7.31 (m, 3H), 4.62-4.67 (m, 1H), 4.49 (br s, 1H), 4.39-4.45 (m, 1H), 3.89-4.23 (m, 4H), 3.67-3.77 (m, 2H), 3.43 (br d, *J =* 14.0 Hz, 2H), 3.24 (br dd, *J* = 14.0, 3.6 Hz, 2H), 2.88-3.03 (m, 5H), 2.73 (br s, 3H), 2.41-2.50 (m, 1H), 2.24-2.41 (m, 1H), 2.20-2.24 (m, 3H), 2.09-2.20 (m, 1H), 1.93-2.05 (m, 2H), 1.76-1.92 (m, 3H), 1.51 (d, *J* = 1.6 Hz, 9H).
(13) To a solution of compound **5-18** (70.0 mg, 113 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **5-19.**
   MS-ESI [M+H]⁺, calculated: 522, found: 522.
(14) To a solution of trifluoroacetate of compound **5-19**(60.0 mg, 94.3 µmol) in dichloromethane (2.0 mL) was added triethylamine (28.6 mg, 283 µmol) and compound **5-20** (17.1 mg, 189 µmol), and the reaction solution was stirred at -78°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **5.**
   MS-ESI [M+H]⁺, calculated: 576, found: 576.
   ¹H NMR (400 MHz, MeOD) *δ* 7.25-7.30 (m, 2H), 7.22 (d, *J* = 17.6 Hz, 1H), 6.80 (br s, 1H), 6.30 (br d, *J* = 16.4 Hz, 1H), 5.84 (br d, *J* = 10.8 Hz, 1H), 5.03 (br s, 1H), 4.69 (br dd, *J* = 12.4, 3.6 Hz, 1H), 4.50 (ddd, *J* = 12.4, 7.2, 1.6 Hz, 1H), 4.17-4.36 (m, 1H), 3.94-4.16 (m, 2H), 3.72-3.84 (m, 3H), 3.59-3.65 (m, 1H), 3.41-3.58 (m, 1H), 3.28 (br d, *J* = 1.6 Hz, 1H), 3.08-3.18 (m, 2H), 2.97-3.06 (m, 5H), 2.84-2.96 (m, 4H), 2.42-2.51 (m, 1H), 2.30-2.40 (m, 1H), 2.24 (d, *J* = 1.6 Hz, 3H), 2.11-2.19 (m, 1H), 2.07 (br dd, *J* = 14.4, 7.6 Hz, 1H), 1.96-2.03 (m, 1H), 1.87-1.95 (m, 1H).

### Example 6 Synthesis of Compounds 6 and 7

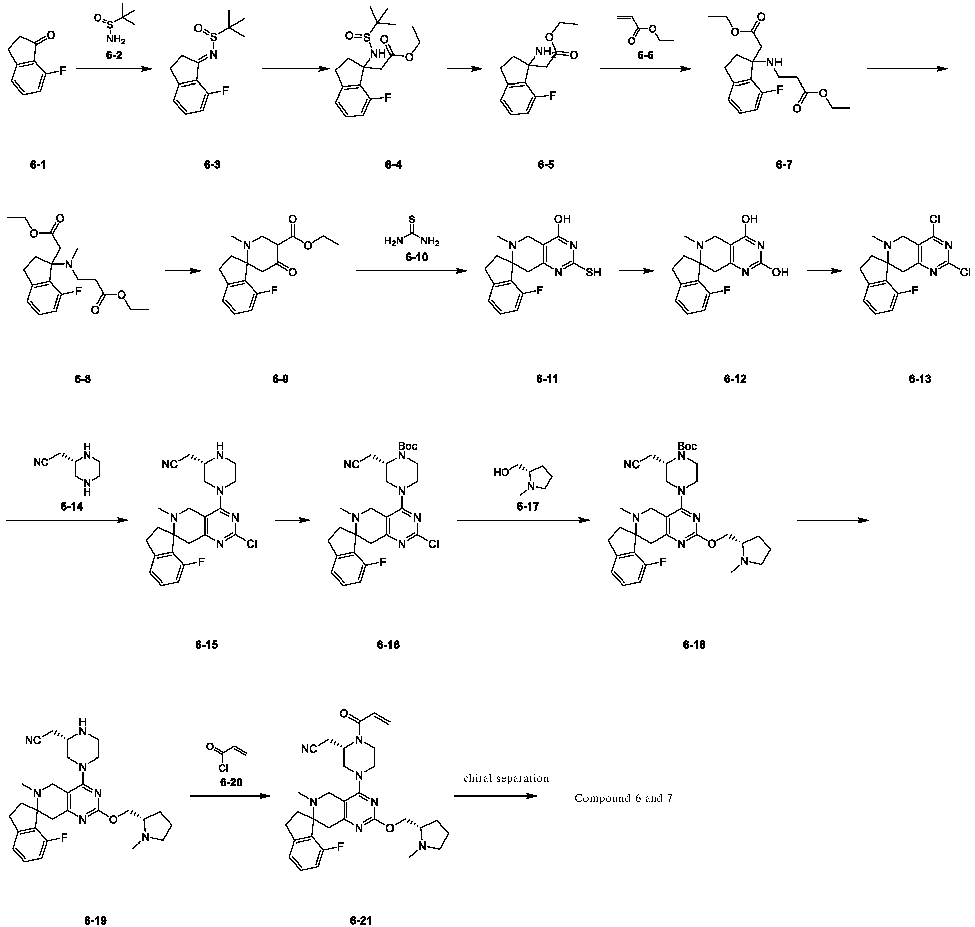

(1) To a solution of compound **6-1** (25.0 g, 166 mmol) in toluene (400 mL) was added compound **6-2** (18.3 g, 151 mmol), tetraethyl titanate (69.0 g, 302 mmol), and the reaction solution was stirred at 120°C for 16 hours under nitrogen protection. Then the reaction solution was added with water (400 mL), and extracted with ethyl acetate (400 mL × 2). The combined organic phase was washed with saturated NaCl aqueous solution (500 mL × 1), dried over anhydrous sodium sulfate, filtered, and concentrated under reduced pressure. The crude product was separated by silica gel column chromatography (petroleum ether/ethyl acetate (1:0 to 0:1) to obtain compound **6-3.**
   MS-ESI [M+H]⁺, calculated: 254, found: 254.
   ¹H NMR (400 MHz, CDCl₃) *δ* 7.42-7.48 (m, 1H), 7.16-7.20 (m, 1H), 6.93-7.00 (m, 1H), 3.46-3.56 (m, 1H), 3.05-3.21 (m, 3H), 1.30-1.36 (s, 9H).
(2) To a solution of ethyl acetate (22.4 g, 254 mmol) in tetrahydrofuran (200 mL) was added dropwise with lithium diisopropylamide (2 mol/L, 50.9 mL, tetrahydrofuran solution) at -78°C under nitrogen protection, and the reaction solution was stirred at -78°C for 1 hour. Then the reaction solution was added dropwise with a solution of compound **6-3** (12.9 g, 50.9 mmol) in tetrahydrofuran (50.0 mL), and continued stirring for 3 hours at -78°C. After warming to 0°C, the reaction solution was quenched with saturated ammonium chloride aqueous solution (200 mL), and extracted with ethyl acetate (200 mL × 1). The combined organic phase was washed with saturated ammonium chloride aqueous solution (200 mL × 1) and saturated NaCl aqueous solution (200 mL × 1), dried over anhydrous sodium sulfate, filtered, and concentrated under reduced pressure. The crude product was separated by silica gel column chromatography (petroleum ether/ethyl acetate = 1:0 to 4:1) to obtain compound **6-4.**
   MS-ESI [M+H]⁺, calculated: 342, found: 342.
   ¹H NMR (400 MHz, CDCl₃) *δ* 7.27-7.30 (m, 1H), 7.01-7.07 (m, 1H), 6.82-6.90 (m, 1H), 5.05-5.11 (s, 1H), 4.15-4.24 (m, 2H), 3.29-3.39 (m, 2H), 2.88-2.98 (m, 1H), 2.64-2.73 (m, 1H), 2.28-2.39 (m, 1H), 1.25-1.30 (t, 3H), 1.16-1.20 (s, 9H).
(3) To a solution of compound **6-4** (12.4 g, 36.3 mmol) in ethanol (120 mL) was added hydrochloric acid in dioxane(4 mol/L, 40.0 mL). Then the reaction solution was stirred at 25°C for 2 hours under nitrogen protection. Then the reaction solution was concentrated under reduced pressure to obtain crude product of hydrochloride of compound **6-5.**
   MS-ESI [M-NH₂+H]⁺, calculated: 221, found: 221.
(4) To a solution of hydrochloride of compound **6-5** (9.94 g, 36.3 mmol) in ethanol (80.0 mL) was added compound **6-6** (37.6 g, 376 mmol), copper oxide (577 mg, 7.26 mmol), and triethylamine (3.67 g, 36.3 mmol), and the reaction solution was reacted at 85°C in a sealed tube for 8 hours under nitrogen protection. Then the reaction solution was added with water (200 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 6:1) to obtain compound **6-7.**
   MS-ESI [M+H]⁺, calculated: 338, found: 338.
   ¹H NMR (400 MHz, CDCl₃) *δ* 7.16-7.22 (m, 1H), 6.96-7.01 (m, 1H), 6.80-6.87 (m, 1H), 4.06-4.17 (m, 4H), 2.95-3.07 (m, 2H), 2.86-2.94 (m, 1H), 2.65-2.81 (m, 3H), 2.26-2.51 (m, 5H), 1.22-1.26 (t, 3H), 1.14-1.20 (t, 3H).
(5) To a solution of compound **6-7** (3.0 g, 8.89 mmol) in ethanol (15.0 mL) was added paraformaldehyde (1.33 g) and sodium cyanoborohydride (1.68 g, 26.6 mmol), and the reaction solution was reacted for 16 hours at 25°C under nitrogen protection. Then the reaction solution was added with water (50.0 mL), and extracted with ethyl acetate (50 mL × 1). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 4:1) to obtain compound **6-8.**
   MS-ESI [M+H]⁺, calculated: 352, found: 352.
   ¹H NMR (400 MHz, CDCl₃) *δ* 7.16-7.22 (m, 1H), 6.93-6.99 (m, 1H), 6.79-6.86 (m, 1H), 4.07-4.14 (m, 2H), 3.88-3.99 (m, 2H), 2.95-3.08 (m, 3H), 2.83-2.92 (m, 1H), 2.72-2.80 (m, 1H), 2.60-2.68 (m, 1H), 2.29-2.46 (m, 4H), 2.19-2.23 (s, 3H), 1.22-1.27 (t, 3H), 1.00-1.06 (t, 3H).
(6) To a solution of compound **6- 8** (3.00 g, 8.54 mmol) in tetrahydrofuran (30.0 mL) was added potassium bis (trimethylsilyl) amide (1 mol/L, 25.6 mL, tetrahydrofuran solution) at -78°C, and the reaction solution was reacted at -78°C for 2 hours under nitrogen protection. Then the reaction solution was added with saturated ammonium chloride aqueous solution (50.0 mL), and extracted with ethyl acetate (50.0 mL × 1). The combined organic phase was washed with saturated NaCl aqueous solution (50.0 mL × 1), dried over anhydrous sodium sulfate, filtered, and concentrated under reduced pressure. The crude product was separated by silica gel column chromatography (petroleum ether/ethyl acetate = 1:0 to 6:1) to obtain compound **6-9.**
   MS-ESI [M+H]⁺, calculated: 306, found: 306.
   ¹H NMR (400 MHz, CDCl₃) *δ* 7.21-7.26 (m, 1H), 6.99-7.02 (m, 1H), 6.87-6.93 (m, 1H), 4.19-4.36 (m, 2H), 3.58-3.64 (m, 1H), 2.85-3.30 (m, 5H), 2.25-2.35 (m, 2H), 2.18-2.22 (m, 3H), 1.72-1.81 (m, 1H), 1.30-1.35 (m, 3H).
(7) To a solution of compound **6-9** (2.00 g, 6.55 mmol) in ethanol (20.0 mL) was added compound **6-10** (997 mg, 13.1 mmol) and sodium ethoxide (1.34 g, 19.6 mmol), and the reaction solution was stirred at 80°C for 12 hours under nitrogen protection. After concentrated under reduced pressure, the reaction solution was added with ethanol (50.0 mL) and its pH was adjusted to 6 with hydrochloric acid (1 mol/L). The resulting solution was precipitated, and filtered, and the filter cake was dried to obtain compound **6-11.**
   MS-ESI [M+H]⁺, calculated: 318, found: 318.
   ¹H NMR (400 MHz, DMSO-d6) *δ* 11.61-12.93 (s, 2H), 7.28-7.36 (m, 1H), 7.06-7.14 (m, 1H), 6.97-7.04 (m, 1H), 3.44-3.53 (m, 2H), 2.79-3.04 (m, 4H), 2.16-2.28 (m, 1H), 1.98-2.11 (s, 3H), 1.61-1.70 (m, 1H).
(8) To a solution of intermediate **6-11** (1.90 g, 5.99 mmol) in water (10.0 mL) was added chloroacetic acid (2.26 g, 23.9 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **6-12.**
   MS-ESI [M+H]⁺, calculated: 302, found: 302.
   ¹H NMR (400 MHz, DMSO-*d*6)*δ* 10.98-11.04 (s, 1H), 10.73-10.80 (s, 1H), 7.29-7.36 (m, 1H), 7.07-7.12 (m, 1H), 6.96-7.04 (m, 1H), 3.39-3.47 (m, 1H), 2.76-3.02 (m, 4H), 2.29-2.37 (m, 1H), 2.18-2.27 (m, 1H), 2.02-2.08 (s, 3H), 1.60-1.68 (m, 1H).
(9) Compound **6-12** (800 mg, 2.66 mmol) was dissolved in phosphorus oxychloride (13.2 g, 86.0 mmol), and the reaction solution was stirred at 80°C for 2 hours under nitrogen protection. Then the reaction solution was concentrated under reduced pressure, added with iced water (10.0 mL) and saturated sodium bicarbonate aqueous solution (50.0 mL), and extracted with dichloromethane (50.0 mL × 1). The organic phase was washed with saturated sodium bicarbonate aqueous solution (50.0 mL × 2) and saturated NaCl aqueous solution (50.0 mL × 1), dried over anhydrous sodium sulfate, filtered, and concentrated under reduced pressure to obtain compound **6-13.**
   MS-ESI [M+H]⁺, calculated: 338, found: 338.
(10) To a solution of compound **6-13** (500 mg, 1.48 mmol) in N-methylpyrrolidone (5.0 mL) was added potassium carbonate (1.02 g, 7.39 mmol) and hydrochloride of compound **6-14** (439 mg, 2.22mmol), and the reaction solution was stirred at 50°C for 12 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure to obtain compound **6-15.**
   MS-ESI [M+H]⁺, calculated: 427, found: 427.
   ¹H NMR (400 MHz, DMSO-*d*6)*δ* 7.26-7.37 (m, 1H), 7.07-7.16 (m, 1H), 6.95-7.05 (m, 1H), 3.36-3.96 (m, 6H), 3.22-3.31 (m, 1H), 2.65-3.11 (m, 8H), 2.23-2.39 (m, 1H), 2.09-2.19 (m, 3H), 1.55-1.83 (m, 1H), 1.19-1.39 (m, 1H).
(11) To a solution of compound **6-15** (1.40 g, 3.28 mmol) in dichloromethane (20.0 mL) was added triethylamine (2.14g, 21.1 mmol) and di-tert-butyl carbonate (3.58 mg, 16.4 mmol), and the reaction solution was stirred at 25 °C for 12 hours. Then the reaction solution was added with dichloromethane (20.0 mL), washed with saturated NaCl aqueous solution (20.0 mL × 2), dried over anhydrous sodium sulfate, and concentrated under reduced pressure. The crude product was separated by silica gel column chromatography (petroleum ether/ethyl acetate = 1:0 to 6:1) to obtain compound **6-16.**
   MS-ESI [M+H]⁺, calculated: 527, found: 527.
(12) To a solution of compound **6-16** (900 mg, 1.71 mmol) in dioxane (10.0 mL) was added compound **6-17** (590 mg, 5.12mmol), cesium carbonate (1.67 g, 5.12 mmol) and (2-dicyclohexylphosphino-2',6'-diisopropoxy-1,1'-biphenyl) [2-(2'-amino-1,1'- biphenyl)] palladium(II) methanesulfonate (285.6 mg, 341 µmol), and the reaction solution was stirred at 110°C for 6 hours under nitrogen protection. Then the reaction solution was added with ethyl acetate (40.0 mL), washed with saturated NaCl aqueous solution (40.0 mL × 2), dried over anhydrous sodium sulfate, filtered, and concentrated under reduced pressure. The crude product was separated by silica gel column chromatography (dichloromethane/methanol =1:0 to 15:1) to obtain compound **6-18.**
   MS-ESI [M+H]⁺, calculated: 606, found: 606.
   ¹H NMR (400 MHz, CDCl₃)*δ* 7.22-7.26 (m, 1H), 7.01-7.06 (m, 1H), 6.87-6.94 (m, 1H), 4.54-4.64 (m, 1H), 4.24-4.40 (m, 1H), 3.78-4.08 (m, 3H), 3.51-3.77 (m, 3H), 3.19-3.39 (m, 3H), 2.80-3.16 (m, 7H), 2.64-2.73 (m, 3H), 2.29-2.35 (s, 3H), 1.79-1.99 (m, 4H), 1.55-1.75 (m, 4H), 1.49-1.55 (s, 9H).
(13) To a solution of compound **6-18** (342 mg, 564 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **6-19.**
   MS-ESI [M+H]⁺, calculated: 506, found: 506.
(14) To a solution of trifluoroacetate of compound **6-19** (110 mg, 217 µmol) in dichloromethane (2.0 mL) was added triethylamine (22.0 mg, 217 µmol) and compound **6-20** (29.5 mg, 326 µmol), and the reaction solution was stirred at -78°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by reverse column chromatography (acetonitrile/ water = 0:1 to 1:19) to obtain compound **6-21.**
   MS-ESI [M+H]⁺, calculated: 560, found: 560.
(15) Compound **6-21** was separated by chiral supercritical fluid chromatography to obtain compounds **6** and **7.**

Separation conditions: Chromatographic column model: AD-H; Column specification: 0.46 cm I .D. × 15 cm L; Injection volume: 2 µL; Mobile phase: carbon dioxide: ethanol (0.1% triethylamine) = 70: 30; Detection wavelength: 254 nm; Column temperature: 25.

**Compound 6:** retention time: 4.071 minutes, 99.42% ee.

MS-ESI [M+H]⁺, calculated: 560, found: 560.

¹H NMR (400 MHz, DMSO-^6) *δ* 7.31 (td, *J* = 7.6, 5.2 Hz, 1H), 7.10 (d, *J* = 7.6 Hz, 1H), 7.03-6.97 (m, 1H), 6.95-6.78 (m, 1H), 6.19 (d, *J* = 16.8 Hz, 1H), 5.77 (d, *J* = 8.8 Hz, 1H), 5.00-4.72 (m, 1H), 4.24 (d, *J* = 6.8 Hz, 1H), 4.00 (dd, *J* = 10.8, 6.4 Hz, 2H), 3.93-3.80 (m, 2H), 3.74-3.58 (m, 2H), 3.53-3.45 (m, 1H), 3.29-3.12 (m, 2H), 3.07-2.96 (m, 4H), 2.96-2.89 (m, 2H), 2.84-2.74 (m, 2H), 2.40-2.28 (m, 4H), 2.20-2.08 (m, 4H), 1.91 (dq, *J* = 12.4, 8.4 Hz, 1H), 1.77-1.68 (m, 1H), 1.68-1.62 (m, 2H), 1.60-1.51 (m, 1H).

**Compound 7:** retention time: 4.436 minutes, 98.76% ee.

MS-ESI [M+H]⁺, calculated: 560, found: 560.

¹H NMR (400 MHz, DMSO-d6) *δ* 7.31 (dt, *J* = 12.8, 6.4 Hz, 1H), 7.11 (d, *J* = 7.2 Hz, 1H), 7.04-6.95 (m, 1H), 6.91-6.79 (m, 1H), 6.18 (d, *J* = 17.2 Hz, 1H), 5.78 (d, *J* = 8.8 Hz, 1H), 5.00-4.55 (m, 1H), 4.27-4.17 (m, 1H), 4.13-3.91 (m, 2H), 3.91-3.72 (m, 2H), 3.67-3.47 (m, 3H), 3.14-2.90 (m, 9H), 2.81 (d, *J =* 17.6 Hz, 1H), 2.39-2.24 (m, 4H), 2.16 (s, 3H), 2.09-1.99 (m, 1H), 1.96-1.85 (m, 1H), 1.83-1.70 (m, 1H), 1.69-1.60 (m, 2H), 1.60-1.49 (m, 1H).

### Example 7 Synthesis of Compounds 8 and 9

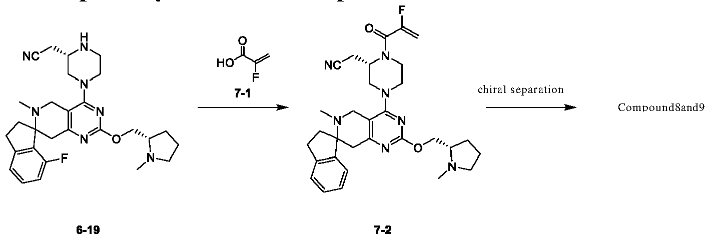

(1) To a solution of compound **6-19** (160 mg, 316 µmol) in dichloromethane (2.0 mL) was added triethylamine (32.0 mg, 316 µmol) and propanephosphonic acid anhydride (503 mg, 791 µmol, 470 µL, 50% ethyl acetate solution) and hydrochloride of compound **7-1** (600 mg, 4.75 mmol), and the reaction solution was stirred at 25°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure to obtain crude compound **7-2.**
   MS-ESI [M+H]⁺, calculated: 578, found: 578.
(2) Compound **7-2** was separated by chiral supercritical fluid chromatography to obtain compounds **8** and **9.**

Separation conditions: Chromatographic column model: AD-H; Column specification: 0.46 cm I .D. × 15 cm L; Injection volume: 2 µL; Mobile phase: carbon dioxide: ethanol (0.1% triethylamine) = 70 : 30; Detection wavelength: 254 nm; Column temperature: 25°C.

**Compound 8:** retention time: 3.689 minutes, 98.44% ee.

MS-ESI [M+H]⁺, calculated: 578, found: 578.

¹H NMR (400 MHz, DMSO-d6) *δ* 7.35-7.27 (m, 1H), 7.10 (d, *J* = 7.6 Hz, 1H), 7.02-6.95 (m, 1H), 5.43-5.35 (m, 1H), 5.35-5.20 (m, 1H), 4.30-4.22 (m, 1H), 4.06 (s, 1H), 3.97-3.88 (m, 1H), 3.86-3.83 (m, 1H), 3.71 (d, *J* = 14.4 Hz, 2H), 3.20 (dd, *J* = 13.6, 3.6 Hz, 1H), 3.05-2.89 (m, 6H), 2.84-2.75 (m, 1H), 2.44-2.32 (m, 4H), 2.26 (s, 1H), 2.13 (s, 3H), 1.98-1.89 (m, 1H), 1.77-1.65 (m, 3H), 1.65---1.52 (m, 1H), 1.52 - 1.41 (m, 2H), 1.20 (s, 1H), 0.91 (t, *J* = 6.8 Hz, 2H).

**Compound 9:** retention time: 4.059 minutes, 97.90% ee.

MS-ESI [M+H]⁺, calculated: 578, found: 578.

¹H NMR (400 MHz, DMSO-d6) *δ* 7.31 (d, *J* = 5.2 Hz, 1H), 7.11 (d, *J* = 8.0 Hz, 1H), 7.05-6.92 (m, 1H), 5.39 (d, *J* = 13.6 Hz, 1H), 5.35-5.17 (m, 1H), 4.22 (d, *J* = 10.4 Hz, 1H), 4.09-3.98 (m, 1H), 3.90 (d, *J* = 13.6 Hz, 1H), 3.85-3.74 (m, 1H), 3.53 (d, *J* = 7.3 Hz, 2H), 3.14-3.06 (m, 2H), 3.06-2.92 (m, 5H), 2.85-2.78 (m, 1H), 2.39-2.28 (m, 4H), 2.22-2.09 (m, 4H), 1.91 (s, 2H), 1.82-1.71 (m, 1H), 1.70-1.62 (m, 2H), 1.58-1.51 (m, 1H), 1.22-1.11 (m, 1H), 1.08-0.77 (m, 2H).

### Example 8 Synthesis of Compound 10

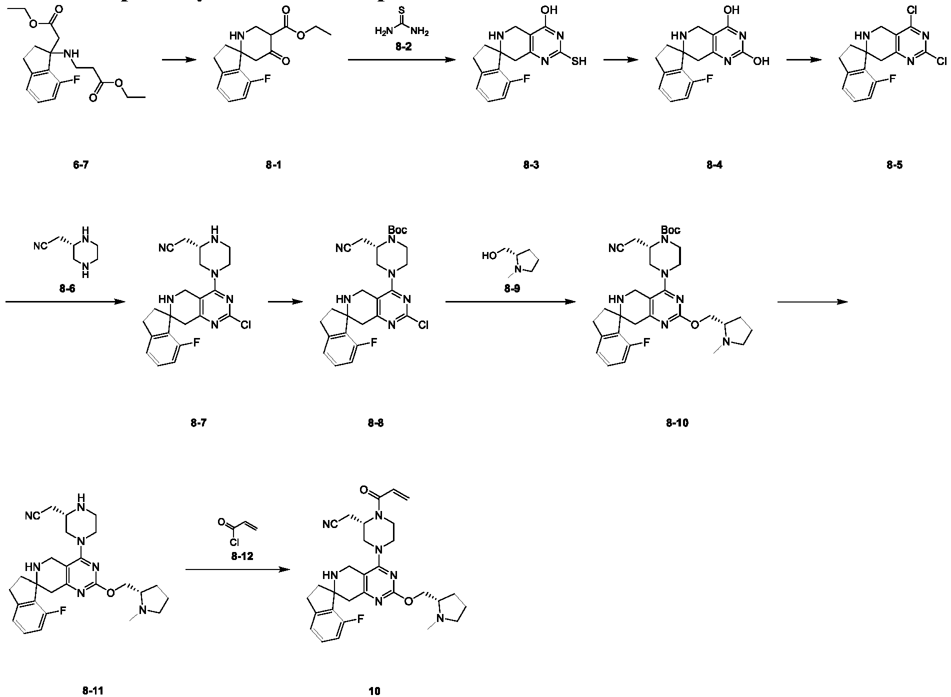

(1) To a solution of compound **6-7** (1.00 g, 2.96 mmol) in tetrahydrofuran (10.0 mL) was added potassium bis (trimethylsilyl) amide (1 mol/L, 9.0 mL, tetrahydrofuran solution) at -78°C, and the reaction solution was reacted at -78°C for 2 hours under nitrogen protection. Then the reaction solution was added with saturated ammonium chloride aqueous solution (50.0 mL), and extracted with ethyl acetate (50.0 mL × 1). The combined organic phase was washed with saturated NaCl aqueous solution (50.0 mL × 1), dried over anhydrous sodium sulfate, filtered, and concentrated under reduced pressure. The crude product was separated by silica gel column chromatography (petroleum ether/ethyl acetate = 1:0 to 6:1) to obtain compound **8-1.**
   MS-ESI [M+H]⁺, calculated: 292, found: 292.
   ¹H NMR (400 MHz, CDCl₃) *δ* 7.18-7.25 (m, 1H), 7.00-7.05 (m, 1H), 6.89 (t, *J =* 9.2 Hz, 1H), 4.22-4.29 (m, 2H), 3.35-3.57 (m, 1H), 3.14-3.27 (m, 1H), 3.01-3.14 (m, 1H), 2.85-2.99 (m, 2H), 2.42-2.69 (m, 2H), 2.34 (br d, *J* = 6.0 Hz, 1H), 2.08-2.20 (m, 2H), 1.30-1.34 (m, 3H).
(2) To a solution of compound **8-1** (970 mg, 3.33 mmol) in ethanol (10.0 mL) was added compound **8-2**(507 mg, 6.66 mmol) and sodium ethoxide (679 mg, 9.98 mmol), and the reaction solution was stirred at 80°C for 16 hours under nitrogen protection. After concentrated under reduced pressure, the reaction solution was adjusted to pH 6 with hydrochloric acid (1 mol/L). The resulting solution was precipitated, and filtered, and the filter cake was dried to obtain compound **8-3.**
   MS-ESI [M+H]⁺, calculated: 304, found: 304.
   ¹H NMR (400 MHz, DMSO-d6) *δ* 12.39 (br s, 1H), 12.25 (br s, 1H), 7.29 (td, *J* = 7.6, 5.2 Hz, 1H), 7.10 (d, *J* = 7.6 Hz, 1H), 6.93-7.01 (m, 1H), 3.50 (br s, 1H), 3.39-3.40 (m, 1H), 3.00-3.08 (m, 1H), 2.81-2.90 (m, 1H), 2.66-2.74 (m, 1H), 2.56 (s, 1H), 1.97-2.16 (m, 2H).
(3) To a solution of intermediate **8-3** (470 mg, 1.55 mmol) in water (10.0 mL) was added chloroacetic acid (560 mg, 5.93 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **8-4.**
   MS-ESI [M+H]⁺, calculated: 288, found: 288.
   ¹H NMR (400 MHz, DMSO-*d*6)*δ* 11.03 (s, 1H), 10.78 (s, 1H), 7.27-7.32 (m, 2H), 7.21 (d, *J* = 1.6 Hz, 1H), 3.43 (br d, *J* = 16.0 Hz, 1H), 3.01 (br d, *J* = 16.0 Hz, 1H), 2.83-2.93 (m, 2H), 2.62 (br d, *J* = 17.6 Hz, 1H), 2.32 (d, *J* = 17.2 Hz, 1H), 2.19 (dt, *J* = 13.2, 8.4 Hz, 1H), 1.97 (s, 3H), 1.65 (ddd, *J* = 13.2, 8.4, 4.4 Hz, 1H).
(4) Compound **8-4** (200 mg, 696 µmol) was dissolved in phosphorus oxychloride (8.25g, 53.8 mmol), and the reaction solution was added with triethylamine (141 mg, 1.39 mmol) and then stirred at 85°C for16 hours under nitrogen protection. Then the reaction solution was added with iced water (10.0 mL) and saturated sodium bicarbonate aqueous solution (50.0 mL), and extracted with dichloromethane (50.0 mL × 1). The organic phase was washed with saturated sodium bicarbonate aqueous solution (50.0 mL × 2) and saturated NaCl aqueous solution (50.0 mL × 1), dried over anhydrous sodium sulfate, filtered, and concentrated under reduced pressure to give compound **8-5.**
   MS-ESI [M+H]⁺, calculated: 324, found: 324.
   ¹H NMR (400 MHz, DMSO-d6) *δ* 7.29 (td, *J* = 7.6, 5.2 Hz, 1H), 7.13 (d, *J* = 7.6 Hz, 1H), 6.89-7.01 (m, 1H), 3.83-3.96 (m, 1H), 3.66-3.77 (m, 1H), 3.18 (s, 1H), 3.04-3.15 (m, 3H), 2.84-2.96 (m, 1H), 2.03-2.15 (m, 2H).
(5) To a solution of compound **8-5** (200 mg, 617 µmol) in N-methylpyrrolidone (5.0 mL) was added potassium carbonate (426 mg, 3.08 mmol) and hydrochloride of compound **8-6**(183 mg, 924 µmol ), and the reaction solution was stirred at 50°C for 12 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure to obtain compound **8-7.**
   MS-ESI [M+H]⁺, calculated: 413, found: 413.
(6) To a solution of compound **8-7** (250 mg, 605µmol ) in dichloromethane (10.0 mL) was added triethylamine (307 mg, 3.03 mmol) and di-tert-butyl carbonate (264 mg, 1.21 mmol), and the reaction solution was stirred at 25 °C for 12 hours. Then the reaction solution was added with dichloromethane (20.0 mL), washed with saturated NaCl aqueous solution (20.0 mL × 2), dried over anhydrous sodium sulfate, and concentrated under reduced pressure. The crude product was separated by silica gel column chromatography (petroleum ether/ethyl acetate = 1:0 to 1:1) to obtain compound **8-8.**
   MS-ESI [M+H]⁺, calculated: 513, found: 513.
   ¹H NMR (400 MHz, CDCl₃) *δ* 7.28-7.43 (m, 1H), 7.03-7.17 (m, 1H), 6.87-6.94 (m, 1H), 4.31-4.95 (m, 2H), 4.18-4.26 (m, 1H), 3.93-4.00 (m, 1H), 3.64-3.91 (m, 2H), 3.32-3.45 (m, 3H), 3.07-3.30 (m, 3H), 2.89-3.07 (m, 2H), 2.66-2.85 (m, 3H), 2.21-2.38 (m, 1H), 1.50-1.52 (m, 9H).
(7) To a solution of compound **8-8** (205 mg, 400 µmol ) in dioxane (5.0 mL) was added compound **8-9** (138 mg, 1.20 mmol), cesium carbonate (391 mg, 1.20 mmol) and (2-dicyclohexylphosphino-2',6'-diisopropoxy-1,1'-biphenyl) [2-(2'-amino-1,1'- biphenyl)] palladium(II) methanesulfonate (66.9 mg, 80.0 µmol), and the reaction solution was stirred at 110°C for 6 hours under nitrogen protection. Then the reaction solution was added with ethyl acetate (40.0 mL), washed with saturated NaCl aqueous solution (40.0 mL × 2), dried over anhydrous sodium sulfate, filtered, and concentrated under reduced pressure. The crude product was separated by preparative thin layer chromatography (dichloromethane/methanol = 1:0 to 10:1) to obtain compound **8-10.**
   MS-ESI [M+H]⁺, calculated: 592, found: 592.
   ¹H NMR (400 MHz, MeOD)*δ* 7.25-7.32 (m, 1H), 7.08-7.17 (m, 1H), 6.88-6.98 (m, 1H), 4.62-4.67 (m, 1H), 4.41 (br s, 2H), 3.93-4.08 (m, 3H), 3.77-3.91 (m, 2H), 3.59-3.75 (m, 1H), 3.09-3.17 (m, 2H), 2.97-3.08 (m, 4H), 2.94 (s, 1H), 2.75-2.90 (m, 2H), 2.64 (br s, 3H), 2.13-2.31 (m, 4H), 1.90 (br s, 2H), 1.80 (br s, 1H), 1.58 (br s, 1H), 1.51 (s, 9H).
(8) To a solution of compound **8-10** (100 mg, 169 µmol) in dichloromethane (3.0 mL) was added trifluoroacetic acid (1.0 mL), and the reaction solution was stirred at 25°C for 0.5 hours under nitrogen protection. Then the reaction solution was concentrated under reduced pressure to obtain crude trifluoroacetate of compound **8-11.**
   MS-ESI [M+H]⁺, calculated: 492, found: 492.
(9) To a solution of trifluoroacetate of compound **8-11** (40.0 mg, 66.1µmol) in dichloromethane (2.0 mL) was added triethylamine (20.1 mg, 199 µmol) and compound **8-12** (6.6 mg, 72.9 µmol), and the reaction solution was stirred at -78°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **10.**
   MS-ESI [M+H]⁺, calculated: 546, found: 546.
   ¹H NMR (400 MHz, MeOD) *δ* 7.32 (tdd, *J* = 8.0, 5.2, 3.2 Hz, 1H), 7.15 (dd, *J* = 7.2, 3.2 Hz, 1H), 6.89-6.98 (m, 1H), 6.72-6.89 (m, 1H), 6.29 (br d, *J* = 16.4 Hz, 1H), 5.83 (br d, *J =* 10.4 Hz, 1H), 5.04 (br s, 1H), 4.64-4.84 (m, 2H), 4.51-4.57 (m, 1H), 4.07-4.27 (m, 2H), 3.90-4.05 (m, 2H), 3.77-3.90 (m, 2H), 3.52-3.74 (m, 2H), 3.10-3.27 (m, 5H), 3.04 (d, *J =* 4.4 Hz, 3H), 2.92-3.03 (m, 3H), 2.27-2.43 (m, 3H), 2.18 (br dd, *J* = 12.8, 6.8 Hz, 1H), 2.10 (br dd, *J* = 14.4, 7.2 Hz, 1H), 2.01-2.07 (m, 1H).

### Example 9 Synthesis of Compound 11

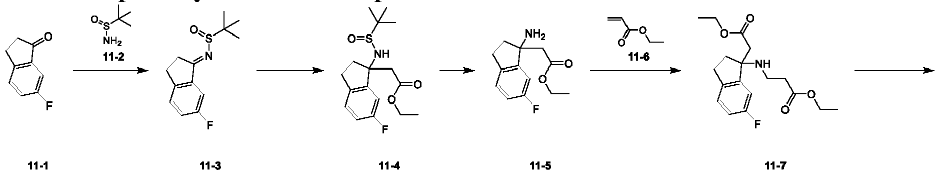

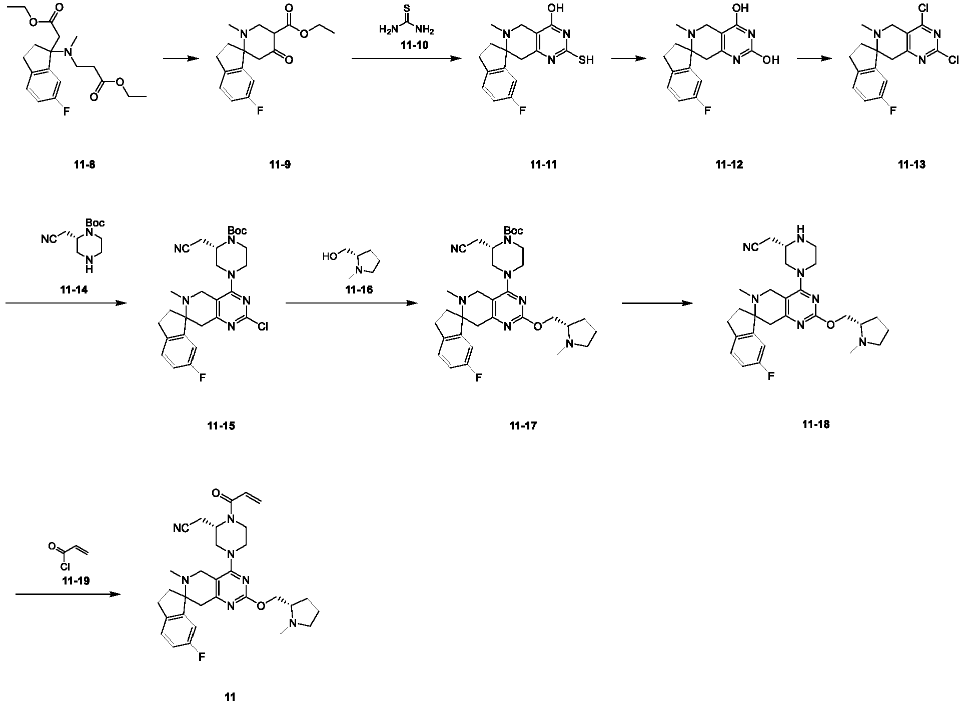

(1) To a solution of compound **11-1** (30.0 g, 200 mmol) in toluene (400 mL) was added compound **11-2** (29.1 g, 240 mmol), tetraethyl titanate (91.4 g, 401 mmol), and the reaction solution was stirred at 120°C for 16 hours under nitrogen protection. Then the reaction solution was added with water (200 mL), and extracted with ethyl acetate (200 mL × 5). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 10:1) to obtain compound **11-3.**
   MS-ESI [M+H]⁺, calculated: 254, found: 254.
   ¹H NMR (400 MHz, CDCl₃) *δ* 7.41 (dd, *J* = 8.0, 2.4 Hz, 1H), 7.35 (dd, *J* = 8.4, 4.8 Hz, 1H), 7.20 (td, *J* = 8.4, 2.4 Hz, 1H), 3.45-3.55 (m, 1H), 3.11-3.17 (m, 1H), 3.07-3.11 (m, 2H), 1.32 (s, 9H).
(2) To a solution of ethyl acetate (32.9 g, 260 mmol) in tetrahydrofuran (200 mL) was added dropwise with lithium diisopropylamide (2 mol/L, 56.0 mL, tetrahydrofuran solution) at -78°C under nitrogen protection, and the reaction solution stirred at -78°C for 1 hour. Then the reaction solution was added dropwise with a solution of compound **11-3** (18.9 g, 74.6 mmol) in tetrahydrofuran (100 mL), and then continued stirring for 3 hours at -65°C. After warming to 0°C, the reaction solution was quenched with saturated ammonium chloride aqueous solution (500 mL), and extracted with ethyl acetate (500 mL × 2). The combined organic phase was washed with saturated ammonium chloride aqueous solution (500 mL × 1) and saturated NaCl aqueous solution (500 mL × 1), dried over anhydrous sodium sulfate, filtered, and concentrated under reduced pressure. The crude product was separated by silica gel column chromatography (petroleum ether/ethyl acetate = 1:0 to 1:1) to obtain compound **11-4.**
   MS-ESI [M+H]⁺, calculated: 342, found: 342.
   ¹H NMR (400 MHz, CDCl₃) *δ* 7.18 (dd, *J* = 8.4, 5.2 Hz, 1H), 6.92-7.00 (m, 1H), 6.88 (dd, *J* = 8.8, 2.4 Hz, 1H), 5.24 (s, 1H), 4.14-4.22 (m, 2H), 3.06-3.17 (m, 1H), 2.82-2.89 (m, 1H), 2.75-2.81 (m, 2H), 2.68-2.74 (m, 1H), 2.34 (ddd, *J =* 13.2, 8.4, 5.2 Hz, 1H), 1.26 (t, *J* = 7.2 Hz, 3H), 1.20 (s, 9H).
(3) To a solution of compound **11-4** (6.90 g, 19.6 mmol) in ethanol (60.0 mL) was added hydrochloric acid in dioxane(4 mol/L, 20.0 mL), and the reaction solution was stirred at 25°C for 1 hour under nitrogen protection. Then the reaction solution was concentrated under reduced pressure to obtain crude product of hydrochloride of compound **11-5.**
   MS-ESI [M-NH₂+H]⁺, calculated: 221, found: 221.
(4) To a solution of hydrochloride of compound **11-5** (5.37 g, 19.6 mmol) in ethanol (30.0 mL) was added compound **11-6** (19.9 g, 198 mmol), copper oxide (312 mg, 3.92 mmol), triethylamine (5.96 g, 58.9 mmol), and the reaction solution was reacted at 85°C in a sealed tube for 8 hours under nitrogen protection. Then the reaction solution was added with water (200 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 4:1) to obtain compound **11-7.**
   MS-ESI [M+H]⁺, calculated: 338, found: 338.
   ¹H NMR (400 MHz, CDCl₃) *δ* 7.12 (dd, *J* = 8.0, 5.2 Hz, 1H), 6.85-6.96 (m, 2H), 4.06-4.15 (m, 4H), 2.81-2.94 (m, 2H), 2.70-2.80 (m, 2H), 2.58-2.70 (m, 2H), 2.49-2.55 (m, 1H), 2.41-2.45 (m, 2H), 2.34 (dt, *J* = 13.6, 8.4 Hz, 1H), 2.17 (ddd, *J* = 13.2, 8.4, 4.4 Hz, 1H), 1.25 (t, *J* = 7.2 Hz, 3H), 1.20 (t, *J* = 7.2 Hz, 3H).
(5) To a solution of compound **11-7** (5.93 g, 17.6 mmol) in ethanol (80.0 mL) was added paraformaldehyde (5.28 g) and sodium cyanoborohydride (3.31 g, 52.7 mmol), and the reaction solution was reacted for 16 hours at 25°C under nitrogen protection. Then the reaction solution was added with water(100 mL), and extracted with ethyl acetate (100 mL × 1). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 9:1) to obtain compound **11-8.**
   MS-ESI [M+H]⁺, calculated: 352, found: 352.
   ¹H NMR (400 MHz, CDCl₃) *δ* 7.09 (dd, *J* = 8.0, 5.2 Hz, 1H), 6.87-6.95 (m, 2H), 4.11 (qd, *J* = 7.2, 2.0 Hz, 2H), 3.91 (qd, *J* = 7.2, 1.2 Hz, 2H), 2.82-2.88 (m, 3H), 2.72-2.76 (m, 1H), 2.62 (td, *J* = 7.2, 3.2 Hz, 2H), 2.38-2.46 (m, 2H), 2.32-2.37 (m, 1H), 2.21-2.28 (m, 1H), 2.18 (s, 3H), 1.25 (t, *J* = 7.2 Hz, 3H), 1.03 (t, *J* = 7.2 Hz, 3H).
(6) To a solution of compound **11- 8** (2.00 g, 5.69 mmol) in tetrahydrofuran (20.0 mL) was added potassium bis (trimethylsilyl) amide (1 mol/L, 17.1 mL, tetrahydrofuran solution) at -78°C, and the reaction solution was reacted at -65°C for 2 hours under nitrogen protection. Then the reaction solution was added with saturated ammonium chloride aqueous solution (100 mL), and extracted with ethyl acetate (100 mL × 1). The combined organic phase was washed with saturated NaCl aqueous solution (100 mL × 1), dried over anhydrous sodium sulfate, filtered, and concentrated under reduced pressure. The crude product was separated by silica gel column chromatography (petroleum ether/ethyl acetate = 1:0 to 10:1) to obtain compound **11-9.**
   MS-ESI [M+H]⁺, calculated: 306, found: 306.
   ¹H NMR (400 MHz, CDCl₃) *δ* 7.10-7.18 (m, 1H), 6.87-7.03 (m, 2H), 4.21-4.32 (m, 2H), 3.49 (d, *J* = 13.2 Hz, 1H), 2.92-3.37 (m, 2H), 2.76-2.92 (m, 2H), 2.49-2.67 (m, 1H), 2.33-2.44 (m, 1H), 2.19-2.31 (m, 1H), 2.06-2.13 (m, 3H), 1.76-1.86 (m, 1H), 1.32 (t, *J* = 7.2 Hz, 3H).
(7) To a solution of compound **11-9** (1.33 g, 4.36 mmol) in ethanol (15.0 mL) was added compound **11-10**(663 mg, 8.71 mmol) and sodium ethoxide (889 mg, 13.1 mmol), and the reaction solution was stirred at 80°C for 12 hours under nitrogen protection. After concentrated under reduced pressure, the reaction solution was adjusted to pH 6 with hydrochloric acid (1 mol/L). The resulting solution was precipitated, and filtered, and the filter cake was dried to obtain compound **11-11.**
   MS-ESI [M+H]⁺, calculated: 318, found: 318.
   ¹H NMR (400 MHz, DMSO-d6) *δ* 7.27 (dd, *J* = 8.4, 5.2 Hz, 1H), 7.07 (td, *J* = 8.8, 2.4 Hz, 1H), 6.98 (d, *J* = 9.2 Hz, 1H), 3.47 (d, *J* = 16.4 Hz, 1H), 3.03 (d, *J* = 16.4 Hz, 1H), 2.81-2.92 (m, 2H), 2.62 (d, *J* = 17.6 Hz, 1H), 2.41 (d, *J* = 17.6 Hz, 1H), 2.19 (dt, *J* = 13.6, 8.4 Hz, 1H), 1.98 (s, 3H), 1.66 (ddd, *J* = 13.2, 8.4, 4.4 Hz, 1H).
(8) To a solution of compound **11-11** (1.36g, 1.08 mmol) in water (15.0 mL) was added chloroacetic acid (1.77g, 18.7 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **11-12.**
   MS-ESI [M+H]⁺, calculated: 302, found: 302.
   ¹H NMR (400 MHz, DMSO-*d*6)*δ* 11.01 (s, 1H), 10.76 (s, 1H), 7.27 (dd, *J =* 8.4, 5.2 Hz, 1H), 7.08 (td, *J* = 8.8, 2.4 Hz, 1H), 6.99 (dd, *J* = 9.2, 2.4 Hz, 1H), 3.44 (d, *J* = 16.0 Hz, 1H), 3.01 (d, *J* = 16.0 Hz, 1H), 2.80-2.91 (m, 2H), 2.61 (d, *J* = 17.6 Hz, 1H), 2.33 (d, *J* = 17.2 Hz, 1H), 2.21 (dt, *J* = 13.2, 8.4 Hz, 1H), 1.98 (s, 3H), 1.67 (ddd, *J* = 13.2, 8.4, 4.4 Hz, 1H).
(9) Compound **11-12** (1.00 g, 3.32 mmol) was dissolved in phosphorus oxychloride (16.5 g, 108 mmol), and the reaction solution was stirred at 80°C for 2 hours under nitrogen protection. Then the reaction solution was concentrated under reduced pressure, added with iced water (100 mL), and extracted with dichloromethane (100 mL × 1). The organic phase was washed with saturated sodium bicarbonate aqueous solution (100 mL × 2) and saturated NaCl aqueous solution (100 mL × 1), dried over anhydrous sodium sulfate, filtered, and concentrated under reduced pressure. The crude product was separated by silica gel column chromatography (petroleum ether/ethyl acetate = 1:0 to 10:1) to obtain compound **11-13.**
   MS-ESI [M+H]⁺, calculated: 338, found: 338.
   ¹H NMR (400 MHz, DMSO-*d*₆) *δ* 7.29 (dd, *J* = 8.4, 5.2 Hz, 1H), 7.09 (td, *J* = 8.8, 2.4 Hz, 1H), 6.98 (dd, *J* = 9.2, 2.4 Hz, 1H), 3.93 (d, *J* = 17.6 Hz, 1H), 3.51 (d, *J* = 17.6 Hz, 1H), 3.12 (d, *J =* 18.0 Hz, 1H), 2.87-2.97 (m, 3H), 2.25 (dt, *J* = 13.2, 8.4 Hz, 1H), 2.11 (s, 3H), 1.66 (dt, *J* = 13.2, 6.4 Hz, 1H).
(10) To a solution of compound **11-13** (200 mg, 591 µmol) in N-methylpyrrolidone (5.0 mL) was added potassium carbonate (245 mg, 1.77 mmol) and compound **11-14** (200 mg, 888 µmol), and the reaction solution was stirred at 50°C for 10 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure. The crude product was separated by silica gel column chromatography (petroleum ether/ethyl acetate = 1:0 to 3:1) to give compound **11-15.**
   MS-ESI [M+H]⁺, calculated: 527, found: 527.
   ¹H NMR (400 MHz, MeOD) *δ* 7.21-7.30 (m, 1H), 7.01 (td, *J* = 8.8, 2.4 Hz, 1H), 6.89-6.98 (m, 1H), 4.63 (s, 1H), 4.09-4.28 (m, 1H), 3.92-4.05 (m, 2H), 3.68-3.85 (m, 2H), 3.45 (dd, *J* = 14.0, 3.6 Hz, 1H), 3.25 (dd, *J* = 13.6, 3.6 Hz, 1H), 3.06-3.20 (m, 1H), 2.80-3.03 (m, 6H), 2.39-2.50 (m, 1H), 2.23 (d, *J* = 1.6 Hz, 3H), 1.89 (ddt, *J* = 13.2, 7.6, 4.0 Hz, 1H), 1.51 (d, *J* = 1.2 Hz, 9H).
(11) To a solution of compound **11-15** (220 mg, 417 µmol ) in dioxane (8.0 mL) was added compound **11-16** (144 mg, 1.20 mmol), cesium carbonate (409 mg, 1.20 mmol) and (2-dicyclohexylphosphino-2',6'-diisopropoxy-1,1'-biphenyl) [2-(2'-amino-1,1'- biphenyl)] palladium(II) methanesulfonate (70.0 mg, 83.7 µmol), and the reaction solution was stirred at 110°C for 4 hours under nitrogen protection. Then the reaction solution was added with ethyl acetate (50.0 mL), washed with saturated NaCl aqueous solution (50.0 mL × 2), dried over anhydrous sodium sulfate, filtered, and concentrated under reduced pressure. The crude product was separated by silica gel column chromatography (dichloromethane/methanol = 1:0 to 9:1) to obtain compound **11-17.**
   MS-ESI [M+H]⁺, calculated: 606, found: 606.
   ¹H NMR (400 MHz, MeOD)*δ* 7.26 (t, *J* = 5.6 Hz, 1H), 6.98-7.08 (m, 1H), 6.86-6.98 (m, 1H), 4.63 (d, *J* = 7.6 Hz, 1H), 4.26-4.46 (m, 2H), 3.92-4.17 (m, 3H), 3.65-3.82 (m, 2H), 3.42 (dd, *J* = 13.6, 3.6 Hz, 1H), 3.14-3.29 (m, 2H), 3.07-3.13 (m, 1H), 2.95-3.06 (m, 3H), 2.88-2.94 (m, 2H), 2.77-2.88 (m, 2H), 2.53 (d, *J* = 4.0 Hz, 3H), 2.34-2.49 (m, 2H), 2.23 (d, *J* = 2.0 Hz, 3H), 2.04-2.16 (m, 1H), 1.78-1.94 (m, 3H), 1.67-1.78 (m, 1H), 1.51 (d, *J* = 1.6 Hz, 9H).
(12) To a solution of compound **11-17** (70.0 mg, 281 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **11-18.**
   MS-ESI [M+H]⁺, calculated: 506, found: 506.
(13) To a solution of trifluoroacetate of compound **11-18** (160.0 mg, 258 µmol) in dichloromethane (5.0 mL) was added triethylamine (78.1 mg, 775 µmol) and compound **11-19** (46.7 mg, 516 µmol), and the reaction solution was stirred at -65°C for 0.5 hours under nitrogen protection. Then the reaction solution was added with dichloromethane (30.0 mL), washed with saturated NaCl aqueous solution (30.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **11.**
   MS-ESI [M+H]⁺, calculated: 560, found: 560.
   ¹H NMR (400 MHz, MeOD) *δ* 7.28 (t, *J =* 6.0 Hz, 1H), 6.93-7.11 (m, 2H), 6.81 (s, 1H), 6.29 (d, *J* = 16.4 Hz, 1H), 5.84 (d, *J* = 10.4 Hz, 1H), 4.90-5.14 (m, 1H), 4.68-4.75 (m, 1H), 4.55 (dd, *J* = 12.4, 7.2 Hz, 1H), 4.17-4.38 (m, 1H), 3.89-4.16 (m, 2H), 3.80 (d, *J* = 15.6 Hz, 3H), 3.65-3.72 (m, 1H), 3.54 (s, 1H), 3.04-3.30 (m, 4H), 3.02 (d, *J =* 5.2 Hz, 3H), 2.89-3.01 (m, 5H), 2.44-2.54 (m, 1H), 2.32-2.41 (m, 1H), 2.26 (s, 3H), 2.13-2.20 (m, 1H), 2.09 (dd, *J* = 14.4, 7.6 Hz, 1H), 1.97-2.05 (m, 1H), 1.88-1.96 (m, 1H).

### Example 10 Synthesis of Compound 12

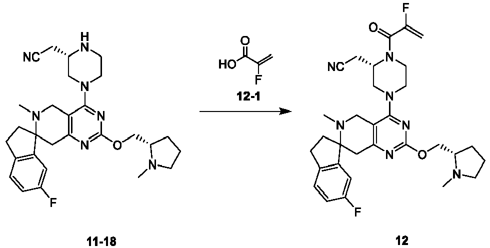

To a solution of compound **12-1** (65.4 mg, 726 µmol) in dichloromethane (5.0 mL) was added triethylamine (73.5 mg, 726 µmol), trifluoroacetate of compound **11-18**(150 mg, 242 µmol) and propanephosphonic acid anhydride (462 mg, 726 µmol, 50% ethyl acetate solution), and the reaction solution was stirred at 25°C for 1 hour. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 70mm × 30 mm 3 µm, A: water (0.225% formic acid); B: acetonitrile, 0%-30%: 8 min) to obtain formate of compound **12.**

MS-ESI [M+H]⁺, calculated: 578, found: 578.

¹H NMR (400 MHz, MeOD) *δ* 7.23-7.31 (m, 1H), 7.02 (td, *J* = 8.8, 2.0 Hz, 1H), 6.95 (ddd, *J* = 11.2, 9.2, 2.4 Hz, 1H), 5.21-5.43 (m, 2H), 4.58 (dt, *J* = 12.0, 4.4 Hz, 1H), 4.43-4.50 (m, 1H), 3.97-4.35 (m, 3H), 3.71-3.82 (m, 2H), 3.39-3.60 (m, 3H), 3.32 (s, 1H), 3.26-3.30 (m, 1H), 3.03-3.23 (m, 2H), 2.88-3.03 (m, 6H), 2.85 (d, *J* = 7.6 Hz, 3H), 2.43-2.53 (m, 1H), 2.25-2.33 (m, 1H), 2.24 (d, *J* = 2.0 Hz, 3H), 1.96-2.10 (m, 2H), 1.85-1.95 (m, 2H).

### Example 11 Synthesis of Compound 13

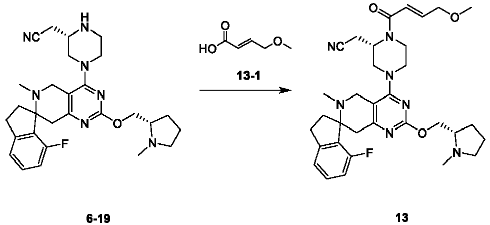

To a solution of compound **13-1** (45.3 mg, 435 µmol) in dichloromethane (2.0 mL) was added triethylamine (14.7 mg, 145 µmol), trifluoroacetate of compound **6-19** (90.0 mg, 145 µmol) and 2-(7-azabenzotriazole)-*N*, *N, N'*, *N'*-tetramethylurea hexafluorophosphate (110 mg, 290 µmol), and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Gemini-NX C18, 70mm × 30 mm 3 µm, A: water (10 mmol/L NH₄HCO₃); B: acetonitrile, 35%-75%: 11 minutes) to obtain compound **13.**

MS-ESI [M+H]⁺, calculated: 604, found: 604.

¹H NMR (400 MHz, MeOD) *δ* 7.28-7.35 (m, 1H), 7.06-7.13 (m, 1H), 6.89-6.98 (t, 1H), 6.10-6.20 (m, 1H), 4.88-5.04 (m, 1H), 4.48-4.54 (m, 1H), 4.29-4.40 (m, 2H), 3.89-4.23 (m, 3H), 3.63-3.71 (m, 5H), 3.40-3.58 (m, 1H), 3.14-3.27 (m, 4H), 2.83-3.13 (m, 7H), 2.71-2.79 (m, 1H), 2.45-2.54 (m, 4H), 2.32-2.39 (m, 1H), 2.25-2.30 (s, 3H), 2.04-2.14 (m, 1H), 1.78-1.93 (m, 3H), 1.66-1.75 (m, 1H).

### Example 12 Synthesis of Compound 14

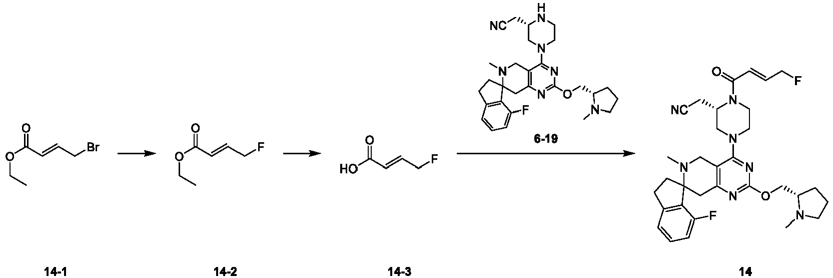

(1) To a solution of compound **14-1** (1.0 g, 5.18 mmol) in acetonitrile (10.0 mL) was added silver fluoride (1.97 g, 15.5 mmol), and the reaction solution was stirred at 25°C for 24 hours under nitrogen protection. Then the reaction solution was filtered and concentrated under reduced pressure to obtain compound **14-2.**
   ¹H NMR (400 MHz, CDCl₃) *δ* 6.90-7.04 (m, 1H), 6.07-6.16 (m, 1H), 5.09-5.14 (m, 1H), 4.98-5.03 (m, 1H), 4.19-4.26 (m, 2H), 1.29-1.33 (m, 3H).
(2) To a solution of compound **14-2** (400 mg, 3.03 mmol) in tetrahydrofuran (3.0 mL) was added lithium hydroxide (381 mg, 9.08 mmol) and water (3.0 mL), and the reaction solution was stirred at 25°C for 12 hours under nitrogen protection. Then the reaction solution was added with hydrochloric acid (20 mL, 1 mol/L), and extracted with ethyl acetate (50.0 mL × 2). The combined organic phase was washed with saturated NaCl aqueous solution (50.0 mL × 2), dried over anhydrous sodium sulfate, filtered, and concentrated under reduced pressure to give compound**14-3**.
   ¹H NMR (400 MHz, DMSO-*d*₆) *δ* 12.36-12.68 (s, 1H), 6.80-6.96 (m, 1H), 5.88-6.04 (m, 1H), 5.03-5.22 (m, 2H).
(3) To a solution of compound **14-3** (45.3 mg, 435 µmol) in dichloromethane (2.0 mL) was added triethylamine (14.7 mg, 145 µmol), trifluoroacetate of compound **6-19** (90.0 mg, 145 µmol) and 2-(7-azabenzotriazole)-N, N, N ', N'-tetramethylurea hexafluorophosphate (110 mg, 290 µmol), and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Gemini-NX C18, 70mm × 30 mm 3 µm, A: water (10 mmol/L NH₄HCO₃); B: acetonitrile, 35%-75%: 11 minutes) to obtain compound **14.**

MS-ESI [M+H]⁺, calculated: 592, found: 592.

¹H NMR (400 MHz, MeOD) *δ* 7.28-7.35 (m, 1H), 7.06-7.13 (m, 1H), 6.89-6.98 (t, 1H), 6.56-6.84 (m, 1H), 5.02-5.19 (m, 1H), 4.28-4.41 (m, 2H), 3.86-4.19 (m, 3H), 3.65-3.74 (m, 2H), 3.34-3.61 (m, 3H), 2.83-3.28 (m, 10H), 2.70-2.80 (m, 1H), 2.43-2.53 (m, 4H), 2.32-2.40 (m, 1H), 2.25-2.31 (s, 3H), 2.04-2.13 (m, 1H), 1.78-1.94 (m, 3H), 1.65-1.75 (m, 1H).

### Example 13 Synthesis of Compound 15

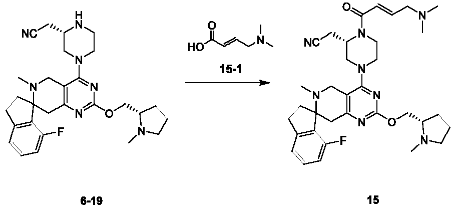

To a solution of compound **15-1** (45.3 mg, 435 µmol) in dichloromethane (2.0 mL) was added triethylamine (14.7 mg, 145 µmol), trifluoroacetate of compound **6-19**(90.0 mg, 145 µmol) and 2-(7-azabenzotriazole)-*N*, *N, N '*, N'-tetramethylurea hexafluorophosphate (110 mg, 290 µmol), and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-20%: 8 minutes) to obtain formate of compound **15.**

MS-ESI [M+H]⁺, calculated: 617, found: 617.

¹H NMR (400 MHz, MeOD) *δ* 7.29-7.37 (m, 1H), 7.07-7.15 (m, 1H), 6.73-7.03 (m, 3H), 4.90-5.20 (m, 1H), 4.65-4.73 (m, 1H), 4.51-4.57 (m, 1H), 3.85-4.47 (m, 3H), 3.61-3.82 (m, 6H), 3.42-3.59 (m, 1H), 3.04-3.29 (m, 6H), 2.87-3.03 (m, 6H), 2.67-2.80 (m, 6H), 2.34-2.56 (m, 2H), 2.26-2.33 (m, 3H), 1.88-2.20 (m, 4H).

### Example 14 Synthesis of Compound 16

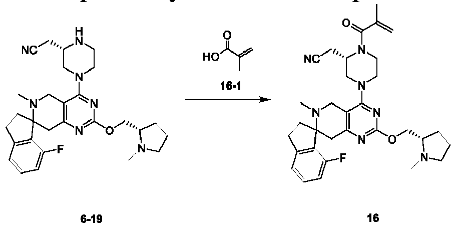

To a solution of compound **16-1** (45.3 mg, 435 µmol) in dichloromethane (2.0 mL) was added triethylamine (14.7 mg, 145 µmol), trifluoroacetate of compound **6-19**(90.0 mg, 145 µmol) and 2-(7-azabenzotriazole)-*N*, *N, N '*, *N*'-tetramethylurea hexafluorophosphate (110 mg, 290 µmol), and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-20%: 8 minutes) to obtain formate of compound **16.**

MS-ESI [M+H]⁺, calculated: 574, found: 574.

¹H NMR (400 MHz, MeOD) *δ* 7.29-7.36 (m, 1H), 7.08-7.14 (m, 1H), 6.90-6.99 (m, 1H), 5.31-5.39 (m, 1H), 5.14-5.24 (m, 1H), 4.89-5.07 (m, 1H), 4.67-4.73 (m, 1H), 4.48-4.56 (m, 1H), 3.89-4.32 (m, 3H), 3.76-3.85 (m, 1H), 3.63-3.74 (m, 3H), 3.34-3.49 (m, 1H), 3.15-3.27 (m, 3H), 2.85-3.14 (m, 9H), 2.45-2.56 (m, 1H), 2.33-2.41 (m, 1H), 2.27-2.32 (m, 3H), 2.02-2.22 (m, 3H), 1.97-2.01 (m, 3H), 1.87-1.95 (m, 1H).

### Example 15 Synthesis of Compound 17

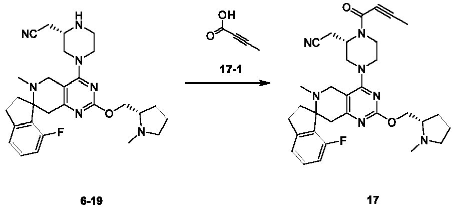

To a solution of compound **17-1** (45.3 mg, 435 µmol) in dichloromethane (2.0 mL) was added triethylamine (14.7 mg, 145 µmol), trifluoroacetate of compound **6-19** (90.0 mg, 145 µmol) and 2-(7-azabenzotriazole)-*N*, *N, N '*, *N*'-tetramethylurea hexafluorophosphate (110 mg, 290 µmol), and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-20%: 8 minutes) to obtain formate of compound **17.**

MS-ESI [M+H]⁺, calculated: 572, found: 572.

¹H NMR (400 MHz, MeOD) *δ* 7.29-7.36 (m, 1H), 7.06-7.15 (m, 1H), 6.90-6.99 (m, 1H), 4.66-4.75 (m, 1H), 4.35-4.58 (m, 2H), 4.01-4.32 (m, 2H), 3.66-3.86 (m, 4H), 3.39-3.60 (m, 1H), 2.85-3.27 (m, 12H), 2.34-2.57 (m, 2H), 2.27-2.33 (m, 3H), 2.06-2.21 (m, 5H), 1.87-2.04 (m, 2H), 1.61-1.75 (m, 1H).

### Example 16 Synthesis of Compound 18

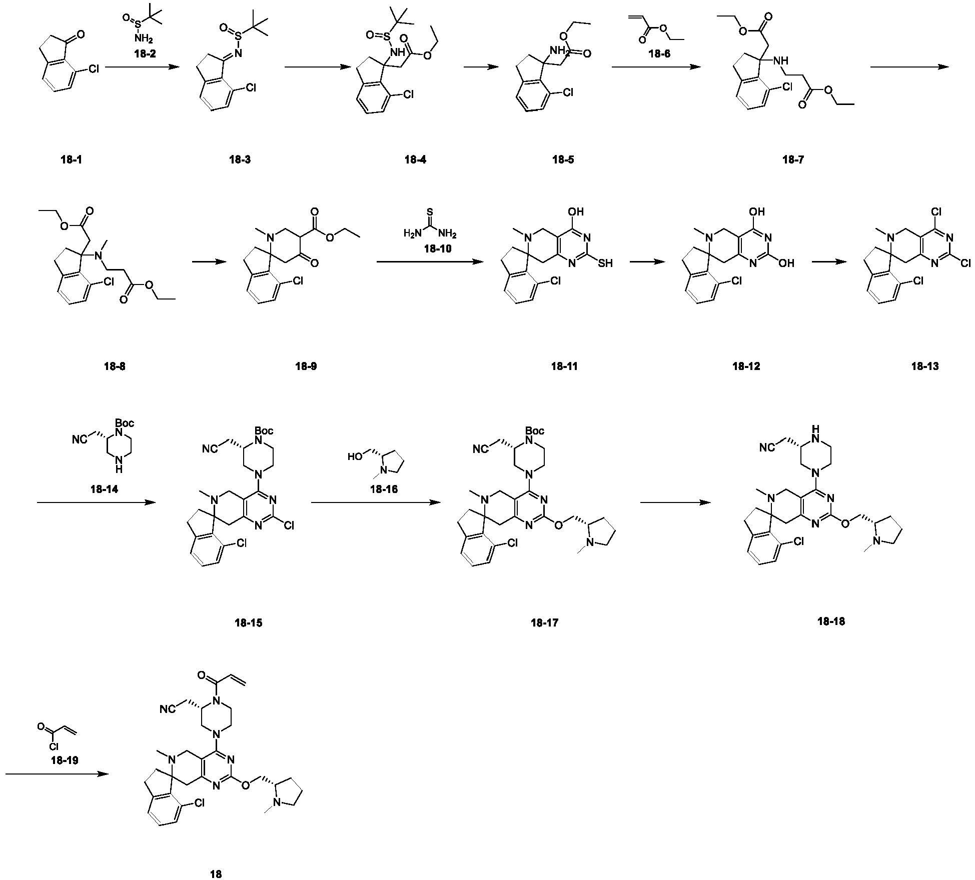

(1) To a solution of compound **18-1** (24.0 g, 144 mmol) in toluene (300 mL) was added compound **18-2** (21.0 g, 173 mmol), and tetraethyl titanate (65.9 g, 289 mmol), and then the reaction solution was stirred at 120°C for 12 hours under nitrogen protection. Then the reaction solution was added with water (100 mL) and ethyl acetate (400 mL). After filtration, the filter cake was washed with ethyl acetate (400 mL × 4). The combined organic phase was washed with saturated NaCl aqueous solution (400 mL × 2), dried over anhydrous sodium sulfate, filtered, and concentrated under reduced pressure. The crude product was separated by silica gel column chromatography (petroleum ether/ethyl acetate = 1:0 to 5:1) to obtain compound **18-3.**
   MS-ESI [M+H]⁺, calculated: 270, found: 270.
   ¹H NMR (400 MHz, CDCl₃) *δ* 7.35-7.40 (m, 1H), 7.27-7.32 (m, 2H), 3.45-3.60 (m, 1H), 3.06-3.15 (m, 3H), 1.36 (s, 9H).
(2) To a solution of ethyl acetate (3.27 g, 37.1 mmol) in tetrahydrofuran (20.0 mL) was added dropwise with lithium diisopropylamide (2 mol/L, 7.4 mL, tetrahydrofuran solution) at -65°C under nitrogen protection, and the reaction solution was stirred at -78°C for 1 hour. Then the reaction solution was added dropwise with a solution of compound **18-3** (2.00 g, 7.41 mmol) in tetrahydrofuran (20.0 mL), and then continued stirring for 2 hours at -65°C. After warming to 0°C, the reaction solution was quenched with saturated ammonium chloride aqueous solution(50.0 mL), and extracted with ethyl acetate (50.0 mL × 2). The combined organic phase was washed with saturated ammonium chloride aqueous solution (50.0 mL × 1) and saturated NaCl aqueous solution (50.0 mL × 1), dried over anhydrous sodium sulfate, filtered, and concentrated under reduced pressure. The crude product was separated by silica gel column chromatography (petroleum ether/ethyl acetate = 1:0 to 3:1) to obtain compound **18-4.**
   MS-ESI [M+H]⁺, calculated: 358, found: 358.
   ¹H NMR (400 MHz, CDCl₃) *δ* 7.21-7.25 (m, 1H), 7.14-7.19 (m, 2H), 5.14 (s, 1H), 4.11-4.20 (m, 2H), 3.68 (d, *J* = 15.6 Hz, 1H), 3.33 (dt, *J* = 16.4, 8.4 Hz, 1H), 2.90 (ddd, *J =* 16.4, 9.2, 3.6 Hz, 1H), 2.78 (d, *J* = 15.6 Hz, 1H), 2.68 (ddd, *J* = 13.2, 9.2, 3.6 Hz, 1H), 2.29-2.38 (m, 1H), 1.23-1.27 (m, 3H), 1.20 (s, 9H).
(3) To a solution of compound **18-4** (1.40 g, 3.91 mmol) in ethanol (15.0 mL) was added hydrochloric acid in dioxane (4 mol/L, 5.0 mL), and the reaction solution was stirred at 25°C for 1 hour under nitrogen protection. Then the reaction solution was concentrated under reduced pressure to obtain crude product of hydrochloride of compound **18-5.**
   MS-ESI [M+H]⁺, calculated: 254, found: 254.
(4) To a solution of hydrochloride of compound **18-5** (1.14 g, 3.93 mmol) in ethanol (12.0 mL) was added compound **18-6** (8.17g, 81.6 mmol), copper oxide (62.5 mg, 786 µmol), and triethylamine (1.19g, 11.8 mmol), and the reaction solution was then reacted at 85°C in a sealed tube for 8 hours under nitrogen protection. Then the reaction solution was added with water (100 mL), and extracted with ethyl acetate (100 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 10:1) to obtain compound **18-7.**
   MS-ESI [M+H]⁺, calculated: 354, found: 354.
   ¹H NMR (400 MHz, CDCl₃) *δ* 7.02-7.18 (m, 3H), 4.09-4.14 (m, 2H), 4.02 (q, *J* = 7.2 Hz, 2H), 2.86-3.03 (m, 3H), 2.72-2.86 (m, 2H), 2.63-2.72 (m, 1H), 2.41-2.50 (m, 4H), 2.29 (m, 1H), 1.22-1.26 (t, *J* = 7.2 Hz, 3H), 1.12 (t, *J* = 7.2 Hz, 3H).
(5) To a solution of compound **18-7** (900 mg, 2.54 mmol) in ethanol (15.0 mL) was added paraformaldehyde (900 mg) and sodium cyanoborohydride (480 mg, 7.64 mmol), and then the reaction solution was reacted for 10 hours at 25°C under nitrogen protection. Then the reaction solution was added with water (30.0 mL), and extracted with ethyl acetate (30.0 mL × 1). The combined organic phase was washed with saturated NaCl aqueous solution (30.0 mL × 2), dried over anhydrous sodium sulfate, filtered, and concentrated under reduced pressure. The crude product was separated by silica gel column chromatography (petroleum ether/ethyl acetate = 1:0 to 15:1) to obtain compound **18-8.**
   MS-ESI [M+H]⁺, calculated: 368, found: 368.
   ¹H NMR (400 MHz, CDCl₃) *δ* 7.08-7.16 (m, 2H), 7.01-7.06 (m, 1H), 4.10 (q, *J* = 7.2 Hz, 2H), 3.80 (dtt, *J* = 10.4, 7.2, 3.6 Hz, 2H), 3.32 (d, *J* = 13.6 Hz, 1H), 2.94 (d, *J* = 13.6 Hz, 1H), 2.87-2.92 (m, 2H), 2.74 (dt, *J* = 12.8, 7.2 Hz, 1H), 2.50-2.56 (m, 1H), 2.43-2.49 (m, 2H), 2.35 (dt, *J* = 14.0, 8.0 Hz, 1H), 2.20-2.28 (m, 1H), 2.14 (s, 3H), 1.24 (t, *J* = 7.2 Hz, 3H), 0.91 (t, *J* = 7.2 Hz, 3H).
(6) To a solution of compound **18- 8** (780 mg, 2.12 mmol) in tetrahydrofuran (10.0 mL) was added potassium bis (trimethylsilyl) amide (1 mol/L, 6.4 mL, tetrahydrofuran solution) at -78°C, and the reaction solution was reacted at -65°C for 2 hours under nitrogen protection. Then the reaction solution was added with saturated ammonium chloride aqueous solution (50.0 mL), and extracted with ethyl acetate (50.0 mL × 1). The combined organic phase was washed with saturated NaCl aqueous solution (50.0 mL × 1), dried over anhydrous sodium sulfate, filtered, and concentrated under reduced pressure. The crude product was separated by silica gel column chromatography (petroleum ether/ethyl acetate = 1:0 to 19:1) to obtain compound **18-9.**
   MS-ESI [M+H]⁺, calculated: 322, found: 322.
   ¹H NMR (400 MHz, CDCl₃) *δ* 7.15-7.20 (m, 2H), 7.10-7.14 (m, 1H), 4.26-4.34 (m, 1H), 4.17-4.25 (m, 1H), 3.61 (d, *J* = 15.2 Hz, 1H), 3.04-3.17 (m, 2H), 2.71-3.04 (m, 3H), 2.24-2.30 (m, 1H), 2.15-2.22 (m, 1H), 2.14 (s, 3H), 1.76 (ddd, *J* = 13.2, 8.4, 2.4 Hz, 1H), 1.31 (t, *J* = 7.2 Hz, 3H).
(7) To a solution of compound **18-9** (475 mg, 1.48 mmol) in ethanol (10.0 mL) was added compound **18-10** (225 mg, 2.96 mmol) and sodium ethoxide (301mg, 4.42 mmol), and the reaction solution was stirred at 80°C for 12 hours under nitrogen protection. After concentrated under reduced pressure, the reaction solution was adjusted to pH 6 with hydrochloric acid (1 mol/L). The resulting solution was precipitated, and filtered, and the filter cake was dried to obtain compound **18-11.**
   MS-ESI [M+H]⁺, calculated: 334, found: 334.
   ¹H NMR (400 MHz, DMSO-d6) *δ* 12.41 (s, 1H), 12.27 (s, 1H), 7.23-7.31 (m, 3H), 3.52 (d, *J =* 16.4 Hz, 1H), 2.88-3.04 (m, 4H), 2.30-2.37 (m, 1H), 2.16-2.25 (m, 1H), 2.03 (s, 3H), 1.66 (t, *J* = 10.0 Hz, 1H).
(8) To a solution of compound **18-11** (320 mg, 959 µmol) in water (10.0 mL) was added chloroacetic acid (410 g, 4.34 mmol), and the reaction solution was stirred at 100°C for16 hours under nitrogen protection. After the reaction solution was filtered, the filtrate was adjusted to pH 8 with saturated sodium bicarbonate aqueous solution to get precipitate. After filtration, the filter cake was dried to obtain compound **18-12.**
   MS-ESI [M+H]⁺, calculated: 318, found: 318.
   ¹H NMR (400 MHz, DMSO-*d*6)*δ* 11.00 (s, 1H), 10.76 (s, 1H), 7.22-7.33 (m, 3H), 3.47 (d, *J* = 15.2 Hz, 1H), 2.85-2.99 (m, 4H), 2.13-2.25 (m, 2H), 2.02 (s, 3H), 1.59-1.69 (m, 1H).
(9) Compound **18-12** (260 mg, 818 µmol) was dissolved in phosphorus oxychloride (8.25g, 53.8 mmol), and the reaction solution was stirred at 80°C for 6 hours under nitrogen protection. Then the reaction solution was diluted with dichloromethane (30.0 mL) and added with saturated sodium bicarbonate aqueous solution (30.0 mL) to adjust pH value to 8. The separated organic phase was washed with saturated sodium bicarbonate aqueous solution (30.0 mL × 2) and saturated NaCl aqueous solution (30.0 mL × 1), dried over anhydrous sodium sulfate, filtered, and concentrated under reduced pressure to give compound **18-13.**
   MS-ESI [M+H]⁺, calculated: 356, found: 356.
   ¹H NMR (400 MHz, CDCl₃) *δ* 7.15-7.26 (m, 3H), 3.72-4.23 (m, 2H), 3.43-3.69 (m, 2H), 3.00-3.08 (m, 1H), 2.81-2.96 (m, 2H), 2.31 (s, 3H), 0.77-1.27 (m, 1H).
(10) To a solution of compound **18-13** (180 mg, 508 µmol) in N-methylpyrrolidone (5.0 mL) was added potassium carbonate (211 mg, 1.53 mmol) and compound **18-14** (137 mg, 608 µmol), and the reaction solution was stirred at 50°C for 10 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure. The crude product was separated by silica gel column chromatography(petroleum ether/ethyl acetate = 1:0 to 6:1) to give compound **18-15.**
   MS-ESI [M+H]⁺, calculated: 543, found: 543.
   ¹H NMR (400 MHz, MeOD) *δ* 7.14-7.34 (m, 3H), 4.64 (s, 1H), 4.10-4.25 (m, 1H), 3.88-4.10 (m, 3H), 3.71 (d, *J* = 9.6 Hz, 2H), 3.33-3.49 (m, 2H), 3.15-3.30 (m, 2H), 3.01 (dt, *J* = 17.6, 8.8 Hz, 3H), 2.75 (dd, *J* = 18.0, 3.6 Hz, 1H), 2.38-2.52 (m, 1H), 2.24 (d, *J* = 0.8 Hz, 3H), 1.78-1.87 (m, 1H), 1.51 (d, *J =* 1.6 Hz, 9H).
(11) To a solution of compound **18-15** (150 mg, 276 µmol) in dioxane (5.0 mL) was added compound **18-16** (95.4 mg, 828 µmol), potassium carbonate (115 mg, 832 µmol), dicyclohexyl(2',4',6'-triisopropyl-[1,1'-biphenyl]-2-yl)phosphine (52.6 mg, 110 µmol) and bis (di benzylidene acetone) palladium (50.6 mg, 55.3 µmol), and the reaction solution was stirred at 110°C for 4 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 10:1) to obtain compound **18-17.**
   MS-ESI [M+H]⁺, calculated: 622, found: 622.
   ¹H NMR (400 MHz, MeOD)*δ* 7.16-7.31 (m, 3H), 4.62 (s, 1H), 4.38-4.54 (m, 2H), 4.13-4.25 (m, 1H), 3.91-4.12 (m, 3H), 3.62-3.72 (m, 2H), 3.36-3.51 (m, 2H), 3.12-3.20 (m, 2H), 2.91-3.08 (m, 4H), 2.65-2.76 (m, 4H), 2.48 (d, *J* = 8.4 Hz, 1H), 2.24 (s, 3H), 2.07-2.22 (m, 2H), 1.93 (d, *J =* 8.4 Hz, 2H), 1.77-1.88 (m, 3H), 1.51 (d, *J* = 2.4 Hz, 9H).
(12) To a solution of compound **18-17** (20.0 mg, 193 µmol) in dichloromethane (3.0 mL) was added trifluoroacetic acid (1.0 mL), and the reaction solution was stirred at 25°C for 1 hour under nitrogen protection. Then the reaction solution was concentrated under reduced pressure to obtain crude trifluoroacetate of compound **18-18.**
   MS-ESI [M+H]⁺, calculated: 522, found: 522.
(13) To a solution of trifluoroacetate of compound **18-18** (50.0 mg, 78.6 µmol) in dichloromethane (2.0 mL) was added triethylamine (23.9 mg, 236 µmol) and compound **18-19** (14.3 mg, 158 µmol), and the reaction solution was stirred at -65°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **18.**
   MS-ESI [M+H]⁺, calculated: 576, found: 576.
   ¹H NMR (400 MHz, MeOD) *δ* 7.21-7.29 (m, 3H), 6.80 (s, 1H), 6.30 (d, *J* = 16.8 Hz, 1H), 5.84 (d, *J* = 10.0 Hz, 1H), 4.67 (d, *J* = 12.4 Hz, 1H), 4.46-4.53 (m, 1H), 4.18-4.38 (m, 1H), 4.00-4.18 (m, 2H), 3.62-3.84 (m, 4H), 3.45-3.62 (m, 2H), 3.33-3.38 (m, 1H), 3.15-3.28 (m, 2H), 2.98-3.14 (m, 4H), 2.96 (d, *J* = 9.2 Hz, 3H), 2.91 (d, *J* = 4.0 Hz, 1H), 2.73 (dd, *J* = 17.6, 3.2 Hz, 1H), 2.44-2.51 (m, 1H), 2.33 (dt, *J* = 8.4, 6.4 Hz, 1H), 2.25 (s, 3H), 2.10-2.17 (m, 1H), 2.02-2.09 (m, 1H), 1.93-2.02 (m, 1H), 1.79-1.88 (m, 1H).

### Example 17 Synthesis of Compound 19

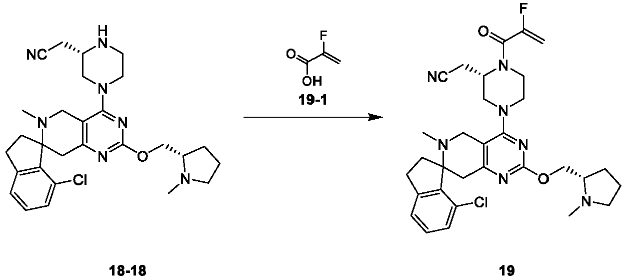

To a solution of compound **19-1** (21.3 mg, 237 µmol) in dichloromethane (2.0 mL) was added triethylamine (23.9 mg, 236 µmol), trifluoroacetate of compound **18-18**(90.0 mg, 145 µmol) and propylphosphonic acid anhydride solution (150 mg, 236 µmol, 50% ethyl acetate solution), and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-20%: 8 min) to obtain formate of compound **19.**

MS-ESI [M+H]⁺, calculated: 594, found: 595.

¹H NMR (400 MHz, MeOD) *δ* 7.21-7.29 (m, 3H), 5.37 (d, *J* = 17.6 Hz, 1H), 5.29 (d, *J* = 7.6 Hz, 1H), 4.64 (d, *J* = 10.0 Hz, 1H), 4.48 (dt, *J* = 12.0, 5.6 Hz, 2H), 4.34 (d, *J* = 13.2 Hz, 1H), 4.08-4.26 (m, 2H), 4.03 (d, *J* = 10.4 Hz, 1H), 3.66-3.73 (m, 2H), 3.48-3.63 (m, 3H), 3.37 (s, 1H), 3.18-3.25 (m, 1H), 2.95-3.07 (m, 4H), 2.92 (d, *J* = 10.0 Hz, 3H), 2.73 (dd, *J* = 18.0, 3.6 Hz, 1H), 2.49 (q, *J* = 10.4 Hz, 1H), 2.28-2.36 (m, 1H), 2.25 (s, 3H), 2.00-2.12 (m, 2H), 1.92-1.99 (m, 1H), 1.79-1.86 (m, 1H), 1.67 (s, 1H).

### Example 18 Synthesis of Compound 20

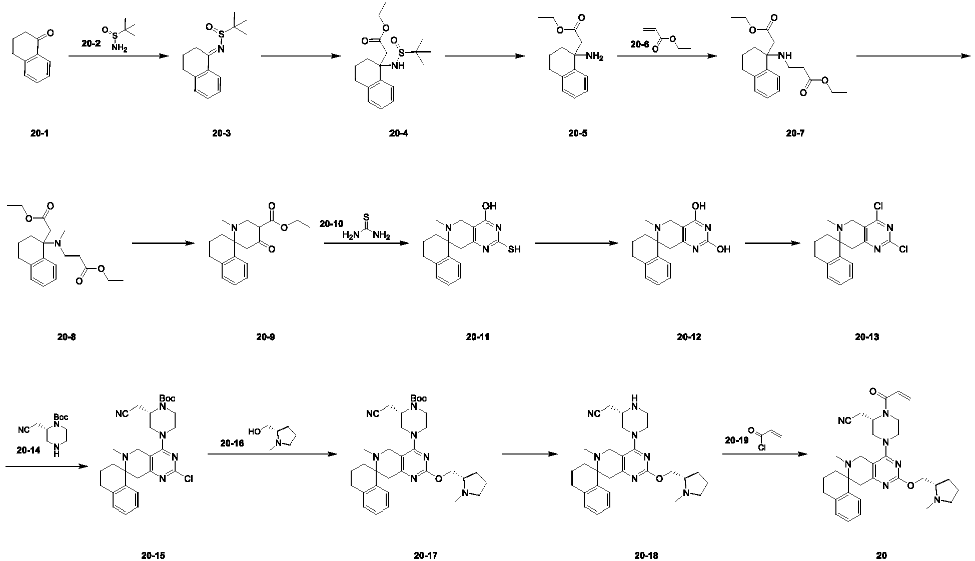

(1) To a solution of compound **20-1** (30.0 g, 205 mmol) in toluene (500 mL) was added compound **20-2** (29.9 g, 247 mmol), tetraethyl titanate (93.6 g, 410 mmol), and the reaction solution was stirred at 110°C for 16 hours under nitrogen protection. Then the reaction solution was added with water(200 mL) and ethyl acetate (300 mL). After filtration, the filter cake was washed with ethyl acetate (300 mL × 4). The combined organic phase was washed with saturated NaCl aqueous solution (300 mL × 2), dried over anhydrous sodium sulfate, filtered, and concentrated under reduced pressure. The crude product was separated by silica gel column chromatography (petroleum ether/ethyl acetate = 1:0 to 11:1) to obtain compound **20-3.**
   MS-ESI [M+H]⁺, calculated: 250, found: 250.
   ¹H NMR (400 MHz, CDCl₃) *δ* 8.17 (d, *J* = 8.0 Hz, 1H), 7.35-7.42 (m, 1H), 7.22-7.26 (m, 1H), 7.19 (d, *J* = 7.6 Hz, 1H), 3.23-3.34 (m, 1H), 3.06 (m, 1H), 2.85-2.90 (m, 2H), 1.95-2.07 (m, 2H), 1.33 (s, 9H).
(2) To a solution of ethyl acetate (60.1 g, 682 mmol) in tetrahydrofuran (400 mL) was added dropwise with lithium diisopropylamide (2 mol/L, 137 mL, tetrahydrofuran solution) at -65°C under nitrogen protection, and the reaction solution was stirred at -65°C for 1 hour. Then the reaction solution was added dropwise with a solution of compound **20-3** (34.0 g, 136 mmol) in tetrahydrofuran (200 mL), and continued stirring for 2 hours at -65°C. After warming to 0°C, the reaction solution was quenched with saturated ammonium chloride aqueous solution(500 mL), and extracted with ethyl acetate (500 mL × 2). The combined organic phase was washed with saturated ammonium chloride aqueous solution (500 mL × 1) and saturated NaCl aqueous solution (500 mL × 1), dried over anhydrous sodium sulfate, filtered, and concentrated under reduced pressure. The crude product was separated by silica gel column chromatography (petroleum ether/ethyl acetate = 1:0 to 2:1) to obtain compound **20-4.**
   MS-ESI [M+H]⁺, calculated: 338, found: 338.
   ¹H NMR (400 MHz, CDCl₃) *δ*7.32-7.40 (m, 1H), 7.13-7.21 (m, 2H), 7.08-7.13 (m, 1H), 5.19 (s, 1H), 4.11-4.20 (m, 2H), 2.86-2.98 (m, 1H), 2.80-2.86 (m, 2H), 2.70-2.78 (m, 1H), 2.42-2.51 (m, 1H), 2.04-2.17 (m, 2H), 1.76-1.86 (m, 1H), 1.24-1.28 (m, 3H), 1.23 (s, 9H).
(3) To a solution of compound **20-4** (6.0 g, 17.8 mmol) in ethanol (60.0 mL) was added hydrochloric acid in dioxane (4 mol/L, 20.0 mL), and the reaction solution was stirred at 25°C for 1 hour under nitrogen protection. Then the reaction solution was concentrated under reduced pressure to obtain crude product of hydrochloride of compound **20-5.**
   MS-ESI [M-NH₂+H]⁺, calculated: 217, found: 217.
(4) To a solution of hydrochloride of compound **20-5** (4.80 g, 17.8 mmol) in ethanol (60.0 mL) was added compound **5-6** (18.2 g, 182 mmol), copper oxide (283 mg, 3.56 mmol), and triethylamine (5.40 g, 53.4 mmol), and the reaction solution was reacted at 85°C in a sealed tube for 8 hours under nitrogen protection. Then the reaction solution was added with water(200 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 10:1) to obtain compound **20-7.**
   MS-ESI [M+H]⁺, calculated: 334, found: 334.
   ¹H NMR (400 MHz, CDCl₃) *δ*7.53 (d, *J* = 6.4 Hz, 1H), 7.09-7.18 (m, 2H), 7.03-7.08 (m, 1H), 4.09-4.15 (m, 4H), 2.73-2.80 (m, 4H), 2.62 (d, *J* = 14.0 Hz, 2H), 2.40-2.46 (m, 3H), 2.08 (d, *J* = 10.0 Hz, 1H), 1.85-1.99 (m, 3H), 1.25 (t, *J* = 7.2 Hz, 3H), 1.21 (t, *J* = 7.2 Hz, 3H).
(5) To a solution of compound **20-7** (4.32 g, 13.0 mmol) in ethanol (60.0 mL) was added paraformaldehyde (3.89 g) and sodium cyanoborohydride (2.45 g, 38.9 mmol), and the reaction solution was reacted for 16 hours at 25°C under nitrogen protection. Then the reaction solution was added with water(100 mL), and extracted with ethyl acetate (100 mL × 1). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 10:1) to obtain compound **20-8.**
   MS-ESI [M+H]⁺, calculated: 348, found: 348.
   ¹H NMR (400 MHz, MeOD) *δ* 7.43-7.52 (m, 1H), 7.05-7.13 (m, 2H), 6.95-7.04 (m, 1H), 4.04-4.12 (m, 2H), 3.73-3.83 (m, 2H), 2.79 (s, 2H), 2.76 (d, *J* = 7.2 Hz, 1H), 2.71 (t, *J* = 6.4 Hz, 2H), 2.56-2.63 (m, 1H), 2.39-2.48 (m, 1H), 2.30-2.38 (m, 1H), 2.21 (s, 3H), 2.10-2.15 (m, 2H), 1.81-1.90 (m, 1H), 1.70-1.80 (m, 1H), 1.22 (t, *J* = 7.2 Hz, 3H), 0.94 (t, *J* = 7.2 Hz, 3H).
(6) To a solution of compound **20- 8** (3.10 g, 8.92 mmol) in tetrahydrofuran (60.0 mL) was added potassium bis (trimethylsilyl) amide (1 mol/L, 17.8 mL mL, tetrahydrofuran solution) at -65°C, and the reaction solution was reacted at -65°C for 2 hours under nitrogen protection. Then the reaction solution was added with saturated ammonium chloride aqueous solution(100 mL), and extracted with ethyl acetate (100 mL × 1). The organic phase was washed successively with saturated ammonium chloride aqueous solution (100 mL × 1) and saturated NaCl aqueous solution (100 mL × 1), dried over anhydrous sodium sulfate, filtered, and concentrated under reduced pressure. The crude product was separated by silica gel column chromatography (petroleum ether/ethyl acetate = 1:0 to 10:1) to obtain compound **20-9.**
   MS-ESI [M+H]⁺, calculated: 302, found: 302.
   ¹H NMR (400 MHz, CDCl₃) *δ*7.49-7.75 (m, 1H), 7.21 (t, *J*=6.4 Hz, 1H), 7.10-7.17 (m, 1H), 7.05 (d, *J* = 7.6 Hz, 1H), 4.20-4.33 (m, 2H), 3.45-3.63 (m, 1H), 3.14-3.28 (m, 1H), 2.64-2.76 (m, 3H), 2.51-2.63 (m, 1H), 2.01-2.14 (m, 3H), 1.80-2.00 (m, 2H), 1.63-1.80 (m, 3H), 1.30-1.34 (m, 3H).
(7) To a solution of compound **20-9** (3.78 g, 12.5 mmol) in ethanol (70.0 mL) was added compound **20-10**(1.91 g, 25.1 mmol) and sodium ethoxide (2.56 g, 37.6 mmol), and the reaction solution was stirred at 80°C for 8 hours under nitrogen protection. After concentrated under reduced pressure, the reaction solution was adjusted to pH 6 with hydrochloric acid (1 mol/L). The resulting solution was precipitated, and filtered, and the filter cake was dried to obtain compound **20-11.**
   MS-ESI [M+H]⁺, calculated: 314, found: 314.
(8) To a solution of intermediate **20-11** (3.36 g, 10.7 mmol) in water (80.0 mL) was added chloroacetic acid (4.05 g, 42.9 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **20-12.**
   MS-ESI [M+H]⁺, calculated: 298, found: 298.
   ¹H NMR (400 MHz, DMSO-d6) *δ* 10.98 (s, 1H), 10.69 (s, 1H), 7.45-7.51 (m, 1H), 7.17-7.24 (m, 1H), 7.11-7.17 (m, 1H), 7.04-7.10 (m, 1H), 3.46-3.52 (m, 1H), 2.99-3.07 (m, 1H), 2.65-2.72 (m, 2H), 2.52-2.55 (m, 2H), 1.95 (s, 3H), 1.84-1.91 (m, 1H), 1.71-1.80 (m, 1H), 1.51-1.64 (m, 2H).
(9) Compound **20-12** (1.00g, 3.36 mmol) was dissolved in phosphorus oxychloride (10 mL), and the reaction solution was stirred at 100°C in a sealed tube under nitrogen protection for 12 hours. Then the reaction solution was concentrated under reduced pressure, added with iced water (100 mL), and extracted with dichloromethane (100 mL × 1). The organic phase was washed with saturated sodium bicarbonate aqueous solution (100 mL × 2) and saturated NaCl aqueous solution (100 mL × 1), dried over anhydrous sodium sulfate, filtered, and concentrated under reduced pressure to obtain compound **20-13.**
   MS-ESI [M+H]⁺, calculated: 334, found: 334.
(10) To a solution of compound **20-13** (782 mg, 2.34 mmol) in *N*-methylpyrrolidone (10.0 mL) was added potassium carbonate (970 mg, 7.02 mmol) and compound **20-14**(633 mg, 2.81 mmol), and the reaction solution was stirred at 50°C for 12 hours under nitrogen protection. Then the reaction solution was poured into water (30.0 mL) and extracted with ethyl acetate (30.0 mL × 2). The organic phases were combined, washed with saturated NaCl aqueous solution (30.0 mL × 2), dried over anhydrous sodium sulfate, filtered, and concentrated under reduced pressure. The crude product was separated by silica gel column chromatography (petroleum ether/ethyl acetate = 1:0 to 0:1) to obtain compound **20-15.**
   MS-ESI [M+H]⁺, calculated: 523, found: 523.
(11) To a solution of compound **20-15** (500 mg, 956 µmol ) in dioxane (10.0 mL) was added compound **20-16** (330 mg, 2.87mmol), cesium carbonate (934 mg, 2.88 mmol ) and (2-dicyclohexylphosphino-2',6'-diisopropoxy-1,1'-biphenyl) [2-(2'-amino-1,1'- biphenyl)] palladium(II) methanesulfonate (160 mg, 191 µmol), and the reaction solution was stirred at 110°C for 12 hours under nitrogen protection. Then the reaction solution was added with ethyl acetate (50.0 mL), washed with saturated NaCl aqueous solution (50.0 mL × 2), dried over anhydrous sodium sulfate, filtered, and concentrated under reduced pressure. The crude product was separated by silica gel column chromatography (dichloromethane/methanol = 1:0 to 9:1) to obtain compound **20-17.**
   MS-ESI [M+H]⁺, calculated: 602, found: 602.
(12) To a solution of compound **20-17** (368 mg, 611 µmol) in dichloromethane (6.0 mL) was added trifluoroacetic acid (3.08 g, 27.0 mmol), and the reaction solution was stirred at 25°C for 0.5 hours under nitrogen protection. Then the reaction solution was concentrated under reduced pressure to obtain crude trifluoroacetate of compound **20-18.**
   MS-ESI [M+H]⁺, calculated: 502, found: 502.
(13) To a solution of trifluoroacetate of compound **20-18**(175 mg, 284 µmol) in dichloromethane (3.0 mL) was added triethylamine (28.8 mg, 284 µmol), then cooled to -78°C and added with compound **20-19** (38.6 mg, 426 µmol), and the reaction solution was stirred at -78°C for 5 minutes under nitrogen protection. Then the reaction solution was added with dichloromethane (30.0 mL), washed with saturated NaCl aqueous solution (30.0 mL × 2), dried over anhydrous sodium sulfate, filtered, and concentrated under reduced pressure. The crude product was separated by preparative high performance liquid chromatography (Xtimate C18, 100mm x 30 mm 10 µm, A: water (0.225% formic acid); B: acetonitrile, 0%-30%: 10 min) to obtain formate of compound **20.**
   MS-ESI [M+H]⁺, calculated: 556, found: 556.
   ¹H NMR (400 MHz, MeOD) *δ* 7.47-7.58 (m, 1H), 7.07-7.28 (m, 3H), 6.70-6.95 (m, 1H), 5.84-5.84 (m, 1H), 5.77-5.90 (m, 1H), 4.66-4.67 (m, 1H), 4.46-4.53 (m, 1H), 4.29-4.41 (m, 1H), 3.96-4.25 (m, 3H), 3.72-3.87 (m, 3H), 3.56-3.70 (m, 2H), 3.20-3.28 (m, 1H), 3.09-3.18 (m, 2H), 3.01-3.08 (m, 2H), 2.94-3.00 (m, 3H), 2.85-2.93 (m, 1H), 2.63-2.84 (m, 3H), 2.29-2.40 (m, 1H), 2.11-2.22 (m, 4H), 1.93-2.10 (m, 4H), 1.75-1.87 (m, 2H).

### Example 19 Synthesis of Compound 21

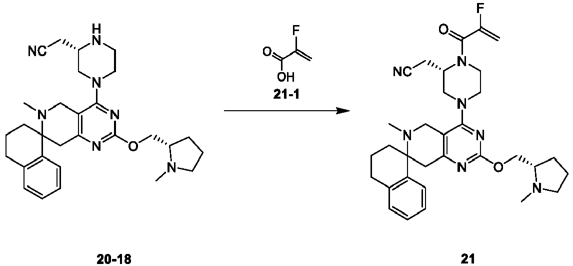

To a solution of compound **20-18** in dichloromethane was added triethylamine to adjust the pH of the solution to 7. Then the reaction solution was cooled to 0°C and added with compound **21-1** (92.2 mg, 1.02 mmol) and propanephosphonic acid anhydride (279 mg, 438 µmol), and the reaction solution was then heated to 25°C and reacted for 0.5 hours. Then the reaction solution was poured into water (30.0 mL), and extracted with dichloromethane (30.0 mL × 2). The organic phases were combined and washed with saturated NaCl aqueous solution (30.0 mL × 1), dried over anhydrous sodium sulfate, filtered, and concentrated under reduced pressure. The crude product was separated by preparative high performance liquid chromatography (Xtimate C18, 100 mm × 30 mm 10 µm, A: water (0.225% formic acid); B: acetonitrile, 0%-30%: 8 min) to obtain formate of compound **21.**
MS-ESI [M+H]⁺, calculated: 574, found: 574.
¹H NMR (400 MHz, MeOD) *δ* 7.52-7.53 (m, 1H), 7.19-7.26 (m, 1H), 7.14-7.18 (m, 1H), 7.09-7.13 (m, 1H), 5.22-5.47 (m, 2H), 4.66-4.70 (m, 1H), 4.46-4.53 (m, 1H), 4.16-4.17 (m, 2H), 3.66-3.86 (m, 4H), 3.46-3.65 (m, 2H), 3.33-3.40 (m, 1H), 3.22-3.29 (m, 1H), 3.05-3.19 (m, 4H), 3.01-3.04 (m, 1H), 2.90-3.00 (m, 4H), 2.72-2.82 (m, 2H), 2.28-2.40 (m, 1H), 2.09-2.15 (m, 4H), 1.92-2.06 (m, 4H), 1.75-1.86 (m, 2H).

### Example 20 Synthesis of Compound 22

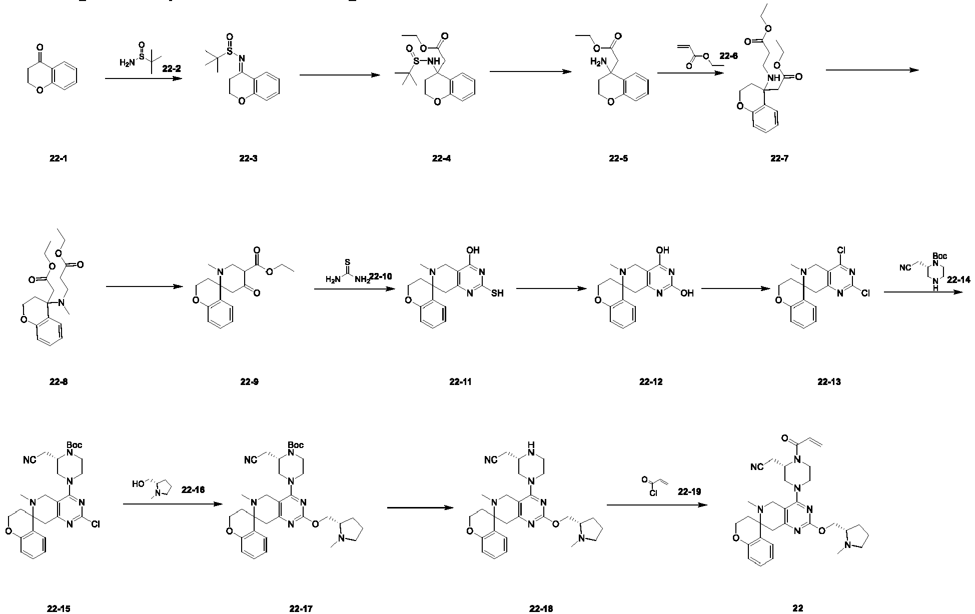

(1) To a solution of compound **22-1** (25.0 g, 168 mmol) in toluene (500 mL) was added compound **22-2** (26.5 g, 219 mmol), and tetraethyl titanate (77.0 g, 337 mmol), and then the reaction solution was stirred at 120°C for 12 hours under nitrogen protection. Then the reaction solution was added with water (400 mL), and filtered. The filtrate was washed with ethyl acetate (400 mL × 2). The combined organic phase was washed with saturated NaCl aqueous solution (500 mL), dried over anhydrous sodium sulfate, filtered, and concentrated under reduced pressure. The crude product was separated by silica gel column chromatography (petroleum ether/ethyl acetate = 1:0 to 0:1) to obtain compound **22-3.**
   MS-ESI [M+H]⁺, calculated: 252, found: 252.
   ¹H NMR (400 MHz, CDCl₃) *δ* 7.98-8.03 (m, 1H), 7.35-7.42 (m, 1H), 6.89-7.02 (m, 2H), 4.27-4.42 (m, 2H), 3.46-3.56 (m, 1H), 3.22-3.34 (m, 1H) , 1.32-1.34 (s, 9H).
(2) To a solution of ethyl acetate (54.6 g, 620 mmol) in tetrahydrofuran (1000 mL) was added dropwise with lithium diisopropylamide (2 mol/L, 155 mL, tetrahydrofuran solution) at -78°C under nitrogen protection, and the reaction solution was stirred at -78°C for 1 hour. Then the reaction solution was added dropwise with a solution of compound **22-3** (39.0 g, 155 mmol) in tetrahydrofuran (500 mL), and continued stirring for 3 hours at -78°C. Then the reaction solution was quenched with saturated ammonium chloride aqueous solution (2000 mL), and extracted with ethyl acetate (2000 mL × 1). The combined organic phase was washed with saturated ammonium chloride aqueous solution (2000 mL) and saturated NaCl aqueous solution (2000 mL), dried over anhydrous sodium sulfate, filtered, and concentrated under reduced pressure. The crude product was separated by silica gel column chromatography (petroleum ether/ethyl acetate = 1:0 to 4:1) to obtain compound **22-4.**
   MS-ESI [M+H]⁺, calculated: 340, found: 340.
(3) To a solution of compound **22-4** (5.00 g, 14.7 mmol) in ethanol (50.0 mL) was added hydrochloric acid in dioxane (4 mol/L, 15.0 mL), and the reaction solution was stirred at 25°C for 2 hours under nitrogen protection. Then the reaction solution was concentrated under reduced pressure to obtain crude product of hydrochloride of compound **22-5.**
   MS-ESI [M-NH₂+H]⁺, calculated: 219, found: 219.
(4) To a solution of hydrochloride of compound **22-5** (4.00 g, 14.7 mmol) in ethanol (40.0 mL) was added compound **22-6** (14.7 g, 147 mmol), copper oxide (234 mg, 2.94 mmol), triethylamine (1.49 g, 14.7 mmol), and the reaction solution was reacted at 85°C in a sealed tube for 8 hours under nitrogen protection. Then the reaction solution was added with water (100 mL), and extracted with ethyl acetate (200 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 6:1) to obtain compound **22-7.**
   MS-ESI [M+H]⁺, calculated: 336, found: 336.
   ¹H NMR (400 MHz, CDCl₃) *δ* 7.34-7.42 (m, 1H), 7.08-7.16 (m, 1H), 6.85-6.95 (m, 1H), 6.77-6.83 (m, 1H), 4.30-4.37 (m, 1H), 4.18-4.26 (m, 1H), 4.11-4.15 (m, 5H), 2.75-2.79 (m, 2H), 2.48-2.59 (m, 2H), 2.42-2.45 (m, 2H), 2.28-2.37 (m, 1H), 2.05-2.12 (m, 1H), 1.25-1.27 (m, 3H), 1.20-1.24 (m, 3H).
(5) To a solution of compound **22-7** (3.00 g, 8.94 mmol) in ethanol (30.0 mL) was added paraformaldehyde (1.34 g) and sodium cyanoborohydride (1.69 g, 26.8 mmol), and the reaction solution was reacted for 16 hours at 25°C under nitrogen protection. Then the reaction solution was added with water (100 mL), and extracted with ethyl acetate (100 mL). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 4:1) to obtain compound **22-8.**
   MS-ESI [M+H]⁺, calculated: 350, found: 350.
   ¹H NMR (400 MHz, CDCl₃) *δ* 7.33-7.37 (m, 1H), 7.09-7.15 (m, 1H), 6.82-6.88 (m, 1H), 6.74-6.81 (m, 1H), 4.26-4.34 (m, 1H), 4.15-4.23 (m, 1H), 4.06-4.14 (m, 2H), 3.88-3.99 (m, 2H), 2.89-2.98 (m, 1H), 2.70-2.81 (m, 3H), 2.32-2.46 (m, 3H), 2.24-2.30 (m, 1H), 2.22-2.24 (m, 3H), 1.20-1.27 (m, 3H), 1.02-1.08 (t, 3H).
(6) To a solution of compound **22-8** (1.75 g, 5.01 mmol) in tetrahydrofuran (30.0 mL) was added potassium bis (trimethylsilyl) amide (1 mol/L, 15 mL, tetrahydrofuran solution) at -78°C, and the reaction solution was reacted at -78°C for 2 hours under nitrogen protection. Then the reaction solution was added with saturated ammonium chloride aqueous solution (70 mL), and extracted with ethyl acetate (500 mL × 1). The combined organic phase was washed with saturated NaCl aqueous solution (50 mL × 1), dried over anhydrous sodium sulfate, filtered, and concentrated under reduced pressure. The crude product was separated by silica gel column chromatography (petroleum ether/ethyl acetate = 1:0 to 6:1) to obtain compound **22-9.**
   MS-ESI [M+H]⁺, calculated: 304, found: 304.
(7) To a solution of compound **22-9** (1.03 g, 3.40 mmol) in ethanol (30.0 mL) was added compound **22-10**(516 mg, 6.79 mmol) and sodium ethoxide (693 mg, 10.1 mmol), and the reaction solution was stirred at 80°C for 12 hours under nitrogen protection. After concentrated under reduced pressure, the reaction solution was adjusted to pH 6 with hydrochloric acid (1 mol/L). The resulting solution was precipitated, and filtered, and the filter cake was dried to obtain compound **22-11.**
   MS-ESI [M+H]⁺, calculated: 316, found: 316.
   ¹H NMR (400 MHz, DMSO-d6) *δ* 11.54-12.84 (m, 2H), 7.37-7.42 (m, 1H), 7.12-7.18 (m, 1H), 6.92-6.99 (m, 1H), 6.75-6.81 (m, 1H), 4.22-4.31 (m, 1H), 3.92-4.01 (t, 1H), 3.51-3.60 (d, 1H), 3.02-3.11 (d, 1H), 2.70-2.77 (s, 2H), 2.03-2.13 (m, 1H), 1.88-1.96 (m, 3H), 1.54-1.63 (d, 1H).
(8) To a solution of intermediate **22-11** (1.05 g, 3.33 mmol) in water (100.0 mL) was added chloroacetic acid (1.26 g, 13.3 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **22-12.**
   MS-ESI [M+H]⁺, calculated: 300, found: 300.
(9) Compound **22-12** (1.70 g, 5.68 mmol) was dissolved in phosphorus oxychloride (16.5 g, 107 mmol), and the reaction solution was stirred at 100°C for 12 hours under nitrogen protection. Then the reaction solution was concentrated under reduced pressure, and extracted with dichloromethane (40 mL). The organic phase was washed with saturated sodium bicarbonate aqueous solution (20 mL × 2) and saturated NaCl aqueous solution (200 mL), dried over anhydrous sodium sulfate, filtered, and concentrated under reduced pressure to obtain compound **22-13.**
   MS-ESI [M+H]⁺, calculated: 336, found: 336.
(10) To a solution of compound **22-13** (1.00 g, 2.97 mmol) in *N*-methylpyrrolidone (10.0 mL) was added potassium carbonate (1.23 g, 8.92 mmol) and compound **22-14(846** mg, 3.57 mmol), and the reaction solution was stirred at 50°C for 12 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure to obtain compound **22-15.**
   MS-ESI [M+H]⁺, calculated: 525, found: 525.
(11) To a solution of compound **22-15** (700 mg, 1.33 mmol) in dioxane (10.0 mL) was added compound **22-16** (460 mg, 4.00 mmol), cesium carbonate (1.30 g, 4.00 mmol) and (2-dicyclohexylphosphino-2',6'-diisopropoxy-1,1'-biphenyl) [2-(2'-amino-1,1'- biphenyl)] palladium(II) methanesulfonate (223 mg, 266 µmol), and the reaction solution was stirred at 110°C for 6 hours under nitrogen protection. Then the reaction solution was added with ethyl acetate (40.0 mL), washed with saturated NaCl aqueous solution (40.0 mL × 2), dried over anhydrous sodium sulfate, filtered, and concentrated under reduced pressure. The crude product was separated by silica gel column chromatography (dichloromethane/methanol = 1:0 to 15:1) to obtain compound **22-17.**
   MS-ESI [M+H]⁺, calculated: 604, found: 604.
(12) To a solution of compound **22-17** (150.0 mg, 248 µmol) in dichloromethane (3.0 mL) was added trifluoroacetic acid (1.54g), and the reaction solution was stirred at 25°C for 1 hour. Then the reaction solution was extracted by ethyl acetate (50 mL × 2) and the organic phase was washed by saturated sodium bicarbonate aqueous solution (50 mL × 1) and saturated NaCl aqueous solution (50 mL× 1), dried over anhydrous sodium sulfate, filtered, and concentrated under reduced pressure to obtain crude trifluoroacetate of compound 22-18.
   MS-ESI [M+H]⁺, calculated: 504, found: 504.
(13) To a solution of trifluoroacetate of compound **22-18** (120.0 mg, 194 µmol) in dichloromethane (2.0 mL) was added triethylamine (14.7 mg, 145 µmol) and compound **22-19** (26.3 mg, 291 µmol), and the reaction solution was stirred at -78°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 0%-30%: 8 min) to obtain formate of compound **22.**
   MS-ESI [M+H]⁺, calculated: 558, found: 558.
   ¹H NMR (400 MHz, MeOD) *δ* 7.40-7.46 (m, 1H), 7.13-7.19 (m, 1H), 6.94-7.00 (m, 1H), 6.73-6.90 (m, 2H), 6.22-6.35 (m, 1H), 5.77-5.88 (m, 1H), 4.95-5.12 (m, 1H), 4.67-4.73 (m, 1H), 4.48-4.55 (m, 1H), 3.99-4.39 (m, 5H), 3.56-3.84 (m, 5H), 3.36-3.53 (m, 1H), 3.09-3.28 (m, 5H), 2.97-3.02 (m, 3H), 2.84-2.93 (m, 1H), 2.31-2.44 (m, 2H), 1.97-2.19 (m, 6H), 1.68-1.79 (m, 1H).

### Example 21 Synthesis of Compound 23

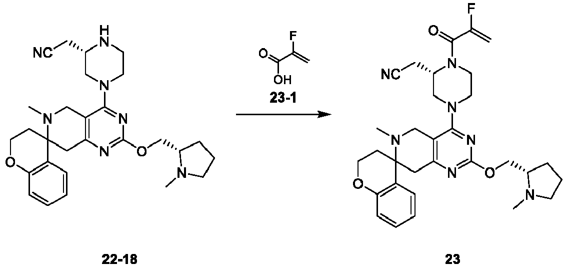

To a solution of compound **22-18** (220 mg, 356 µmol) in dichloromethane (2.0 mL) was added triethylamine (14.7 mg, 145 µmol), compound **23-1**(160.0 mg, 1.78 µmol) and propanephosphonic acid anhydride solution (679.98 mg, 1.07mol, 50% ethyl acetate ) at 0°C, and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Genimi NX C18, 150mm × 40mm 5 µm, A: water (0.05% hydrochloric acid); B: acetonitrile, 58%-35%: 10 min) to obtain hydrochloride of compound **23.**

MS-ESI [M+H]⁺, calculated: 576, found: 576.

¹H NMR (400 MHz, MeOD) *δ* 7.83-7.96 (m, 1H), 7.38-7.44 (m, 1H), 7.11-7.19 (m, 1H), 6.97-7.04 (m, 1H), 5.19-5.51 (m, 2H), 4.95-5.16 (m, 2H), 4.37-4.72 (m, 4H), 3.98-4.30 (m, 4H), 3.59-3.82 (m, 5H), 3.19-3.27 (m, 1H), 3.03-3.13 (m, 4H), 2.67-2.76 (m, 4H), 2.37-2.54 (m, 2H), 1.77-2.30 (m, 6H).

### Example 22 Synthesis of Compound 24

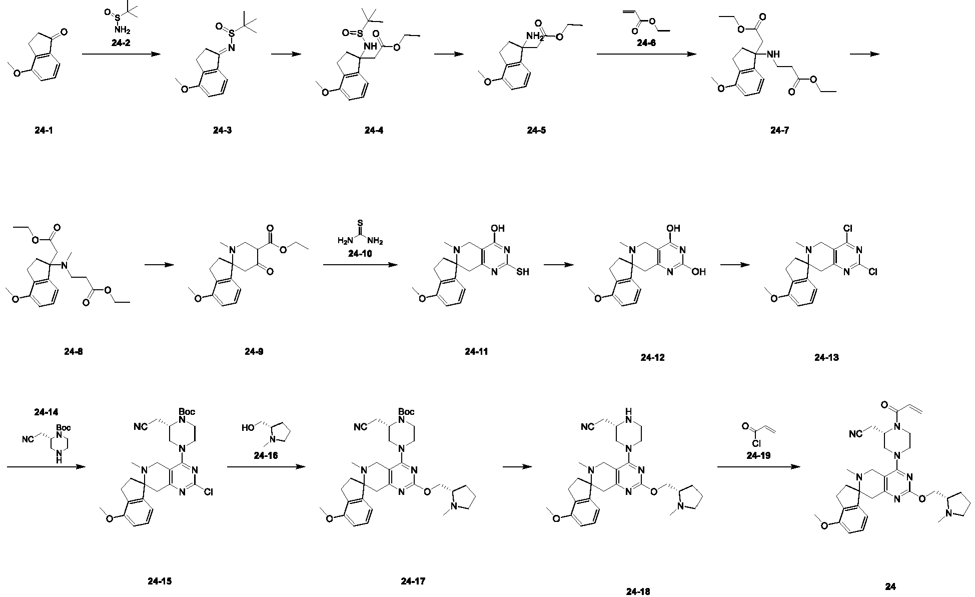

(1) To a solution of compound **24-1** (20.0 g, 123 mmol) in toluene (500 mL) was added compound **24-2** (19.4 g, 160 mmol), and tetraethyl titanate (56.2 g, 246 mmol), and the reaction solution was stirred at 120°C for 12 hours under nitrogen protection. Then the reaction solution was added with water (400 mL), and filtered. The filtrate was washed with ethyl acetate (400 mL × 2). The combined organic phase was washed with saturated NaCl aqueous solution (500 mL), dried over anhydrous sodium sulfate, filtered, and concentrated under reduced pressure. The crude product was separated by silica gel column chromatography (petroleum ether/ethyl acetate = 1:0 to 0:1) to obtain compound **24-3.**
   MS-ESI [M+H]⁺, calculated: 266, found: 266.
   ¹H NMR (400 MHz, CDCl₃) *δ* 7.38-7.43 (m, 1H), 7.28-7.34 (m, 1H), 6.93-6.98 (m, 1H), 3.87-3.90 (m, 3H), 3.40-3.51 (m, 1H), 3.01-3.08 (m, 3H), 1.31-1.34 (s, 9H).
(2) To a solution of ethyl acetate (15.8 g, 179 mmol) in tetrahydrofuran (500 mL) was added dropwise with lithium diisopropylamide (2 mol/L, 44.8 mL, tetrahydrofuran solution) at -78°C under nitrogen protection, and the reaction solution was stirred at -78°C for 1 hour. Then the reaction solution was added dropwise with a solution of compound **24-3** (11.9 g, 44.8 mmol) in tetrahydrofuran (500 mL), and continued stirring for 3 hours at -78°C. Then the reaction solution was quenched with saturated ammonium chloride aqueous solution(1000 mL), and extracted with ethyl acetate (1000 mL × 1). The combined organic phase was washed with saturated ammonium chloride aqueous solution (1000 mL) and saturated NaCl aqueous solution (1000 mL), dried over anhydrous sodium sulfate, filtered, and concentrated under reduced pressure. The crude product was separated by silica gel column chromatography (petroleum ether/ethyl acetate = 1:0 to 4:1) to obtain compound **24-4.**
   MS-ESI [M+H]⁺, calculated: 354, found: 354.
(3) To a solution of compound **24-4** (8.00 g, 22.6 mmol) in ethanol (50.0 mL) was added hydrochloric acid in dioxane (4 mol/L, 15.0 mL), and the reaction solution was stirred at 25°C for 2 hours under nitrogen protection. Then the reaction solution was concentrated under reduced pressure to obtain crude product of hydrochloride of compound **24-5.**
   MS-ESI [M-NH₂+H]⁺, calculated: 233, found: 233.
(4) To a solution of hydrochloride of compound **24-5** (6.40 g, 22.4 mmol) in ethanol (40.0 mL) was added compound **24-6** (22.4 g, 223 mmol), copper oxide (356 mg, 4.48 mmol), and triethylamine (2.27 g, 22.4 mmol), and the reaction solution was reacted at 85°C in a sealed tube for 8 hours under nitrogen protection. Then the reaction solution was added with water (100 mL), and extracted with ethyl acetate (200 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 6:1) to obtain compound **24-7.**
   MS-ESI [M+H]⁺, calculated: 350, found: 350.
(5) To a solution of compound **24-7** (3.80 g, 10.8 mmol) in ethanol (30.0 mL) was added paraformaldehyde (1.60 g) and sodium cyanoborohydride (2.05 g, 32.6 mmol), and the reaction solution was reacted for 16 hours at 25°C under nitrogen protection. Then the reaction solution was added with water (100 mL), and extracted with ethyl acetate (100 mL). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 4:1) to obtain compound **24-8.**
   MS-ESI [M+H]⁺, calculated: 364, found: 364.
   ¹H NMR (400 MHz, CDCl₃) *δ* 7.12-7.21 (m, 1H), 6.82-6.89 (m, 1H), 6.67-6.75 (m, 1H), 4.08-4.16 (m, 3H), 3.91-3.94 (m, 1H), 3.80-3.83 (m, 3H), 2.75-2.94 (m, 4H), 2.60-2.68 (m, 2H), 2.27-2.48 (m, 4H), 2.13-2.21 (m, 3H), 1.24-1.27 (m, 3H), 1.01-1.07 (t, 3H) .
(6) To a solution of compound **24-8** (4.60 g, 12.6 mmol) in tetrahydrofuran (100.0 mL) was added potassium bis (trimethylsilyl) amide (1 mol/L, 38 mL, tetrahydrofuran solution) at -78°C, and the reaction solution was reacted at -78°C for 2 hours under nitrogen protection. Then the reaction solution was added with saturated ammonium chloride aqueous solution (70 mL), and extracted with ethyl acetate (200 mL). The combined organic phase was washed with saturated NaCl aqueous solution (50 mL), dried over anhydrous sodium sulfate, filtered, and concentrated under reduced pressure. The crude product was separated by silica gel column chromatography (petroleum ether/ethyl acetate = 1:0 to 6:1) to obtain compound **24-9.**
   MS-ESI [M+H]⁺, calculated: 318, found: 318.
(7) To a solution of compound **24-9** (2.00 g, 6.30 mmol) in ethanol (30.0 mL) was added compound **24-10** (959 mg, 12.6 mmol) and sodium ethoxide (1.29 g, 18.9 mmol), and the reaction solution was stirred at 80°C for 12 hours under nitrogen protection. After concentrated under reduced pressure, the reaction solution was adjusted to pH 6 with hydrochloric acid (1 mol/L). The resulting solution was precipitated, and filtered. The filter cake was dried to obtain compound **24-11.**
   MS-ESI [M+H]⁺, calculated: 330, found: 330.
   ¹H NMR (400 MHz, DMSO-*d*6) *δ* 12.19-12.46 (m, 2H), 7.21-7.27 (m, 1H), 6.83-6.88 (m, 1H), 6.75-6.82 (m, 1H), 3.77-3.81 (m, 3H), 3.43-3.53 (m, 1H), 2.98-3.10 (m, 1H), 2.58-2.83 (m, 3H), 2.38-2.45 (m, 1H), 2.09-2.19 (m, 1H), 1.91-2.02 (s, 3H), 1.58-1.69 (m, 1H).
(8) To a solution of intermediate **24-11** (1.72 g, 5.22 mmol) in water (10.0 mL) was added chloroacetic acid (1.97 g, 20.8 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **24-12.**
   MS-ESI [M+H]⁺, calculated: 314, found: 314.
   ¹H NMR (400 MHz, DMSO-*d*6) *δ* 10.95-11.02 (s, 1H), 10.70-10.76 (s, 1H), 7.20-7.26 (t, 1H), 6.77-6.87 (dd, 2H), 3.78-3.81 (m, 3H), 3.37-3.46 (m, 1H), 2.96-3.04 (m, 1H), 2.55-2.87 (m, 3H), 2.27-2.35 (m, 1H), 2.09-2.20 (m, 1H), 1.94-1.99 (s, 3H), 1.57-1.68 (m, 1H).
(9) Compound **24-12** (1.34 g, 4.28 mmol) was dissolved in phosphorus oxychloride (16.5 g, 107 mmol), and the reaction solution was stirred at 100°C for 12 hours under nitrogen protection. Then the reaction solution was concentrated under reduced pressure, and extracted with dichloromethane (40 mL). The organic phase was washed with saturated sodium bicarbonate aqueous solution (20 mL × 2) and saturated NaCl aqueous solution (100 mL× 2), dried over anhydrous sodium sulfate, filtered, and concentrated under reduced pressure to obtain compound **24-13.**
   MS-ESI [M+H]⁺, calculated: 350, found: 350.
(10) To a solution of compound **24-13** (1.12 g, 3.20 mmol) in *N*-methylpyrrolidone (10.0 mL) was added potassium carbonate (1.33 g, 9.59 mmol) and compound**24-14** (864 mg, 3.84 mmol), and the reaction solution was stirred at 50°C for 12 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure to obtain compound **24-15.**
   MS-ESI [M+H]⁺, calculated: 539, found: 539.
(11) To a solution of compound **24-15** (700 mg, 1.30 mmol) in dioxane (10.0 mL) was added compound **24-16** (448 mg, 3.90 mmol), cesium carbonate (1.27 g, 3.90 mmol) and (2-dicyclohexylphosphino-2',6'-diisopropoxy-1,1'-biphenyl) [2-(2'-amino-1,1'- biphenyl)] palladium(II) methanesulfonate (217 mg, 259 µmol), and the reaction solution was stirred at 110°C for 6 hours under nitrogen protection. Then the reaction solution was added with ethyl acetate (40.0 mL), washed with saturated NaCl aqueous solution (40.0 mL × 2), dried over anhydrous sodium sulfate, filtered, and concentrated under reduced pressure. The crude product was separated by silica gel column chromatography (dichloromethane/methanol = 1:0 to 15:1) to obtain compound **24-17.**
   MS-ESI [M+H]⁺, calculated: 618, found: 618.
(12) To a solution of compound **24-17** (430 mg, 696 µmol) in dichloromethane (3.0 mL) was added trifluoroacetic acid (1.54g), and the reaction solution was stirred at 25°C for 1 hour under nitrogen protection. Then the reaction solution was extracted with ethyl acetate (50 mL × 2) and the organic phase was washed by saturated sodium bicarbonate aqueous solution (50 mL) and saturated NaCl aqueous solution (50 mL), dried over anhydrous sodium sulfate, filtered, and concentrated under reduced pressure to obtain crude trifluoroacetate of compound **24-18.**
   MS-ESI [M+H]⁺, calculated: 518, found: 518.
(13) To a solution of trifluoroacetate of compound **24-18**(100.0 mg, 158 µmol) in dichloromethane (2.0 mL) was added triethylamine (16.0 mg, 158 µmol) and compound **24-19** (21.4 mg, 237 µmol), and the reaction solution was stirred at -78°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm ×3 µm, A: water (0.225% formic acid); B: acetonitrile, 0%-30%: 8 min) to obtain formate of compound **24.**
   MS-ESI [M+H]⁺, calculated: 558, found: 572.
   ¹H NMR (400 MHz, MeOD) *δ* 7.24-7.31 (m, 1H), 6.78-6.94 (m, 3H), 6.23-6.36 (m, 1H), 5.79-5.88 (m, 1H), 4.61-4.86 (m, 2H), 4.52-4.60 (m, 1H), 3.91-4.36 (m, 4H), 3.76-3.90 (m, 6H), 3.58-3.74 (m, 2H), 3.06-3.28 (m, 4H), 3.01-3.04 (m, 3H), 2.86-3.00 (m, 4H), 2.35-2.52 (m, 2H), 2.28-2.32 (m, 3H), 1.92-2.21 (m, 4H).

### Example 23 Synthesis of Compound 25

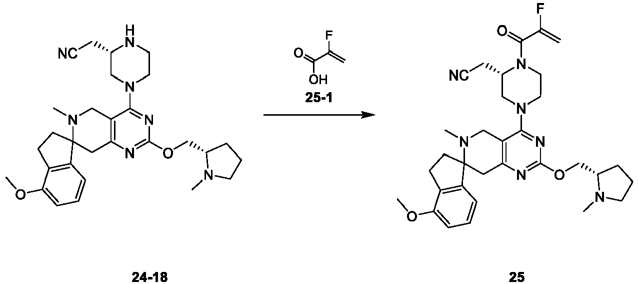

To a solution of compound **24-18** (100 mg, 158 µmol) in dichloromethane (2.0 mL) was added triethylamine (14.7 mg, 145 µmol), compound **25-1**(71.2 mg, 791 µmol) and propanephosphonic acid anhydride solution(302 mg, 474 µmol, 50% ethyl acetate solution) at 0°C, and the reaction solution was stirred at 25°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm ×3 µm, A: water (0.225% formic acid); B: acetonitrile, 0%-30%: 8 min) to obtain formate of compound **25.**
MS-ESI [M+H]⁺, calculated: 590, found: 590.
¹H NMR (400 MHz, MeOD) *δ* 7.21-7.32 (m, 1H), 6.83-6.90 (m, 2H), 5.23-5.43 (m, 2H), 4.69-4.74 (m, 1H), 4.50-4.57 (m, 1H), 4.00-4.36 (m, 3H), 3.63-3.88 (m, 8H), 3.40-3.58 (m, 1H), 2.81-3.29 (m, 12H), 2.34-2.50 (m, 2H), 2.23-2.28 (m, 3H), 1.89-2.21 (m, 4H).

### Example 24 Synthesis of Compounds 26 and 27

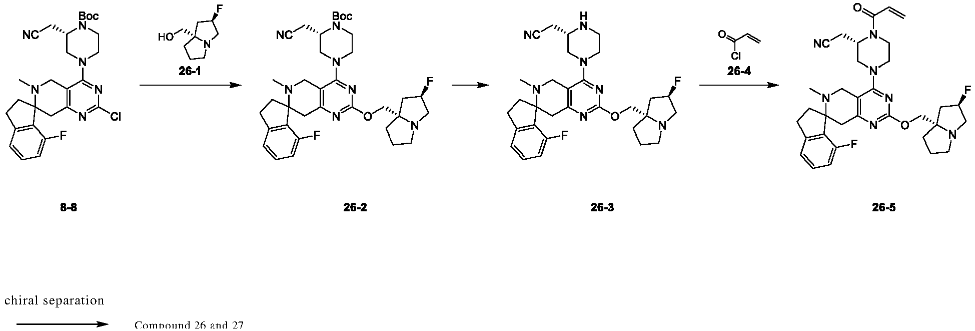

(1) To a solution of compound **8-8** (3.00 g, 5.69 mmol) in dioxane (80.0 mL) was added compound **26-1** (1.81 mg, 11.4 mmol), cesium carbonate (5.56 g, 17.1 mmol) and (2-dicyclohexylphosphino-2',6'-diisopropoxy-1,1'-biphenyl) [2-(2'-amino-1,1'-biphenyl)] palladium(II) methanesulfonate (300 mg, 359 µmol), and the reaction solution was stirred at 110°C for 4 hours under nitrogen protection. Then the reaction solution was added with ethyl acetate (200 mL), washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 8:1) to give compound **26-2.**
   MS-ESI [M+H]⁺, calculated: 650, found: 650.
(2) To a solution of compound **26-2** (3.24 g, 4.99 mmol) in dichloromethane (50.0 mL) was added hydrochloric acid in dioxane (1.25 mL, 4 mol/L), and the reaction solution was stirred at 25°C for 0.5 hours under nitrogen protection. Then the reaction solution was concentrated under reduced pressure to obtain crude product of hydrochloride of compound **26-3.**
   MS-ESI [M+H]⁺, calculated: 550, found: 550.
(3) To a solution of hydrochloride of compound **26-3** (2.60 g, 4.44 mmol) in dichloromethane (30.0 mL) was added triethylamine (1.35 g, 13.3 mmol) and compound **26-4** (803 mg, 8.87 µmol), and then the reaction solution was stirred at -78°C for 0.5 hours under nitrogen protection. Then the reaction solution was added with dichloromethane (60.0 mL), washed with saturated NaCl aqueous solution (30.0 mL × 2), dried over anhydrous sodium sulfate, filtered, and concentrated under reduced pressure. The crude product was separated by silica gel column chromatography (dichloromethane/methanol = 1:0 to 25:1) to obtain compound **26-5.**
(4) Compound **26-5** was separated by chiral supercritical fluid chromatography to obtain compounds **26** and **27.**

Separation conditions: Chromatographic column model: Chiralpak OX-3; Column specification: 100 × 4.6mm I.D., 3µm; Injection volume: 10 µL; Mobile phase: carbon dioxide: isopropyl alcohol (0.05% diethylamine) = 60 : 40; Detection wavelength: 254 nm; Column temperature: 35°C.

**Compound 6:** retention time 2.047 minutes, 99.28%ee.

MS-ESI [M+H]⁺, calculated: 604, found: 604.

¹H NMR (400 MHz, MeOD) *δ* 7.31 (td, *J* = 7.6, 5.2 Hz, 1H), 7.10 (d, *J* = 7.6 Hz, 1H), 6.91-6.97 (m, 1H), 6.71-6.90 (m, 1H), 6.28 (d, *J* = 16.4 Hz, 1H), 5.83 (d, *J* = 10.4 Hz, 1H), 5.19-5.37 (m, 1H), 5.03 (s, 1H), 4.54 (s, 1H), 4.16-4.22 (m, 1H), 4.10 (d, *J* = 10.4 Hz, 3H), 3.52-3.79 (m, 3H), 3.39-3.51 (m, 1H), 3.21-3.28 (m, 2H), 3.19 (d, *J* = 9.6 Hz, 2H), 3.11 (d, *J* = 3.2 Hz, 1H), 3.04-3.09 (m, 1H), 2.99-3.04 (m, 2H), 2.89-2.98 (m, 2H), 2.85 (s, 1H), 2.49 (dt, *J* = 13.2, 8.8 Hz, 1H), 2.27-2.32 (m, 3H), 2.19-2.27 (m, 1H), 2.12-2.19 (m, 1H), 2.05-2.12 (m, 1H), 1.93-2.02 (m, 2H), 1.84-1.93 (m, 2H).

**Compound 7:** retention time 3.558 minutes, 98.36% ee.

MS-ESI [M+H]⁺, calculated: 604, found: 604.

¹H NMR (400 MHz, MeOD) *δ* 7.32 (td, *J* = 7.6, 5.2 Hz, 1H), 7.10 (d, *J* = 7.6 Hz, 1H), 6.91-6.97 (m, 1H), 6.72-6.91 (m, 1H), 6.29 (d, *J* = 16.4 Hz, 1H), 5.83 (d, *J* = 10.4 Hz, 1H), 5.22-5.39 (m, 1H), 5.07 (s, 1H), 4.50-4.78 (m, 1H), 4.18-4.27 (m, 2H), 4.14 (d, *J* = 10.4 Hz, 1H), 3.91-4.13 (m, 2H), 3.62-3.74 (m, 2H), 3.37-3.61 (m, 1H), 3.25-3.29 (m, 2H), 3.20-3.24 (m, 2H), 3.08-3.20 (m, 3H), 3.06 (d, *J* = 5.2 Hz, 1H), 2.98-3.05 (m, 2H), 2.90 (d, *J* = 18.0 Hz, 1H), 2.48 (dt, *J* = 13.2, 8.4 Hz, 1H), 2.28-2.37 (m, 1H), 2.24-2.28 (m, 3H), 2.18-2.24 (m, 1H), 2.10-2.16 (m, 1H), 1.94-2.03 (m, 2H), 1.90 (ddd, *J* = 13.2, 8.4, 4.4 Hz, 2H).

### Example 25 Synthesis of Compound 28 and 29

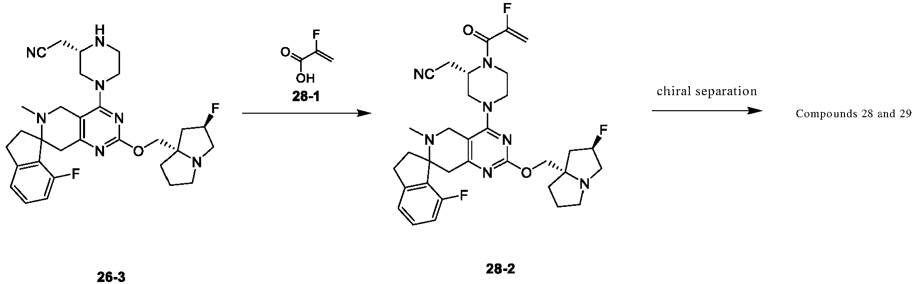

To a solution of compound **28-1** (848 mg, 9.42 mmol) in ethyl acetate (20.0 mL) was added 4A molecular sieve (1.80 g), triethylamine (1.59 g, 1.57 mmol), hydrochloride of compound **26-3**(1.84 g, 3.14 mmol) and propanephosphonic acid anhydride solution(5.99 g, 9.42 mmol, 50% ethyl acetate solution), and the reaction solution was stirred at 25°C for 0.5 hours. After filtration, the filter cake was washed with ethyl acetate (10.0 mL× 3), and the filtrate was washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 24:1) to obtain compound **28-2.**

(4) Compound **28-2** was separated by chiral supercritical fluid chromatography to obtain compounds **28** and **29.**

**Separation conditions:** Chromatographic column model: Chiralpak OX-3; Column specification: 50 × 4.6mm I.D., 3µm; Injection volume: 10 µL; Mobile phase: A: carbon dioxide, B: ethanol (0.05% diethylamine), B%: 5%-40% gradient elution for 2.5 minutes, 40% fixed concentration elution for 0.5 minutes, 5% fixed concentration elution for 1 minute; Detection wavelength: 254 nm; Column temperature: 35°C.

**Compound 28:** retention time 1.457 minutes, 100%ee.

MS-ESI [M+H]⁺, calculated: 622, found: 622.

¹H NMR (400 MHz, MeOD) *δ* 7.32 (td, *J* = 7.6, 5.2 Hz, 1H), 7.10 (d, *J* = 7.6 Hz, 1H), 6.90-6.97 (m, 1H), 5.34-5.41 (m, 1H), 5.28-5.34 (m, 1H), 5.19-5.28 (m, 1H), 4.92 (s, 1H), 4.79-4.88 (m, 1H), 4.15-4.23 (m, 1H), 4.13 (s, 1H), 4.06-4.11 (m, 2H), 3.90-4.06 (m, 1H), 3.69 (s, 2H), 3.45 (d, *J* = 13.2 Hz, 1H), 3.23-3.29 (m, 1H), 3.17-3.23 (m, 2H), 3.16 (d, *J* = 5.2 Hz, 1H), 3.10-3.15 (m, 1H), 3.06-3.10 (m, 1H), 3.04 (d, *J* = 8.8 Hz, 2H), 2.98-3.01 (m, 1H), 2.93-2.98 (m, 1H), 2.87 (d, *J* = 18.0 Hz, 1H), 2.49 (dt, *J* = 13.2, 8.8 Hz, 1H), 2.27-2.32 (m, 3H), 2.19-2.27 (m, 1H), 2.13-2.18 (m, 1H), 2.06-2.12 (m, 1H), 1.96-2.02 (m, 1H), 1.92-1.96 (m, 1H), 1.88-1.92 (m, 1H), 1.79-1.88 (m, 1H).

**Compound 29:** retention time 1.588 minutes, 95.44%ee.

MS-ESI [M+H]⁺, calculated: 622, found: 622.

¹H NMR (400 MHz, MeOD) *δ* 7.32 (td, *J* = 7.6, 5.2 Hz, 1H), 7.11 (d, *J* = 7.6 Hz, 1H), 6.91-6.97 (m, 1H), 5.38 (s, 1H), 5.29-5.35 (m, 1H), 5.25 (d, *J* = 13.6 Hz, 1H), 4.91 (s, 1H), 4.86-4.87 (m, 1H), 4.24 (d, *J* = 14.0 Hz, 1H), 4.14-4.22 (m, 2H), 3.96-4.12 (m, 2H), 3.63-3.71 (m, 2H), 3.27 (d, *J* = 3.6 Hz, 2H), 3.24 (d, *J* = 3.2 Hz, 2H), 3.20 (s, 1H), 3.13-3.18 (m, 2H), 3.06-3.13 (m, 2H), 2.99-3.06 (m, 2H), 2.89 (d, *J* = 18.0 Hz, 1H), 2.44-2.51 (m, 1H), 2.28 (s, 3H), 2.22-2.26 (m, 1H), 2.19 (s, 1H), 2.12 (d, *J* = 9.2 Hz, 1H), 1.96-2.04 (m, 2H), 1.90 (td, *J* = 8.8, 4.4 Hz, 2H).

### Example 26 Synthesis of Compound 30

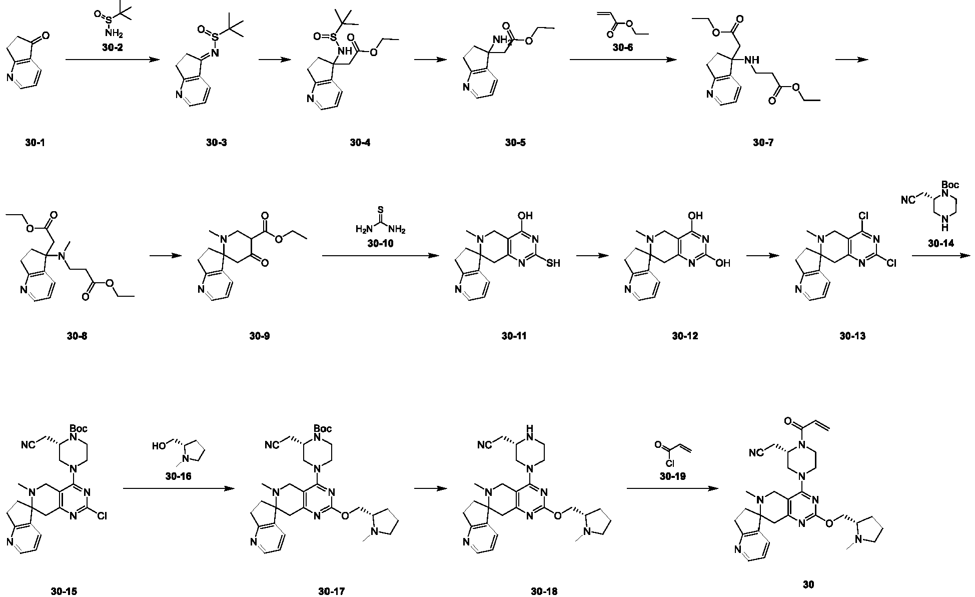

(1) To a solution of compound **30-1** (20.0 g, 150 mmol) in toluene (500 mL) was added compound **30-2** (21.9 g, 181 mmol), and tetraethyl titanate (68.5 g, 300 mmol), and then the reaction solution was stirred at 110°C for 12 hours under nitrogen protection. Then the reaction solution was added with water (200 mL) and ethyl acetate (500 mL). After filtration, the filter cake was washed with ethyl acetate (500 mL × 4). The combined organic phase was washed with saturated NaCl aqueous solution (500 mL × 2), dried over anhydrous sodium sulfate, filtered, and concentrated under reduced pressure. The crude product was separated by silica gel column chromatography (petroleum ether/ethyl acetate = 1:0 to 1:2) to obtain compound **30-3.**
   MS-ESI [M+H]⁺, calculated: 237, found: 237.
   ¹H NMR (400 MHz, CDCl₃) *δ* 8.71 (dd, *J* = 4.8, 1.6 Hz, 1H), 8.05 (dd, *J* = 7.6, 1.6 Hz, 1H), 7.28 (dd, *J* = 8.0, 4.8 Hz, 1H), 3.49-3.59 (m, 1H), 3.25-3.31 (m, 2H), 3.11-3.20 (m, 1H), 1.33 (s, 9H).
(2) To a solution of ethyl acetate (24.3 g, 276 mmol) in tetrahydrofuran (150 mL) was added dropwise with lithium diisopropylamide (2 mol/L, 55mL, tetrahydrofuran solution) at -65°C under nitrogen protection, and the reaction solution was stirred at -65°C for 1 hour. Then the reaction solution was added dropwise with a solution of compound **30-3** (13.0 g, 55.0 mmol) in tetrahydrofuran (150 mL), and then continued stirring for 2 hours at -65°C. After warming to 0°C, the reaction solution was quenched with saturated ammonium chloride aqueous solution (200 mL), and extracted with ethyl acetate (200 mL × 2). The combined organic phase was washed with saturated ammonium chloride aqueous solution (200 mL × 1) and saturated NaCl aqueous solution (200 mL × 1), dried over anhydrous sodium sulfate, filtered, and concentrated under reduced pressure. The crude product was separated by silica gel column chromatography (dichloromethane/ methanol = 1:0 to 32:1) to obtain compound **30-4.**
   MS-ESI [M+H]⁺, calculated: 325, found: 325.
   ¹H NMR (400 MHz, MeOD) *δ* 8.42 (d, *J* = 5.2 Hz, 1H), 7.77-7.83 (m, 1H), 7.25-7.33 (m, 1H), 4.08-4.19 (m, 2H), 3.17-3.25 (m, 1H), 2.97-3.15 (m, 2H), 2.85-2.97 (m, 1H), 2.60-2.69 (m, 1H), 2.41-2.54 (m, 1H), 1.17-1.22 (m, 12H).
(3) To a solution of compound **30-4** (7.40 g, 22.8 mmol) in ethanol (60.0 mL) was added hydrochloric acid in dioxane (4 mol/L, 20.0 mL), and the reaction solution was stirred at 25°C for 1 hour under nitrogen protection. Then the reaction solution was concentrated under reduced pressure to obtain crude product of hydrochloride of compound **30-5.**
   MS-ESI [M+H]⁺, calculated: 221, found: 221.
(4) To a solution of hydrochloride of compound **30-5** (5.86 g, 22.8 mmol) in ethanol (50.0 mL) was added compound **5-6** (23.1 g, 231 mmol), copper oxide (363 mg, 4.56 mmol), and triethylamine (6.94 g, 68.6 mmol), and the reaction solution was reacted at 85°C in a sealed tube for 8 hours under nitrogen protection. Then the reaction solution was added with water (200 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 2:1) to obtain compound **30-7.**
   MS-ESI [M+H]⁺, calculated: 321, found: 321.
   ¹H NMR (400 MHz, MeOD) *δ* 8.37 (d, *J* = 4.4 Hz, 1H), 7.75 (dd, *J* = 7.6, 1.2 Hz, 1H), 7.26 (dd, *J* = 7.6, 5.2 Hz, 1H), 4.13 (q, *J* = 7.2 Hz, 2H), 4.06 (q, *J* = 7.2 Hz, 2H), 2.97-3.10 (m, 2H), 2.82-2.87 (m, 1H), 2.75-2.80 (m, 1H), 2.71 (dt, *J* = 10.8, 6.0 Hz, 1H), 2.48-2.59 (m, 1H), 2.43-2.48 (m, 2H), 2.28-2.39 (m, 2H), 1.24 (t, *J* = 7.2 Hz, 3H), 1.15 (t, *J* = 7.2 Hz, 3H).
(5) To a solution of compound **30-7** (1.00 g, 3.12 mmol) in ethanol (15.0 mL) was added paraformaldehyde (937 mg) and sodium cyanoborohydride (589 mg, 9.37 mmol), and the reaction solution was reacted for 14 hours at 25°C under nitrogen protection. Then the reaction solution was added with water (100 mL), and extracted with ethyl acetate (100 mL × 1). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **30-8.**
   MS-ESI [M+H]⁺, calculated: 335, found: 335.
   ¹H NMR (400 MHz, MeOD) *δ*8.36 (dd, *J* = 5.2, 1.6 Hz, 1H), 7.72 (dd, *J* = 7.6, 1.6 Hz, 1H), 7.25 (dd, *J* = 7.6, 5.2 Hz, 1H), 4.07-4.13 (m, 2H), 3.90 (qd, *J* = 7.2, 1.2 Hz, 2H), 2.98-3.04 (m, 2H), 2.94-2.97 (m, 1H), 2.84-2.89 (m, 1H), 2.64-2.71 (m, 1H), 2.57-2.63 (m, 1H), 2.40-2.46 (m, 3H), 2.25-2.32 (m, 1H), 2.20 (s, 3H), 1.24 (t, *J* = 7.2 Hz, 3H), 1.02 (t, *J* = 7.2 Hz, 3H).
(6) To a solution of compound **30- 8** (900 mg, 2.69 mmol) in tetrahydrofuran (20.0 mL) was added potassium bis (trimethylsilyl) amide (1 mol/L, 8.0 mL, tetrahydrofuran solution) at -65°C, and the reaction solution was reacted at -65°C for 2 hours under nitrogen protection. Then the reaction solution was added with saturated ammonium chloride aqueous solution(100 mL), and extracted with ethyl acetate (100 mL × 1). The organic phase was washed successively with saturated ammonium chloride aqueous solution (100 mL × 1) and saturated NaCl aqueous solution (100 mL × 1), dried over anhydrous sodium sulfate, filtered, and concentrated under reduced pressure. The crude product was separated by silica gel column chromatography (petroleum ether/ethyl acetate = 1:0 to 3:1) to obtain compound 30-9.
   MS-ESI [M+H]⁺, calculated: 289, found: 289.
   ¹H NMR (400 MHz, MeOD) *δ* 8.42 (dd, *J* = 5.2, 1.6 Hz, 1H), 7.71 (dd, *J* = 7.6, 1.6 Hz, 1H), 7.32 (dd, *J* = 7.6, 5.2 Hz, 1H), 4.28 (q, *J* = 7.2 Hz, 2H), 3.45 (d, *J* = 15.2 Hz, 1H), 3.32-3.39 (m, 1H), 3.22-3.28 (m, 1H), 3.04-3.11 (m, 2H), 2.58-2.69 (m, 1H), 2.44-2.49 (m, 1H), 2.34-2.40 (m, 1H), 2.07 (s, 3H), 1.85-1.93 (m, 1H), 1.32 (t, *J* = 7.2 Hz, 3H).
(7) To a solution of compound **30-9** (510 mg, 1.77 mmol) in ethanol (8.0 mL) was added compound **30-10** (269 mg, 3.53 mmol) and sodium ethoxide (361 mg, 5.30 mmol), and the reaction solution was stirred at 80°C for 16 hours under nitrogen protection. After concentrated under reduced pressure, the reaction solution was adjusted to pH 6 with hydrochloric acid (1 mol/L). The resulting solution was precipitated, and filtered. The filter cake was dried to obtain compound **30-11.**
   MS-ESI [M+H]⁺, calculated: 301, found: 301.
   ¹H NMR (400 MHz, DMSO-*d*₆) *δ* 12.77 (s, 1H), 12.64 (s, 1H), 8.66-8.73 (m, 1H), 8.59 (s, 1H), 7.56 (s, 1H), 4.10 (s, 1H), 3.96 (d, *J* = 14.4 Hz, 1H), 3.43 (d, *J* = 18.8 Hz, 4H), 3.18 (s, 2H), 2.89 (d, *J* = 16.0 Hz, 1H), 2.67 (s, 1H), 2.20 (s, 1H).
(8) To a solution of intermediate **30-11** (531 mg, 1.77 mmol) in water (10.0 mL) was added chloroacetic acid (670 mg, 7.09 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **30-12.**
   MS-ESI [M+H]⁺, calculated: 285, found: 285.
   ¹H NMR (400 MHz, DMSO-d6) *δ* 10.92 (s, 2H), 8.44 (dd, *J* = 4.8, 1.6 Hz, 1H), 7.58 (dd, *J* = 7.6, 1.6 Hz, 1H), 7.24 (dd, *J* = 7.6, 4.8 Hz, 1H), 3.39 (s, 1H), 3.07 (d, *J* = 16.0 Hz, 1H), 2.90-2.99 (m, 2H), 2.61 (d, *J* = 17.2 Hz, 1H), 2.39 (d, *J* = 17.6 Hz, 1H), 2.21 (dt, *J* = 13.6, 8.4 Hz, 1H), 1.96 (s, 3H), 1.70 (ddd, *J* = 13.6, 8.0, 5.6 Hz, 1H).
(9) Compound **30-12** (300 mg, 1.06 mmol) was dissolved in phosphorus oxychloride (9.90 g, 64.6 mmol), and the reaction solution was stirred at 100°C in a sealed tube under nitrogen protection for 12 hours. Then the reaction solution was concentrated under reduced pressure, slowly added with iced water(50 mL), and extracted with dichloromethane (50 mL × 1). The organic phase was washed with saturated sodium carbonate solution (20 mL × 2) and saturated NaCl aqueous solution (20 mL × 1), dried over anhydrous sodium sulfate, filtered, and concentrated under reduced pressure to obtain compound **30-13.**
   MS-ESI [M+H]⁺, calculated: 321, found: 321.
(10) To a solution of compound **30-13** (338 mg, 1.05 mmol) in *N*-methylpyrrolidone (5.0 mL) was added potassium carbonate (436 mg, 3.15 mmol) and compound **30-14**(284 mg, 1.26 mmol), and the reaction solution was stirred at 50°C for 3 hours under nitrogen protection. Then the reaction solution was poured into water (30.0 mL) and extracted with ethyl acetate (30.0 mL × 2). The organic phases were combined, washed with saturated NaCl aqueous solution (30.0 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **30-15.**
   MS-ESI [M+H]⁺, calculated: 510, found: 510.
   ¹H NMR (400 MHz, MeOD) *δ* 8.44 (d, *J* = 4.4 Hz, 1H), 7.65-7.74 (m, 1H), 7.28-7.37 (m, 1H), 4.43 (s, 2H), 3.98-4.08 (m, 2H), 3.87 (d, *J* = 14.4 Hz, 2H), 3.68-3.80 (m, 2H), 2.97 (d, *J* = 5.2 Hz, 2H), 2.93 (s, 2H), 2.83-2.87 (m, 2H), 2.82 (d, *J* = 4.0 Hz, 2H), 2.45-2.54 (m, 1H), 2.21 (s, 3H), 1.48 (s, 9H).
(11) To a solution of compound **30-15** (300 mg, 588 µmol ) in dioxane (10.0 mL) was added compound **30-16** (203 mg, 1.76 mmol), cesium carbonate (575 mg, 1.76 mmol ) and (2-dicyclohexylphosphino-2',6'-diisopropoxy-1,1'-biphenyl) [2-(2'-amino-1,1'- biphenyl)] palladium(II) methanesulfonate (98.4 mg, 118 µmol), and the reaction solution was stirred at 110°C for 4 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 5:1) to obtain compound **30-17.**
   MS-ESI [M+H]⁺, calculated: 589, found: 589.
(12) To a solution of compound **30-17** (110 mg, 187 µmol) in dichloromethane (3.0 mL) was added trifluoroacetic acid (1.54 g, 13.5 mmol), and the reaction solution was stirred at 25°C for 1 hour under nitrogen protection. Then the reaction solution was concentrated under reduced pressure to obtain crude trifluoroacetate of compound **30-18.**
   MS-ESI [M+H]⁺, calculated: 489, found: 489.
(13) To a solution of trifluoroacetate of compound **30-18**(90.0 mg, 149 µmol) in dichloromethane (5.0 mL) was added triethylamine (45.0 mg, 445 µmol), then cooled to -78°C and added with compound **30-19** (27.0 mg, 298 µmol), and the reaction solution was stirred at -65°C for 0.5 hours under nitrogen protection. Then the reaction solution was added with dichloromethane (30.0 mL), washed with saturated NaCl aqueous solution (30.0 mL × 2), dried over anhydrous sodium sulfate, filtered, and concentrated under reduced pressure. The crude product was separated by preparative high performance liquid chromatography (Gemini-NX C18, 75 × 30mm× 3 µm, A: water (10 mmM ammonium bicarbonate); B: acetonitrile, 10%-40%: 10 min) to obtain compound **30.**
   MS-ESI [M+H]⁺, calculated: 543, found: 543.
   ¹H NMR (400 MHz, MeOD) *δ* 8.44 (d, *J* = 4.8 Hz, 1H), 7.62-7.75 (m, 1H), 7.27-7.37 (m, 1H), 6.83 (d, *J* = 13.2 Hz, 1H), 6.29 (d, *J* = 16.4 Hz, 1H), 5.84 (d, *J* = 10.4 Hz, 1H), 4.43-4.68 (m, 1H), 4.29-4.41 (m, 2H), 4.19 (d, *J* = 14.0 Hz, 1H), 4.10 (s, 1H), 4.00 (d, *J* = 10.0 Hz, 1H), 3.71-3.82 (m, 2H), 3.44-3.64 (m, 1H), 3.27 (s, 1H), 3.15-3.23 (m, 1H), 3.04-3.15 (m, 4H), 2.88-2.99 (m, 3H), 2.74 (dt, *J* = 14.0, 6.8 Hz, 1H), 2.50-2.56 (m, 1H), 2.49 (d, *J* = 2.4 Hz, 3H), 2.35 (qd, *J* = 8.8, 2.0 Hz, 1H), 2.21 (s, 3H), 2.09 (dq, *J* = 12.4, 8.4 Hz, 1H), 1.91-2.01 (m, 1H), 1.77-1.86 (m, 2H), 1.63-1.75 (m, 1H).

### Example 27 Synthesis of Compound 31

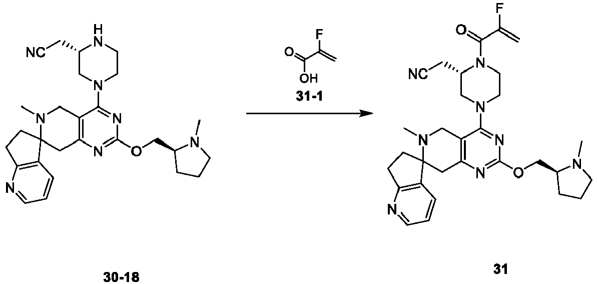

To a solution of compound **31-1** (58.3 mg, 648 µmol) in dichloromethane (5.0 mL) was added 4A molecular sieve(130 mg), triethylamine (65.6 mg, 648 µmol), trifluoroacetate of compound **30-18**(130 mg, 216 µmol) and propanephosphonic acid anhydride solution(549 mg, 863 µmol, 50% ethyl acetate solution), and the reaction solution was stirred at 25°C for 0.5 hours. After filtration, the filter cake was washed with ethyl acetate (10.0 mL × 3), and the filtrate was washed with saturated NaCl aqueous solution (20.0 mL × 2), dried over anhydrous sodium sulfate, filtered, and concentrated under reduced pressure. The crude product was separated by preparative high performance liquid chromatography (Phenomenex Gemini-NX C18, 75 mm × 30 mm 3 µm, A: water (10 mmol/L ammonium bicarbonate); B: acetonitrile, 25%-50%: 8 minutes) to obtain compound **31.**

MS-ESI [M+H]⁺, calculated: 561, found: 561.

¹H NMR (400 MHz, MeOD) *δ* 8.44 (d, *J* = 4.8 Hz, 1H), 7.69 (ddd, *J* = 13.2, 7.6, 1.6 Hz, 1H), 7.33 (dt, *J* = 7.6, 5.2 Hz, 1H), 5.32-5.44 (m, 1H), 5.20-5.32 (m, 1H), 4.28-4.39 (m, 2H), 4.14-4.26 (m, 1H), 4.10 (d, *J* = 5.2 Hz, 1H), 3.93-4.07 (m, 1H), 3.63-3.80 (m, 2H), 3.37-3.57 (m, 1H), 3.32-3.34 (m, 1H), 3.20-3.30 (m, 1H), 3.04-3.20 (m, 5H), 2.95-3.04 (m, 1H), 2.94 (d, *J* = 6.4 Hz, 2H), 2.76 (dt, *J* = 13.6, 6.8 Hz, 1H), 2.50 (d, *J* = 2.4 Hz, 3H), 2.47 (d, *J* = 3.6 Hz, 1H), 2.33-2.42 (m, 1H), 2.21 (d, *J* = 1.2 Hz, 3H), 2.09 (dq, *J* = 12.4, 8.4 Hz, 1H), 1.91-2.01 (m, 1H), 1.77-1.87 (m, 2H), 1.65-1.76 (m, 1H).

### Example 28 Synthesis of Compounds 32 and 33

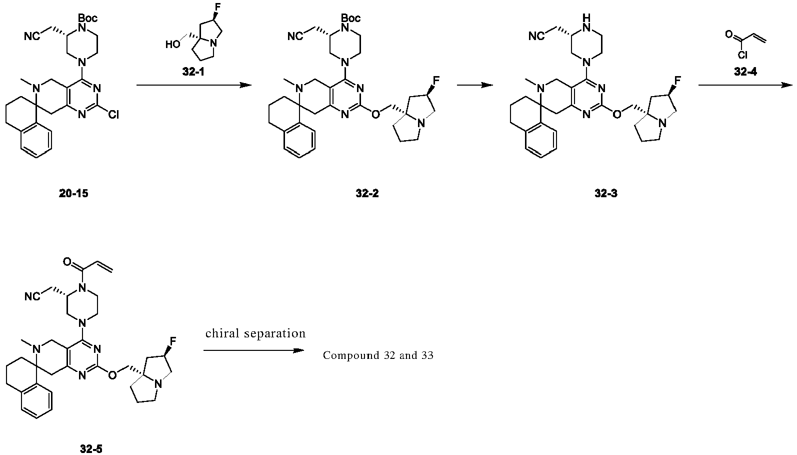

(1) To a solution of compound **20-15** (800 mg, 1.53 mmol) in dioxane (15.0 mL) was added compound **32-1** (487 mg, 3.06 mmol), cesium carbonate (382 mg, 2.76 mmol) and (2-dicyclohexylphosphino-2',6'-diisopropoxy-1,1'-biphenyl) [2-(2'-amino-1,1'- biphenyl)] palladium methanesulfonate (80.0 mg, 95.7 µmol), and the reaction solution was stirred at 110°C for 3 hours under nitrogen protection. Then the reaction solution was added with ethyl acetate (80.0 mL), washed with water (50.0 mL × 1) and saturated NaCl aqueous solution (50.0 mL × 3), dried over anhydrous sodium sulfate, filtered, and concentrated under reduced pressure. The crude product was separated by silica gel column chromatography (dichloromethane/methanol = 1:0 to 16:1) to obtain compound **32-2.**
   MS-ESI [M+H]⁺, calculated: 646, found: 646.
   ¹H NMR (400 MHz, CDCl₃) *δ* 7.53 (t, *J* = 7.2 Hz, 1H), 7.19-7.24 (m, 1H), 7.14 (t, *J* = 6.8 Hz, 1H), 7.06-7.09 (m, 1H), 4.59 (s, 1H), 4.09 (s, 2H), 3.96 (s, 1H), 3.63-3.83 (m, 3H), 3.34 (dd, *J* = 14.0, 3.2 Hz, 1H), 3.23 (s, 2H), 3.16 (d, *J* = 8.0 Hz, 1H), 3.05-3.14 (m, 3H), 2.79-3.05 (m, 4H), 2.74 (d, *J* = 5.6 Hz, 2H), 2.69 (d, *J* = 2.8 Hz, 1H), 2.27 (s, 1H), 2.21 (s, 1H), 2.10-2.16 (m, 3H), 1.89-1.99 (m, 4H), 1.84 (d, *J* = 10.0 Hz, 2H), 1.77 (s, 1H), 1.67-1.76 (m, 2H), 1.51 (s, 9H).
(2) To a solution of compound **32-2** (810 g, 1.25 mmol) in dichloromethane (10.0 mL) was added hydrochloric acid in dioxane (3.12 mL, 4 mol/L), and the reaction solution was stirred at 25°C for 1 hour under nitrogen protection. Then the reaction solution was concentrated under reduced pressure to obtain crude product of hydrochloride of compound **32-3.**
   MS-ESI [M+H]⁺, calculated: 546, found: 546.
(3) To a solution of hydrochloride of compound **32-3**(830 g, 1.43 mmol) in dichloromethane (3.0 mL) was added triethylamine (433 mg, 4.28 mmol) and compound **32-4** (258 mg, 2.85 mmol), and then the reaction solution was stirred at -78°C for 1 hour under nitrogen protection. Then the reaction solution was added with dichloromethane (60.0 mL), washed with saturated NaCl aqueous solution (30.0 mL × 3), dried over anhydrous sodium sulfate, filtered, and concentrated under reduced pressure. The crude product was separated by silica gel column chromatography (dichloromethane/methanol = 1:0 to 16:1) to obtain compound **32-5.**
(4) Compound **32-5** was separated by chiral supercritical fluid chromatography to obtain compounds **32** and **33.**

Separation conditions: Chromatographic column model:Chiralpak OX-3; Column specification: 50 × 4.6mm I.D., 3µm; Injection volume: 10 µL; Mobile phase: A: Carbon dioxide, B: Ethanol (0.05% diethylamine), B%: 5%-40% gradient elution for 2.5 minutes, 40% fixed concentration elution for 0.5 minutes, 5% fixed concentration elution for 1 minute; Detection wavelength: 254 nm; Column temperature: 35°C.

**Compound32:** retention time 1.759 minutes, 100%ee.

MS-ESI [M+H]⁺, calculated: 600, found: 600.

¹H NMR (400 MHz, MeOD) *δ* 7.51 (d, *J* = 8.0 Hz, 1H), 7.19-7.25 (m, 1H), 7.13-7.18 (m, 1H), 7.07-7.12 (m, 1H), 6.80 (s, 1H), 6.29 (d, *J* = 16.4 Hz, 1H), 5.83 (d, *J* = 10.4 Hz, 1H), 5.21-5.42 (m, 1H), 5.02 (s, 1H), 4.23-4.53 (m, 1H), 4.22 (s, 1H), 4.13 (d, *J* = 8.4 Hz, 2H), 4.09 (s, 1H), 3.75-3.87 (m, 2H), 3.57-3.75 (m, 1H), 3.35-3.57 (m, 2H), 3.15 (d, *J* = 15.2 Hz, 1H), 3.04-3.12 (m, 2H), 2.93-3.04 (m, 2H), 2.88 (s, 2H), 2.76 (d, *J* = 5.6 Hz, 2H), 2.26-2.39 (m, 1H), 2.13-2.26 (m, 2H), 2.12 (s, 3H), 2.03-2.10 (m, 1H), 2.01 (d, *J* = 8.4 Hz, 2H), 1.92-1.99 (m, 2H), 1.90 (d, *J* = 6.8 Hz, 1H), 1.66-1.84 (m, 2H).

**Compound33:** retention time: 1.966 minutes, 99.36%ee.

MS-ESI [M+H]⁺, calculated: 600, found: 600.

¹H NMR (400 MHz, MeOD) *δ* 7.51 (d, *J* = 7.6 Hz, 1H), 7.19-7.25 (m, 1H), 7.13-7.18 (m, 1H), 7.07-7.12 (m, 1H), 6.82 (d, *J* = 10.0 Hz, 1H), 6.29 (d, *J* = 16.4 Hz, 1H), 5.84 (d, *J* = 10.4 Hz, 1H), 5.25-5.43 (m, 1H), 5.07 (s, 1H), 4.32 (d, *J* = 14.0 Hz, 1H), 4.18-4.29 (m, 2H), 4.11 (d, *J* = 10.0 Hz, 1H), 4.00 (d, *J* = 11.2 Hz, 1H), 3.70-3.85 (m, 2H), 3.46-3.69 (m, 1H), 3.36-3.45 (m, 2H), 3.20 (dd, *J* = 14.0, 3.6 Hz, 2H), 3.14 (d, *J* = 8.4 Hz, 1H), 3.07-3.10 (m, 1H), 3.03 (s, 1H), 2.99 (s, 1H), 2.94 (s, 1H), 2.74-2.81 (m, 2H), 2.29-2.43 (m, 1H), 2.23-2.29 (m, 1H), 2.16-2.23 (m, 1H), 2.12 (s, 3H), 2.08 (d, *J* = 10.0 Hz, 1H), 2.04 (d, *J* = 5.2 Hz, 2H), 1.95-2.00 (m, 2H), 1.92 (d, *J* = 6.8 Hz, 1H), 1.71-1.83 (m, 2H).

### Example 29 Synthesis of Compound 34

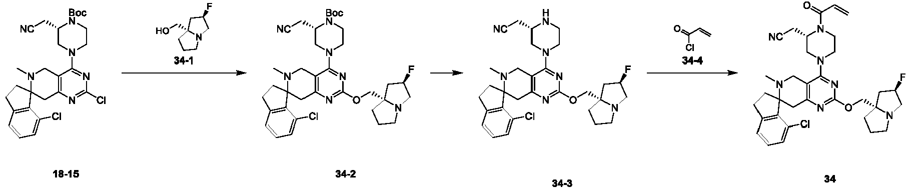

(1) To a solution of compound **18-15** (500 mg, 920 µmol) in dioxane (10.0 mL) was added compound **34-1** (220 mg,1.38mmol), potassium carbonate (382 mg, 2.76 mmol), dicyclohexyl(2',4',6'-triisopropyl-[1,1'-biphenyl]-2-yl)phosphine (176 mg, 369 µmol) and bis (dibenzylidene acetone) palladium(169 mg, 185 µmol), and the reaction solution was stirred at 110°C for 4 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 10:1) to obtain compound **34-2.**
   MS-ESI [M+H]⁺, calculated: 666, found: 666.
(2) To a solution of compound **34-2** (485 g, 728 µmol) in dichloromethane (6.0 mL) was added trifluoroacetic acid (2.0 mL), and the reaction solution was stirred at 25°C for 1 hour under nitrogen protection. Then the reaction solution was concentrated under reduced pressure to obtain crude trifluoroacetate of compound **34-3.**
   MS-ESI [M+H]⁺, calculated: 566, found: 566.
(3) To a solution of trifluoroacetate of compound **34-3**(150 g, 221 µmol) in dichloromethane (3.0 mL) was added triethylamine (67.0 mg, 662 µmol) and compound **34-4** (39.9 mg, 441 µmol), and the reaction solution was stirred at -65°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm 3 µm, A: water (0.225% formic acid); B: acetonitrile, 7%-27%: 7 min) to obtain formate of compound **34.**
   MS-ESI [M+H]⁺, calculated: 620, found: 620.
   ¹H NMR (400 MHz, MeOD) *δ* 7.20-7.30 (m, 3H), 6.82 (d, *J* = 11.6 Hz, 1H), 6.29 (d, *J* = 16.8 Hz, 1H), 5.84 (d, *J* = 10.4 Hz, 1H), 5.41-5.64 (m, 1H), 5.03 (s, 1H), 4.38-4.64 (m, 3H), 4.02-4.23 (m, 2H), 3.83-3.95 (m, 1H), 3.76-3.83 (m, 2H), 3.71-3.76 (m, 2H), 3.50-3.70 (m, 1H), 3.32-3.44 (m, 3H), 3.08-3.28 (m, 2H), 2.97-3.08 (m, 2H), 2.76-2.96 (m, 2H), 2.73 (d, *J* = 18.0 Hz, 1H), 2.55-2.68 (m, 1H), 2.43-2.55 (m, 2H), 2.28-2.41 (m, 2H), 2.25 (d, *J* = 2.4 Hz, 3H), 2.10-2.20 (m, 1H), 1.77-1.91 (m, 1H).

### Example 30 Synthesis of Compound 35

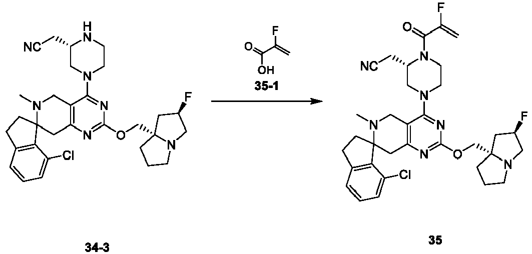

To a solution of compound **35-1** (108 mg, 1.20mmol) in ethyl acetate(10.0 mL) was added 4A molecular sieve(270 mg), triethylamine(121 mg, 1.20 mmol), trifluoroacetic acid of compound **34-3** (270 mg, 397 µmol) and propanephosphonic acid anhydride solution (1.02 g, 1.60 mmol, 50% ethyl acetate solution), and the reaction solution was stirred at 25°C for 0.5 hours. After filtration, the filter cake was washed with ethyl acetate (10.0 mL × 3), and the filtrate was washed with saturated NaCl aqueous solution (20.0 mL × 2), dried over anhydrous sodium sulfate, filtered, and concentrated under reduced pressure. The crude product was separated by preparative high performance liquid chromatography (Phenomenex Gemini-NX C18, 75 mm × 30 mm 3 µm, A: water (10 mmol/L ammonium bicarbonate); B: acetonitrile, 40%-70%: 10 minutes) to obtain compound **35.**

MS-ESI [M+H]⁺, calculated: 638, found: 638.

¹H NMR (400 MHz, MeOD) *δ* 7.23-7.30 (m, 3H), 5.28-5.45 (m, 2H), 5.13-5.25 (m, 1H), 3.90-3.98 (m, 2H), 3.83-3.90 (m, 2H), 3.77-3.83 (m, 1H), 3.60-3.77 (m, 1H), 3.50 (dd, *J* = 14.8, 8.8 Hz, 1H), 3.18-3.23 (m, 1H), 3.13 (dd, *J* = 18.4, 5.6 Hz, 2H), 3.05 (s, 3H), 2.93-3.01 (m, 4H), 2.77-2.84 (m, 1H), 2.53-2.71 (m, 2H), 2.35-2.43 (m, 1H), 2.28-2.34 (m, 1H), 2.12 (d, *J* = 4.4 Hz, 3H), 2.06-2.09 (m, 1H), 1.99 (s, 1H), 1.94 (s, 1H), 1.77-1.83 (m, 1H), 1.66-1.76 (m, 3H).

### Example 31 Synthesis of Compounds 36 and 37

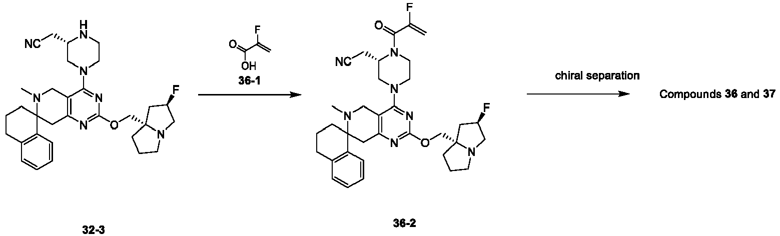

(1) To a solution of compound **35-1** (1.02 g, 11.3 mmol) in ethyl acetate (20.0 mL) was added 4A molecular sieve (2.0 g), triethylamine (1.15 g, 11.3 mmol), hydrochloride of compound **32-3**(2.2 g, 3.78 mmol) and propanephosphonic acid anhydride solution (9.62 g, 15.1 mmol, 50% ethyl acetate solution), and the reaction solution was stirred at 25°C for 0.5 hours. After filtration, the filter cake was washed with ethyl acetate (10.0 mL× 3), and the filtrate was washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 16:1) to obtain compound **36-2.**
(2) Compound **36-2** was separated by chiral supercritical fluid chromatography to obtain compounds **36** and **37.**

Separation conditions: Chromatographic column model:Chiralpak OD-3; column specification: 50 × 4.6mm I.D., 3µm; Injection volume: 4.0 µL; Mobile phase: A: Carbon dioxide, B: Ethanol (0.05% diethylamine), B%: 5%-40% gradient elution for 2.5 minutes, 40% fixed concentration elution for 0.5 minutes, 5% fixed concentration elution for 1 minute; Detection wavelength: 254 nm; column temperature: 35°C.

**Compound 36:** retention time: 1.567 minutes, 98.46%ee.

MS-ESI [M+H]⁺, calculated: 618, found: 618.

¹H NMR (400 MHz, MeOD) *δ* 7.51 (d, *J* = 7.6 Hz, 1H), 7.19-7.25 (m, 1H), 7.15 (t, *J* = 7.2 Hz, 1H), 7.07-7.12 (m, 1H), 5.37-5.46 (m, 1H), 5.30-5.36 (m, 1H), 5.25-5.30 (m, 1H), 4.86 (s, 1H), 4.61 (s, 1H), 4.25-4.32 (m, 1H), 4.22 (d, *J* = 11.2 Hz, 1H), 4.11-4.19 (m, 2H), 4.10 (s, 1H), 3.77 (d, *J* = 3.2 Hz, 2H), 3.51 (d, *J* = 15.2 Hz, 1H), 3.39-3.49 (m, 2H), 3.33-3.38 (m, 1H), 3.13 (d, *J* = 1.6 Hz, 1H), 3.05-3.12 (m, 2H), 3.00 (s, 2H), 2.90-2.95 (m, 1H), 2.73-2.80 (m, 2H), 2.31-2.45 (m, 1H), 2.30 (d, *J* = 7.6 Hz, 1H), 2.18 (d, *J* = 8.4 Hz, 1H), 2.12 (s, 3H), 2.07 (s, 1H), 2.04 (s, 1H), 2.00 (s, 1H), 1.97 (d, *J* = 3.2 Hz, 2H), 1.75-1.85 (m, 2H).

**Compound 37:** retention time: 1.778 minutes, 99.36%ee.

MS-ESI [M+H]⁺, calculated: 618, found: 618.

¹H NMR (400 MHz, MeOD) *δ* 7.51 (d, *J* = 7.2 Hz, 1H), 7.17-7.23 (m, 1H), 7.14 (td, *J* = 7.2, 1.2 Hz, 1H), 7.04-7.10 (m, 1H), 5.34-5.41 (m, 1H), 5.29-5.34 (m, 1H), 5.18-5.28 (m, 1H), 4.27 (d, *J* = 13.6 Hz, 1H), 4.10-4.25 (m, 2H), 4.01-4.10 (m, 1H), 3.80-4.01 (m, 1H), 3.74 (q, *J* = 14.8 Hz, 2H), 3.32-3.62 (m, 1H), 3.25-3.29 (m, 1H), 3.24 (d, *J* = 8.4 Hz, 1H), 3.19-3.22 (m, 2H), 3.17 (d, *J* = 6.8 Hz, 2H), 3.08 (s, 1H), 3.01-3.06 (m, 1H), 2.98 (s, 1H), 2.87-2.98 (m, 1H), 2.69-2.79 (m, 2H), 2.20-2.47 (m, 1H), 2.14-2.20 (m, 1H), 2.11 (s, 3H), 2.09 (s, 1H), 1.99-2.07 (m, 1H), 1.98 (s, 1H), 1.95 (d, *J* = 4.0 Hz, 2H), 1.87-1.93 (m, 1H), 1.80-1.87 (m, 1H), 1.57-1.80 (m, 2H).

### Example 32 Synthesis of Compound 38

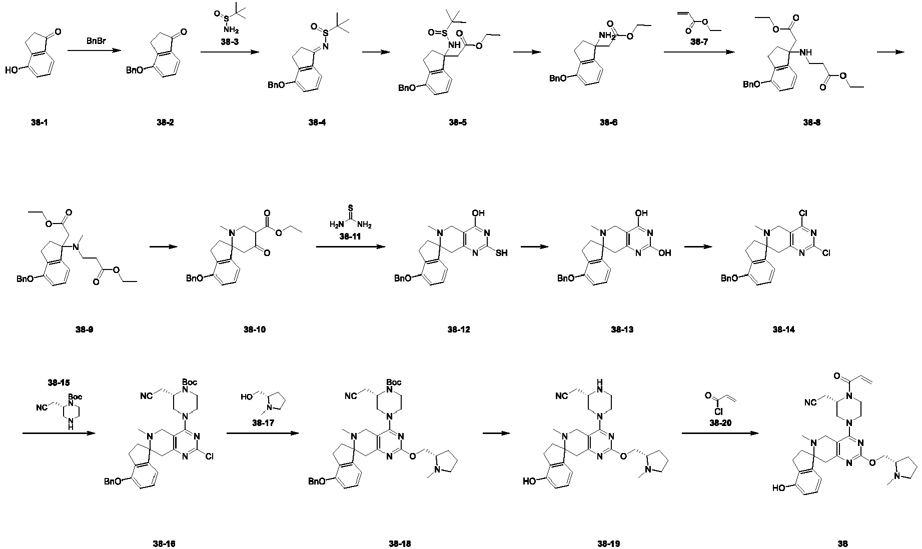

(1) To a solution of compound **38-1** (25.0 g, 168 mmol) in acetonitrile (250 mL) was added benzyl bromide (43.3 g, 253 mmol) and cesium carbonate (110 g, 338 mmol), and the reaction solution was stirred at 20°C for 12 hours under nitrogen protection. Then the reaction solution was concentrated under reduced pressure, added with water (50.0 mL), and extracted with ethyl acetate (40.0 mL × 2). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **38-2.**
   MS-ESI [M+H]⁺, calculated: 239, found: 239.
   ¹H NMR (400 MHz, CDCl₃) *δ* 7.32-7.40(m, 4H), 7.30-7.31 (m, 2H), 7.20-7.26 (m, 1H), 7.02-7.04 (d, *J* = 8 Hz, 1H), 5.12 (s, 2H), 3.03-3.06 (m, 2H), 2.62-2.65 (m, 2H).
(2) To a solution of compound **38-2** (20.0 g, 83.9 mmol) in toluene (200 mL) was added compound **38-3** (13.2 g, 109 mmol), and tetraethyl titanate (38.3 g, 168 mmol), and then the reaction solution was stirred at 120°C for 12 hours under nitrogen protection. Then the reaction solution was added with water (400 mL), and filtered. The filter cake was washed with ethyl acetate (400 mL × 2). The combined organic phase was washed with saturated NaCl aqueous solution (500 mL), dried over anhydrous sodium sulfate, filtered, and concentrated under reduced pressure. The crude product was separated by silica gel column chromatography (petroleum ether/ethyl acetate = 1:0 to 3:1) to obtain compound **38-4.**
   MS-ESI [M+H]⁺, calculated: 342, found: 342.
   ¹H NMR (400 MHz, CDCl₃) *δ* 7.26-7.44 (m, 7H), 6.99-7.01 (d, *J* = 8 Hz, 1H), 5.15 (s, 2H), 3.42-3.50 (m, 1H), 3.07-3.14 (m, 3H), 1.32 (s, 9H).
(3) To a solution of ethyl acetate (13.7 g, 156 mmol) in tetrahydrofuran (50.0 mL) was added dropwise with lithium diisopropylamide (2 mol/L, 39.0 mL, tetrahydrofuran solution) at -78°C under nitrogen protection, and the reaction solution was stirred at -78°C for 1 hour. Then the reaction solution was added dropwise with a solution of compound **38-4** (13.3 g, 39.0 mmol) in tetrahydrofuran (500 mL), and continued stirring for 3 hours at -78°C. Then the reaction solution was quenched with saturated ammonium chloride aqueous solution(1000 mL), and extracted with ethyl acetate (1000 mL × 1). The combined organic phase was washed with saturated ammonium chloride aqueous solution (1000 mL× 1) and saturated NaCl aqueous solution (1000 mL), dried over anhydrous sodium sulfate, filtered, and concentrated under reduced pressure. The crude product was separated by silica gel column chromatography (petroleum ether/ethyl acetate = 1:0 to 1:1) to obtain compound **38-5.**
   MS-ESI [M+H]⁺, calculated: 430, found: 430.
(4) To a solution of compound **38-5** (6.80 g, 15.8 mmol) in ethanol (54.0 mL) was added hydrochloric acid in dioxane (4 mol/L, 18.0 mL), and the reaction solution was stirred at 25°C for 0.5 hour under nitrogen protection. Then the reaction solution was concentrated under reduced pressure to obtain crude product of hydrochloride of compound **38-6.**
   MS-ESI [M-NH₂+H]⁺, calculated: 309, found: 309.
(5) To a solution of hydrochloride of compound **38-6** (5.70 g, 15.8 mmol) in ethanol (40.0 mL) was added compound **38-7** (15.8 g, 158 mmol), copper oxide (250 mg, 3.15 mmol), and triethylamine (1.60 g, 15.7 mmol), and the reaction solution was reacted at 78°C in a sealed tube for 12 hours under nitrogen protection. Then the reaction solution was added with water (200 mL), and extracted with ethyl acetate (100 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 1:1) to obtain compound **38-8.**
   MS-ESI [M+H]⁺, calculated: 426, found: 426.
(6) To a solution of compound **38-8** (6.20 g, 14.6 mmol) in ethanol (60.0 mL) was added paraformaldehyde (2.20 g) and sodium cyanoborohydride (2.75 g, 43.7 mmol), and the reaction solution was reacted for 12 hours at 30°C under nitrogen protection. Then the reaction solution was added with water (100 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **38-9.**
   MS-ESI [M+H]⁺, calculated: 440, found: 440.
   ¹H NMR (400 MHz, CDCl₃) *δ* 7.40-7.45 (m, 2H), 7.34-7.40 (m, 2H), 7.28-7.33 (m, 1H), 7.11-7.16 (t, 1H), 6.86-6.88 (d, *J* = 7.2 Hz, 1H), 6.75-6.77 (d, *J* = 8.0 Hz, 1H), 5.02-5.11 (m, 2H), 4.07-4.13 (m, 2H), 3.83-3.97 (m, 2H), 2.83-2.97 (m, 3H), 2.71-2.81 (m, 2H), 2.61-2.69 (m, 2H), 2.36-2.47 (m, 3H), 2.22-2.35 (m, 2H), 1.79-1.88 (m, 1H), 1.24 (m, 3H), 1.00-1.04 (t, 3H).
(7) To a solution of compound **38-9** (5.80 g, 13.2 mmol) in tetrahydrofuran (60.0 mL) was added potassium bis (trimethylsilyl) amide (1 mol/L, 39.6 mL, tetrahydrofuran solution) at -78°C, and the reaction solution was reacted at -78°C for 1 hour under nitrogen protection. Then the reaction solution was added with saturated ammonium chloride aqueous solution (300 mL), and extracted with ethyl acetate (200 mL × 2). The combined organic phase was washed with saturated NaCl aqueous solution (200 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **38-10.**
   MS-ESI [M+H]⁺, calculated: 394, found: 394.
(8) To a solution of compound **38-10** (3.90 g, 9.91 mmol) in ethanol (40.0 mL) was added compound **38-11**(1.51 g, 11.8 mmol) and sodium ethoxide (2.02 g, 29.7 mmol), and the reaction solution was stirred at 80°C for 12 hours under nitrogen protection. After concentrated under reduced pressure, the reaction solution was adjusted to pH 6 with hydrochloric acid (1 mol/L). The resulting solution was precipitated, and filtered. The filter cake was dried to obtain compound **38-12.** MS-ESI [M+H]⁺, calculated: 406, found: 406.
(9) To a solution of intermediate **38-12** (3.50 g, 8.64 mmol) in water (40.0 mL) was added chloroacetic acid (7.34 g, 77.6 mmol), and the reaction solution was stirred at 100°C for 12 hours under nitrogen protection. After the reaction solution was filtered, the filtrate was adjusted to pH 8 with saturated sodium bicarbonate aqueous solution to get precipitate. After filtration, the filter cake was dried to obtain compound **38-13.**
   MS-ESI [M+H]⁺, calculated: 390, found: 390.
   ¹H NMR (400 MHz, DMSO-d6) *δ* 11.37 (s, 1H), 11.17 (s, 1H), 7.45-7.58 (m, 3H), 7.32-7.43 (m, 4H), 7.11 (br s, 1H), 5.17 (s, 2H), 4.05-4.17 (m, 1H), 3.80-3.96 (m, 1H), 3.24-3.29 (m, 1H), 2.88-3.03 (m, 2H), 2.61-2.74 (m, 1H), 2.54-2.60 (m, 1H), 2.43-2.48 (m, 3H), 1.96-2.17 (m, 1H).
(10) Compound **38-13** (1.00 g, 2.57 mmol) was dissolved in phosphorus oxychloride (16.5 g, 107 mmol), and the reaction solution was stirred at 80°C for 12 hours under nitrogen protection. Then the reaction solution was concentrated under reduced pressure, and extracted with dichloromethane (100 mL× 2). The organic phase was washed with saturated sodium bicarbonate aqueous solution (100 mL × 2) and saturated NaCl aqueous solution (100 mL× 2), dried over anhydrous sodium sulfate, filtered, and concentrated under reduced pressure to obtain compound **38-14.**
   MS-ESI [M+H]⁺, calculated: 426, found: 426.
(11) To a solution of compound **38-14** (1.10 g, 2.58 mmol) in *N*-methylpyrrolidone (10.0 mL) was added potassium carbonate (1.07 g, 7.74 mmol) and compound **38-15(697** mg, 3.10 mmol), and the reaction solution was stirred at 50°C for 12 hours under nitrogen protection. Then the reaction solution was added with water (30.0 mL), and extracted with ethyl acetate (20.0 mL × 2). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 1:1) to obtain compound **38-16.**
   MS-ESI [M+H]⁺, calculated: 615, found: 615.
(12) To a solution of compound **38-16** (790 mg, 1.28 mmol) in dioxane (10.0 mL) was added compound **38-17** (445 mg, 3.85 mmol), cesium carbonate (1.26 g, 3.85 mmol) and (2-dicyclohexylphosphino-2',6'-diisopropoxy-1,1'-biphenyl) [2-(2'-amino-1,1'- biphenyl)] palladium(II) methanesulfonate (215 mg, 257 µmol), and the reaction solution was stirred at 110°C for 6 hours under nitrogen protection. Then the reaction solution was added with water (30.0 mL), and extracted with ethyl acetate (40.0 mL × 2). The combined organic phase was washed with saturated NaCl aqueous solution (40.0 mL × 2), dried over anhydrous sodium sulfate, filtered, and concentrated under reduced pressure. The crude product was separated by silica gel column chromatography (dichloromethane/ methanol = 1:0 to 10:1) to obtain compound **38-18.**
   MS-ESI [M+H]⁺, calculated: 694, found: 694.
(13) Compound **38-18** (200 g, 288 µmol) was added into trifluoroacetic acid (10.0 mL), and the reaction solution was stirred at 50°C for 3 hours under nitrogen protection. Then the reaction solution was concentrated under reduced pressure to obtain the crude trifluoroacetate of compound **38-19.**
   MS-ESI [M+H]⁺, calculated: 504, found: 504.
(14) To a solution of trifluoroacetate of compound **38-19**(70.0 mg, 139 µmol) in dichloromethane (1.0 mL) was added triethylamine (42.2 mg, 417 µmol) and compound **38-20** (15.1 mg, 167 µmol), and the reaction solution was stirred at -78°C for 0.5 hours under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm ×3 µm+YMC AQ, 100 mm × 30 mm ×10 µm, A: water (0.05% hydrochloric acid); B: methanol, 0%-40%: 20 min) to obtain hydrochloride of compound **38.**

MS-ESI [M+H]⁺, calculated: 558, found: 558.

¹H NMR (400 MHz, MeOD) *δ* 7.24-7.31 (m, 2H), 6.84 (br s, 2H), 6.25-6.37 (m, 1H), 5.82-5.92 (m, 1H), 5.01-5.19 (m, 2H), 4.77 (br s, 3H), 4.13 (br s, 2H), 3.99-4.06 (m, 1H), 3.85 (br d, *J* = 6.8 Hz, 3H), 3.45-3.68 (m, 2H), 3.33-3.44 (m, 1H), 3.23-3.29 (m, 1H), 3.11-3.22 (m, 2H), 3.10 (d, *J* = 1.4 Hz, 3H), 2.98-3.08 (m, 2H), 2.73-2.85 (m, 5H), 2.39-2.52 (m, 2H), 2.14-2.29 (m, 2H), 2.04-2.12 (m, 1H).

### Example 33 Synthesis of Compound 39

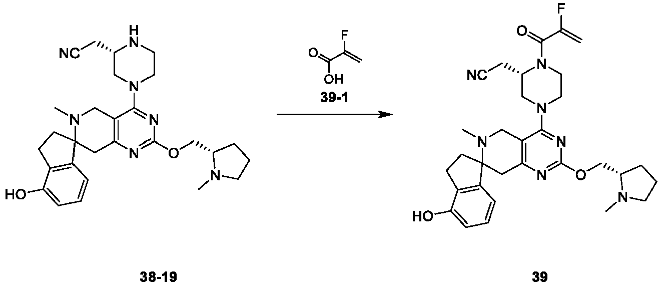

To a solution of trifluoroacetate of compound **38-19** (90.0 mg, 180 µmol) in dichloromethane (2.0 mL) was added triethylamine (18.1 mg, 180 µmol), compound **39-1**(113 mg, 1.25 mmol) and propanephosphonic acid anhydride solution (341 mg, 536 µmol, 50% ethyl acetate solution) at 0°C, and the reaction solution was stirred at 0°C for 0.5 hours. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Phenomenex Luna C18, 100mm × 30 mm ×3 µm, A: water (0.225% formic acid); B: acetonitrile, 0%-30%: 8 min) to obtain formate of compound **39.**

MS-ESI [M+H]⁺, calculated: 576, found: 576.

¹H NMR (400 MHz, MeOD) *δ* 6.95-7.39 (m, 2H), 6.76-6.91 (m, 1H), 5.22-5.47 (m, 2H), 4.54-4.67 (m, 5H), 4.32-4.50 (m, 2H), 3.99-4.26 (m, 3H), 3.68-3.92 (m, 3H), 3.42-3.62 (m, 3H), 3.18-3.27 (m, 2H), 3.10-3.17 (m, 2H), 2.93-3.07 (m, 4H), 2.52-2.86 (m, 3H), 2.37 (br s, 1H), 2.10-2.27 (m, 3H), 1.94-2.08 (m, 1H).

### Example 34 Synthesis of Compounds 40 and 41

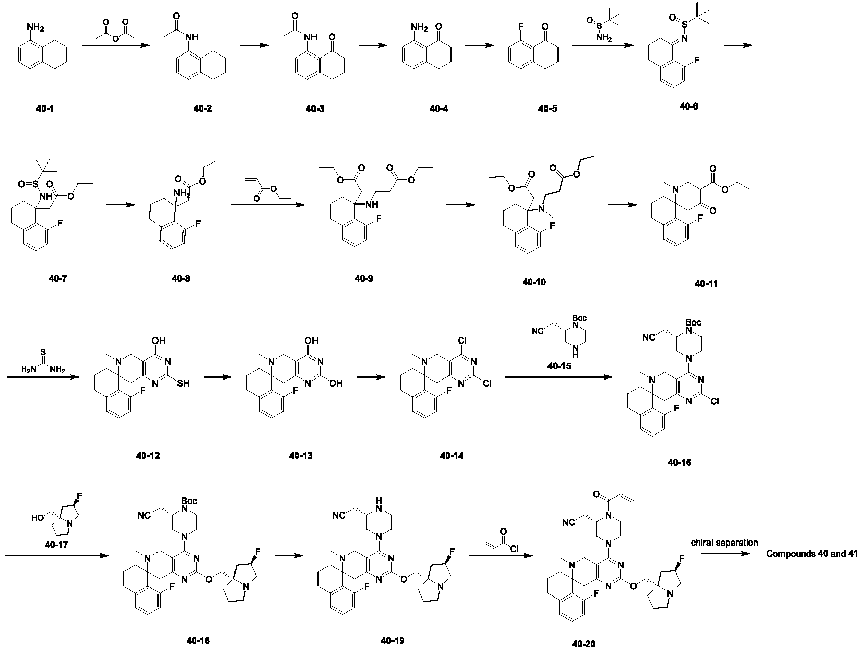

(1) To a solution of compound **40-1**(100 g, 679 mmol) in ethanol (1.0 L) was added acetic anhydride (139 g, 1.36 mol), and the reaction solution was stirred at 30°C for 1 hour under nitrogen protection. Then the reaction solution was concentrated under reduced pressure to remove ethanol to obtain crude compound **40-2.**
   MS-ESI [M+H]⁺, calculated: 190, found: 190.
(2) To a solution of compound **40-2**(101 g, 534 mmol) in acetone (1.0 L) was added magnesium sulfate (89.1 g, 740 mmol), water (300mL) and potassium permanganate (253 g, 1.60 mol), and the reaction solution was stirred at 25°C for 3 hours under nitrogen protection. After filtration, the residue was added with water (600 mL) and extracted with dichloromethane (500 mL × 1). The organic phase was washed with saturated NaCl aqueous solution (500 mL × 1), dried over anhydrous sodium sulfate, filtered, concentrated under reduced pressure. The crude product was separated by silica gel column chromatography (petroleum ether/ethyl acetate = 1:0 to 10:1) to obtain compound **40-3.**
   ¹H NMR (400 MHz, CDCl₃) *δ* 8.60 (d, *J* = 8.4 Hz, 1H), 7.45 (t, *J* = 8.0 Hz, 1H), 6.94 (d, *J* = 7.6, 0.8 Hz, 1H), 2.98 (t, *J* = 6.0 Hz, 2H), 2.66-2.75 (m, 2H), 2.24 (s, 3H), 2.03-2.15 (m, 2H).
(3) To a solution of compound **40-3** (140 g, 689 mmol) in water (1.0 L) was added hydrochloric acid (12 mol/L, 459 mL), and the reaction solution was stirred at 110°C for 3 hours under nitrogen protection. Then the reaction solution was concentrated under reduced pressure, added with sodium hydroxide aqueous solution (2 mol/L) to adjust pH to 8. After filtration, the filter cake was washed with water (100 mL × 1) to obtain compound **40-4.**
   MS-ESI [M+H]⁺, calculated: 162, found: 162.
(4) To a solution of compound **40-4** (90.0 g, 558 mmol) in dichloromethane (1.0 L) was added boron trifluoride diethyl etherate (119 g, 838 mmol), and the reaction solution was stirred at 0°C for 20 minutes. Then the reaction solution was added with isoamyl nitrite (74.9g, 726 mmol), and continued stirring for 40 minutes at 0°C under nitrogen protection. Methyl tert-butyl ether (200 mL) was added. After filtration, the filter cake was washed with methyl tert-butyl ether (200 mL), and dissolved in xylene (100 mL). The resulting solution was stirred at 120°C for 30 minutes. Then the reaction solution was adjusted to pH 8 with the addition of sodium hydroxide aqueous solution (2 mol/L), and extracted with ethyl acetate (200 mL × 3). The combined organic phase was washed with saturated NaCl aqueous solution (500 mL × 1), dried over anhydrous sodium sulfate, filtered, and concentrated under reduced pressure. The crude product was separated by silica gel column chromatography (petroleum ether/ethyl acetate = 1:0 to 10:1) to obtain compound **40-5.**
   MS-ESI [M+H]⁺, calculated: 165, found: 165.
   ¹H NMR (400 MHz, CDCl₃) *δ* 7.42 (d, *J* = 8.0, 5.2 Hz, 1H), 7.06 (d, *J* = 7.6 Hz, 1H), 6.98 (d, *J* = 11.2, 8.4 Hz, 1H), 2.98 (t, *J* = 6.0 Hz, 2H), 2.64-2.69 (m, 2H), 2.09-2.17 (m, 2H).
(5) A solution of compound **40-5** (40.0 g, 244 mmol) in toluene (400 mL) was added with tert-butyl sulfinamide (35.4 g, 292 mmol), and tetraethyl titanate (111 g, 487 mmol), and then stirred at 100°C for 3 hours under nitrogen protection. Then the reaction solution was added with water (200 mL), and filtered. The filter cake was washed with ethyl acetate (200 mL × 3). The combined organic phase was washed with saturated NaCl aqueous solution (300 mL), dried over anhydrous sodium sulfate, filtered, and concentrated under reduced pressure. The crude product was separated by silica gel column chromatography (petroleum ether/ethyl acetate = 1:0 to 5:1) to obtain compound **40-6.**
   MS-ESI [M+H]⁺, calculated: 268, found: 268.
   ¹H NMR (400 MHz, CDCl₃) *δ* 7.32 (td, *J* = 8.0, 5.2 Hz, 1H), 6.93-7.01 (m, 2H), 3.35 (d, *J* = 17.6, 8.0, 6.0 Hz, 1H), 3.08 (d, *J* = 5.6 Hz, 1H), 2.83-2.90 (m, 2H), 1.91-2.01 (m, 2H), 1.34 (s, 9H).
(6) To a solution of ethyl acetate (49.4 g, 561 mmol) in tetrahydrofuran (200 mL) was added dropwise with lithium diisopropylamide (2 mol/L, 112 mL, tetrahydrofuran solution) at -78°C under nitrogen protection, and the reaction solution was stirred at -78°C for 1 hour. Then the reaction solution was added dropwise with a solution of compound **40-6** (30.0 g, 112 mmol) in tetrahydrofuran (100 mL), and continued stirring for 2 hours at -78°C. Then the reaction solution was quenched with saturated ammonium chloride aqueous solution (200 mL), and extracted with ethyl acetate (200 mL × 2). The combined organic phase was washed with saturated ammonium chloride aqueous solution (200 mL× 1) and saturated NaCl aqueous solution (100 mL× 3), dried over anhydrous sodium sulfate, filtered, and concentrated under reduced pressure. The crude product was separated by silica gel column chromatography (petroleum ether/ethyl acetate = 1:0 to 3:1) to obtain compound **40-7.**
   MS-ESI [M+H]⁺, calculated: 356, found: 356.
   ¹H NMR (400 MHz, CDCl₃) *δ* 7.25 (d, *J* = 8.0, 5.6 Hz, 1H), 7.00 (d, *J* = 7.6 Hz, 1H), 6.88 (d, *J* = 12.4, 8.4 Hz, 1H), 4.01-4.13 (m, 2H), 3.03-3.21 (m, 2H), 2.88 (d, *J =* 16.8, 4.8 Hz, 1H), 2.70-2.80 (m, 1H), 2.19-2.34 (m, 2H), 2.10 (m, *J* = 13.6, Hz, 1H), 1.71-1.82 (m, 1H), 1.20 (s, 9H), 1.14 (t, *J* = 7.2 Hz, 3H).
(7) To a solution of compound **40-7** (20.0 g, 56.3 mmol) in ethanol (100 mL) was added hydrochloric acid in dioxane (4 mol/L, 141 mL), and the reaction solution was stirred at 25°C for 1 hour under nitrogen protection. Then the reaction solution was concentrated under reduced pressure to obtain crude product of hydrochloride of compound **40-8.**
(8) To a solution of hydrochloride of compound **40-8** (18.0 g, 62.6 mmol) in ethanol (200 mL) was added ethyl acrylate (93.9 g, 188 mmol), copper oxide (995 mg, 12.5 mmol), and triethylamine (19.0 g, 188 mmol), and the reaction solution was reacted at 85°C in a sealed tube for 10 hours under nitrogen protection. Then the reaction solution was added with water (100 mL), and extracted with ethyl acetate (100 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 2:1) to obtain compound **40-9.**
   MS-ESI [M+H]⁺, calculated: 352, found: 352.
(9) To a solution of compound **40-9** (15.0 g, 42.7 mmol) in ethanol (200 mL) was added paraformaldehyde (5.50 g), acetic acid (2.56 g, 42.7 mmol) and sodium cyanoborohydride (8.05 g, 128 mmol), and the reaction solution was reacted for 12 hours at 30°C under nitrogen protection. Then the reaction solution was added with water (100 mL), and extracted with ethyl acetate (100 mL). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **40-10.**
   ¹H NMR (400 MHz, CDCl₃) *δ* 7.11 (d, *J* = 7.6, 5.2 Hz, 1H), 6.76-6.89 (m, 2H), 4.05-4.13 (m, 2H), 3.79-3.93 (m, 2H), 2.68-2.87 (m, 6H), 2.38 (m, *J* = 14.8, 7.6 Hz, 2H), 2.23 (s, 3H), 2.08-2.18 (m, 2H), 1.69-1.87 (m, 2H), 1.18-1.29 (m, 3H), 0.97 (t, *J* = 7.2 Hz, 3H).
(10) To a solution of compound **40- 8** (10.0 g, 27.4 mmol) in tetrahydrofuran (100 mL) was added potassium bis (trimethylsilyl) amide (1 mol/L, 82.1 mL, tetrahydrofuran solution) at -78°C, and the reaction solution was reacted at 0°C for 2 hours under nitrogen protection. Then the reaction solution was added with saturated ammonium chloride aqueous solution (300 mL), and extracted with ethyl acetate (300 mL). The combined organic phase was washed with saturated NaCl aqueous solution (300 mL), dried over anhydrous sodium sulfate, filtered, and concentrated under reduced pressure. The crude product was separated by silica gel column chromatography (petroleum ether/ethyl acetate = 1:0 to 5:1) to obtain compound **40-11.**
   MS-ESI [M+H]⁺, calculated: 320, found: 320.
(11) To a solution of compound **40-11** (8.00 g, 25.0 mmol) in ethanol (20.0 mL) was added with thiourea (3.81 g, 50.1 mmol) and sodium ethoxide (5.11 g, 75.2 mmol), and the reaction solution was stirred at 80°C for 12 hours under nitrogen protection. After being concentrated under reduced pressure, the reaction solution was adjusted to pH 6 with hydrochloric acid (1 mol/L). The resulting solution was precipitated, and filtered. The filter cake was dried to obtain compound **40-12.**
   MS-ESI [M+H]⁺, calculated: 332, found: 332.
(12) To a solution of intermediate **40-12** (9.00 g, 27.2 mmol) in water (60.0 mL) was added chloroacetic acid (18.0 g, 190 mmol), and the reaction solution was stirred at 100°C for 12 hours under nitrogen protection. After the reaction solution was filtered, the filtrate was adjusted to pH 8 with saturated sodium bicarbonate aqueous solution to get precipitate. After filtration, the filter cake was dried to obtain compound **40-13.**
   MS-ESI [M+H]⁺, calculated: 316, found: 316.
   ¹H NMR (400 MHz, MeOD) *δ*7.24 (m, *J* = 7.6, 5.2 Hz, 1H), 6.89-7.01 (m, 2H), 3.58-3.66 (m, 1H), 3.08-3.26 (m, 2H), 2.60-2.84 (m, 3H), 2.11 (s, 3H), 1.89-2.02 (m, 2H), 1.61-1.81 (m, 2H).
(13) Compound **40-13** (8.00 g, 25.4 mmol) was dissolved in phosphorus oxychloride (15.6g), and the reaction solution was stirred at 100°C for 3 hours under nitrogen protection. Then the reaction solution was concentrated under reduced pressure, and extracted with dichloromethane (200 mL). The organic phase was washed with saturated sodium bicarbonate aqueous solution (200 mL × 2) and saturated NaCl aqueous solution (200 mL× 2), dried over anhydrous sodium sulfate, filtered, and concentrated under reduced pressure to obtain compound **40-14.**
   MS-ESI [M+H]⁺, calculated: 352, found: 352.
(14) To a solution of compound **40-14** (600 mg, 17.0 mmol) in *N*-methylpyrrolidone (50.0 mL) was added potassium carbonate (7.06 g, 51.1 mmol) and compound **40-15** (4.99 g, 22.1 mmol), and the reaction solution was stirred at 50°C for 3 hours under nitrogen protection. Then the reaction solution was added with ethyl acetate (200 mL), washed with saturated NaCl aqueous solution (200 mL × 5), dried over anhydrous sodium sulfate, filtered, and concentrated under reduced pressure. The crude product was separated by silica gel column chromatography (petroleum ether/ethyl acetate = 1:0 to 10:1) to give compound **40-16.**
   MS-ESI [M+H]⁺, calculated: 541, found: 541.
(15) To a solution of compound **40-16** (1.00 g, 1.85 mmol) in dioxane (20.0 mL) was added compound **40-17** (442 mg, 2.78 mmol), cesium carbonate (1.81 g, 5.54 mmol) and (2-dicyclohexylphosphino-2',6'-diisopropoxy-1,1'-biphenyl) [2-(2'-amino-1,1'-biphenyl)] palladium(II) methanesulfonate (100 mg, 120 µmol), and the reaction solution was stirred at 110°C for 3 hours under nitrogen protection. Then the reaction solution was added with ethyl acetate (50.0 mL), washed with saturated NaCl aqueous solution (50.0 mL × 3), dried over anhydrous sodium sulfate, filtered, and concentrated under reduced pressure. The crude product was separated by silica gel column chromatography (dichloromethane/methanol = 1:0 to 30:1) to obtain compound **40-18.**
   MS-ESI [M+H]⁺, calculated: 664, found: 664.
   ¹H NMR (400 MHz, MeOD) *δ* 7.22 (td, *J* = 7.6, 5.2 Hz, 1H), 6.98 (d, *J* = 7.6 Hz, 1H), 6.91 (dd, *J* = 12.4, 8.0 Hz, 1H), 5.13-5.43 (m, 1H), 4.60 (d, *J* = 12.4 Hz, 2H), 4.04-4.23 (m, 3H), 3.84-4.04 (m, 2H), 3.56-3.77 (m, 2H), 3.40 (dd, *J* = 13.6, 3.6 Hz, 1H), 3.22-3.29 (m, 2H), 3.10-3.22 (m, 3H), 2.94-3.05 (m, 3H), 2.71-2.86 (m, 3H), 2.22-2.36 (m, 1H), 2.13-2.18 (m, 3H), 2.04-2.13 (m, 2H), 1.94-2.02 (m, 3H), 1.83-1.93 (m, 2H), 1.56-1.82 (m, 2H), 1.51 (d, *J* = 2.4 Hz, 9H).
(16) To a solution of compound **40-18** (910 g, 1.37 mmol) in dichloromethane (10.0 mL) was added hydrochloric acid in dioxane (4 mol/L, 5.0 mmol), and the reaction solution was stirred at 25°C for 2 hours under nitrogen protection. Then the reaction solution was adjusted to pH 8 by saturated sodium bicarbonate aqueous solution and extracted with dichloromethane (20 mL× 2). The organic phase was washed with saturated sodium bicarbonate aqueous solution (20 mL) and saturated NaCl aqueous solution (50 mL), dried over anhydrous sodium sulfate, filtered, and concentrated under reduced pressure to obtain compound **40-19.**
   MS-ESI [M+H]⁺, calculated: 564, found: 564.
(17) To a solution compound **40-19**(672 g, 1.19 mmol) in dichloromethane (10.0 mL) was added triethylamine (362 mg, 3.58 mmol) and acryloyl chloride (216 mg, 2.39 mmol), and then the reaction solution was stirred at -65°C for 0.5 hour under nitrogen protection. Then the reaction solution was added with dichloromethane (20.0 mL), washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 30:1) to obtain compound **40-20.**
(18) Compound **40-20** was separated by chiral supercritical fluid chromatography to obtain compounds **40** and **41.**

Separation conditions: Chromatographic column model:(S,S)-Whelk-0-1.8; Column specification: 50 × 4.6mm I.D., 1.8 µm; Injection volume: 10.0 µL; Mobile phase: A: carbon dioxide, B: ethanol (0.05% diethylamine), B%: 40% elution for 7 min; Detection wavelength: 254 nm; Column temperature: 35°C.

**Compound 40:** retention time 2.328 minutes, 100%ee.

MS-ESI [M+H]⁺, calculated: 618, found: 618.

¹H NMR (400 MHz, MeOD) *δ* 7.22 (m, *J* = 7.6, 5.2 Hz, 1H), 6.98 (d, *J* = 7.6 Hz, 1H), 6.91 (dd, *J* = 12.4, 8.0 Hz, 1H), 6.28 (d, *J* = 16.8 Hz, 1H), 5.83 (d, *J* = 10.4 Hz, 1H), 5.18-5.37 (m, 1H), 5.03 (s, 1H), 4.70-4.85 (m, 2H), 4.10-4.32 (m, 3H), 3.90-4.10 (m, 2H), 3.65-3.80 (m, 2H), 3.38-3.51 (m, 1H), 3.18-3.28 (m, 3H), 3.08-3.18 (m, 2H), 2.95-3.02 (m, 2H), 2.89 (s, 2H), 2.67-2.84 (m, 2H), 2.16-2.41 (m, 2H), 2.16 (s, 3H), 2.03-2.13 (m, 2H), 1.94-2.03 (m, 3H), 1.85-1.93 (m, 2H), 1.68-1.79 (m, 1H).

**Compound 41:** retention time 1.677 minutes, 100%ee.

MS-ESI [M+H]⁺, calculated: 618, found: 618.

¹H NMR (400 MHz, MeOD) *δ* 7.22 (td, *J* = 7.6, 5.2 Hz, 1H), 6.98 (d, *J* = 7.6 Hz, 1H), 6.92 (dd, *J* = 12.4, 8.0 Hz, 1H), 6.29 (d, *J* = 16.8 Hz, 1H), 5.83 (d, *J* = 10.4 Hz, 1H), 5.14-5.41 (m, 1H), 5.08 (s, 1H), 4.70-4.84 (m, 2H), 4.11-4.40 (m, 3H), 3.92-4.11 (m, 2H), 3.60-3.78 (m, 2H), 3.37-3.60 (m, 1H), 3.21-3.29 (m, 3H), 3.17-3.21 (m, 2H), 3.12-3.17 (m, 2H), 2.94-3.01 (m, 2H), 2.72-2.86 (m, 2H), 2.17-2.34 (m, 2H), 2.16 (s, 3H), 2.03-2.13 (m, 2H), 1.94-2.02 (m, 3H), 1.82-1.92 (m, 2H), 1.68-1.80 (m, 1H).

### Example 35 synthesis of Compounds 42 and 43

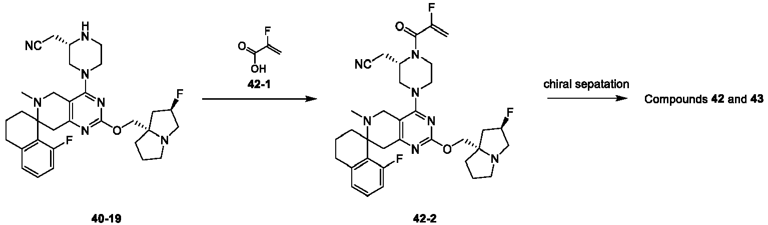

(1) To a solution of compound **40-19** (1.50 g, 2.66 mmol) in ethyl acetate (15.0 mL) was added triethylamine (809 mg, 7.99 mmol), compound **42-1** (719 mg, 7.98 mmol), 4A molecular sieve(1.50 g) and propanephosphonic acid anhydride solution (6.78 g, 10.6 mmol, 50% ethyl acetate solution), and the reaction solution was stirred at 25°C for 0.5 hours. Then the reaction solution was added with ethyl acetate (50.0 mL), washed with saturated NaCl aqueous solution (50.0 mL × 2), dried over anhydrous sodium sulfate, filtered, and concentrated under reduced pressure. The crude product was separated by silica gel column chromatography (dichloromethane/methanol = 1:0 to 30:1) to obtain compound **42-2.**
(2) Compound **42-2** was separated by chiral supercritical fluid chromatography to obtain compounds **42** and **43.**

Separation conditions: Chromatographic column model:Chiralpak OX-3; column specification: 100 × 4.6mm I.D., 3 µm; Injection volume: 10.0 µL; Mobile phase: A: Carbon dioxide, B: Ethanol (0.05% diethylamine), B%: 40% fixed concentration elution for 4 min; detection wavelength: 254 nm; column temperature: 35°C.

**Compound 42:** retention time 1.385 minutes, 100%ee.

MS-ESI [M+H]⁺, calculated: 636, found: 636.

¹H NMR (400 MHz, MeOD) *δ* 7.22 (td, *J* = 7.6, 5.2 Hz, 1H), 6.98 (d, *J* = 7.6 Hz, 1H), 6.92 (dd, *J* = 12.8, 8.0 Hz, 1H), 5.29-5.44 (m, 2H), 5.18-5.28 (m, 1H), 4.11-4.19 (m, 2H), 4.05-4.11 (m, 2H), 3.71-3.78 (m, 1H), 3.63-3.68 (m, 1H), 3.46 (d, *J* = 13.2 Hz, 1H), 3.32-3.33 (m, 1H), 3.22-3.30 (m, 3H), 3.13-3.22 (m, 2H), 2.99-3.12 (m, 3H), 2.89-2.99 (m, 2H), 2.75-2.88 (m, 2H), 2.22-2.33 (m, 1H), 2.18 (s, 1H), 2.16 (s, 3H), 2.04-2.14 (m, 2H), 1.95-2.04 (m, 3H), 1.88-1.88 (m, 3H), 1.76-1.94 (m, 1H).

**Compound 43:** retention time 2.071 minutes, 100%ee.

MS-ESI [M+H]⁺, calculated: 636, found: 636.

¹H NMR (400 MHz, MeOD) *δ* 7.22 (td, *J* = 7.6, 5.2 Hz, 1H), 6.98 (d, *J* = 7.6 Hz, 1H), 6.91 (dd, *J* = 12.8, 8.0 Hz, 1H), 5.29-5.43 (m, 2H), 5.26 (s, 1H), 4.27 (d, *J* = 13.6 Hz, 1H), 4.18 (q, *J* = 10.4 Hz, 2H), 3.99 (d, *J* = 11.2 Hz, 1H), 3.71-3.79 (m, 1H), 3.59-3.68 (m, 1H), 3.38-3.56 (m, 1H), 3.36 (d, *J* = 2.4 Hz, 1H), 3.27 (d, *J* = 3.6 Hz, 3H), 3.21-3.27 (m, 2H), 3.05-3.20 (m, 3H), 2.85-3.05 (m, 2H), 2.65-2.84 (m, 2H), 2.26-2.40 (m, 1H), 2.19-2.26 (m, 1H), 2.16 (s, 3H), 2.04-2.15 (m, 2H), 1.96-2.04 (m, 3H), 1.77-1.96 (m, 3H), 1.65-1.77 (m, 1H).

### Example 36 Synthesis of Compounds 44 and 45

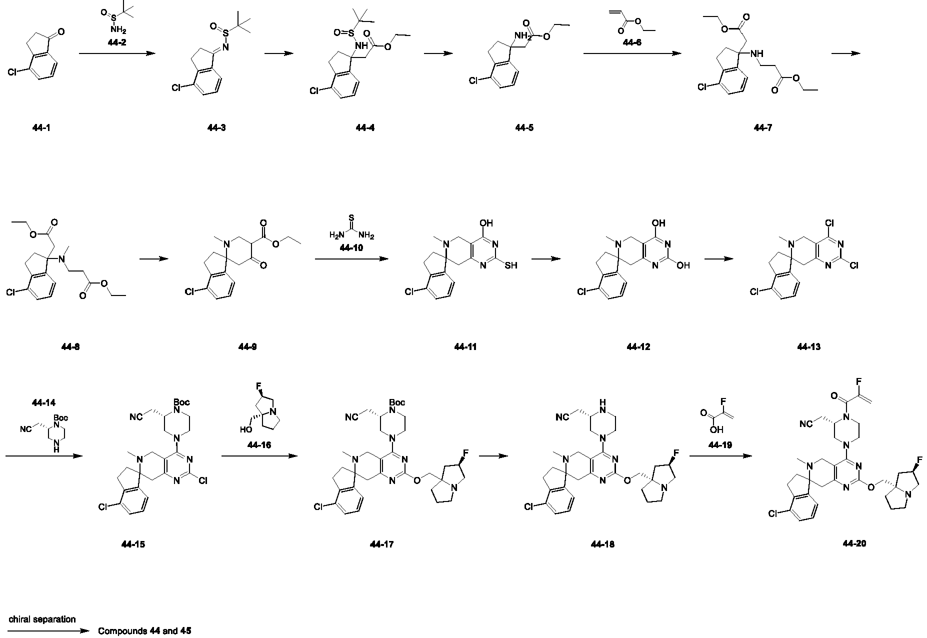

(1) To a solution of compound **44-1** (5.00 g, 30.0 mmol) in toluene (50.0 mL) was added compound **44-2** (4.36 g, 36.0 mmol), tetraethyl titanate (13.7 g, 60.0 mmol), and the reaction solution was stirred at 110°C for 12 hours under nitrogen protection. Then the reaction solution was added with water (150 mL), and filtered. The filter cake was washed with ethyl acetate (150 mL × 2). The combined organic phase was washed with saturated NaCl aqueous solution (150 mL), dried over anhydrous sodium sulfate, filtered, and concentrated under reduced pressure. The crude product was separated by silica gel column chromatography (petroleum ether/ethyl acetate = 1:0 to 10:1) to obtain compound **44-3.**
   MS-ESI [M+H]⁺, calculated: 270, found: 270.
   ¹H NMR (400 MHz, CDCl₃) *δ* 7.70 (d, *J* = 7.6 Hz, 1H), 7.50 (d, *J* = 8.0 Hz, 1H), 7.30 (t, *J* = 7.6 Hz, 1H), 3.46-3.56 (m, 1H), 3.06-3.19 (m, 3H), 1.33 (s, 9H).
(2) To a solution of ethyl acetate (8.16g, 92.6 mmol) in tetrahydrofuran (200 mL) was added dropwise with lithium diisopropylamide (2 mol/L, 18.5 mL, tetrahydrofuran solution) at -78°C under nitrogen protection, and the reaction solution was stirred at -65°C for 1 hour. Then the reaction solution was added dropwise with a solution of compound **44-3** (5.0 g, 18.5 mmol) in tetrahydrofuran (500 mL), and then continued stirring for 2 hours at -65°C. Then the reaction solution was quenched with saturated ammonium chloride aqueous solution(50.0 mL), and extracted with ethyl acetate (100 mL). The combined organic phase was washed with saturated ammonium chloride aqueous solution (100 mL× 1) and saturated NaCl aqueous solution (100 mL), dried over anhydrous sodium sulfate, filtered, and concentrated under reduced pressure. The crude product was separated by silica gel column chromatography (petroleum ether/ethyl acetate = 1:0 to 6:1) to obtain compound **44-4.**
   MS-ESI [M+H]⁺, calculated: 358, found: 358.
(3) To a solution of compound **44-4** (2.30 g, 6.43 mmol) in ethanol (15.0 mL) was added hydrochloric acid in dioxane (4 mol/L, 10.0 mL), and the reaction solution was stirred at 25°C for 1 hour under nitrogen protection. Then the reaction solution was concentrated under reduced pressure to obtain crude product of hydrochloride of compound **44-5.**
(4) To a solution of hydrochloride of compound **44-5** (2.30 g, 9.06 mmol) in ethanol (20.0 mL) was added compound **44-6** (9.08 g, 90.6 mmol), copper oxide (144 mg, 1.81 mmol), triethylamine (2.75 g, 27.2 mmol), and the reaction solution was reacted at 85°C in a sealed tube for 12 hours under nitrogen protection. Then the reaction solution was added with water (100 mL), and extracted with ethyl acetate (200 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 6:1) to obtain compound **44-7.**
   MS-ESI [M+H]⁺, calculated: 354, found: 354.
   ¹H NMR (400 MHz, CDCl₃) *δ* 7.19-7.23 (m, 1H), 7.12-7.19 (m, 2H), 4.06-4.16 (m, 4H), 2.95-3.06 (m, 1H), 2.81-2.93 (m, 1H), 2.67-2.79 (m, 2H), 2.59-2.67 (m, 1H), 2.48-2.55 (m, 1H), 2.42-2.48 (m, 2H), 2.34 (dt, *J* = 13.6, 8.0 Hz, 1H), 2.19 (ddd, *J* = 13.6, 8.8, 4.4 Hz, 1H), 1.25 (t, *J* = 7.2 Hz, 3H), 1.20 (t, *J* = 7.2 Hz, 3H).
(5) To a solution of compound **44-7** (2.00 g, 5.65 mmol) in ethanol (20.0 mL) was added paraformaldehyde (2.00 g) and sodium cyanoborohydride (1.07 g, 17.0 mmol), and the reaction solution was reacted for 10 hours at 25°C under nitrogen protection. Then the reaction solution was added with water (100 mL), and extracted with ethyl acetate (100 mL). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 6:1) to obtain compound **44-8.**
   MS-ESI [M+H]⁺, calculated: 368, found: 368.
   ¹H NMR (400 MHz, CDCl₃) *δ* 7.18-7.24 (m, 1H), 7.12-7.17 (m, 2H), 4.11 (q, *J* = 7.2 Hz, 2H), 3.82-3.98 (m, 2H), 2.83-3.02 (m, 3H), 2.71-2.81 (m, 1H), 2.56-2.68 (m, 2H), 2.23-2.48 (m, 4H), 2.17 (s, 3H), 1.25 (t, *J* = 7.2 Hz, 3H), 1.02 (t, *J* = 7.2 Hz, 3H).
(6) To a solution of compound **44- 8** (1.50 g, 4.08 mmol) in tetrahydrofuran (15.0 mL) was added potassium bis (trimethylsilyl) amide (1 mol/L, 8.16 mL, tetrahydrofuran solution) at -78°C, and the reaction solution was reacted at -65°C for 2 hours under nitrogen protection. Then the reaction solution was added with saturated ammonium chloride aqueous solution (50.0 mL), and extracted with ethyl acetate (50.0 mL). The combined organic phase was washed with saturated NaCl aqueous solution (50.0 mL), dried over anhydrous sodium sulfate, filtered, and concentrated under reduced pressure. The crude product was separated by silica gel column chromatography (petroleum ether/ethyl acetate = 1:0 to 6:1) to obtain compound **44-9.**
   MS-ESI [M+H]⁺, calculated: 322, found: 322.
(7) To a solution of compound **44-9** (1.08 g, 3.36 mmol) in ethanol (10.0 mL) was added compound **44-10**(510 mg, 6.71 mmol) and sodium ethoxide (685 mg, 10.1 mmol), and the reaction solution was stirred at 80°C for 12 hours under nitrogen protection. After being concentrated under reduced pressure, the reaction solution was adjusted to pH 6 with hydrochloric acid (1 mol/L). The resulting solution was precipitated, and filtered. The filter cake was dried to obtain compound **44-11.**
   MS-ESI [M+H]⁺, calculated: 334, found: 334.
   ¹H NMR (400 MHz, DMSO-*d*6) *δ* 12.42-12.50 (m, 1H), 12.31 (br d, *J* = 2.0 Hz, 1H), 7.13-7.51 (m, 3H), 3.36-3.42 (m, 2H), 2.85-2.99 (m, 2H), 2.63-2.70 (m, 1H), 2.53 (br d, *J* = 2.0 Hz, 1H), 2.29-2.37 (m, 1H), 2.16-2.28 (m, 1H), 1.88-2.15 (m, 3H).
(8) To a solution of intermediate **44-11** (848 mg, 2.54 mmol) in water (10.0 mL) was added chloroacetic acid (1.20 g, 12.7 mmol), and the reaction solution was stirred at 100°C for 16 hours under nitrogen protection. After the reaction solution was filtered, the filtrate was adjusted to pH 8 with saturated sodium bicarbonate aqueous solution to get precipitate. After filtration, the filter cake was dried to obtain compound **44-12.**
   MS-ESI [M+H]⁺, calculated: 318, found: 318.
   ¹H NMR (400 MHz, DMSO-d6) *δ* 11.03 (br s, 1H), 10.78 (br s, 1H), 7.28 (s, 2H), 7.15-7.20 (m, 1H), 2.75-3.25 (m, 4H), 2.31-2.40 (m, 2H), 2.20 (dt, *J* = 13.6, 8.0 Hz, 1H), 1.96 (s, 3H), 1.69 (ddd, *J* = 13.6, 8.8, 4.8 Hz, 1H).
(9) Compound **44-12** (236 mg, 743 µmol) was dissolved in phosphorus oxychloride (13.3 g, 86.7 mmol), and the reaction solution was stirred at 80°C for 6 hours under nitrogen protection. Then the reaction solution was concentrated under reduced pressure, and extracted with dichloromethane (10.0 mL). The organic phase was washed with saturated sodium bicarbonate aqueous solution (10.0 mL × 2) and saturated NaCl aqueous solution (10.0 mL× 2), dried over anhydrous sodium sulfate, filtered, and concentrated under reduced pressure to obtain compound **44-13.**
   MS-ESI [M+H]⁺, calculated: 354, found: 354.
(10) To a solution of compound **44-13** (600 mg, 1.69 mmol) in *N*-methylpyrrolidone (10.0 mL) was added potassium carbonate (701 mg, 5.08 mmol) and compound **44-14** (419 mg, 1.86 mmol), and the reaction solution was stirred at 50°C for 10 hours under nitrogen protection. Then the reaction solution was added with ethyl acetate (30.0 mL), washed with saturated NaCl aqueous solution (30.0 mL × 5), dried over anhydrous sodium sulfate, filtered, and concentrated under reduced pressure. The crude product was separated by silica gel column chromatography (petroleum ether/ethyl acetate = 1:0 to 3:1) to give compound **44-15.**
   MS-ESI [M+H]⁺, calculated: 543, found: 543.
(11) To a solution of compound **44-15** (200 mg, 367 µmol) in dioxane (5.00 mL) was added compound **44-16** (175 mg, 1.10 mmol), potassium carbonate (127 mg, 1.10mmol), tris(dibenzylidene acetone) dipalladium (67.4 mg, 73.6 µmol) and 2-dicyclohexylphosphine-2', 6'-diisopropoxy-1,1'-biphenyl (68.6 mg, 147 µmol), and the reaction solution was stirred at 110°C for 4 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by silica gel column chromatography (dichloromethane/methanol = 1:0 to 3:1) to obtain compound **44-17.**
   MS-ESI [M+H]⁺, calculated: 666, found: 666.
(12) To a solution of compound **44-17** (74.0 g, 111 µmol) in dichloromethane (3.0 mL) was added trifluoroacetic acid (2.85 g, 24.9 mmol), and the reaction solution was stirred at 25°C for 1 hour under nitrogen protection. Then the reaction solution was extracted with ethyl acetate (50 mL × 2) and the organic phase was washed by saturated sodium bicarbonate aqueous solution (50 mL) and saturated NaCl aqueous solution (50 mL), dried over anhydrous sodium sulfate, filtered, and concentrated under reduced pressure to obtain crude compound **44-18.**
   MS-ESI [M+H]⁺, calculated: 566, found: 566.
(13)To a solution of compound **44-18** (65.0 mg, 114 µmol) in ethyl acetate (3.0 mL) was added triethylamine (34.8 mg, 344 µmol), compound **44-19** (31.0 mg, 344 µmol), 4A molecular sieve(40.0 mg) and propanephosphonic acid anhydride solution (219 mg, 344 µmol, 50% ethyl acetate solution) at 0°C, and the reaction solution was stirred at 25°C for 1 hour. Then the reaction solution was added with dichloromethane (10.0 mL), washed with saturated NaCl aqueous solution (10.0 mL × 2), dried over anhydrous sodium sulfate, filtered, and concentrated under reduced pressure. The crude product was separated by preparative high performance liquid chromatography (Phenomenex Luna C18, 75 mm × 30 mm ×3 µm, A: water (10 mmol/L ammonium bicarbonate); B: acetonitrile, 41%-61%: 11 min) to obtain compound **44-20.**
(14) Compound **44-20** was separated by chiral supercritical fluid chromatography to obtain compounds **44** and **45.**

Separation conditions: Chromatographic column model:Chiralpak IC-3; column specification: 50 × 4.6mm I.D., 3 µm; Injection volume: 8.0 µL; Mobile phase: A: Carbon dioxide, B: Ethanol (0.05% diethylamine), B%: 40% fixed concentration elution for 4 min; detection wavelength: 254 nm; column temperature: 35°C.

**Compound 44:** retention time: 1.342 minutes, 98.64% ee.

MS-ESI [M+H]⁺, calculated: 638, found: 638.

¹H NMR (400 MHz, MeOD) *δ* 7.24-7.32 (m, 2H), 7.16 (dd, *J* = 7.2, 1.2 Hz, 1H), 5.32-5.40 (m, 1H), 5.23-5.31 (m, 1H), 4.09-4.31 (m, 4H), 4.00 (br d, *J* = 12.4 Hz, 1H), 3.71 (s, 2H), 3.27 (br s, 2H), 3.20-3.23 (m, 1H), 2.90-3.16 (m, 8H), 2.39-2.50 (m, 1H), 2.16-2.25 (m, 5H), 2.08-2.15 (m, 1H), 1.96-2.04 (m, 2H), 1.91 (ddd, *J* = 13.6, 8.8, 4.4 Hz, 2H), 1.25-1.36 (m, 4H).

**Compound 45:** retention time: 1.807 minutes, 97.24% ee.

MS-ESI [M+H]⁺, calculated: 638, found: 638.

¹H NMR (400 MHz, MeOD) *δ* 7.24-7.32 (m, 2H), 7.16 (dd, *J* = 7.2, 1.2 Hz, 1H), 5.32-5.40 (m, 1H), 5.23-5.31 (m, 1H), 4.09-4.31 (m, 4H), 4.00 (br d, *J* = 12.4 Hz, 1H), 3.71 (s, 2H), 3.27 (br s, 2H), 3.20-3.23 (m, 1H), 2.90-3.16 (m, 8H), 2.39-2.50 (m, 1H), 2.16-2.25 (m, 5H), 2.08-2.15 (m, 1H), 1.96-2.04 (m, 2H), 1.91 (ddd, *J* = 13.6, 8.8, 4.4 Hz, 2H), 1.25-1.36 (m, 4H).

### Example 37 Synthesis of Compounds 46 and 47

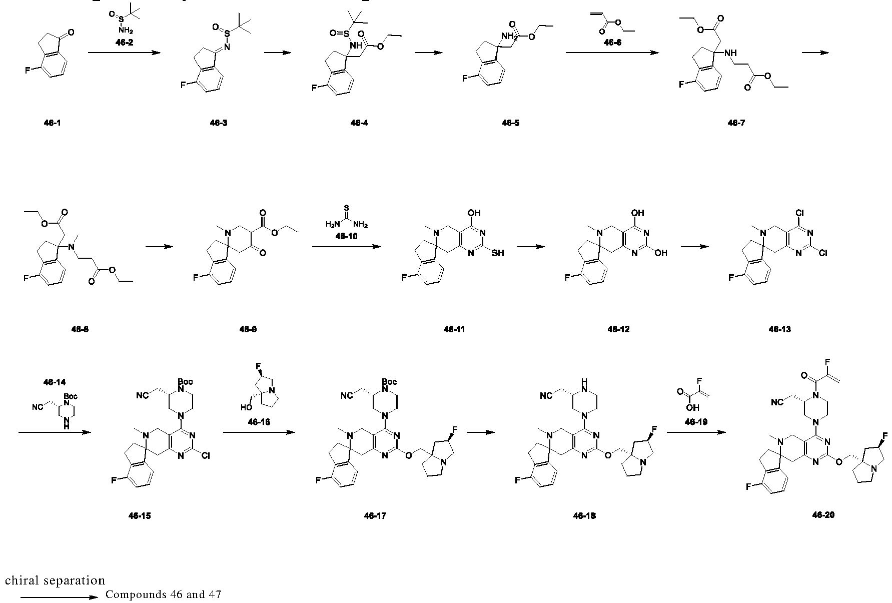

(1) To a solution of compound **46-1** (21.0 g, 140 mmol) in toluene (300 mL) was added compound **46-2** (20.3 g, 168 mmol), tetraethyl titanate (63.8 g, 280 mmol), and the reaction solution was stirred at 110°C for 2 hours under nitrogen protection. Then the reaction solution was added with water (200 mL), and filtered. The filter cake was washed with ethyl acetate (500 mL × 3). The combined organic phase was washed with saturated NaCl aqueous solution (150 mL), dried over anhydrous sodium sulfate, filtered, and concentrated under reduced pressure. The crude product was separated by silica gel column chromatography (petroleum ether/ethyl acetate = 1:0 to 2:1) to obtain compound **46-3.**
   MS-ESI [M+H]⁺, calculated: 254, found: 254.
   ¹H NMR (400 MHz, CDCl₃) *δ* 7.59 (d, *J* = 8.0 Hz, 1H), 7.28-7.34 (m, 1H), 7.13-7.20 (m, 1H), 3.44-3.55 (m, 1H), 3.10-3.20 (m, 3H), 1.32 (s, 9H).
(2) To a solution of ethyl acetate (27.0g, 306 mmol) in tetrahydrofuran (200 mL) was added dropwise with lithium diisopropylamide (2 mol/L, 61.2 mL, tetrahydrofuran solution) at -78°C under nitrogen protection, and the reaction solution was stirred at -78°C for 1 hour. Then the reaction solution was added dropwise with a solution of compound **46-3** (15.5 g, 61.2 mmol) in tetrahydrofuran (200 mL), and continued stirring for 2 hours at -78°C. Then the reaction solution was quenched with saturated ammonium chloride aqueous solution (200 mL), and extracted with ethyl acetate (100 mL × 3). The combined organic phase was washed with saturated ammonium chloride aqueous solution (200 mL× 1) and saturated NaCl aqueous solution (100 mL× 3), dried over anhydrous sodium sulfate, filtered, and concentrated under reduced pressure. The crude product was separated by silica gel column chromatography (petroleum ether/ethyl acetate = 1:0 to 2:1) to obtain compound **46-4.**
   MS-ESI [M+H]⁺, calculated: 342, found: 342.
   ¹H NMR (400 MHz, CDCl₃) *δ* 7.16-7.24 (m, 1H), 6.91-7.02 (m, 2H), 4.12-4.22 (m, 2H), 3.13-3.24 (m, 1H), 2.83-2.97 (m, 2H), 2.67-2.80 (m, 2H), 2.30-2.41 (m, 1H), 1.25 (t, *J* = 8.0 Hz, 3H), 1.19 (s, 9H).
(3) To a solution of compound **46-4** (4.50 g, 13.2 mmol) in ethanol (30.0 mL) was added hydrochloric acid in dioxane (4 mol/L, 10.0 mL), and the reaction solution was stirred at 25°C for 1 hour under nitrogen protection. Then the reaction solution was concentrated under reduced pressure to obtain crude product of hydrochloride of compound **46-5.**
(4) To a solution of hydrochloride of compound **46-5** (6.32 g, 13.2 mmol) in ethanol (40.0 mL) was added compound **46-6** (13.8 g, 138 mmol), copper oxide (209 mg, 2.63 mmol), and triethylamine (2.66 g, 26.3 mmol), and the reaction solution was reacted at 80°C in a sealed tube for 12 hours under nitrogen protection. Then the reaction solution was added with water (100 mL), and extracted with ethyl acetate (150 mL × 2). The combined organic phase was washed with saturated NaCl aqueous solution (100 mL × 2), dried over anhydrous sodium sulfate, filtered, and concentrated under reduced pressure. The crude product was separated by silica gel column chromatography (petroleum ether/ethyl acetate = 1:0 to 2:1) to obtain compound **46-7.**
   MS-ESI [M+H]⁺, calculated: 338, found: 338.
   ¹H NMR (400 MHz, CDCl₃) *δ* 7.14-7.21 (m, 1H), 6.98-7.07 (m, 1H), 6.90 (t, *J* = 8.0 Hz, 1H), 4.07-4.15 (m, 4H), 2.94-3.06 (m, 1H), 2.84-2.92 (m, 1H), 2.61-2.79 (m, 3H), 2.49-2.56 (m, 1H), 2.40-2.47 (m, 2H), 2.30-2.39 (m, 1H), 2.16-2.25 (m, 1H), 1.22-1.26 (m, 3H), 1.20 (t, *J* = 8.0 Hz, 3H).
(5) To a solution of compound **46-7** (4.10 g, 12.2 mmol) in ethanol (50.0 mL) was added paraformaldehyde (3.65 g), sodium cyanoborohydride (2.29 g, 36.5 mmol), and acetic acid (1.46 g, 24.3 mmol), and the reaction solution was reacted for 15 hours at 35°C under nitrogen protection. Then the reaction solution was added with water (30.0 mL), and extracted with ethyl acetate (100 mL). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 2:1) to obtain compound **46-8.**
   MS-ESI [M+H]⁺, calculated: 352, found: 352.
   ¹H NMR (400 MHz, CDCl₃) *δ* 7.12-7.19 (m, 1H), 7.02 (d, *J* = 8.0 Hz, 1H), 6.89 (t, *J* = 8.0 Hz, 1H), 4.05-4.15 (m, 2H), 3.84-3.96 (m, 2H), 2.84-2.98 (m, 3H), 2.72-2.81 (m, 1H), 2.56-2.68 (m, 2H), 2.28-2.47 (m, 4H), 2.16 (s, 3H), 1.20-1.27 (m, 3H), 0.99-1.05 (m, 3H).
(6) To a solution of compound **46-8** (3.40 g, 9.68 mmol) in tetrahydrofuran (50.0 mL) was added potassium bis (trimethylsilyl) amide (1 mol/L, 29.0 mL, tetrahydrofuran solution) at -78°C, and the reaction solution was reacted at -78°C for 2 hours under nitrogen protection. Then the reaction solution was added with saturated ammonium chloride aqueous solution (50.0 mL), and extracted with dichloromethane (50.0 mL). The combined organic phase was washed with saturated NaCl aqueous solution (50.0 mL), dried over anhydrous sodium sulfate, filtered, and concentrated under reduced pressure. The crude product was separated by silica gel column chromatography (petroleum ether/ethyl acetate = 1:0 to 2:1) to obtain compound **46-9.**
   MS-ESI [M+H]⁺, calculated: 306, found: 306.
   ¹H NMR (400 MHz, CDCl₃) *δ* 7.19-7.26 (m, 1H), 7.02-7.14 (m, 1H), 6.88-7.01 (m, 1H), 4.21-4.32 (m, 2H), 3.47-3.57 (m, 1H), 3.13-3.33 (m, 2H), 2.88-3.06 (m, 2H), 2.61-2.73 (m, 1H), 2.34-2.44 (m, 1H), 2.22-2.33 (m, 1H), 2.06-2.13 (m, 3H), 1.77-1.89 (m, 1H), 1.30-1.34 (m, 3H).
(7) To a solution of compound **46-9** (2.50 g, 8.19 mmol) in ethanol (50.0 mL) was added compound **46-10**(1.25 g, 16.4 mmol) and sodium ethoxide (1.67 g, 24.6 mmol), and the reaction solution was stirred at 80°C for 15 hours under nitrogen protection. After being concentrated under reduced pressure, the reaction solution was adjusted to pH 6 with hydrochloric acid (1 mol/L). The resulting solution was precipitated, and filtered. The filter cake was dried to obtain compound **46-11.**
   MS-ESI [M+H]⁺, calculated: 318, found: 318.
   ¹H NMR (400 MHz, DMSO-*d*6) *δ* 7.28-7.35 (m, 1H), 7.03-7.12 (m, 2H), 3.41-3.51 (m, 1H), 3.06 (d, *J* = 16.0 Hz, 1H), 2.82-2.99 (m, 2H), 2.59-2.69 (m, 1H), 2.51-2.53 (m, 1H), 2.16-2.27 (m, 1H), 1.98 (s, 3H), 1.68-1.76 (m, 1H).
(8) To a solution of intermediate **46-11** (2.20 g, 6.93 mmol) in water (50.0 mL) was added chloroacetic acid (3.30 g, 34.9 mmol), and the reaction solution was stirred at 100°C for 15 hours under nitrogen protection. After the reaction solution was filtered, the filtrate was adjusted to pH 8 with saturated sodium bicarbonate aqueous solution to get precipitate. After filtration, the filter cake was dried to obtain compound **46-12.**
   MS-ESI [M+H]⁺, calculated: 302, found: 302.
   ¹H NMR (400 MHz, DMSO-*d*6) *δ* 11.02 (s, 1H), 10.78 (s, 1H), 7.27-7.35 (m, 1H), 7.02-7.12 (m, 2H), 3.43 (s, 1H), 2.99-3.06 (m, 1H), 2.82-2.96 (m, 2H), 2.54-2.63 (m, 1H), 2.33-2.43 (m, 1H), 2.17-2.27 (m, 1H), 1.97 (s, 3H), 1.65-1.77 (m, 1H).
(9) Compound **46-12** (400 mg, 1.33 mmol) was dissolved in phosphorus oxychloride (10.0 mL), and the reaction solution was stirred at 80°C for 12 hours under nitrogen protection. Then the reaction solution was concentrated under reduced pressure, and extracted with dichloromethane (20.0 mL). The organic phase was washed with saturated sodium bicarbonate aqueous solution (20.0 mL × 2) and saturated NaCl aqueous solution (20.0 mL× 2), dried over anhydrous sodium sulfate, filtered, and concentrated under reduced pressure to obtain compound **46-13.**
   MS-ESI [M+H]⁺, calculated: 338, found: 338.
(10) To a solution of compound **46-13** (600 mg, 1.31 mmol) in *N*-methylpyrrolidone (20.0 mL) was added potassium carbonate (701 mg, 3.94 mmol) and compound **46-14**(444 mg, 1.97 mmol), and the reaction solution was stirred at 50°C for 5 hours under nitrogen protection. Then the reaction solution was added with ethyl acetate (50.0 mL), washed with saturated NaCl aqueous solution (30.0 mL × 5), dried over anhydrous sodium sulfate, filtered, and concentrated under reduced pressure. The crude product was separated by silica gel column chromatography (dichloromethane/methanol = 1:0 to 10:1) to obtain compound **46-15.**
   MS-ESI [M+H]⁺, calculated: 527, found: 527.
   ¹H NMR (400 MHz, CDCl₃) *δ* 7.20-7.26 (m, 1H), 6.93-7.07 (m, 2H), 3.96-4.07 (d, *J* = 12.0 Hz, 2H), 3.59-3.85 (m, 2H), 3.38-3.52 (m, 1H), 3.21-3.31 (m, 1H), 2.94-3.12 (m, 6H), 2.61-2.74 (m, 2H), 2.30-2.43 (m, 1H), 2.18-2.28 (m, 3H), 1.82-1.94 (m, 2H), 1.50 (s, 9H).
(11) To a solution of compound **46-15** (500 mg, 949 µmol) in dioxane (15.0 mL) was added compound **46-16** (227 mg, 1.42 mmol), cesium carbonate (927 mg, 2.85 mmol) and (2-dicyclohexylphosphino-2',6'-diisopropoxy-1,1'-biphenyl) [2-(2'-amino-1,1'-biphenyl)] palladium(II) methanesulfonate (159 mg, 190 µmol), and the reaction solution was stirred at 110°C for 4 hours under nitrogen protection. Then the reaction solution was added with ethyl acetate (50.0 mL), washed with saturated NaCl aqueous solution (50.0 mL × 2), dried over anhydrous sodium sulfate, filtered, and concentrated under reduced pressure. The crude product was separated by silica gel column chromatography (dichloromethane/methanol = 1:0 to 10:1) to obtain compound **46-17.**
   MS-ESI [M+H]⁺, calculated: 650, found: 650.
   ¹H NMR (400 MHz, CDCl₃) *δ* 7.18-7.25 (m, 1H), 6.90-7.03 (m, 2H), 4.13-4.18 (m, 1H), 3.91-4.08 (m, 2H), 3.73-3.89 (m, 1H), 3.62-3.72 (m, 1H), 3.55 (br d, *J* = 12.0 Hz, 1H), 3.13-3.48 (m, 4H), 2.86-3.11 (m, 6H), 2.57-2.78 (m, 2H), 2.28-2.43 (m, 2H), 2.12-2.27 (m, 5H), 1.59-2.02 (m, 8H), 1.42-1.57 (m, 9H).
(12) To a solution of compound **46-17** (300 g, 462 µmol) in dichloromethane (5.0 mL) was added trifluoroacetic acid (1.54 g, 13.5 mmol), and the reaction solution was stirred at 25°C for 2 hours under nitrogen protection. Then the reaction solution was extracted with ethyl acetate (50 mL × 2) and the organic phase was washed by saturated sodium bicarbonate aqueous solution (50 mL) and saturated NaCl aqueous solution (50 mL), dried over anhydrous sodium sulfate, filtered, and concentrated under reduced pressure to obtain crude compound **46-18.**
   MS-ESI [M+H]⁺, calculated: 550, found: 550.
(13) To a solution of compound **46-18** (150 mg, 273 µmol) in ethyl acetate(3.0 mL) was added triethylamine (82.8 mg, 819 µmol), compound **46-19**(73.7 mg, 819 µmol), 4A molecular sieve(150 mg) and propanephosphonic acid anhydride solution(521 mg, 819 µmol, 50% ethyl acetate solution) at 0°C, and the reaction solution was stirred at 25°C for 1 hour. Then the reaction solution was added with dichloromethane (10.0 mL), washed with saturated NaCl aqueous solution (10.0 mL × 2), dried over anhydrous sodium sulfate, filtered, and concentrated under reduced pressure. The crude product was separated by silica gel column chromatography (dichloromethane/methanol = 1:0 to 10:1) to obtain compound **46-20.**
(14) Compound **46-20** was separated by chiral supercritical fluid chromatography to obtain compounds **46** and **47.**

Separation conditions: Chromatographic column model: Chiralpak OX-3; column specification: 10 × 4.6mm I.D., 3 µm; Injection volume: 8.0 µL; Mobile phase: A: carbon dioxide, B: ethanol (0.05% diethylamine), B%: 5%-40% gradient elution for 4 minutes, 40% fixed concentration elution for 1 minute, 10% fixed concentration elution for 1 minute; Detection wavelength: 254 nm; Column temperature: 35°C.

**Compound 46:** retention time 3.680 minutes, 100%ee.

MS-ESI [M+H]⁺, calculated: 622, found: 622.

¹H NMR (400 MHz, MeOD) *δ* 7.27-7.34 (m, 1H), 6.98-7.09 (m, 1H), 6.98-7.03 (m, 1H), 5.25-5.45 (m, 3H), 4.89-5.01 (m, 1H), 4.26-4.40 (m, 2H), 4.04-4.23 (m, 3H), 3.69-3.82 (m, 2H), 3.44-3.62 (m, 4H), 3.08-3.29 (m, 3H), 3.03-3.08 (m, 1H), 2.90-3.02 (m, 5H), 2.33-2.53 (m, 3H), 2.23 (s, 4H), 2.10-2.17 (m, 2H), 1.98-2.06 (m, 1H), 1.86-2.06 (m, 1H).

**Compound 47:** retention time: 4.052 minutes, 98.44%ee.

MS-ESI [M+H]⁺, calculated: 622, found: 622.

¹H NMR (400 MHz, MeOD) *δ* 7.24-7.33 (m, 1H), 6.95-7.08 (m, 2H), 5.19-5.43 (m, 3H), 4.25 (d, *J* = 12.0 Hz, 1H), 4.03-4.22 (m, 3H), 3.96-4.03 (m, 1H), 3.71 (s, 2H), 3.18-3.28 (m, 4H), 3.09-3.17 (m, 2H), 2.96-3.08 (m, 3H), 2.93 (d, *J* = 4.0 Hz, 2H), 2.42-2.52 (m, 1H), 2.27-2.39 (m, 1H), 2.16-2.27 (m, 4H), 2.05-2.15 (m, 1H), 1.84-2.03 (m, 4H), 1.29 (s, 3H).

### Example 38 Synthesis of Compounds 48 and 49

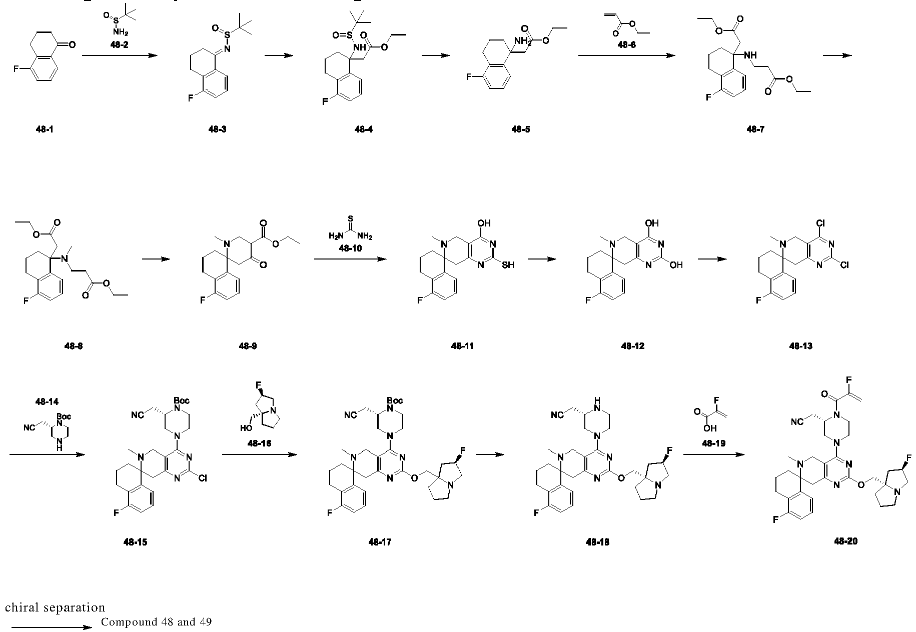

(1) To a solution of compound **48-1** (5.00 g, 30.4 mmol) in toluene (150 mL) was added compound **48-2** (3.69 g, 30.4 mmol), tetraethyl titanate (13.8 g, 60.9 mmol), and the reaction solution was stirred at 120°C for 10 hours under nitrogen protection. Then the reaction solution was added with water (300 mL), and filtered. The filter cake was washed with ethyl acetate (300 mL × 3). The combined organic phase was washed with saturated NaCl aqueous solution (300 mL), dried over anhydrous sodium sulfate, filtered, and concentrated under reduced pressure. The crude product was separated by silica gel column chromatography (petroleum ether/ethyl acetate = 1:0 to 0:1) to obtain compound **48-3.**
   MS-ESI [M+H]⁺, calculated: 268, found: 268.
   ¹H NMR (400 MHz, CDCl₃) *δ* 7.95 (d, *J* = 7.64 Hz, 1H), 7.18-7.24 (m, 1H), 7.11-7.17 (m, 1H), 3.22-3.32 (m, 1H), 3.03-3.11 (m, 1H), 2.77-2.95 (m, 2H), 1.91-2.10 (m, 2H) 1.32 (s, 9H).
(2) To a solution of ethyl acetate (8.57 g, 97.2 mmol) in tetrahydrofuran (50 mL) was added dropwise with lithium diisopropylamide (2 mol/L, 19.4 mL, tetrahydrofuran solution) at -78°C under nitrogen protection, and the reaction solution was stirred at -78°C for 1 hour. Then the reaction solution was added dropwise with a solution of compound **48-3** (15.5 g, 61.2 mmol) in tetrahydrofuran (200 mL), and continued stirring for 2 hours at -78°C. Then the reaction solution was quenched with saturated ammonium chloride aqueous solution (200 mL), and extracted with ethyl acetate (100 mL × 3). The combined organic phase was washed with saturated ammonium chloride aqueous solution (200 mL× 1) and saturated NaCl aqueous solution (100 mL× 3), dried over anhydrous sodium sulfate, filtered, and concentrated under reduced pressure. The crude product was separated by silica gel column chromatography (petroleum ether/ethyl acetate = 1:0 to 0:1) to obtain compound **48-4.**
   MS-ESI [M+H]⁺, calculated: 356, found: 356.
   ¹H NMR (400 MHz, CDCl₃) *δ* 7.08-7.20 (m, 2H), 6.92 (d, *J* = 9.2, 1.6 Hz, 1H), 5.17 (s, 1H), 4.13-4.21 (m, 2H), 2.81-2.88 (m, 2H), 2.68-2.80 (m, 2H), 2.39-2.49 (m, 1H), 2.11-2.18 (m, 1H), 2.04-2.10 (m, 1H), 1.74-1.86 (m, 1H), 1.25-1.28 (m, 3H), 1.22-1.24 (m, 9H).
(3) To a solution of compound **48-4** (3.70 g, 10.4 mmol) in ethanol (30.0 mL) was added hydrochloric acid in dioxane (4 mol/L, 10.0 mL), and the reaction solution was stirred at 25°C for 1 hour under nitrogen protection. Then the reaction solution was concentrated under reduced pressure to obtain crude product of hydrochloride of compound **48-5.**
(4) To a solution of hydrochloride of compound **48-5** (2.60 g, 10.3 mmol) in ethanol (40.0 mL) was added compound **48-6** (10.3 g, 103 mmol), copper oxide (164 mg, 2.07 mmol), and triethylamine (3.14 g, 31.0 mmol), and the reaction solution was reacted at 85°C in a sealed tube for 12 hours under nitrogen protection. Then the reaction solution was added with water (50.0 mL), and extracted with ethyl acetate (50.0 mL × 2). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 0:1) to obtain compound **48-7.**
   MS-ESI [M+H]⁺, calculated: 352, found: 352.
   ¹H NMR (400 MHz, CDCl₃) *δ* 7.36 (s, 1H), 7.14 (q, 1H), 6.81-6.94 (m, 1H), 4.06-4.19 (m, 4H), 2.72-2.86 (m, 3H), 2.57-2.69 (m, 2H), 2.43 (s, 3H), 2.02-2.11 (m, 1H), 1.94 (d, *J* = 4.8 Hz, 2H), 1.84 (d, *J* = 10.8, 5.6 Hz, 1H) 1.25 (m, 3H), 1.21 (t, *J* = 7.2 Hz, 3H).
(5) To a solution of compound **48-7** (1.90 g, 5.41 mmol) in ethanol (30.0 mL) was added paraformaldehyde (1.62 g) and sodium cyanoborohydride (1.02 g, 16.2 mmol), and the reaction solution was reacted for 16 hours at 30°C under nitrogen protection. Then the reaction solution was added with water (100 mL), and extracted with ethyl acetate (100 mL). The combined organic phase was washed with saturated NaCl aqueous solution (50.0 mL × 2), dried over anhydrous sodium sulfate, filtered, and concentrated under reduced pressure. The crude product was separated by silica gel column chromatography (petroleum ether/ethyl acetate = 1:0 to 0:1) to obtain compound **48-8.**
   MS-ESI [M+H]⁺, calculated: 366, found: 366.
   ¹H NMR (400 MHz, CDCl₃) *δ* 7.31 (d, *J* = 8.0 Hz, 1H), 7.05-7.12 (m, 1H), 6.81-6.88 (m, 1H), 4.09 (q, *J* = 7.2 Hz, 2H), 3.81-3.92 (m, 2H), 2.69-2.81 (m, 4H), 2.54-2.68 (m, 2H), 2.31-2.48 (m, 2H), 2.21 (s, 3H), 2.04-2.13 (m, 2H), 1.81 (q, *J* = 6.4 Hz, 2H), 1.23 (t, *J* = 7.2 Hz, 3H), 1.01 (t, *J* = 7.2 Hz, 3H).
(6) To a solution of compound **48-8** (1.70 g, 4.65 mmol) in tetrahydrofuran (30.0 mL) was added potassium bis (trimethylsilyl) amide (1 mol/L, 9.30 mL, tetrahydrofuran solution) at -78°C, and the reaction solution was reacted at -78°C for 2 hours under nitrogen protection. Then the reaction solution was added with saturated ammonium chloride aqueous solution (50.0 mL), and extracted with ethyl acetate (50.0 mL). The combined organic phase was washed with saturated NaCl aqueous solution (50.0 mL), dried over anhydrous sodium sulfate, filtered, and concentrated under reduced pressure. The crude product was separated by silica gel column chromatography (petroleum ether/ethyl acetate = 1:0 to 0:1) to obtain compound **48-9.**
   MS-ESI [M+H]⁺, calculated: 320, found: 320.
   ¹H NMR (400 MHz, CDCl₃) *δ* 7.26-7.56 (m, 1H), 7.13-7.24 (m, 1H), 6.81-7.01 (m, 1H), 4.08-4.36 (m, 2H), 3.42-3.61 (m, 1H), 3.09-3.21 (m, 1H), 2.86-2.96 (m, 1H), 2.49-2.77 (m, 2H), 2.26-2.48 (m, 1H), 2.01 (s, 3H), 1.61-1.81 (m, 3H), 1.59 ( s, 2H), 1.28-1.35 (m, 3H).
(7) To a solution of compound **48-9** (1.20 g, 3.76 mmol) in ethanol (20.0 mL) was added compound **48-10** (572 mg, 7.51 mmol) and sodium ethoxide (767 mg, 11.2 mmol), and the reaction solution was stirred at 80°C for 15 hours under nitrogen protection. After being concentrated under reduced pressure, the reaction solution was adjusted to pH 6 with hydrochloric acid (1 mol/L). The resulting solution was precipitated, and filtered. The filter cake was dried to obtain compound **48-11.**
   MS-ESI [M+H]⁺, calculated: 332, found: 332.
(8) To a solution of intermediate **48-11** (1.10 g, 3.25 mmol) in water (30.0 mL) was added chloroacetic acid (1.25 g, 13.2 mmol), and the reaction solution was stirred at 100°C for 12 hours under nitrogen protection. After the reaction solution was filtered, the filtrate was adjusted to pH 8 with saturated sodium bicarbonate aqueous solution to get precipitate. After filtration, the filter cake was dried to obtain compound **48-12.**
   MS-ESI [M+H]⁺, calculated: 316, found: 316.
   ¹H NMR (400 MHz, DMSO-d6) *δ* 11.03 (s, 1H), 10.75 (s, 1H), 7.38 (d, *J* = 7.6 Hz, 1H), 7.20-7.32 (m, 1H), 7.04 (t, *J* = 8.4 Hz, 1H), 3.52 (d, *J* = 14.8 Hz, 1H), 3.05 (d, *J* = 14.0 Hz, 1H), 2.81 (d, *J* = 16.4 Hz, 1H), 2.52-2.59 (m, 2H), 2.40-2.48 (m, 1H), 1.94 (s, 4H), 1.70-1.80 (m, 1H), 1.47-1.61 (m, 2H).
(9) Compound **48-12** (200 mg, 634 µmol) was dissolved in phosphorus oxychloride (2.0 mL), and the reaction solution was stirred at 80°C for 12 hours under nitrogen protection. Then the reaction solution was concentrated under reduced pressure, and extracted with dichloromethane (20.0 mL). The organic phase was washed with saturated sodium bicarbonate aqueous solution (20.0 mL × 2) and saturated NaCl aqueous solution (20.0 mL× 2), dried over anhydrous sodium sulfate, filtered, and concentrated under reduced pressure to obtain compound **48-13.**
   MS-ESI [M+H]⁺, calculated: 352, found: 352.
(10) To a solution of compound **48-13** (150 mg, 425 µmol) in *N*-methylpyrrolidone (5.0 mL) was added potassium carbonate (176 mg, 1.28 mmol) and compound **48-14** (115 mg, 551 µmol), and the reaction solution was stirred at 50°C for 5 hours under nitrogen protection. Then the reaction solution was added with ethyl acetate (50.0 mL), washed with saturated NaCl aqueous solution (30.0 mL × 5), dried over anhydrous sodium sulfate, filtered, and concentrated under reduced pressure. The crude product was separated by silica gel column chromatography (petroleum ether/ethyl acetate = 1:0 to 0:1) to give compound **48-15.**
   MS-ESI [M+H]⁺, calculated: 541, found: 541.
   ¹H NMR (400 MHz, CDCl₃) *δ* 7.35 (s, 1H), 7.20 (s, 1H), 6.83-7.01 (m, 1H), 4.57 (s, 1H), 4.00 (s, 2H), 3.82 (d, *J* = 11.2 Hz, 1H), 3.72 (dd, *J* = 6.0, 3.2 Hz, 1H), 3.30-3.66 (m, 2H), 3.08-3.29 (m, 3H), 2.80-3.07 (m, 3H), 2.65 (d, *J* = 6.8 Hz, 2H), 2.42-2.52 (m, 1H), 2.12 (s, 2H), 2.02 (s, 1H), 1.19-1.94 (m, 1H), 1.64-1.78 (m, 2H), 1.50 (d, *J* = 2.0 Hz, 9H).
(11) To a solution of compound **48-15** (440 mg, 813 µmol ) in dioxane (10.0 mL) was added compound **48-16** (388 mg, 2.44 mmol), cesium carbonate (794 mg, 2.44 mmol) and (2-dicyclohexylphosphino-2',6'-diisopropoxy-1,1'-biphenyl) [2-(2'-amino-1,1'- biphenyl)] palladium(II) methanesulfonate (136 mg, 162 µmol), and the reaction solution was stirred at 110°C for 4 hours under nitrogen protection. Then the reaction solution was added with ethyl acetate (50.0 mL), washed with saturated NaCl aqueous solution (50.0 mL × 2), dried over anhydrous sodium sulfate, filtered, and concentrated under reduced pressure. The crude product was separated by silica gel column chromatography (dichloromethane/methanol = 1:0 to 10:1) to obtain compound **48-17.**
   MS-ESI [M+H]⁺, calculated: 664, found: 664.
(12) To a solution of compound **48-17** (270 g, 406 µmol) in dichloromethane (5.0 mL) was added hydrochloric acid in dioxane (4 mol/L, 36.7 µmol), and the reaction solution was stirred at 25°C for 2 hours under nitrogen protection. Then the reaction solution was adjusted to pH 8 by saturated sodium bicarbonate aqueous solution and extracted with dichloromethane (20 mL× 2). The organic phase was washed with saturated sodium bicarbonate aqueous solution (20 mL) and saturated NaCl aqueous solution (50 mL), dried over anhydrous sodium sulfate, filtered, and concentrated under reduced pressure. The crude product was separated by silica gel column chromatography (dichloromethane/methanol = 1:0 to 10:1) to obtain compound **48-18.**
   MS-ESI [M+H]⁺, calculated: 564, found: 564.
(13) To a solution of compound **48-18** (200 mg, 354 µmol) in ethyl acetate(1.0 mL) was added triethylamine (17.9 mg, 177 µmol), compound **48-19**(35.9 mg, 354 µmol), 4A molecular sieve (100 mg) and propanephosphonic acid anhydride solution (677 mg, 819 µmol, 50% ethyl acetate solution) at 0°C, and the reaction solution was stirred at 25°C for 1 hour. Then the reaction solution was added with dichloromethane (10.0 mL), washed with saturated NaCl aqueous solution (10.0 mL × 2), dried over anhydrous sodium sulfate, filtered, and concentrated under reduced pressure. The crude product was separated by silica gel column chromatography (dichloromethane/methanol = 1:0 to 10:1) to obtain compound **48-20.**
(14) Compound **48-20** was separated by chiral supercritical fluid chromatography to obtain compounds **48** and **49.**

Separation conditions: Chromatographic column model: Chiralpak OD-3; column specification: 50 × 4.6mm I.D., 3 µm; Injection volume: 8.0 µL; Mobile phase: A: carbon dioxide, B: ethanol (0.05% diethylamine), B%: 5%-40% gradient elution for 2.5 minutes, 40% fixed concentration elution for 0.5 minutes, 5% fixed concentration elution for 1 minute; Detection wavelength: 254 nm; Column temperature: 35°C.

**Compound 48:** retention time: 1.743 minutes, 98.58%ee.

MS-ESI [M+H]⁺, calculated: 636, found: 636.

¹H NMR (400 MHz, MeOD) *δ*7.37 (d, *J* = 8.0 Hz, 1H), 7.20-7.27 (m, 1H), 6.94 (t, *J* = 8.8 Hz, 1H), 5.24-5.42 (m, 3H), 4.38-4.63 (m, 1H), 4.20-4.25 (m, 1H), 4.08-4.19 (m, 3H), 3.77 (s, 2H), 3.46-3.60 (m, 1H), 3.35 (d, *J* = 2.8 Hz, 1H), 3.28 (s, 1H), 3.04-3.14 (m, 3H), 2.89-3.02 (m, 4H), 2.43-2.55 (m, 1H), 2.27-2.39 (m, 1H), 2.15-2.26 (m, 2H), 2.12 (s, 3H), 1.97-2.08 (m, 4H), 1.85-1.97 (m, 2H), 1.70-1.84 (m, 2H), 1.28-1.53 (m, 2H).

**Compound 49:** retention time: 1.937 minutes, 98.18%ee.

MS-ESI [M+H]⁺, calculated: 636, found: 636.

¹H NMR (400 MHz, MeOD) *δ* 7.61 (d, *J* = 8.0 Hz, 1H), 7.39-7.46 (m, 1H), 7.16 (t, *J* = 8.4 Hz, 1H), 5.63 (t, *J* = 3.6 Hz, 0.5H), 5.50 (d, *J* = 3.2 Hz, 0.5H), 5.30-5.43 (m, 2H), 4.62 (s, 1H), 4.53-4.59 (m, 3H), 3.94-4.03 (m, 2H), 3.83-3.93 (m, 3H), 3.41-3.49 (m, 4H), 3.38 (s, 1H), 3.10-3.28 (m, 2H), 2.87-3.10 (m, 3H), 2.57-2.64 (m, 4H), 2.51-2.57 (m, 1H), 2.40 (d, J= 8.4 Hz, 1H), 2.25-2.37 (m, 3H), 2.13-2.24 (m, 4H), 1.81-1.91 (m, 1H), 1.28-1.51 (m, 1H).

### Example 39 Synthesis of Compounds 50 and 51

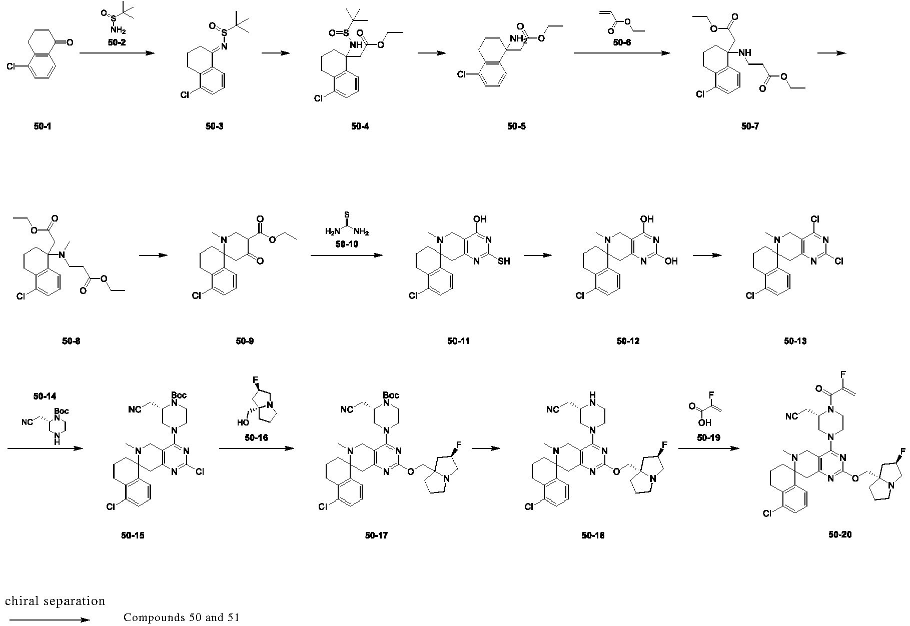

(1) To a solution of compound **50-1** (20.0 g, 111 mmol) in toluene (240 mL) was added compound **50-2** (16.1 g, 133 mmol), tetraethyl titanate (50.5 g, 221 mmol), and the reaction solution was stirred at 110°C for 13 hours under nitrogen protection. Then the reaction solution was added with water (200 mL), and filtered. The filter cake was washed with ethyl acetate (100 mL × 3). The combined organic phase was washed with saturated NaCl aqueous solution (100 mL), dried over anhydrous sodium sulfate, filtered, and concentrated under reduced pressure. The crude product was separated by silica gel column chromatography (petroleum ether/ethyl acetate = 1:0 to 5:1) to obtain compound **50-3.**
   MS-ESI [M+H]⁺, calculated: 284, found: 284.
   ¹H NMR (400 MHz, CDCl₃) *δ* 8.07-8.14 (m, 1H), 7.49 (dd, *J* = 7.6, 1.2 Hz, 1H), 7.21 (t, *J* = 8.0 Hz, 1H), 3.03-3.33 (m, 2H), 2.86-3.02 (m, 2H), 2.05-2.13 (m, 1H), 1.96-2.04 (m, 1H), 1.33 (s, 9H).
(2) To a solution of ethyl acetate (26.9 g, 305 mmol) in tetrahydrofuran (320 mL) was added dropwise with lithium diisopropylamide (2 mol/L, 60.9 mL, tetrahydrofuran solution) at -78°C under nitrogen protection, and the reaction solution was stirred at -78°C for 1 hour. Then the reaction solution was added dropwise with a solution of compound **50-3** (15.5 g, 61.2 mmol) in tetrahydrofuran (200 mL), and continued stirring for 3 hours at -78°C. Then the reaction solution was quenched with saturated ammonium chloride aqueous solution (200 mL), and extracted with ethyl acetate (100 mL × 3). The combined organic phase was washed with saturated ammonium chloride aqueous solution (200 mL× 1) and saturated NaCl aqueous solution (100 mL× 3), dried over anhydrous sodium sulfate, filtered, and concentrated under reduced pressure. The crude product was separated by silica gel column chromatography (petroleum ether/ethyl acetate = 1:0 to 3:1) to obtain compound **50-4.**
   MS-ESI [M+H]⁺, calculated: 372, found: 372.
   ¹H NMR (400 MHz, CDCl₃) *δ* 7.31 (dd, *J* = 17.2, 8.0 Hz, 2H), 7.09-7.16 (m, 1H), 4.11-4.19 (m, 2H), 2.75-2.90 (m, 4H), 2.41-2.49 (m, 1H), 2.06-2.18 (m, 2H), 1.78-1.89 (m, 1H), 1.25-1.28 (m, 3H), 1.24 (s, 9H).
(3) To a solution of compound **50-4** (17.1 g, 46.0 mmol) in ethanol (80.0 mL) was added hydrochloric acid in dioxane (4 mol/L, 38.9 mL), and the reaction solution was stirred at 25°C for 1 hour under nitrogen protection. Then the reaction solution was concentrated under reduced pressure to obtain crude product of hydrochloride of compound **50-5.**
(4) To a solution of hydrochloride of compound **50-5** (4.60 g, 15.1 mmol) in ethanol (30.0 mL) was added compound **50-6** (15.1 g, 151 mmol), copper oxide (241 mg, 3.02 mmol), and triethylamine (1.53 g, 15.1 mmol), and the reaction solution was reacted at 85°C in a sealed tube for 12 hours under nitrogen protection. Then the reaction solution was added with water (90.0 mL), and extracted with ethyl acetate (40.0 mL × 3). The combined organic phase was washed with saturated NaCl aqueous solution (30.0 mL × 1), dried over anhydrous sodium sulfate, filtered, and concentrated under reduced pressure. The crude product was separated by silica gel column chromatography (petroleum ether/ethyl acetate = 1:0 to 3:1) to obtain compound **50-7.**
   MS-ESI [M+H]⁺, calculated: 368, found: 368.
(5) To a solution of compound **50-7** (2.30 g, 6.25 mmol) in ethanol (20.0 mL) was added paraformaldehyde (1.88 g) and sodium cyanoborohydride (1.18 g, 18.8 mmol), and the reaction solution was reacted for 25 hours at 25°C under nitrogen protection. Then the reaction solution was added with water (30.0 mL), and extracted with ethyl acetate (15.0 mL × 3). The combined organic phase was washed with saturated NaCl aqueous solution (15.0 mL × 2), dried over anhydrous sodium sulfate, filtered, and concentrated under reduced pressure. The crude product was separated by silica gel column chromatography (petroleum ether/ethyl acetate = 1:0 to 3:1) to obtain compound **50-8.**
   MS-ESI [M+H]⁺, calculated: 382, found: 382.
   ¹H NMR (400 MHz, CDCl₃) *δ* 7.48 (d, *J* = 8.0 Hz, 1H), 7.20-7.24 (m, 1H), 7.05-7.11 (m, 1H), 4.10 (q, *J* = 7.2 Hz, 2H), 3.79-3.96 (m, 2H), 2.70-2.86 (m, 5H), 2.54-2.65 (m, 1H), 2.31-2.50 (m, 2H), 2.22 (s, 3H), 1.99-2.16 (m, 2H), 1.84 (quin, *J* = 6.4 Hz, 2H), 1.24 (t, *J* = 7.2 Hz, 3H), 1.01 (t, *J* = 7.2 Hz, 3H).
(6) To a solution of compound **50-8** (1.70 g, 4.45 mmol) in tetrahydrofuran (35.0 mL) was added potassium bis (trimethylsilyl) amide (1 mol/L, 13.4 mL, tetrahydrofuran solution) at -78°C, and the reaction solution was reacted at -78°C for 2 hours under nitrogen protection. Then the reaction solution was added with saturated ammonium chloride aqueous solution (15.0 mL), and extracted with ethyl acetate (50.0 mL). The combined organic phase was washed with saturated NaCl aqueous solution (50.0 mL), dried over anhydrous sodium sulfate, filtered, and concentrated under reduced pressure. The crude product was separated by silica gel column chromatography (petroleum ether/ethyl acetate = 1:0 to 0:1) to obtain compound **50-9.**
   MS-ESI [M+H]⁺, calculated: 336, found: 336.
   ¹H NMR (400 MHz, CDCl₃) *δ* 7.47-7.59 (m, 1H), 7.23-7.26 (m, 1H), 7.11-7.22 (m, 1H), 4.20-4.34 (m, 2H), 3.46-3.64 (m, 1H), 2.93-3.32 (m, 3H), 2.44-2.73 (m, 3H), 2.01 (d, *J* = 14.4 Hz, 4H), 1.66-1.84 (m, 3H), 1.33 (t, *J* = 7.2 Hz, 3H).
(7) To a solution of compound **50-9** (1.29 g, 3.84 mmol) in ethanol (25.0 mL) was added compound **50-10**(585 mg, 7.68 mmol) and sodium ethoxide (784 mg, 11.5 mmol), and the reaction solution was stirred at 80°C for 15 hours under nitrogen protection. After being concentrated under reduced pressure, the reaction solution was adjusted to pH 6 with hydrochloric acid (1 mol/L). The resulting solution was precipitated, and filtered. The filter cake was dried to obtain compound **50-11.**
   MS-ESI [M+H]⁺, calculated: 348, found: 348.
   ¹H NMR (400 MHz, CDCl₃) *δ* 7.54 (dd, *J* = 7.6, 1.2 Hz, 1H), 7.28-7.33 (m, 1H), 7.20-7.27 (m, 1H), 7.04-7.19 (m, 1H), 3.53 (d, *J* = 16.4 Hz, 2H), 3.02 (d, *J* = 16.4 Hz, 1H), 2.88 (d, *J* = 17.6 Hz, 1H), 2.52-2.64 (m, 2H), 1.91-1.96 (m, 1H), 1.89 (s, 3H), 1.47-1.73 (m, 3H).
(8) To a solution of intermediate **50-11** (1.18 g, 3.39 mmol) in water (75.0 mL) was added chloroacetic acid (3.21 g, 33.9 mmol), and the reaction solution was stirred at 110°C for 12 hours under nitrogen protection. After the reaction solution was filtered, the filtrate was adjusted to pH 8 with saturated sodium bicarbonate aqueous solution to get precipitate. After filtration, the filter cake was dried to obtain compound **50-12.**
   MS-ESI [M+H]⁺, calculated: 332, found: 332.
(9) Compound **50-12** (1.20 g, 3.62 mmol) was dissolved in phosphorus oxychloride (10.0 mL), and the reaction solution was stirred at 80°C for 12 hours under nitrogen protection. Then the reaction solution was concentrated under reduced pressure, and extracted with dichloromethane (20.0 mL). The organic phase was washed with saturated sodium bicarbonate aqueous solution (20.0 mL × 2) and saturated NaCl aqueous solution (20.0 mL× 2), dried over anhydrous sodium sulfate, filtered, and concentrated under reduced pressure to obtain compound **50-13.**
   MS-ESI [M+H]⁺, calculated: 368, found: 368.
(10) To a solution of compound **50-13** (520 mg, 1.41 mmol) in *N*-methylpyrrolidone (5.0 mL) was added potassium carbonate (585 mg, 4.23 mmol) and compound **50-14**(381 mg, 1.69 mmol), and the reaction solution was stirred at 50°C for 4 hours under nitrogen protection. Then the reaction solution was added with ethyl acetate (50.0 mL), washed with saturated NaCl aqueous solution (30.0 mL × 5), dried over anhydrous sodium sulfate, filtered, and concentrated under reduced pressure. The crude product was separated by silica gel column chromatography (petroleum ether/ethyl acetate = 1:0 to 4:1) to obtain compound **50-15.**
   MS-ESI [M+H]⁺, calculated: 557, found: 557.
   ¹H NMR (400 MHz, CDCl₃) *δ* 7.42-7.56 (m, 1H), 7.29-7.42 (m, 1H), 7.11-7.24 (m, 1H), 4.50-4.68 (m, 1H), 3.92-4.09 (m, 2H), 3.56 (s, 3H), 3.28-3.54 (m, 1H), 2.96-3.26 (m, 5H), 2.44-2.79 (m, 3H), 2.06-2.19 (m, 3H), 1.62-1.99 (m, 4H), 1.51 (d, *J* = 2.4 Hz, 9H).
(11) To a solution of compound **50-15** (230 mg, 412 µmol) in dioxane (5.00 mL) was added compound **50-16** (197 mg, 1.24 mmol), potassium carbonate (171 mg, 1.24 mmol), tris(dibenzylidene acetone) dipalladium (75.6 mg, 82.5 µmol) and 2-dicyclohexylphosphine-2',6'-diisopropoxy-1,1'-biphenyl (77.0 mg, 165 µmol), and the reaction solution was stirred at 90°C for 5 hours under nitrogen protection. Then the reaction solution was added with ethyl acetate (20.0 mL), washed with saturated NaCl aqueous solution (20.0 mL × 2), dried over anhydrous sodium sulfate, filtered, and concentrated under reduced pressure. The crude product was separated by preparative high performance liquid chromatography (Xtimate C18, 150 mm × 40 mm 10 µm, A: water (0.225% formic acid); B: acetonitrile, 20%-50%: 10 min) to obtain formate of compound **50-17.**
   MS-ESI [M+H]⁺, calculated: 680, found: 680.
(12) To a solution of formate of compound **50-17**(80 g, 118 µmol) in dichloromethane (1.0 mL) was added trifluoroacetic acid (1.0 mL), and the reaction solution was stirred at 25°C for 1 hour under nitrogen protection. Then the reaction solution was concentrated under reduced pressure to remove trifluoroacetic acid, then adjusted to pH 8 by saturated sodium bicarbonate aqueous solution and extracted with dichloromethane (20 mL× 2). The organic phase was washed with saturated sodium bicarbonate aqueous solution (20 mL) and saturated NaCl aqueous solution (50 mL), dried over anhydrous sodium sulfate, filtered, and concentrated under reduced pressure to obtain compound **50-18.**
   MS-ESI [M+H]⁺, calculated: 580, found: 580.
(13) To a solution of compound **50-18** (55.0 mg, 94.8 µmol) in ethyl acetate(1.0 mL) was added triethylamine (57.6 mg, 79.2 µmol), compound **50-19**(25.6 mg, 284 µmol), 4A molecular sieve (100 mg) and propanephosphonic acid anhydride solution (181 mg, 284 µmol, 50% ethyl acetate solution) at 0°C, and the reaction solution was stirred at 25°C for 1 hour. Then the reaction solution was added with ethyl acetate (10.0 mL), washed with saturated NaCl aqueous solution (10.0 mL × 2), dried over anhydrous sodium sulfate, filtered, and concentrated under reduced pressure. The crude product was separated by preparative high performance liquid chromatography (Phenomenex C18, 75 mm × 30 mm 3 µm, A: water (10 mmol/L ammonium bicarbonate); B: acetonitrile, 38%-78%: 28 min) to obtain compound **50-20.**
(14) Compound **50-20** was separated by chiral supercritical fluid chromatography to obtain compounds **50** and **51.**

Separation conditions: Chromatographic column model: Chiralpak OD-3; column specification: 50 × 4.6mm I.D., 3 µm; Injection volume: 8.0 µL; Mobile phase: A: Carbon dioxide, B: Ethanol (0.05% diethylamine), B%: 5%-40% gradient elution for 2.5 minutes, 40% fixed concentration elution for 0.5 minutes, 5% fixed concentration elution for 1 minute; Detection wavelength: 254 nm; Column temperature: 35°C.

**Compound 50:** retention time: 1.657 minutes, 99.30%ee.

MS-ESI [M+H]⁺, calculated: 652, found: 652.

¹H NMR (400 MHz, MeOD) *δ* 7.53 (d, *J* = 7.6 Hz, 1H), 7.26-7.32 (m, 1H), 7.17-7.25 (m, 1H), 5.11-5.48 (m, 3H), 4.30 (d, *J* = 13.6 Hz, 1H), 4.10-4.22 (m, 2H), 4.00 (d, *J* = 11.2 Hz, 1H), 3.70-3.82 (m, 2H), 3.61 (q, *J* = 7.2 Hz, 1H), 2.90-3.31 (m, 11H), 2.55 (ddd, *J* = 17.2, 12.0, 5.2 Hz, 1H), 2.06-2.39 (m, 6H), 1.84-2.06 (m, 5H), 1.77 (d, *J* = 11.2 Hz, 2H), 1.15-1.33 (m, 2H).

**Compound 51:** retention time: 1.810 minutes, 100%ee.

MS-ESI [M + H]⁺, calculated: 652, found: 652.

¹H NMR (400 MHz, MeOD) *δ* 7.53 (d, *J* = 7.6 Hz, 1H), 7.26-7.32 (m, 1H), 7.17-7.25 (m, 1H), 5.11-5.48 (m, 3H), 4.30 (d, *J* = 13.6 Hz, 1H), 4.10-4.22 (m, 2H), 4.00 (d, *J* = 11.2 Hz, 1H), 3.70-3.82 (m, 2H), 3.61 (q, *J* = 7.2 Hz, 1H), 2.90-3.31 (m, 11H), 2.55 (ddd, *J* = 17.2, 12.0, 5.2 Hz, 1H), 2.06-2.39 (m, 6H), 1.84-2.06 (m, 5H), 1.77 (d, *J* = 11.2 Hz, 2H), 1.15-1.33 (m, 2H).

### Test Example 1

### Inhibitory effect of compounds on the proliferation of NCI-H358 cells

1. Experimental principle: NCI-H358 cellis human non-small cell lung cancer cell line expressing KRAS G12C. The compounds of the present invention inhibit the proliferation of NCI-H358 cells by covalently binding to KRAS G12C.
2. Test materials: NCI-H358 cells were purchased from ATCC; CellTiter-GloR was purchased from Promega (item: G7571); RPMI-1640 was purchased from ATCC (item: 30-2001): fetal bovine serum (FBS) was purchased from EXCELL (item: FND500); Penicillin-streptomycin was purchased from Gibco (item: 15140-122); 0.25% trypsin-ethylenediaminetetraacetic acid digestive juice (Trypsin-EDTA) was purchased from Gibco (item: 25200-072); dimethyl sulfoxide (DMSO) was purchased from Sigma (item: D2650); 96-well plate was purchased from Corning (item: 3610); Incubator was purchased from Thermo (model: 3111); Inverted microscope was purchased from Nikon (item: TS-100); Automatic cell counter was purchased from Life technologies (model: Countess II); Enzyme plate reader was purchased from PerkinElmer (model: Envision); The data processing software was GraphPad Prism 5.0.
3. Test method:
   Cells in logarithmic growth phase were resuspended in growth medium (RPMI-1640 + 10% FBS) and diluted to target density (2000 cells/mL). The above cell suspension was inoculated into 96-well plates at 100 µL per well; incubated overnight in a 37°C, 5% CO₂ incubator. Medium was used as background control.

The test compound was dissolved in DMSO to prepare a stock solution at a concentration of 10 mmol/L. The stock solution was first diluted to 2 mmol/L with DMSO, and then diluted in 3-fold gradient for a total of 10 concentrations. 3 µL of each concentration of the above solution was taken and diluted with 197 µL of growth medium. Cells were then inoculated at 50 µL/well in the 96-well plate. The cells to which the test compound was added were incubated in a 37°C, 5% CO₂ incubator for 72 hours. The 96-well plate was equilibrated at room temperature, and 40 µL CellTiter-Glo reagent was added to each well. Then, the plate was mixed on the vortex for 2 minutes, incubated at room temperature for 60 minutes. The luminescence value was read on an EnVision microplate reader, and the IC₅₀of the compound was calculated using the GraphPad Prism 5.0 software.

The test results were shown in Table 1 (the number of each compound as indicated in the examples), wherein A represents IC₅₀ < 100 nM, B represents 100 nM < IC₅₀ < 1 µM, and C represents 1 µM<IC₅₀ < 10 µM.

**Table 1**

| Compound | | H358 IC₅₀(nM) |
|---|---|---|
| 1 | | C |
| Formate of 3 | | A |
| Formate of 4 | | C |
| 5 | | B |
| 6 | | A |
| 7 | | B |
| 8 | | B |
| Formate of 10 | | B |
| Formate of 11 | | C |
| Formate of 12 | | C |
| | 14 | C |
| Formate of 15 | | B |
| Formate of 17 | | B |
| Formate of 18 | | A |
| Formate of 19 | | B |
| Formate of 20 | | A |
| Formate of 21 | | B |
| Formate of 22 | | B |
| Hydrochloride of 23 | | C |
| Formate of 24 | | B |
| Formate of 25 | | C |
| | 26 | A |
| | 28 | A |
| | 32 | A |
| | 33 | A |
| Formate of 34 | | A |
| | 35 | A |
| | 36 | A |
| | 37 | B |
| | 40 | A |
| | 41 | B |
| | 42 | A |
| | 43 | B |
| | 44 | A |
| | 45 | B |
| | 46 | A |
| | 47 | C |
| | 48 | A |
| | 49 | C |
| | 50 | A |
| | 51 | C |

It can be seen from the test data in Table 1 that the compounds of formula I of the present invention shows a better inhibitory effect on the growth of H358 cells and has the potential to be prepared as drugs for the treatment and prevention of cancer.

### Test Example 2

### Determination of pharmacokinetic properties of the compound in rats

1. Test purpose: to determine the pharmacokinetic characteristics of some of the example compound of the present invention in SD rats after single intravenous injection(IV) and oral administration(PO) by LC-MS/MS method.
2. Test material: the test drug is self-made from the example compounds of the present invention; the positive compound AMG-510 and MRTX-849 were purchased from Chengdu Zhihui Zhongxin Biopharmaceutical Co., Ltd.; SD male rats were obtained from the Laboratory Animal Management Department of Shanghai Family Planning Research Institute. Animal production license number (SCXK (Shanghai) 2018-0006).
3. Test method:
   Preparation of 0.5% methyl cellulose: 5g of methyl cellulose were weighed and dissolved in 1000 mL of purified water.

Preparation of oral formulation: A required amount of test compound was weighed and dissolved in 0.5% methyl cellulose solution to obtain a suspension or solution of 0.5 mg/mL.

Preparation of intravenous injection formulation: A required amount of test compound was weighed, and dissolved in 0.5 mL of dimethyl sulfoxide to prepare a solution of 4 mg/mL. 0.25 mL of the above solution was added into 0.5 mL of polyethylene glycol 15 hydroxystearate (solutol) and 4.25 mL of normal saline to obtain a solution of 0.2 mg/mL.

Administration: SD male rats were orally administered (PO) after fasting overnight, and the single intravenous injection (IV) group did not need fasting. The dosage and volume administered were shown in the table below. Feeding 4 hours after adminisration.

| **Route of administration** | **Number of animal** | **Dosage** | **Concentration of administration** | **Volume of administration** |
|---|---|---|---|---|
| | | **(mg/kg)** | **(mg/mL)** | **(mL/kg)** |
| IV | 3 | 1 | 0.2 | 5 |
| PO | 3 | 5 | 0.5 | 10 |

Sample Collection: blood is collected through jugular vein or other suitable vein, and 0.2 mL of blood was collected at each time point. Samples were placed into tubes containing ethylenediaminetetraacetic acid dipotassium salt and kept on ice before centrifugation. Within 1 hour after blood collection, the blood samples were centrifuged at 2-8°C and 6800g for 6 minutes and stored at -80°C. The time point for blood collection was 0.083, 0.25, 0.5, 1, 2, 4, 8 and 24 hours after intravenous administration, and 0.25, 0.5, 1, 2, 4, 6, 8 and 24 hours after oral administration. The test results were shown in Table 2.

**Table 2**

| **Compound** | Intravenous injection, 1 mg/kg | | | | | Oral gavage, 5 mg/kg | | | |
|---|---|---|---|---|---|---|---|---|---|
| | t_{1/2} h | Cmax ng/mL | AUC_{(0-∞)} h*ng/mL | Vz L/kg | CL mL/min/kg | t_{1/2} h | Cmax ng/mL | AUC_{(0-∞)} h^{∗}ng/mL | F % |
| **AMG510** | 0.3 6 | 762.12 | 318.99 | 1.57 | 50.73 | 1.81 | 1258.08 | 584.27 | 35.41 |
| **MRTX849** | 3.1 6 | 136.78 | 347.91 | 13.14 | 48.14 | 2.80 | 23.31 | 237.61 | 13.66 |
| **26** | 1.5 5 | 379.11 | 246.53 | 9.30 | 68.35 | 1.00 | 162.55 | 384.60 | 30.99 |
| **28** | 1.8 2 | 547.05 | 725.88 | 3.63 | 23.20 | 1.34 | 801.74 | 2854.83 | 78.41 |

The data in Table 2 showed that some of the example compounds of the present invention have excellent pharmacokinetic property in rats, even better than that of the positive compound.

### Test Example 3

### Whole Blood Stability Assay of Compounds

1. Test purpose: to determine the whole blood stability of the compounds by monitoring the clearance rate of the parent compound after incubation of some of the example compounds of the invention with dog and rat whole blood.
2. Test materials: the test drug is self-made from the example compounds of the present invention; the positive compound AMG-510 and MRTX-849 were purchased from Chengdu Zhihui Zhongxin Biopharmaceutical Co., Ltd.
3. Test method:
   Solution preparation: A required amount of test compound was weighed, and dissolved in dimethyl sulfoxide to prepare a stock solution of 4 mg/mL. Then, the stock solution of the test compound was diluted to 1 mmol/L and 0.2 mmol/L with 70% acetonitrile aqueous solution, respectively, as working solution.

The working solution was added to the whole blood. The concentration of each test compound was 5 µmol/L. 90 µL was taken and then mixed evenly with 90 µL of water. Then 600 µL of acetonitrile termination solution containing propranolol as internal standard was added. Incubated at 37°C in a water bath and shook gently. At 30, 60, 120 and 240 minutes, 90 µL of each mixture was placed on a clean 96-well plate prefilled with 90 µL of water, mixed well, and then 600 µL of acetonitrile termination solution containing propranolol as internal standard was added and centrifuged at 5000 g for 15 min. 80 µL of the supernatant was taken and placed in a 96-well plate preloaded with 160 µL of ultrapure water, then analyzed by LC-MS/MS.

The test results were shown in Table 3.

**Table 3**

| Compound | Half-Life in Whole Blood of Rat (min) | Half-Life in Whole Blood of Dog (min) |
|---|---|---|
| **AMG510** | 111.3 | 87.5 |
| **MRTX849** | 783.9 | 369.0 |
| **28** | 258.9 | 380.4 |

The above data showed that the compounds of some examples of the present invention have excellent whole blood stability.

### Test Example 4

### H358 pERK inhibitory activity

1. Test purpose: to detect the inhibitory effect of the test compound on the phosphorylation level of ERK in NCI-H358 cells.
2. Test materials: the test drug was self-made from the example compounds of the present invention; the positive compound AMG-510 was purchased from Chengdu Zhihui Zhongxin Biomedical Co., Ltd.; the NCI-H358 cells were purchased from Wuhan Procell Life Science&Technology Co.,Ltd.; the 96-well cell culture plate was purchased from Corning; the incubator was purchased from Thermo Fisher Scientific; the PRMI1640 culture medium was purchased from Biological Industries; dimethyl sulfoxide was purchased from Sinopharm Chemical Reagent Co.,Ltd.; pipettes was purchased from Thermo Fwasher Scientific; 384 microplate was purchased from Greiner; phospho-ERK (Thr202/Tyr204) kit was purchased from Cwasbio; the multi-label analyzer was purchased from PerkinElmer.
3. Test method:
   NCI-H358 cells suspension was seeded at 80 µL per well in a clear 96-well cell culture plate, with 10000 cells each well. The cell culture plate was placed in an incubator and incubated overnight at 37°C under 5% CO₂. After incubation, the cell supernatant was discarded, 80 µL of PRMI1640 medium containing 0.02% serum was added to each well, and the cell culture plate was placed in an incubator and incubated overnight at 37 ° C, under 5% CO₂. The test compound was diluted with 100%DMSO to 4 mmol/L as the first concentration, and then diluted 5-fold to the 8th concentration using a pipette. 2 µL of compound or DMSO (as negative control) was taken and added with 158µL of cell culture medium. After mixing evenly, 20 µL of the compound solution was taken and added to corresponding cell plate well. The cell wells of the blank group were incubated without any compound and DMSO, and the cell plates were placed in the incubator at 37°C with 5% CO₂ and incubated for 1 hour. At the end of incubation, the cell supernatant was discarded, and 50 µL of 1X cell lysate was added to each well. The cell plate was incubated and shook at room temperature for 30 minutes. Phosphorylated ERK1/2 Eu cryptate antibody and phosphorylated ERK1/2 d2 antibody were diluted 20-fold using assay buffer. 16 µL cell lysate supernatant was taken from each well and added into a new 384 white microplate followed by the addition of 2 µL phosphorylated ERK1/2 Eu cryptate antibody dilution and 2 µL phosphorylated ERK1/2 d2 antibody dilution, and then incubated at room temperature for 4 hours. After incubation, the signals at 615 nm and 665 nm were read using a multi-label analyzer. The percent inhibition was calculated according to the following formula: Ratio = (signal 665nm/signal 615nm) × 10000; the percent inhibition = (ratio of test compound-ratio of negative control)/(ratio of blank-ratio of negative control) × 100%.

The test results were shown in Table 4.

**Table 4**

| Compound | H358 pERK IC₅₀(nM) |
|---|---|
| AMG510 | 88.33 |
| MRTX849 | 67.61 |
| **26** | 2.98 |
| **28** | 42.73 |

The above data showed that the compounds of some examples of the present invention have good inhibitory activity on H358 ERK phosphorylation.

### Test Example 5

### Cell selectivity test

1. Test purpose: to detect the proliferation inhibition of the test compounds on NCI-H23, NCI-H2122, HCC827, Mia paca2, A549 and SW837 cell lines.
2. Experimental materials: the test drug was made by the example compounds of the present invention; the positive compounds AMG-510 and MRTX849 were purchased from Chengdu Zhihui Zhongxin Biomedical Co., Ltd.; NCI-H23, NCI-H2122, HCC827, Mia paca2, A549 and SW837 cell lines were purchased from ATCC; CellTiter-Glo were purchased from Promega (Art.No. G7571); DMEM culture medium was purchased from ATCC (Art.No.30-2002); RPMI-1640 culture medium was purchased from ATCC (Art.No. 30-2001); F-12K culture medium was purchased from ATCC (Art.No. 30-2004); Leibovitz's L-15 culture medium was purchased from ATCC (item: 30-2008); Fetal bovine serum (FBS) was purchased from EXCELL (Art.No. FND500); Penicillin-streptomycin was purchased from Gibco (Art.No. 15140-122); 0.25% trypsin-ethylenediaminetetraacetic acid digestive juice (Trypsin-EDTA) was purchased from Gibco (Art.No. 25200-072); dimethyl sulfoxide (DMSO) was purchased from Sigma (Art.No. D2650); 96-well cell plate was purchased from Corning (Art.No. 3610); incubator was purchased from NuAire (model number: NU-5700E); Biosafety cabinet was purchased from AuAire (model number: NU-543-600S); Inverted microscope was purchased from Nikon (Art.No.TS-100); Automated cell counter was purchased from Life technologies (model number: Countess II); Microplate reader was purchased from PerkinElmer (model number: Envision); the data processing software was GraphPad Prism 5.0.
3. Experimental methods:
   The cells in logarithmic growth phase were re-suspended in growth medium (the growth medium for NCI-H23, NCI-H2122 and HCC827 was RPMI-1640 + 10% FBS; the growth medium for Mia paca2 was DMEM + 10% FBS +2.5% horse serum; the growth medium for A549 was F-12K + 10% FBS; the growth medium for SW837 was Leibovitz's L-15 + 10% FBS), and diluted to target density (800 cells/well, 1500 cells/well, 3000 cells/well, 2000 cells/well, 1000 cells/well, and 2000 cells/well for NCI-H23, NCI-H2122, HCC827, Mia paca2, A549, and SW837 cells, respectively). The above cell suspension was inoculated into 96-well plates at 100 µL per well; incubated overnight in a 37°C, 5% CO₂ incubator. Medium was used as background control; DMSO was used as negative control.

The test compound was dissolved in DMSO to prepare a stock solution with a concentration of 10 mmol/L. The stock solution was first diluted to 2 mmol/L with DMSO, and then diluted in 5-fold gradient for a total of 8 concentrations. 3 µL of each concentration of the above solutions were taken and diluted with 197 µL of growth medium. 50 µL/well was then added to the cell-seeded 96-well plate. The cells to which the test compound was added were incubated in a 37°C, 5% CO₂ incubator for 72 hours. The 96-well plate was equilibrated at room temperature, and 40 µL CellTiter-Glo reagent was added to each well. Then, the plate was mixed on the vortex for 2 minutes, incubated at room temperature for 60 minutes. The luminescence values were read on an EnVision microplate reader, and the IC₅₀ of the compounds were calculated using the GraphPad Prism 5.0 software.

The test results were shown in Table 5.

**Table 5**

| Cell line (KRAS mutation) | IC₅₀ (nM) | | |
|---|---|---|---|
| | AMG510 | MRTX849 | **28** |
| NCI-H23 (G12C) | 7.55 | 27.28 | 3.89 |
| NCI-H2122 (G12C) | 27.02 | 40.27 | 20.30 |
| Mia paca2(G12C) | 15.67 | 45.15 | 8.61 |
| SW837(G12C) | 284.90 | 16.65 | 7.29 |
| A549(G 12S) | > 10000 | 3498 | > 10000 |
| HCC827(wt) | > 10000 | 2495 | > 10000 |

The above data showed that some of the example compounds of the present invention have good selectivity for KRAS G12C mutant cell lines.

### Test Example 6

### Pharmacokinetics study in mice

1. Test purpose: to determine the pharmacokinetic characteristics of some of the example compounds of the present invention in ICR male mice after single intravenous injection (IV) and oral administration (PO) by LC-MS/MS method.
2. Test materials: the test drugs are self-made from the example compound of the present invention, the positive compound AMG-510 is purchased from Chengdu Zhihui Zhongxin Biopharmaceutical Co., Ltd., and the ICR male mice are from Shanghai Sippe-Bk Lab Animal Co.,Ltd. (Sino-British SIPPR Lab Animal Ltd, Shanghai).
3. Test method:
   Preparation of 0.5% methyl cellulose: 5g of methyl cellulose were weighed and dissolved in 1000 mL of purified water.

Preparation of oral formulation: a required amount of the test compound was weighed and dissolved in 0.5% methyl cellulose solution to obtain a suspension or solution of 0.5 mg/mL.

Preparation of intravenous injection formulation: a required amount of test compound was weighed, and dissolved in 0.5 mL of dimethyl sulfoxide to prepare a solution of 4 mg/mL. 0.06 mL of the above solution was taken, and 0.12 mL of polyethylene glycol 15 hydroxystearate (solutol) and 1.02 mL of normal saline were added thereto to obtain a solution of 0.2 mg/mL.

Administration: ICR male mice were orally administered (PO) after overnight fasting, and the single intravenous (IV) group did not need fasting. The dosage and volume administered were shown in the table below. Feeding 4 hours after adminisration.

| **route of administration** | **Number of animal** | **Dosage** | **Concentration of administration** | **Volume of administration** |
|---|---|---|---|---|
| | | **(mg/kg)** | **(mg/mL)** | **(mL/kg)** |
| IV | 3 | 1 | 0.2 | 5 |
| PO | 3 | 5 | 0.5 | 10 |

Sample Collection: blood was collected via the jugular vein or other suitable vein, and 0.03 mL blood was collected at each time point. Samples were placed into tubes containing ethylenediaminetetraacetic acid dipotassium salt and kept on ice before centrifugation. Within 1 hour after blood collection, the blood samples were centrifuged at 2-8°C and 6800g for 6 minutes and stored at -80°C. The time points for blood collection were 0.083, 0.25, 0.5, 1, 2, 4, 8 and 24 hours after intravenous and oral administration.

The test results were shown in Table 6.

**Table 6**

| **Compound** | Intravenous injection, 1 mg/kg | | | | | Oral gavage, 5 mg/kg | | | |
|---|---|---|---|---|---|---|---|---|---|
| | t_{1/2} h | Cmax ng/mL | AUC_{(0-∞)} h*ng/mL | Vz L/kg | CL mL/min/kg | t_{1/2} h | Cmax ng/mL | AUC_{(0-∞)} h*ng/mL | F % |
| **AMG510** | 0.22 | 622.56 | 226.81 | 1.42 | 74.67 | 0.36 | 7.25 | 6.12 | 0.33 |
| **28** | 1.59 | 575.45 | 623.02 | 3.71 | 26.92 | 1.42 | 1444.02 | 2678.57 | 86.09 |

The data in Table 6 showed that some of the example compounds of the present invention have excellent pharmacokinetic properties in mice, even better than the positive compound.

### Test Example 7

### Pharmacokinetic study in dogs

1. The test purpose: to determine the pharmacokinetic characteristics of some of the example compounds of the present invention in beagle dogs after single intravenous injection(IV) and oral administration(PO) by LC-MS/MS method.
2. Test materials: the test drug was self-made from the example compounds of the present invention; Beagle dogs were obtained from Medicilon Colony: 999M-004.
3. Test method:
   Preparation of 0.5% methyl cellulose: 5g of methyl cellulose were weighed and dissolved in 1000 mL of purified water.

Preparation of oral formulation: a required amount of test compound was weighed and dissolved in 0.5% methyl cellulose solution to obtain a suspension or solution of 1 mg/mL.

Preparation of intravenous injection formulation: an appropriate amount of the test compound was weighed, and dissolved in dimethyl sulfoxide to prepare a solution of 10 mg/mL. 3 mL of the above solution was taken, and 6 mL of polyethylene glycol 15 hydroxystearate (solutol) and 51 mL of normal saline were added thereto to obtain a solution of 0.5 mg/mL.

Administration: Beagle dogs were orally (PO) administered after overnight fasting, and the single intravenous (IV) group did not need fasting. The dosage and volume administered were shown in the table below. Feeding 4 hours after adminisration.

| **route of administration** | **Number of animal** | **Dosage** | **Concentration of administration** | **Volume of administration** |
|---|---|---|---|---|
| | | **(mg/kg)** | **(mg/mL)** | **(mL/kg)** |
| IV | 3 | 1 | 0.5 | 2 |
| PO | 3 | 5 | 1 | 5 |

Sample Collection: blood was collected via the jugular vein or other suitable vein, and 1 mL blood was collected at each time point. Samples were placed into tubes containing ethylenediaminetetraacetic acid dipotassium salt and kept on ice before centrifugation. Within 1 hour after blood collection, blood samples were centrifuged at 2-8°C and 2200 g for 10 minutes and stored at -80°C. The time point for blood collection was 0.083, 0.25, 0.5, 1, 2, 4, 8 and 24 hours after intravenous administration, and 0.25, 0.5, 1, 2, 4, 6, 8 and 24 hours after oral administration, respectively. The test results were shown in Table 7.

**Table 7**

| **Compound** | Intravenous injection, 1 mg/kg | | | | | Oral gavage, 5 mg/kg | | | |
|---|---|---|---|---|---|---|---|---|---|
| | t_{1/2} h | Cₘₐₓ ng/mL | AUC_{(0-∞)} h*ng/mL | Vz L/kg | CL mL/min/kg | t_{1/2} h | Cₘₐₓ ng/mL | AUC_{(0-∞)} h*ng/mL | F % |
| **28** | 1.79 | 575.49 | 928.81 | 2.72 | 18.37 | 5.88 | 191.42 | 1511.42 | 32.30 |

The data in Table 7 showed that some of the example compounds of the present invention have excellent pharmacokinetic properties in dogs.

### Test Example 8

### Brain tissue distribution test

1. Test purpose: to determine the ability of some of the example compounds of the present invention to cross the blood-brain barrier after a single oral administration (PO) by LC-MS/MS.
2. Test materials: the test drugs were self-made from the example compounds of the present invention, the positive compounds AMG-510 and MRTX849 were purchased from Chengdu Zhihui Zhongxin Biopharmaceutical Co., Ltd., and SD rats were obtained from Sino-British SIPPR Lab Animal Ltd, Shanghai.
3. Test method:
   5 g of methyl cellulose was weighed and dissolved in 1000 mL of purified water to obtain 0.5% methyl cellulose. A required amount of the test compound was weighed and dissolved in 0.5% methylcellulose solution to obtain a suspension of 2 mg/mL.

Administration: SD rats were orally administered (PO) after overnight fasting. The dosage and volume administered were shown in the table below. Feeding 4 hours after adminisration.

| **route of administration** | **Number of animal** | **Dosage** | **Concentration of administration** | **Volume of administration** |
|---|---|---|---|---|
| | | **(mg/kg)** | **(mg/mL)** | **(mL/kg)** |
| PO | 6 | 20 | 2 | 10 |

Sample Collection: 0.5 mL of blood was collected at each time point via the jugular vein. Samples were placed into tubes containing ethylenediamine tetraacetic acid dipotassium salt (K₂EDTA) and kept on ice before centrifugation. Within 1 hour after blood collection, blood samples were centrifuged at 2-8°C and 6800 g for 6 minutes and stored at -80°C. After the rats were euthanized by carbon dioxide inhalation, the brains of the rats were collected, quickly frozen on dry ice, and stored at -80°C before bioanalysis. The time points for blood collection were 0.5 and 2 hours after oral administration. The test results were shown in Table 8.

**Table 8**

| Compound | Brain concentration 0.5 hours after administration (ng/g) | Brain concentration 2 hours after administration (ng/g) |
|---|---|---|
| AMG510 | Below detection limit | Below detection limit |
| MRTX849 | 8.00 | 69.79 |
| **28** | 133.97 | 92.81 |

The data in Table 8 showed that some of the example compounds of the present invention have excellent brain exposure.

### Test Example 9

### CYP inhibition test

1. Test purpose: to evaluate the inhibitory ability of some of the example compounds of the present invention against CYP1A2, 2B6, 2C8, 2C9, 2C19, 2D6 and 3A4 via human liver microsomes.
2. Test materials: the test drug was self-made from the example compounds of the present invention, the positive compounds AMG-510 and MRTX849 were purchased from Chengdu Zhihui Zhongxin Biopharmaceutical Co., Ltd., and the human liver microsomes were purchased from Xenotech.
3. Test method:
   Solution preparation:
   1. K/Mg buffer: the components were 0.1 mol/L potassium phosphate and 5 mmol/L magnesium chloride with pH 7.4±0.1, preheated.
   2. Solution of the test compound: the test compound was dissolved in dimethyl sulfoxide to prepare a stock solution of 10 mmol/L. 8 µL of 10 mmol/L stock solution was transferred to a 96-well plate containing 12 µL of acetonitrile as the highest concentration point, at 3 -fold dilution, for 8 concentrations.
   3. NADPH solution: 66.7 mg of NADPH was dissolved in 10 mL of 0.1 mol/L K/Mg buffer to obtain 8 mmol/L NADPH solution with pH 7.4.
   4. Human liver microsomal stock solution: human liver microsomes were dissolved in 0.1 mol/L K/Mg buffer to obtain 20 mg/mL stock solution.
   5. Human liver microsomal working solution: 10 µL of 20 mg/mL human liver microsomal stock solution was taken and added into 990 µL of 0.1 mol/L K/Mg buffer to prepare 0.2 mg/mL human liver microsomal solution.
   6. Substrate solution:
      CYP 1A2 substrate solution (120 µM): 8 µL of 30 mmol/L phenacetin stock solution was taken and diluted with 1992 µL K/Mg buffer;
      CYP 2B6 substrate solution (280 µM): 40 µL of 14 mmol/L bupropion stock solution was taken and diluted with 1960 µL K/Mg buffer;
      CYP 2C8 substrate solution (40 µM, containing 1.6 mg/ml human liver microsomes); 8 µL of 10 mmol/L paclitaxel stock solution was taken and added into 160 µL human liver microsomal stock solution and 1832 µL of K/Mg buffer;
      CYP 2C9 substrate solution (40 µM): 8 µL of 10 mmol/L diclofenac stock solution was taken and diluted with 1992 µL of K/Mg buffer;
      CYP 2C19 substrate solution (140 µM, containing 1.6 mg/ml HLM); 40 µL of 7 mmol/L mephenytoin stock solution was taken and added into 160 µL of human liver microsomal stock solution and 1800 µL of K/Mg buffer;
      CYP 2D6 substrate solution (20 µM); 8 µL of 5 mmol/L dextromethorphan stock solution was taken and diluted with 1992 µL K/Mg buffer;
      CYP 3A4M substrate solution (20 µM); 8 µL of 5 mmol/L midazolam stock solution was taken and diluted with 1992 µL K/Mg buffer;
      CYP 3A4T substrate solution (320 µM); 40 µL of 16 mmol/L testosterone stock solution was taken and diluted with 1960 µL K/Mg buffer.
      Incubation and detection:
         600 µL of 0.2 mg/mL human liver microsomal solution and 3 µL of stepwise diluted test compound solution were added to the 96-well plate. 30 µL of the above solution was added on the 96-well plate followed by the addition of 15 µL of the corresponding substrate solution. The 96-well plate and the NADPH solution were pre-incubated at 37°C for 5 minutes, and 15 µL of NADPH solution was added to the 96-well plate to start the reaction. The assay plate was incubated at 37°C for 5 minutes for 3A4, 10 minutes for 1A2, 2B6 and 2C9, 20 minutes for 2C8 and 2D6, and 45 minutes for 2C19. Then the reaction was terminated by 180 µL of acetonitrile containing internal standard substance. After quenching, the plate was shook for 10 minutes (600 rpm/min) and then centrifuged at 6000 rpm for 15 minutes. 80 µL of supernatant was taken from each well and transferred to a 96-well sample plate containing 120 µL of ultrapure water for LC-MS/MS analysis.

The test results were shown in Table 10.

**Table 10**

| CYP | IC₅₀(µM) | | |
|---|---|---|---|
| | AMG510 | MRTX849 | 28 |
| 1A2 | >10 | >10 | >10 |
| 2B6 | >10 | 3.93 | >10 |
| 2C19 | >10 | >10 | >10 |
| 2C8 | >10 | >10 | >10 |
| 2C9 | >10 | 1.35 | >10 |
| 2D6 | >10 | 9.72 | >10 |
| 3A4-M | 5.07 | 6.83 | >10 |
| 3A4-T | >10 | >10 | >10 |

The data in Table 10 showed that some of the example compounds of the present invention have no inhibitory activity on each subtype of CYP enzyme, and there was no risk of drug-drug interactions.

### Test Example 10

### Plasma protein binding assay

1. Test purpose: to evaluate the protein binding rate of the test compound in human, dog, monkey, rat and mouse plasma by equilibrium dialysis;
2. Experimental materials: the test drug was self-made from the example compound of the present invention; the positive compounds AMG-510 and MRTX849 were purchased from Chengdu Zhihui Zhongxin Biopharmaceutical Co., Ltd.
3. Experimental methods:
   0.1 mol/L sodium phosphate and 0.15 mol/L sodium chloride buffer with pH 7.4±0.1 was preheated;
   Preparation of plasma containing test substance: the test compound was dissolved in DMSO to prepare a 10 mmol/l stock solution. 5 µL of stock solution was taken and diluted with 95 µL of acetonitrile to obtain solution A (0.5 mmol/L). 8 µL of solution A was taken and diluted with 192 µL of 0.1mol/L sodium phosphate buffer to obtain solution B (0.02 mmol/L). 570 µL of plasma was added into a 96-well plate followed by the addition of 30 µL of solution B. The final concentration was 1 µmol/L with 0.01% DMSO.

Preparation of T0 plasma samples (in triplicate): 25 µL of plasma containing test substance was taken and added into a 96-well plate, followed by the addition of 25 µL of buffer and 200 µL of acetonitrile solution containing internal standard (the internal standard of MRTX849 and compound **28** is tolbutamide; the internal standard of AMG510 is verapamil). The plate was shook at 600 rpm for 10 minutes, covered and stored in a freezer (-20°C).

T5 sample preparation (in triplicate): the dialysis device was added with 100 µL of buffer on its receiver side and 100 µL of plasma containing the test compound on its doner side. And the dialysis device was then shook at 37°C and 120 rpm for 5 hours. At the end of culture, 25 µL of plasma containing the test substance was taken from the plasma side and then added to a 96-well plate, followed by the addition of 25 µL of buffer and 200 µL of acetonitrile solution containing internal standard. 25 µL of buffer was taken from the buffer side and then added to a 96-well plate, followed by the addition of 25 µL of blank plasma and 200 µL of acetonitrile solution containing internal standard. All samples were rotated at 600 rpm for 10 minutes and then centrifuged at 6000 rpm. 100 µL of supernatant was taken from each well and transferred to a 96-well sample plate pre-loaded with 100 µL of ultrapure water for LC-MS/MS analysis. The specific test results were shown in Table 11.

**Table 11**

| species | Plasma protein binding rate (%) | | |
|---|---|---|---|
| | AMG510 | MRTX849 | **28** |
| Mice | 90.9 | 99.6 | 97.5 |
| Rat | 85.9 | 99.0 | 95.5 |
| Dog | 92.1 | 98.8 | 67.5 |
| Monkev | 98.3 | 98.7 | 71.7 |
| Human | 92.5 | 98.2 | 61.8 |

### Test Example 11

### hERG experiment

1. Test purpose: to test the inhibition of hERG current on HEK293 cells stably transfected with hERG potassium channel by manual membrane clamp;
2. Experimental materials: the positive compounds AMG-510 and MRTX849 were purchased from Chengdu Zhihui Zhongxin Biopharmaceutical Co., Ltd.; HEK293 cells were from the Academy of Military Medical Sciences. Manual membrane clamp system amplifier was purchased from Molecular Devices (Model No. MultiClamp700B); Manual membrane clamp system digital-to-analog converter was purchased from Molecular Devices (Model No. Axon Digidata1550B); Rapid perfusion system was purchased from ALA Scientific Ins. (Model No. ALA-VM8); Micro-manipulator was purchased from Sutter Instrument Co (model No. MP-225); Inverted microscope was purchased from Nikon (model No. Ti2-U); Micropipette Puller was purchased from NARISHIGE (model number PC-10).
3. Experimental methods:
   Preparation of electrophysiological solution:
   Extracellular fluid: N-2-hydroxyethylpiperazine-N' -2-ethanesulfonic acid 10 mmol/L, sodium chloride 145 mmol/L, potassium chloride 4 mmol/L, calcium chloride 2 mmol/L, magnesium chloride 1 mmol/L, glucose 10 mmol/L, the pH was adjusted to 7.3-7.4 with sodium hydroxide; the osmotic pressure was adjusted to 290-310 mOsm; stored at 4 °C after filtration.

Electrode internal fluid (mM): potassium chloride 120 mmol/L, potassium hydroxide 31.25 mmol/L, calcium chloride 5.374 mmol/L, magnesium chloride 1.75 mmol/L, ethylene glycol bis (β-aminoethyl ether)-N,N,N',N'-tetraacetic acid 10 mmol/L, N-2-hydroxyethylpiperazine-N'-2-ethanesulfonic acid 10 mmol/L, adenosine triphosphate disodium salt 4 mmol/L, the pH was adjusted to 7.2-7.3 with potassium hydroxide; the osmotic pressure was adjusted to 290-310 mOsm; after filtration, it was divided and stored at -20 °C.

Cell preparation:
After the HEK-293-hERG cells were subcultured to a suitable state, the cells were washed with phosphate buffer salt, digested and separated with Tryple solution. The cells were resuspended with culture medium and stored in a centrifuge tube, the supernatant was discarded after centrifugation, then the cells were resuspended with extracellular liquid for later use, and stored at 2-8 °C. Prior to membrane clamp recording, the cells were added dropwise to culture dish to ensure that the cells had a certain density and were individually separated.

Electrophysiological experiments:
The whole-cell membrane clamp technique was applied to record hERG currents. The cell suspension was added into small Petri dishes and placed on an inverted microscope stage. After cells were adhered, they were perfused with extracellular fluid at a recommended flow rate of 1-2 mL/min. The glass microelectrode was drawn in two steps by a micropipette puller, and its resistance value in water was 2-5 MΩ after filling with electrode internal fluid.

After the whole-cell recording mode was established, the clamp potential was maintained at -80 mV. The hERG tail current was elicited by applying a depolarizing voltage to +60 mV for 850 ms and then repolarizing to -50 mV for 1275 ms. Such a set of pulse was repeated every 15 seconds throughout the experiment. After the current was stabilized, the drug was continuously extracellularly perfused from low concentration to high concentration (0.3, 1, 3, 10 and 30 µmol/L). The perfusion started from low concentration and continued until the drug effect was stable, and then proceeded to the next concentration.

Data collection and analysis:
Stimulus release and signal acquisition were performed on PatchMaster software. The signal was amplified by a diaphragm clamp amplifier. Further data analysis and curve fitting were performed using FitMaster, EXCEL, Graphpad Prism or SPSS 21.0. Data was expressed as mean and standard deviation. The IC₅₀ value was obtained by fitting the Hill equation (if applicable). If the maximum inhibition rate of all concentrations of the test substance was less than 50%, the IC₅₀ value was not calculated.

Test Results: The IC₅₀ of AMG 510, MRTX849 and compound **28** were > 30 µM, 0.99 µM and> 30 µM, respectively.

### Test Example 12

### In vivo efficacy test on H358

1. Test purpose: to evaluate the in vivo efficacy of the test substance in the female BALB/c nude mouse model of subcutaneous transplantation of NCI-H358 tumor.
2. Experimental materials: the test drug was self-made compound of the example of the present invention; the positive compounds AMG-510 and MRTX849 were purchased from Chengdu Zhihui Zhongxin Biopharmaceutical Co., Ltd.; BALB/c nude mice were purchased from Shanghai Lingchang Biotechnology Co., Ltd;
3. Experimental methods:
   BALB/c nude mice, female, 6-8 weeks old, weight 18-22 g. NCI-H358 tumor cells were resuspended in phosphate buffer to prepare a 0.1 mL (containing 5 × 10⁶ cells) cell suspension, then subcutaneously inoculated on the right nape of each mouse (5 × 10⁶ cells /mouse) (+ 50% Matrigel). The mice were randomly grouped and administered intragastrically when the mean volume of tumor reached approximately 150-200 mm³, and the dosing frequency was once a day. The tumor diameter was measured twice a week with vernier calipers. The tumor volume was calculated by formula: V = 0.5 a×b², a and b represented the long diameter and short diameter of the tumor, respectively.

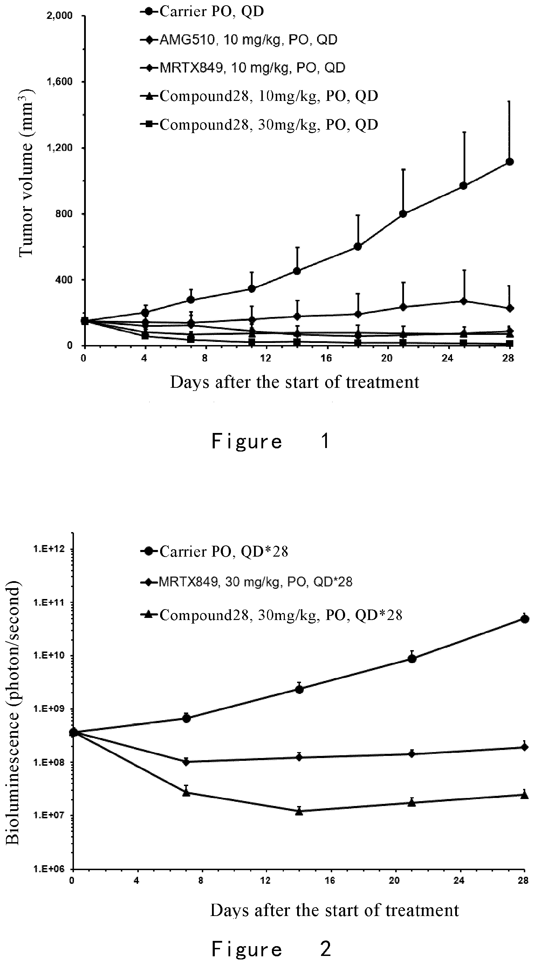

The results were shown in Figure 1, and the results showed that compound 28 of the present invention exhibited equivalent therapeutic effect to that of the positive control drug MRTX849 in the animal model of subcutaneous transplantation of NCI-H358 tumors, and was superior to that of the positive control drug AMG 510.

### Test Example 13

### Pharmacodynamic experiment of NCI-H1373-luc lung cancer brain metastasis model

1. Test purpose: to evaluate the in vivo efficacy of the test substance in the BALB/c nude mouse NCI-H1373-luc intracranial inoculation model.
2. Experimental materials: the test drug was self-made compound of the present invention; the positive compound MRTX849 was purchased from Chengdu Zhihui Zhongxin Biopharmaceutical Co., Ltd.; BALB/c nude mice (female, 6-8 weeks old, weight 18-22g) were purchased from Beijing Weitong Lihua Experimental Animal Technology Co., Ltd.
3. Experimental methods:
   NCI-H1373-luc cells were cultured in vitro with a culture medium supplemented by 10% fetal bovine serum, at 37 °C, 5% CO₂. The cells were routinely passaged 2 times a week. When cells were maintained in the exponential growth phase, cells were harvested, counted, and seeded. BALB/c nude mice were anesthetized, fixed on a brain stereotaxic instrument, and the skin of the brain was sterilized. A sagittal incision of about 2mm was made at the parietal bone and occipital bone to expose the skull. The skull was perforated 2mm to the right of the bregma and 1mm to the anterior coronal suture with a 25G sharp needle. The cell suspension was extracted with a syringe, and the needle was inserted vertically into the previous perforation to a depth of 3mm. The cell suspension (3 µL, containing 3 × 10⁵ NCI-H1373-luc cells and 20% Matrigel, for 3 min)was injected slowly. After injection, the syringe was retained for 1 minute, and then withdrawn slowly to reduce the backflow of cell fluid. The perforation was sealed with adhesive, the scalp was reset, and the incision was sutured. The animals should be observed until fully awakened after the operation. When the fluorescence value of the tumor reached about 3E +08, the mice were randomly grouped and administered intragastrically, and the dosing frequency was once a day. Imaging was
performed with IVIS once a week.

The results were shown in Figure 2, and the results showed that the therapeutic effect of compound 28 of the present invention was better than that of positive control drug MRTX849 in the BALB/c nude mouse NCI-H1373-luc intracranial inoculation animal model.

The applicant declares that the present invention illustrates the spirocyclic compound, the pharmaceutical composition thereof and the use thereof through the above examples, but the present invention is not limited to the above-described examples, that is, it does not mean that the present invention must rely on the above-described examples to be implemented. Those skilled in the art should understand that any improvement of the present invention, equivalent replacement of each raw material of the product of the present invention, addition of auxiliary components, selection of specific methods, etc., all fall within the scope of protection and disclosure of the present invention.

## Claims

1. A compound of formula I, or pharmaceutically acceptable salts thereof, enantiomers, diastereomers, tautomers, cis-trans isomers, solvates, polymorphs, deuterated compounds, or combinations thereof, 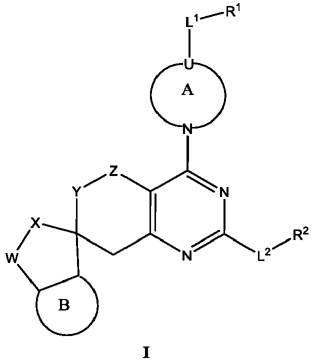 wherein,
A is a 4-12 membered saturated or partially saturated monocyclic ring, fused ring, bridged ring or spiro ring, wherein the saturated or partially saturated monocyclic ring, fused ring, bridged ring, or spiro ring is optionally substituted by one or more R³;
each R³ is independently oxo, C₁₋₃ alkyl, C₂₋₄ alkenyl, C₂₋₄ alkynyl, cyano, -C(O)OR⁴, -CON(R⁴)₂, or -N(R⁴)₂, wherein the C₁₋₃ alkyl is optionally substituted by cyano, halogen, -OR⁴, or -N(R⁴)₂;
each R⁴ is independently hydrogen or C₁₋₃ alkyl;
U is N or CH;
L¹ is absent or NR^{N}, and when U is N, L¹ is absent, and when U is CH, L¹ is NR^{N};
R^{N} is hydrogen or C₁₋₃ alkyl;
R¹ is 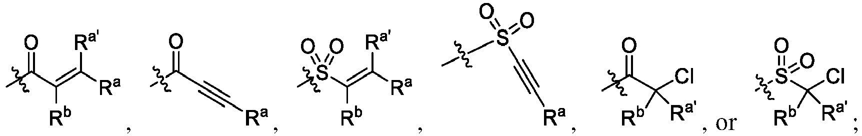
wherein,
R^{a} is hydrogen, halogen, optionally substituted C₁₋₃ alkyl, -N(R⁵)₂, optionally substituted 4-6 membered saturated heterocyclyl, C₁₋₃ alkoxy, C₁₋₃ alkylthio or acetyl, wherein the substituents for optionally substituted described in R^{a} is selected from the group consisting of: methyl, ethyl, -N(R⁵)₂, halogen, C₁₋₃ alkoxy, and 4-6 membered saturated heterocyclyl;
each R⁵ is independently hydrogen or C₁₋₃ alkyl;
R^{a'} and R^{b} are independently hydrogen, halogen or C₁₋₃ alkyl;
L² is absent, -O-, -S-, -NR⁶-, -SO-, -SO₂- or -CO-, wherein, R⁶ is hydrogen or C₁₋₄ alkyl;
R² is hydrogen, optionally substituted C₁₋₄ alkyl, optionally substituted saturated or unsaturated 3-8 membered carbocyclyl, optionally substituted saturated or unsaturated 4-12 membered heterocyclyl, optionally substituted saturated or unsaturated 3-8 membered carbocyclyl fused 6-10 membered aryl, optionally substituted saturated or unsaturated 3-8 membered carbocyclyl fused 5-10 membered heteroaryl, optionally substituted saturated or unsaturated 3-8 membered heterocyclyl fused 6-10 membered aryl, optionally substituted saturated or unsaturated 3-8 membered heterocyclyl fused 5-10 membered heteroaryl, optionally substituted 6-10 membered aryl or optionally substituted 5-10 membered heteroaryl; wherein the substituents for optionally substituted described in R² is selected from: deuterium, halogen, hydroxy, cyano, oxo, C₁₋₃ alkoxy, -NR^{c}R^{d}, -CO₂R⁷, -CONR^{e}R^{f}, C₁₋₄ alkyl sulfoxide, C1-C4 alkyl sulfonyl, -SO₂NR^{g}R^{h}, optionally substituted 3-8 membered saturated or unsaturated carbocyclyl, and optionally substituted 4-8 membered saturated and unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl;
W is -(CR⁸R⁹)-, -NR¹⁰-, O or S, wherein R⁸, R⁹ and R¹⁰ are independently hydrogen or C₁₋₃ alkyl;
X is -(CR¹¹R¹²) ₘ- or -(C=O)-, wherein each R¹¹ is independently hydrogen or C₁₋₃ alkyl, and each R¹² is independently hydrogen or C₁₋₃ alkyl;
m=1, 2 or 3;
B is a 6-10 membered aryl or a 5-8 membered heteroaryl, wherein the 6-10 membered aryl or the 5-8 membered heteroaryl is optionally substituted by one or more R¹³;
each R¹³ is independently halogen, hydroxy, amino, C1-C3 alkylamino, (C1-C3 alkyl)₂amino, C₁₋₃ alkyl, C₁₋₆ haloalkyl, C₃₋₆ carbocyclyl, C₁₋₃ alkoxy, or C₁₋₃ haloalkoxy;
Y is -NR¹⁴-, wherein R¹⁴ is hydrogen, C₁₋₆ alkyl, C₁₋₆ haloalkyl or C₃₋₆ carbocyclyl;
Z is -(CR¹⁵R¹⁶)-, wherein R¹⁵ and R¹⁶ are independently hydrogen or C₁₋₃ alkyl.

2. The compound of Formula I according to claim 1, or pharmaceutically acceptable salts thereof, enantiomers, diastereomers, tautomers, cis-trans isomers, solvates, polymorphs, deuterated compounds, or combinations thereof, wherein
R² is hydrogen, optionally substituted C₁₋₄ alkyl, optionally substituted saturated or unsaturated 3-8 membered carbocyclyl, optionally substituted saturated or unsaturated 4-12 membered heterocyclyl, optionally substituted saturated or unsaturated 3-8 membered heterocyclyl fused 6-10 membered aryl, optionally substituted saturated or unsaturated 3-8 membered heterocyclyl fused 5-10 membered heteroaryl, optionally substituted 6-10 membered aryl or optionally substituted 5-10 membered heteroaryl, wherein the substituents for optionally substituted described in the R² are selected from: deuterium, halogen, hydroxyl, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl and optionally substituted 4-8 membered saturated or unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
each R^{g} and R^{h} are independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl;
preferably, L² is absent, -O-, -S-, or -NR⁶-, wherein R⁶ is hydrogen or C₁₋₄ alkyl;
preferably, R^{a} is hydrogen, fluorine, optionally substituted C₁₋₃ alkyl, optionally substituted 4-6 membered saturated heterocyclyl or acetyl;
wherein the substituents for optionally substituted described in R^{a} are selected from the group consisting of methyl, ethyl, -N(R⁵)₂, halogen, C₁₋₃ alkoxy and 4-6 membered saturated heterocyclyl;
each R⁵ is independently hydrogen or C₁₋₃ alkyl;
preferably, R^{a'} and R^{b} are independently hydrogen, fluorine or C₁₋₃ alkyl;
preferably, W is -(CR⁸R⁹)- or -NR¹⁰-, wherein R⁸, R⁹ and R¹⁰ are independently hydrogen or C₁₋₃ alkyl;
preferably, X is -(CR¹¹R¹²) ₘ-; wherein each R¹¹ is independently hydrogen or C₁₋₃ alkyl, and each R¹² is independently hydrogen or C₁₋₃ alkyl; m is 1, 2 or 3; further preferably, m is 1 or 2.

3. The compound of Formula I according to claim 1 or 2, or pharmaceutically acceptable salts thereof, enantiomers, diastereomers, tautomers, cis-trans isomers, solvates, polymorphs, deuterated compounds, or combinations thereof, wherein the moiety represented by 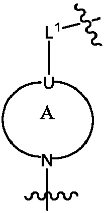 is selected from the group consisting of: 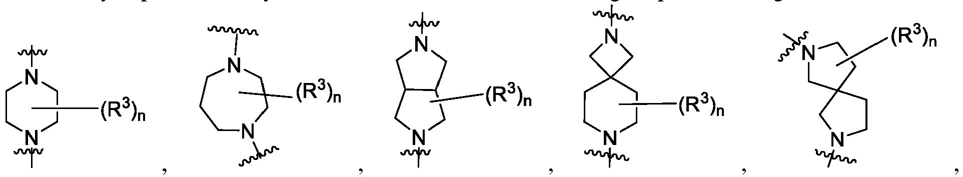 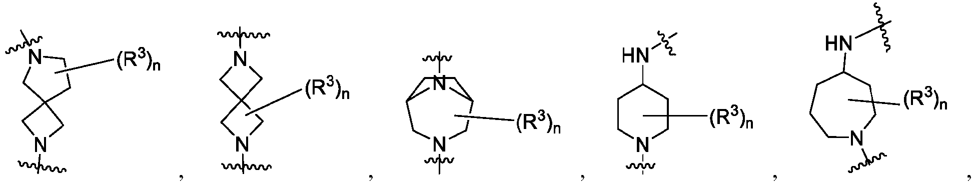 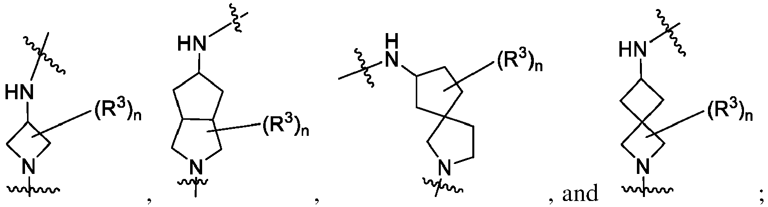
n represents the number of R³, and n is 0, 1, 2 or 3;
each R³ is independently oxo, C₁₋₃ alkyl, C₂₋₄ alkenyl, C₂₋₄ alkynyl, cyano, -C(O)OR⁴, -CON(R⁴)₂, or -N(R⁴)₂, wherein the C₁₋₃ alkyl may be optionally substituted by cyano, halogen, -OR⁴, or -N(R⁴)₂;
each R⁴ is independently hydrogen or C1-C3 alkyl;
preferably, the moiety represented by 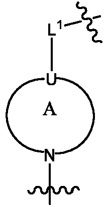 is selected from the group consisting of:

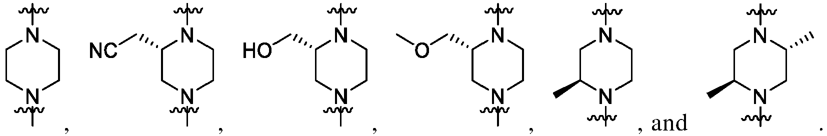

4. The compound of Formula I according to claim 1, or pharmaceutically acceptable salts thereof, enantiomers, diastereomers, tautomers, cis-trans isomers, solvates, polymorphs, deuterated compounds, or combinations thereof, wherein
R¹ is 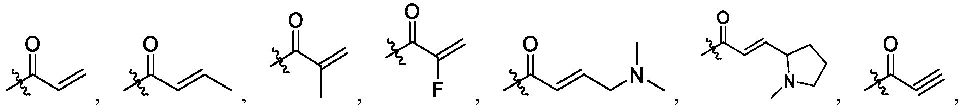 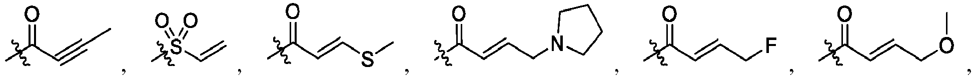 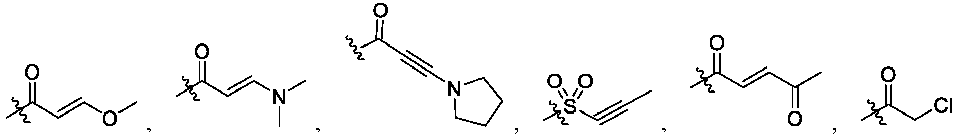 or 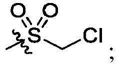
preferably, R¹ is 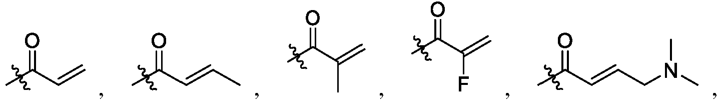 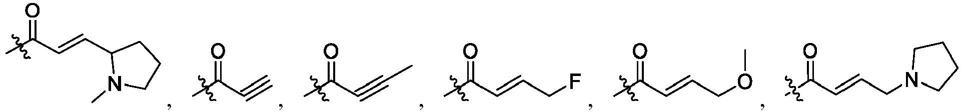 or

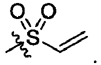

5. The compound of Formula I according to claim 1, or pharmaceutically acceptable salts thereof, enantiomers, diastereomers, tautomers, cis-trans isomers, solvates, polymorphs, deuterated compounds, or combinations thereof, wherein
R² is hydrogen, optionally substituted C₁₋₄ alkyl, optionally substituted 6-10 membered aryl or optionally substituted 5-10 membered heteroaryl;
wherein the substituents for optionally substituted described in R² are selected from the group consisting of: halogen, hydroxy, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl, and optionally substituted 4-8 membered saturated or unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl;
preferably, R² is 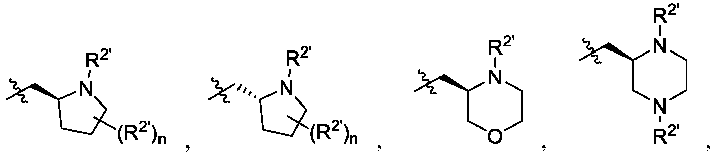 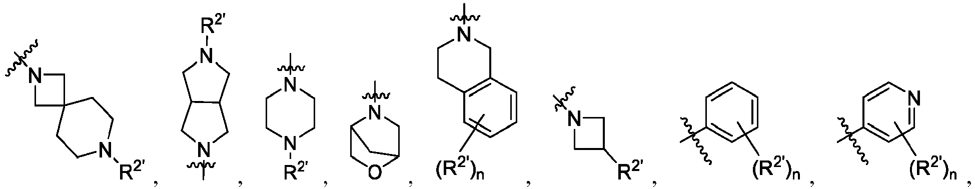 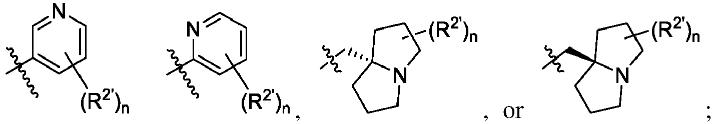 wherein n represents the number of R²', and selected from: 0, 1, 2 and 3; each R² is independently halogen, C₁₋₃ alkyl, C₁₋₃ haloalkyl, hydroxy, cyano, oxo, C₁₋₃ alkoxy, -NR^{c}R^{d}, -CO₂R⁷, -CONR^{e}R^{f}, C₁₋₄ alkyl sulfoxide, C₁₋₄ alkyl sulfonyl, -SO₂NR^{g}R^{h}, optionally substituted 3-8 membered saturated or unsaturated carbocyclyl or optionally substituted 4-8 membered saturated or unsaturated heterocyclyl;
R^{c} and R^{d} are each independently selected from hydrogen, optionally substituted C₁₋₆ alkyl, optionally substituted 3-8 membered carbocyclyl and optionally substituted 4-8 membered heterocyclyl, or R^{c} and R^{d} together with N to which they are connected form an optionally substituted 4-8 membered heterocycle;
R^{e} and R^{f} are each independently selected from hydrogen, optionally substituted C₁₋₆ alkyl, optionally substituted 3-8 membered carbocyclyl and optionally substituted 4-8 membered heterocyclyl, or R^{e} and R^{f} together with N to which they are connected form an optionally substituted 4-8 membered heterocycle;
R^{g} and R^{h} are each independently selected from hydrogen, optionally substituted C₁₋₆ alkyl, optionally substituted 3-8 membered carbocyclyl and optionally substituted 4-8 membered heterocyclyl, or R^{g} and R^{h} together with N to which they are connected form an optionally substituted 4-8 membered heterocycle;
R⁷ is hydrogen or optionally substituted C₁₋₄ alkyl.

6. The compound according to any of claims 1-5, or pharmaceutically acceptable salts thereof, enantiomers, diastereomers, tautomers, cis-trans isomers, solvates, polymorphs, deuterated compounds, or combinations thereof, wherein
the spiro ring represented by 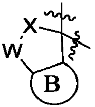 is selected from the group consisting of: 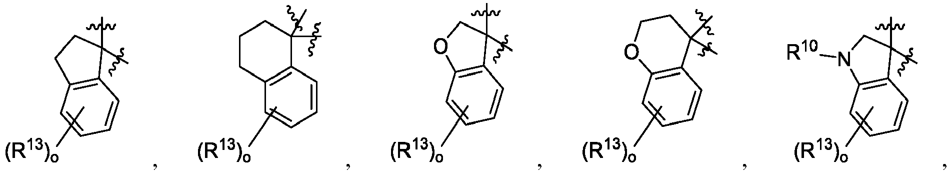 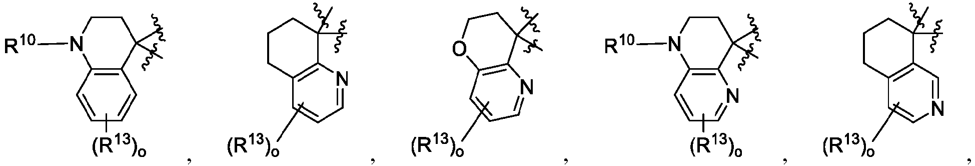 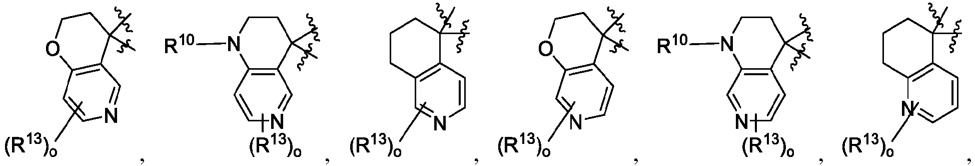 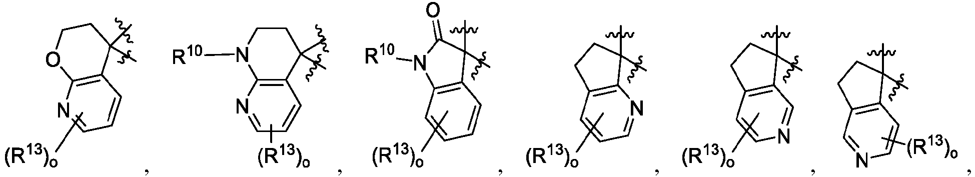 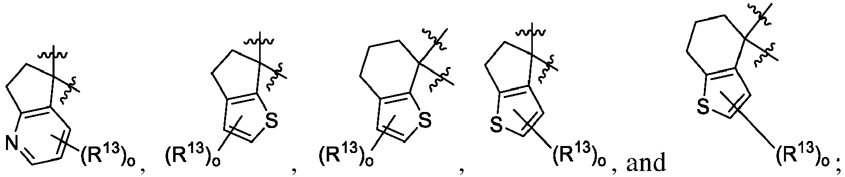
R¹⁰ is independently hydrogen or C₁₋₃ alkyl;
o represents the number of R¹³, which is 0, 1, 2 or 3;
each R¹³ is independently selected from halogen, C₁₋₆ alkyl, C₁₋₆ haloalkyl, C₃₋₆ carbocyclyl, C₁₋₃ alkoxy, and C₁₋₃ haloalkoxy.

7. The compound according to claim 1, or pharmaceutically acceptable salts thereof, enantiomers, diastereomers, tautomers, cis-trans isomers, solvates, polymorphs, deuterated compounds, or combinations thereof, wherein, the compound is selected from the group consisting of:

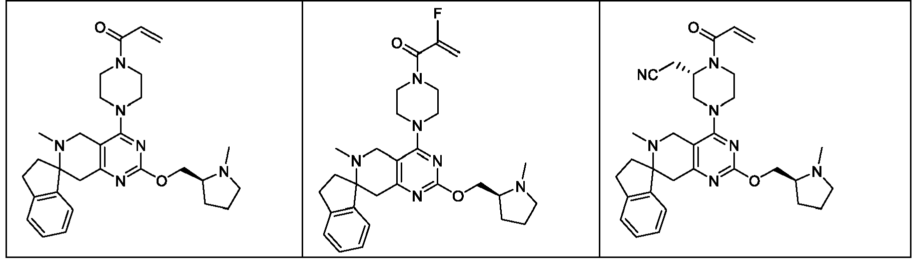

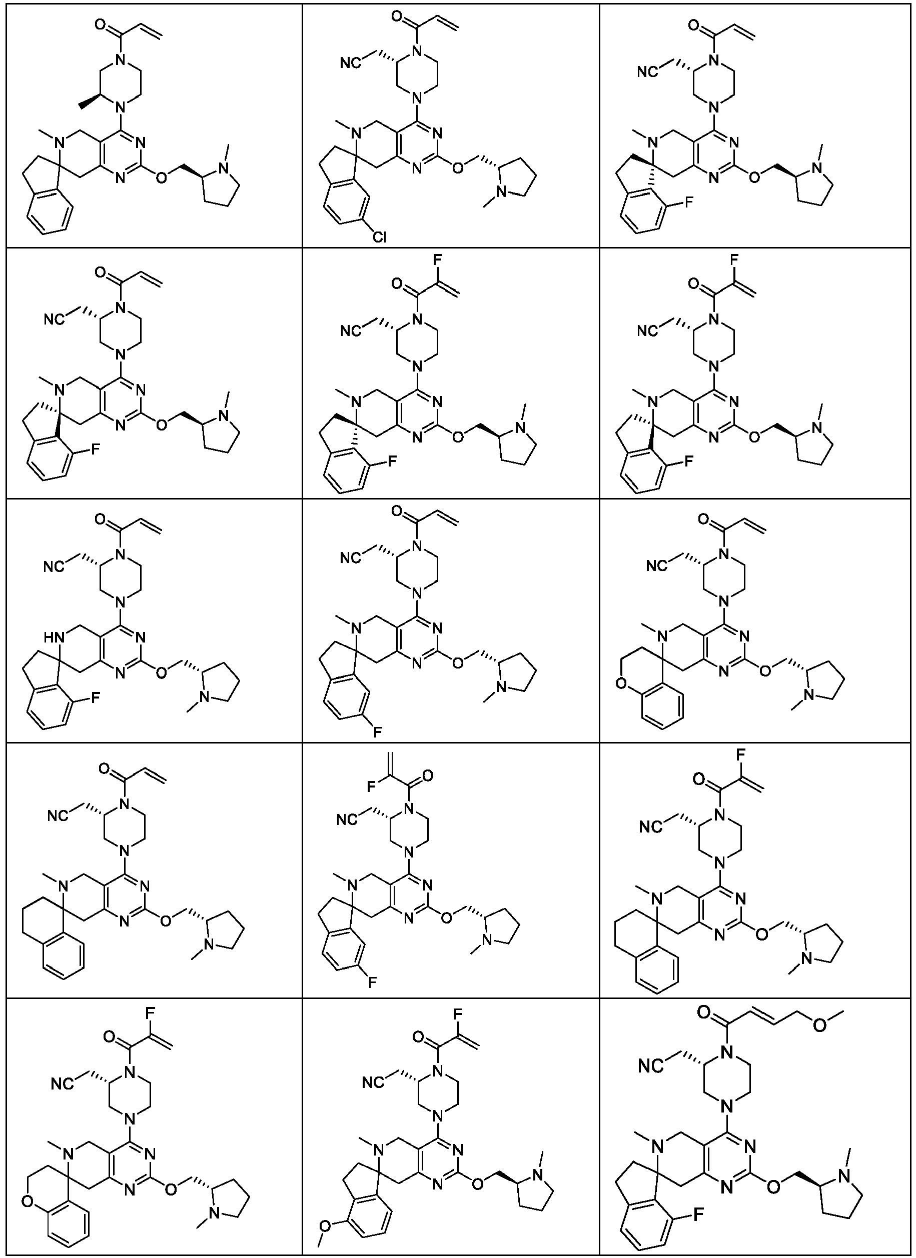

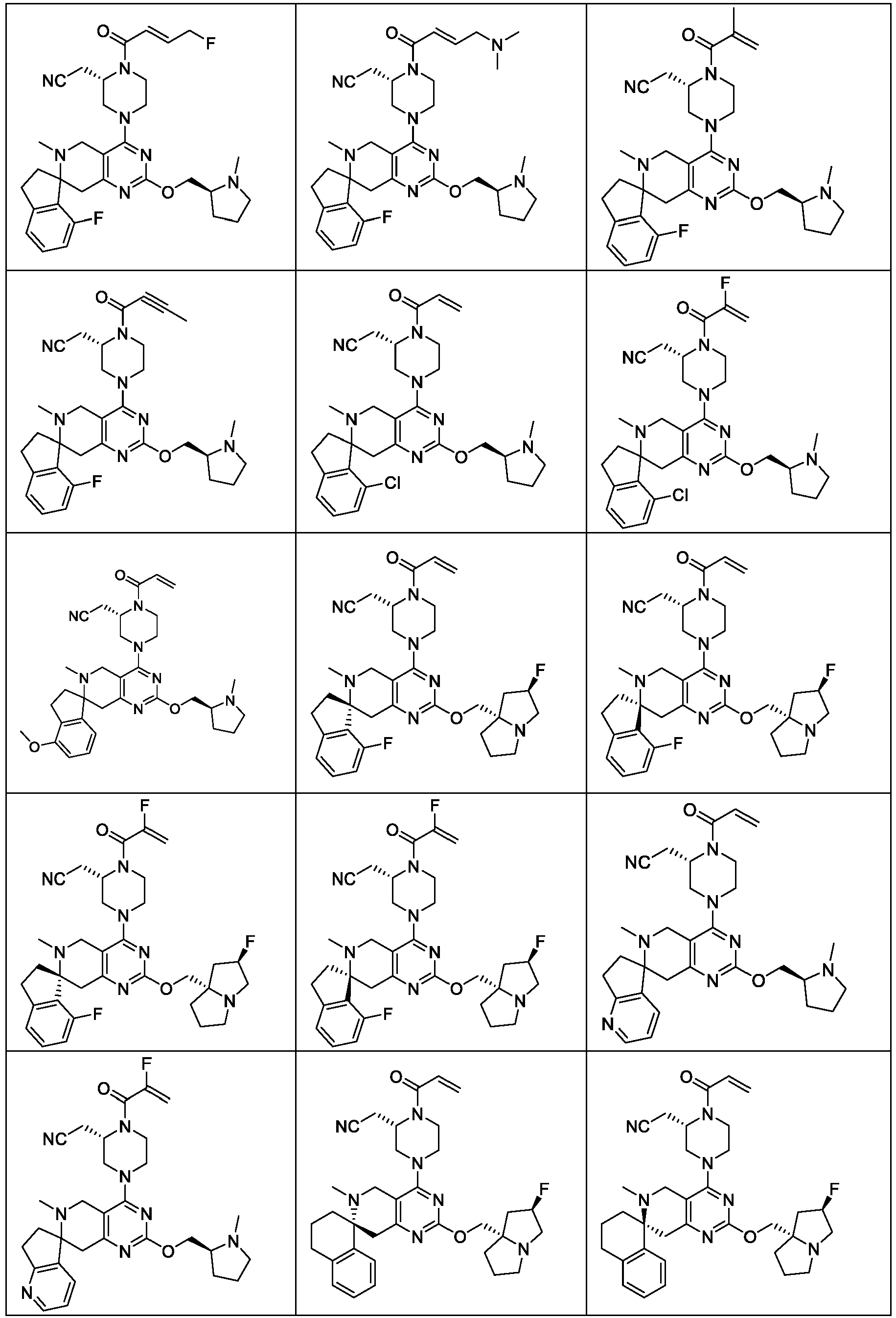

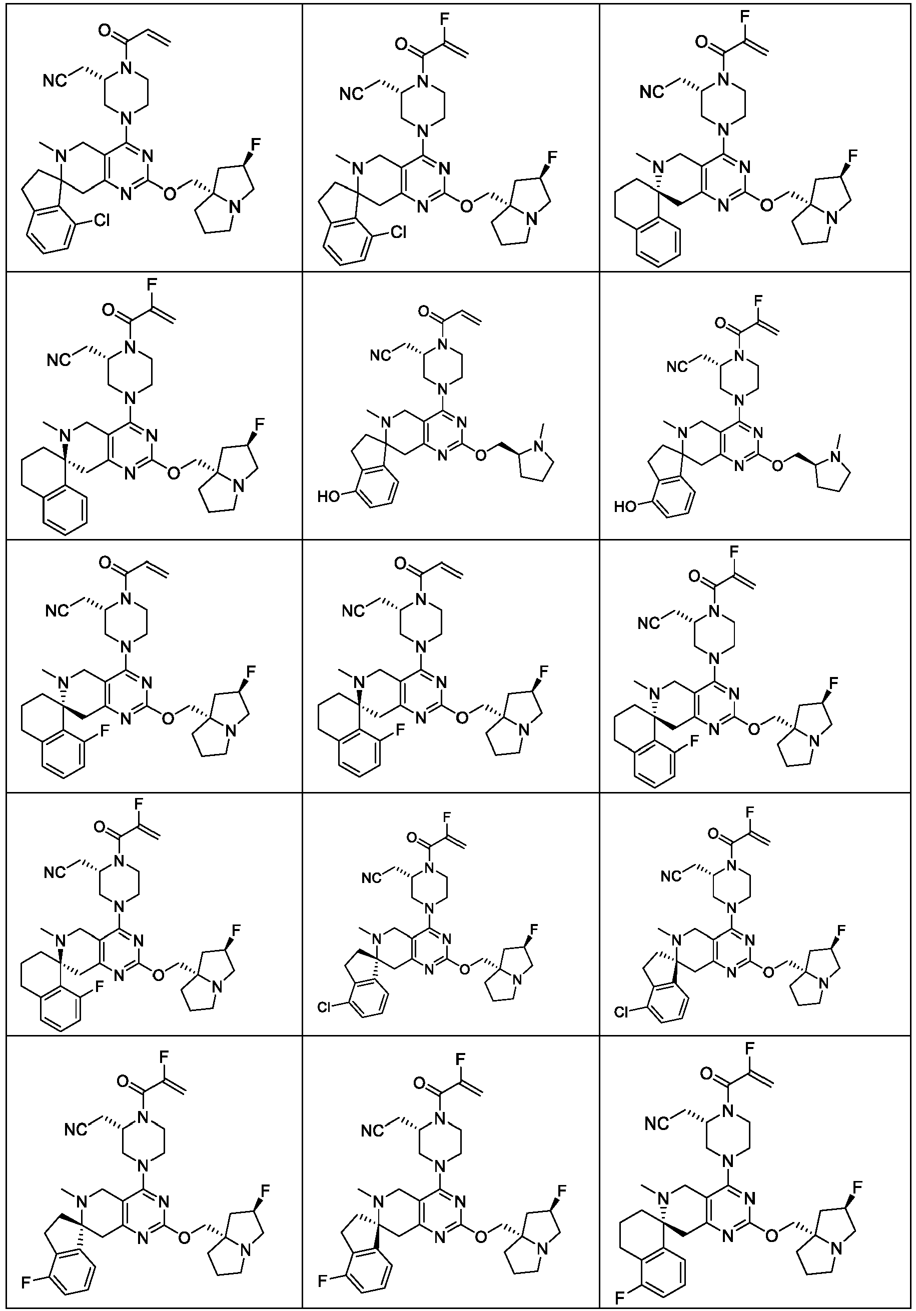

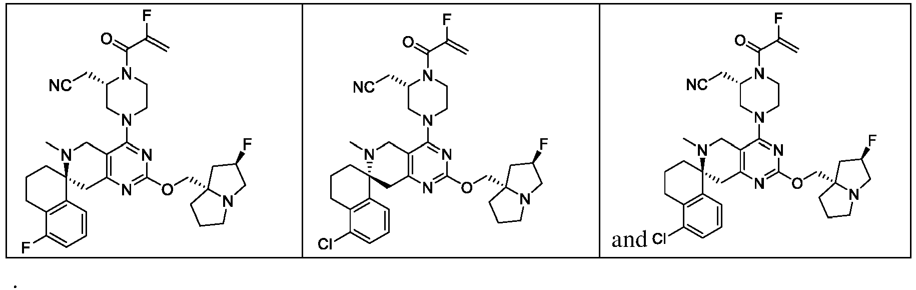

8. A pharmaceutical composition, comprising:
(1) a therapeutically effective amount of one or more of the compounds according to any of claims 1-7, or pharmaceutically acceptable salts thereof, enantiomers, diastereomers, tautomers, cis-trans isomers, solvates, polymorphs, and deuterated compounds thereof as active ingredients; and
(2) optionally, pharmaceutically acceptable excipients.

9. A use of the compound according to any one of claims 1 to 7, or pharmaceutically acceptable salts thereof, enantiomers, diastereomers, tautomers, cis-trans isomers, solvates, polymorphs or deuterated compounds thereof, or the pharmaceutical composition according to claim 8 in the preparation of medicaments for preventing or treating cancer mediated by KRAS G12C mutation.

10. The use according to claim 9, wherein, the cancer is selected from the group consisting of lung cancer, pancreatic cancer, colorectal cancer, leukemia, Ewing's sarcoma, breast cancer, prostate cancer, T-cell lymphoma, B-cell lymphoma, malignant rhabdomyoma, synovial sarcoma, endometrioma, gastric cancer, liver cancer, renal cancer, melanoma, ovarian cancer, brain glioma, cholangiocarcinoma, nasopharyngeal carcinoma, cervical cancer, head and neck cancer, esophageal cancer, thyroid cancer and bladder cancer, in particular, selected from non-small cell lung cancer, pancreatic cancer, and colorectal cancer.
